# EUROPEAN PATENT APPLICATION

(11) **EP 4 786 483 A1**
(43) Date of publication of application: **05.08.2026**
(21) Application number: 24870765.5
(22) Date of filing: 25.09.2024
(51) Int. Cl.: C07K 5/02, C07K 7/02, A61K 47/68, A61P 35/00

(54) **AURISTATIN DERIVATIVE AND ANTIBODY-DRUG CONJUGATE THEREOF**

(30) Priority: 26.09.2023 CN 202311256590; 25.01.2024 CN 202410109076; 20.06.2024 CN 202410806735; 15.08.2024 CN 202411124033; 14.09.2024 CN 202411296153
(71) Applicant: Shanghai Qilu Pharmaceutical Research and Development Centre Ltd., Shanghai 201203 (CN)
(72) Inventor: WANG, Junfei, Shanghai 201203 (CN); HUANG, Ying, Shanghai 201203 (CN); BI, Xiaoyang, Shanghai 201203 (CN); CUI, Changyi, Shanghai 201203 (CN); QIN, Wenwu, Shanghai 201203 (CN); HAN, Fengying, Shanghai 201203 (CN); LI, Mengjia, Shanghai 201203 (CN); HUANG, Wenshu, Shanghai 201203 (CN); MA, Cuicui, Shanghai 201203 (CN); MA, Lina, Shanghai 201203 (CN); ZHANG, Meijuan, Shanghai 201203 (CN); CHEN, Dong, Shanghai 201203 (CN); WANG, Xinmei, Shanghai 201203 (CN); AI, Xianwei, Shanghai 201203 (CN); YANG, Yang, Shanghai 201203 (CN); SUN, Daqing, Shanghai 201203 (CN); TAO, Weikang, Shanghai 201203 (CN)
(74) Representative: Studio Torta S.p.A.
(86) International application number: PCT/CN2024/121050
(87) International publication number: WO 2025/067221

(57) **Abstract**

The present disclosure relates to a cytotoxic compound and an antibody-drug conjugate thereof, and relates in particular to an auristatin derivative, a preparation method for an antibody-drug conjugate thereof, and use.

## Description

The present disclosure claims priority to the Chinese Patent Application No. 202311256590.X entitled "AURISTATIN DERIVATIVE AND ANTIBODY-DRUG CONJUGATE THEREOF" and filed with China National Intellectual Property Administration on September 26, 2023, the Chinese Patent Application No. 202410109076.1 entitled "AURISTATIN DERIVATIVE AND ANTIBODY-DRUG CONJUGATE THEREOF" and filed with China National Intellectual Property Administration on January 25, 2024, the Chinese Patent Application No. 202410806735.7 entitled "AURISTATIN DERIVATIVE AND ANTIBODY-DRUG CONJUGATE THEREOF" and filed with China National Intellectual Property Administration on June 20, 2024, the Chinese Patent Application No. 202411124033.7 entitled "AURISTATIN DERIVATIVE AND ANTIBODY-DRUG CONJUGATE THEREOF" and filed with China National Intellectual Property Administration on August 15, 2024, and the Chinese Patent Application No. 202411296153.5 entitled "AURISTATIN DERIVATIVE AND ANTIBODY-DRUG CONJUGATE THEREOF" and filed with China National Intellectual Property Administration on September 14, 2024, which are incorporated herein by reference in their entirety.

### TECHNICAL FIELD

The present disclosure belongs to the field of pharmaceuticals, and particularly relates to an auristatin derivative and a preparation method therefor and use of an antibody-drug conjugate thereof.

### BACKGROUND

An antibody-drug conjugate (ADC) is a type of targeted biologic agent that conjugates a target-specific monoclonal antibody (Ab) to a cytotoxic drug (payload) with a high-killing effect via a specific linker. It uses the monoclonal antibody as a carrier to efficiently transport the small-molecule cytotoxic drug to target tumor cells in a targeted manner. ADC drugs combine the advantages of highly targeted and selective antibodies with highly potent anti-tumor cytotoxic drugs. While retaining the tumor-killing properties of small-molecule cytotoxic drugs, they selectively reduce the off-target toxic and side effects of the small-molecule cytotoxic drugs, effectively improving the benefit-to-risk ratio of anti-tumor therapy.

Auristatin derivatives are a class of depsipeptide derivatives isolated from the *Dolabella auricularia.* They primarily inhibit tumor growth by acting on tubulin, and their activity is about 1000 times greater than that of traditional chemotherapeutic drugs. MMAE (monomethyl auristatin E) and MMAF (monomethyl auristatin F) are the two most commonly used auristatin compounds in ADC research and development.

Despite significant progress made in the clinical development of ADCs in recent years, the design and development of ADCs still face many challenges and involve numerous clinical adverse reactions. For example, the most common adverse reactions for Trastuzumab Deruxtecan (trade name Enhertu) include upper respiratory tract infections, anemia, neutropenia, thrombocytopenia, leukopenia, interstitial lung disease, and the like. Brentuximab Vedotin (trade name Adcetris) presents adverse reactions such as hematologic abnormalities and gastrointestinal diseases. Therefore, there remains a need to develop safer and more effective cytotoxic drugs and antibody-drug conjugates thereof.

### SUMMARY

The present disclosure provides a cytotoxic drug represented by formula (I) or a stereoisomer, isotopic derivative, solvate, nitrogen oxide, prodrug, or pharmaceutically acceptable salt thereof: 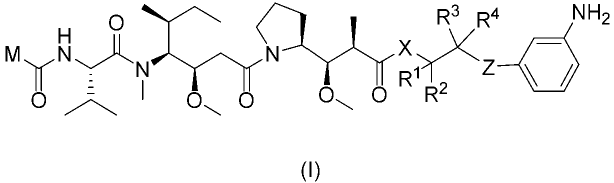
wherein M is selected from C₁₋₆ alkyl, C₃₋₆ cycloalkyl, and 3- to 8-membered heterocyclyl; the C₁₋₆ alkyl, C₃₋₆ cycloalkyl, or 3- to 8-membered heterocyclyl is optionally further substituted with a substituent selected from C₁₋₄ alkyl and NR^{m1}R^{m2}, and the 3- to 8-membered heterocyclyl contains 1-3 heteroatoms selected from N, O, and S;
R^{m1} and R^{m2} are each independently selected from H and C₁₋₄ alkyl;
X is selected from O and -N(R^{x1})-;
R^{x1} is selected from H, OH, C₁₋₄ alkyl, C₁₋₄ alkoxy, C₂₋₄ alkenyl, C₂₋₄ alkynyl, and C₃₋₆ cycloalkyl; the C₁₋₄ alkyl or C₃₋₆ cycloalkyl is optionally further substituted with 1-3 substituents selected from halogen, CN, deuterium, and C₃₋₆ cycloalkyl;
R³ and R⁴ are each independently selected from H and OH;
or, X and R³, together with the atoms to which they are attached, form a 4- to 8-membered heterocyclic ring, and the 4- to 8-membered heterocyclic ring contains 1-3 heteroatoms or groups selected from N, O, S, and S(O)₂;
or, X and R⁴, together with the atoms to which they are attached, form a 4- to 8-membered heterocyclic ring, and the 4- to 8-membered heterocyclic ring contains 1-3 heteroatoms or groups selected from N, O, S, and S(O)₂;
R¹ and R² are each independently selected from H, OH, C₁₋₄ alkyl, C₂₋₄ alkynyl, C₂₋₄ alkenyl, C₃₋₆ cycloalkyl, 3- to 8-membered heterocyclyl, -COOR^{1a}, and -CONR^{1b}R^{1c}; the C₁₋₄ alkyl, C₂₋₄ alkenyl, C₃₋₆ cycloalkyl, or 3- to 8-membered heterocyclyl is optionally further substituted with 1-3 substituents selected from CN, OH, N₃, halogen, -COOR^{1a}, -CONR^{1b}R^{1c}, and C₁₋₄ alkyl, and the 3- to 8-membered heterocyclyl contains 1-3 heteroatoms selected from N, O, and S;
R^{1a}, R^{1b}, and R^{1c} are each independently selected from H and C₁₋₄ alkyl;
or, R¹ and R², together with the carbon atom to which they are attached, form C₃₋₆ cycloalkyl or 3- to 8-membered heterocyclyl;
Z is selected from a chemical bond and -(CH₂)ₙ-, and n is selected from 1 and 2.

In some embodiments, M is selected from C₁₋₆ alkyl, C₃₋₆ cycloalkyl, and 3- to 8-membered heterocyclyl; the C₁₋₆ alkyl, C₃₋₆ cycloalkyl, or 3- to 8-membered heterocyclyl is optionally further substituted with a substituent selected from C₁₋₄ alkyl and NR^{m1}R^{m2}, and the 3- to 8-membered heterocyclyl contains 1-3 heteroatoms selected from N, O, and S;
R^{m1} and R^{m2} are each independently selected from H and C₁₋₄ alkyl;
X is selected from O and -N(R^{x1})-;
R^{x1} is selected from H, OH, C₁₋₄ alkyl, C₁₋₄ alkoxy, C₂₋₄ alkenyl, and C₂₋₄ alkynyl; the C₁₋₄ alkyl is optionally further substituted with 1-3 substituents selected from halogen and CN;
R³ and R⁴ are each independently selected from H and OH;
or, X and R³, together with the atoms to which they are attached, form a 4- to 8-membered heterocyclic ring, and the 4- to 8-membered heterocyclic ring contains 1-3 heteroatoms or groups selected from N, O, S, and S(O)₂;
or, X and R⁴, together with the atoms to which they are attached, form a 4- to 8-membered heterocyclic ring, and the 4- to 8-membered heterocyclic ring contains 1-3 heteroatoms or groups selected from N, O, S, and S(O)₂;
R¹ and R² are each independently selected from H, OH, C₁₋₄ alkyl, C₂₋₄ alkynyl, C₂₋₄ alkenyl, C₃₋₆ cycloalkyl, 3- to 8-membered heterocyclyl, -COOR^{1a}, and -CONR^{1b}R^{1c}; the C₁₋₄ alkyl, C₂₋₄ alkenyl, C₃₋₆ cycloalkyl, or 3- to 8-membered heterocyclyl is optionally further substituted with 1-3 substituents selected from CN, OH, N₃, halogen, -COOR^{1a}, and -CONR^{1b}R^{1c}, and the 3- to 8-membered heterocyclyl contains 1-3 heteroatoms selected from N, O, and S;
R^{1a}, R^{1b}, and R^{1c} are each independently selected from H and C₁₋₄ alkyl;
or, R¹ and R², together with the carbon atom to which they are attached, form C₃₋₆ cycloalkyl or 3- to 8-membered heterocyclyl;
Z is selected from a chemical bond and -(CH₂)ₙ-, and n is selected from 1 and 2.

In some embodiments, provided is the cytotoxic drug represented by formula (I) or the stereoisomer, isotopic derivative, solvate, nitrogen oxide, prodrug, or pharmaceutically acceptable salt thereof: 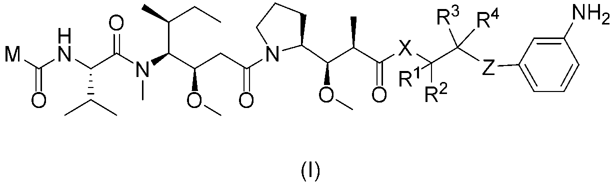
wherein M is selected from C₁₋₆ alkyl, C₃₋₆ cycloalkyl, and 3- to 8-membered heterocyclyl; the C₁₋₆ alkyl, C₃₋₆ cycloalkyl, or 3- to 8-membered heterocyclyl is optionally further substituted with a substituent selected from C₁₋₄ alkyl and NR^{m1}R^{m2}, and the 3- to 8-membered heterocyclyl contains 1-3 heteroatoms selected from N, O, and S;
R^{m1} and R^{m2} are each independently selected from H and C₁₋₄ alkyl;
X is selected from O and -N(R^{x1})-;
R^{x1} is selected from H, OH, C₁₋₄ alkyl, C₁₋₄ alkoxy, C₂₋₄ alkenyl, and C₂₋₄ alkynyl; the C₁₋₄ alkyl is optionally further substituted with 1-3 substituents selected from halogen and CN;
R¹ and R² are each independently selected from H, OH, C₁₋₄ alkyl, C₂₋₄ alkynyl, C₂₋₄ alkenyl, C₃₋₆ cycloalkyl, 3- to 8-membered heterocyclyl, -COOR^{1a}, and -CONR^{1b}R^{1c}; the C₁₋₄ alkyl, C₂₋₄ alkenyl, C₃₋₆ cycloalkyl, or 3- to 8-membered heterocyclyl is optionally further substituted with 1-3 substituents selected from CN, OH, N₃, halogen, -COOR^{1a}, and -CONR^{1b}R^{1c}, and the 3- to 8-membered heterocyclyl contains 1-3 heteroatoms selected from N, O, and S;
R^{1a}, R^{1b}, and R^{1c} are each independently selected from H and C₁₋₄ alkyl;
or, R¹ and R², together with the carbon atom to which they are attached, form C₃₋₆ cycloalkyl or 3- to 8-membered heterocyclyl;
R³ and R⁴ are each independently selected from H and OH;
Z is selected from a chemical bond and -(CH₂)ₙ-, and n is selected from 1 and 2.

In some embodiments, provided is the cytotoxic drug represented by formula (I) or the stereoisomer, isotopic derivative, solvate, nitrogen oxide, prodrug, or pharmaceutically acceptable salt thereof, wherein M is selected from C₁₋₄ alkyl, C₃₋₆ cycloalkyl, and 3- to 6-membered heterocyclyl; the C₁₋₄ alkyl, C₃₋₆ cycloalkyl, or 3- to 6-membered heterocyclyl is optionally further substituted with a substituent selected from C₁₋₄ alkyl and NR^{m1}R^{m2}, and the 3- to 6-membered heterocyclyl contains 1-3 heteroatoms selected from N, O, and S; R^{m1} and R^{m2} are each independently selected from H and C₁₋₂ alkyl.

In some embodiments, M is selected from 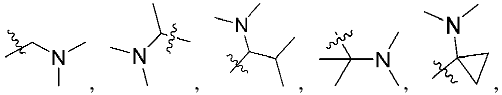 and

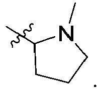

In some embodiments, R^{x1} is selected from H, -CH₃, OH, -OCH₃,

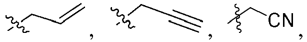

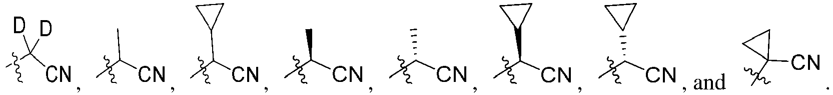

In some embodiments, X is selected from O, -NH-, -N(CH₃)-,

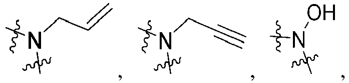

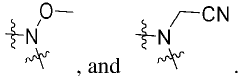

In some embodiments, X is selected from

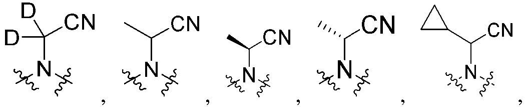

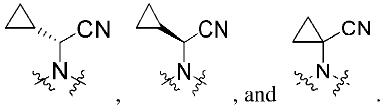

In some embodiments, R¹ and R² are each independently selected from H, -CH₃, -CH₂OH, -CH₂F, - CHF₂, -CF₃, -CH₂Cl, 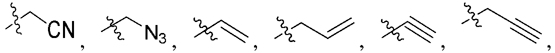 -COOH, -COOCH₃, - CONH₂, -CONHCH₃, 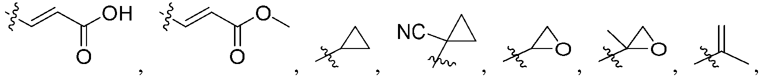 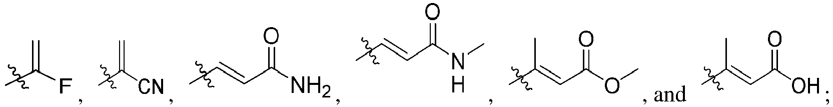 or, R¹ and R², together with the carbon atom to which they are attached, form 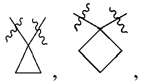 or

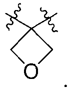

In some embodiments, R³ and R⁴ are each independently selected from H and OH, and preferably, R³ and R⁴ are each independently H.

In some embodiments, X and R³, together with the atoms to which they are attached, form a 5- to 6-membered heterocyclic ring, and the 5- to 6-membered heterocyclic ring contains 1-3 heteroatoms or groups selected from N, O, S, and S(O)₂;
or, X and R⁴, together with the atoms to which they are attached, form a 5- to 6-membered heterocyclic ring, and the 5- to 6-membered heterocyclic ring contains 1-3 heteroatoms or groups selected from N, O, S, and S(O)₂.

In some embodiments, X and R³, together with the atoms to which they are attached, form 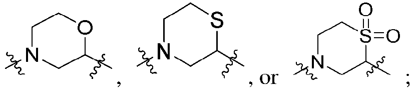 or, X and R⁴, together with the atoms to which they are attached, form 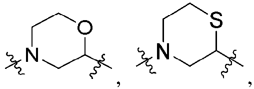 or

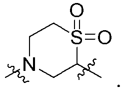

In some embodiments, Z is selected from a chemical bond and -CH₂-.

In some embodiments, provided is the cytotoxic drug represented by formula (I) or the stereoisomer, isotopic derivative, solvate, nitrogen oxide, prodrug, or pharmaceutically acceptable salt thereof, which is selected from a compound of general formula (Ia), (Ib), or (Ic): 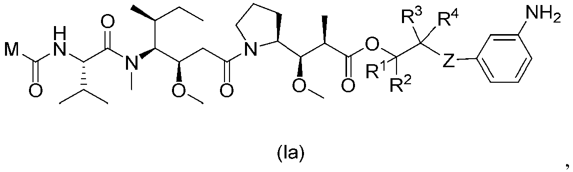 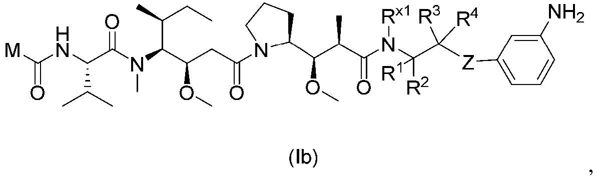 or 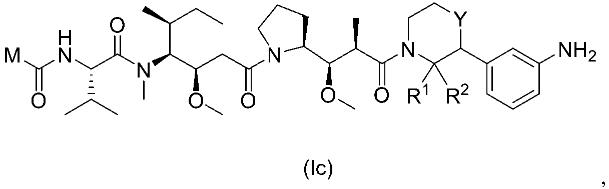 wherein Y is selected from O, S, and S(O)₂, and M, R¹, R², R³, R⁴, R^{x1}, and Z are as defined in formula (I).

In some embodiments, provided is the cytotoxic drug represented by formula (I) or the stereoisomer, isotopic derivative, solvate, nitrogen oxide, prodrug, or pharmaceutically acceptable salt thereof, which is selected from a compound of general formula (IIa), (IIb), or (IIc): 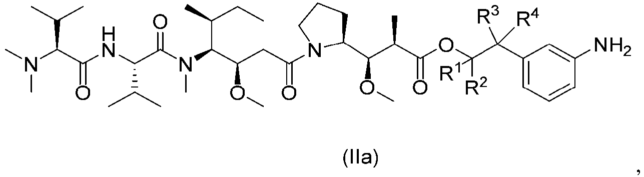 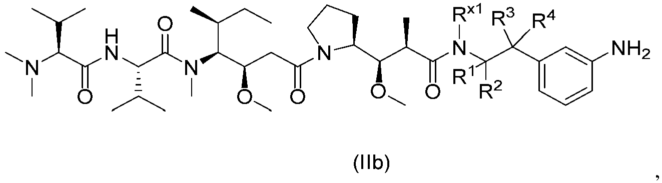 or 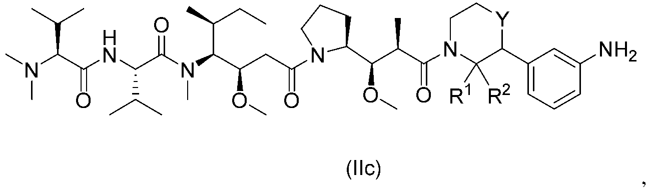 wherein Y is selected from O, S, and S(O)₂, and R¹, R², R³, R⁴, and R^{x1} are as defined in formula (I).

In some embodiments, provided is the cytotoxic drug represented by formula (I) or the stereoisomer, isotopic derivative, solvate, nitrogen oxide, prodrug, or pharmaceutically acceptable salt thereof, which is selected from a compound of general formula (IIIa): 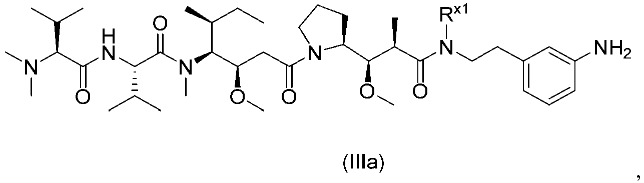 wherein R^{x1} is selected from OH, C₁₋₄ alkyl, C₁₋₄ alkoxy, and C₃₋₆ cycloalkyl, and the C₁₋₄ alkyl or C₃₋₆ cycloalkyl is further substituted with 1-3 substituents selected from deuterium, methyl, CN, and cyclopropyl; preferably, R^{x1} is selected from -OCH₃,

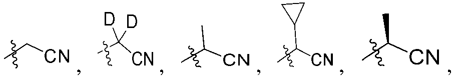

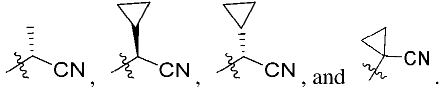

In some embodiments, the cytotoxic drug or the stereoisomer, isotopic derivative, solvate, nitrogen oxide, prodrug, or pharmaceutically acceptable salt thereof is selected from: 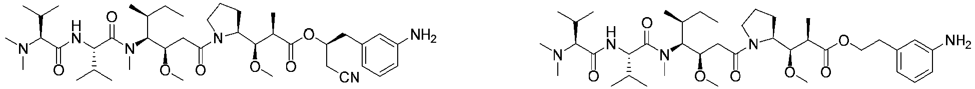 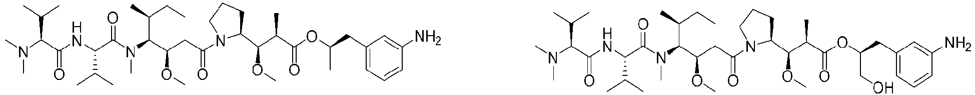 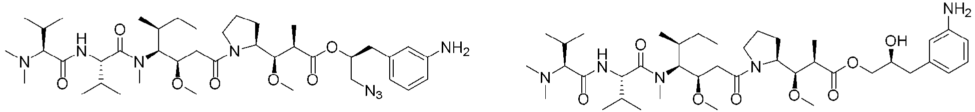 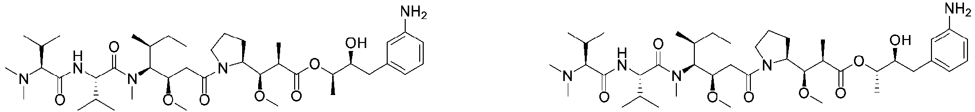 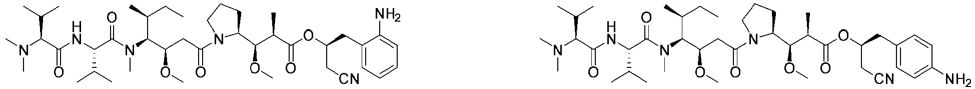 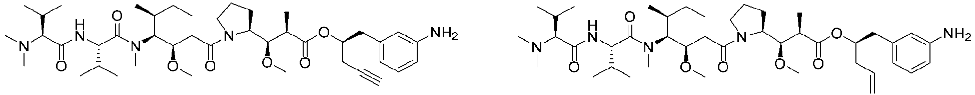 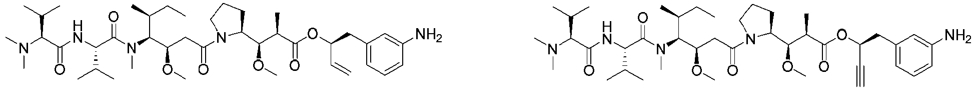 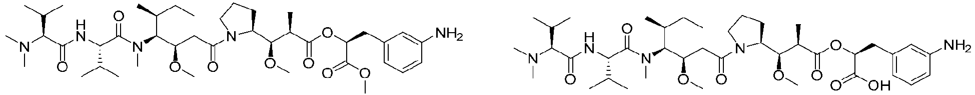 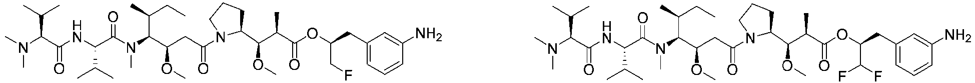 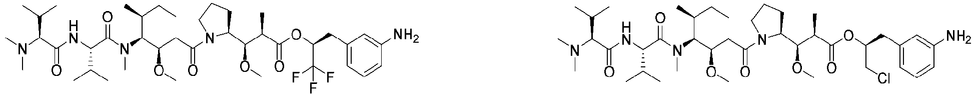 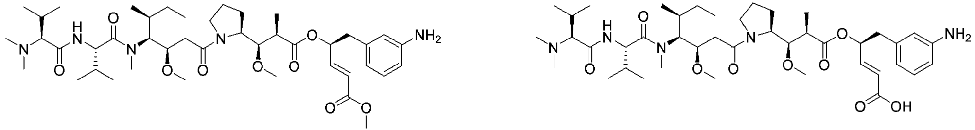 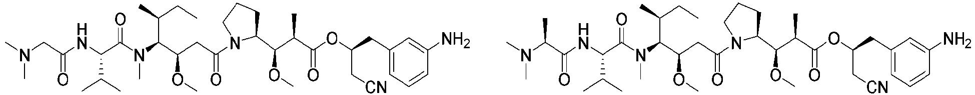 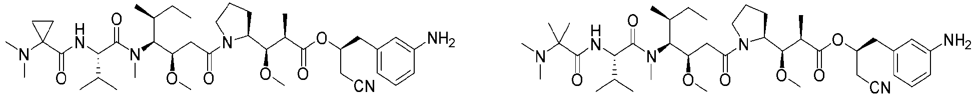 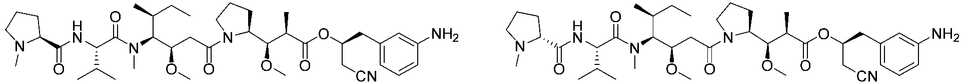 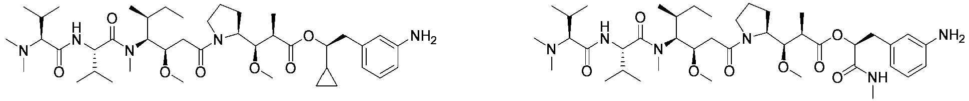 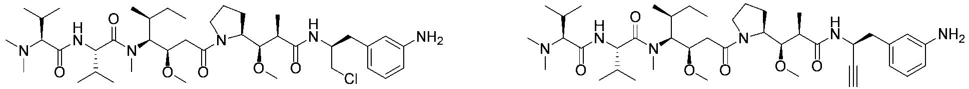 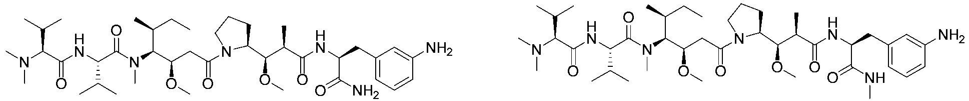 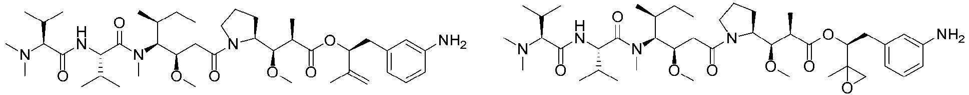 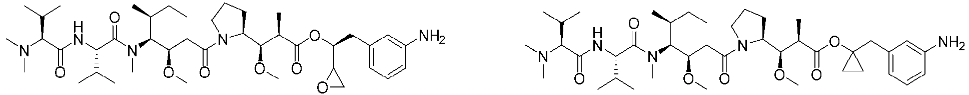 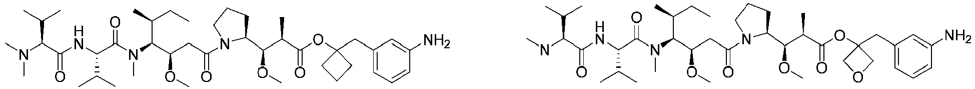 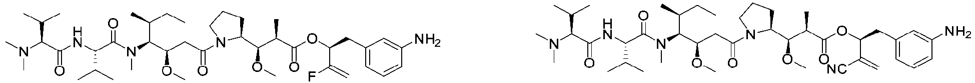 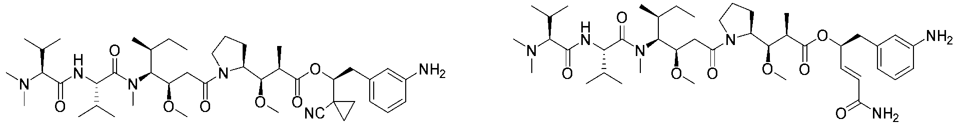 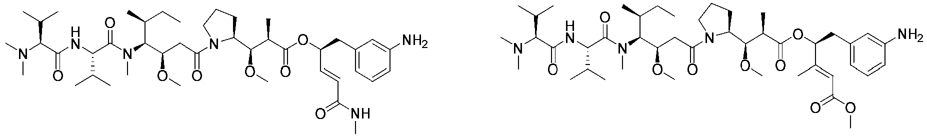 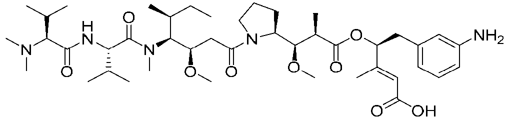 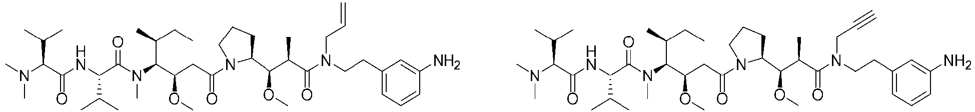 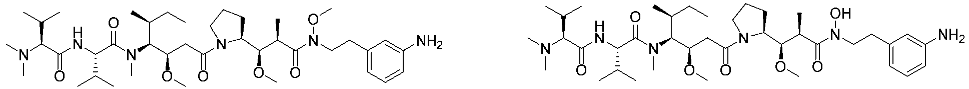 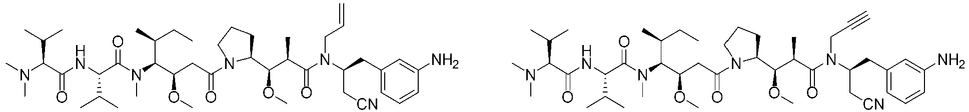 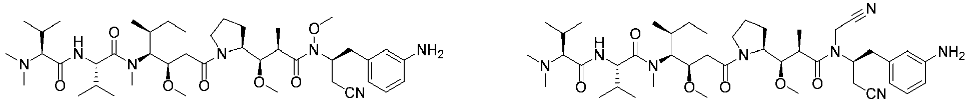 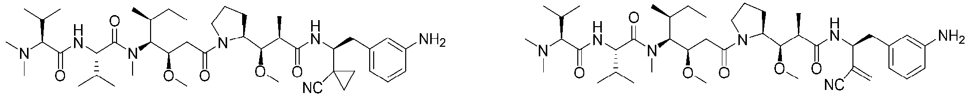 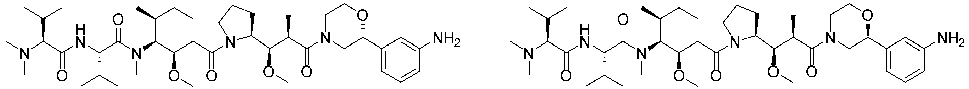 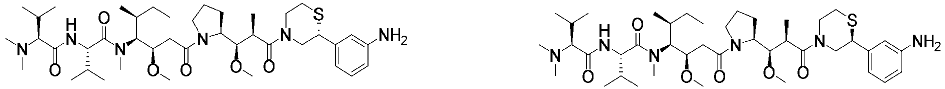 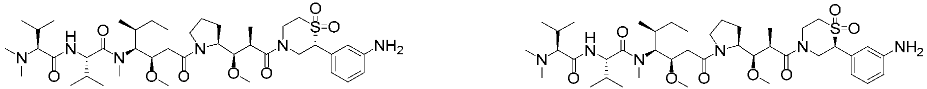 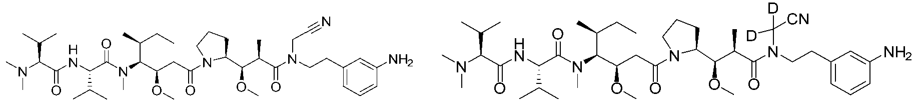 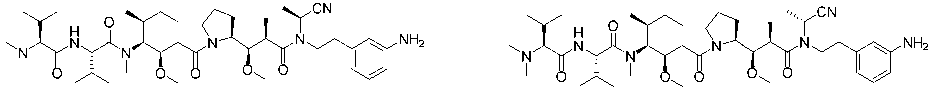 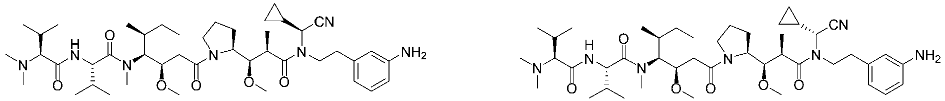 and

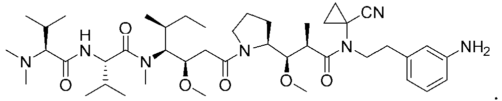

The present disclosure also provides an antibody-drug conjugate or a stereoisomer, isotopic derivative, solvate, nitrogen oxide, prodrug, or pharmaceutically acceptable salt thereof, which is selected from a structure represented by formula (A): 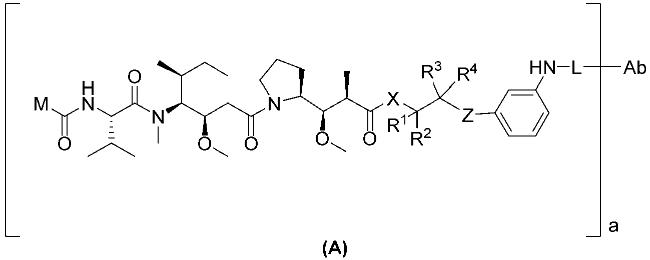
wherein Ab is an antibody or an antigen-binding fragment;
L is a linking group, with the left end thereof linked to NH on the cytotoxic drug and the right end thereof linked to Ab;
a is selected from a numerical value of 1-10;
M, R¹, R², R³, R⁴, X, and Z are as defined in formula (I).

In some embodiments, the antibody-drug conjugate or the stereoisomer, isotopic derivative, solvate, nitrogen oxide, prodrug, or pharmaceutically acceptable salt thereof is selected from a structure represented by formula (A-1): 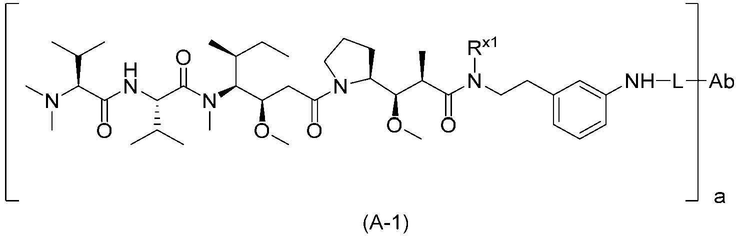
wherein Ab is a Her2 antibody; preferably, Ab is trastuzumab;
R^{x1} is selected from H, -CH₃, OH, -OCH₃, 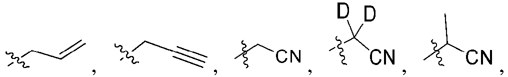 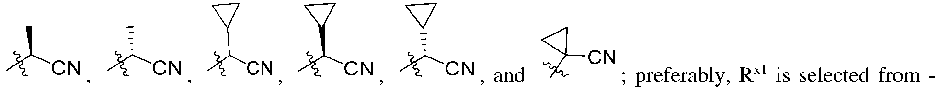 OCH₃, 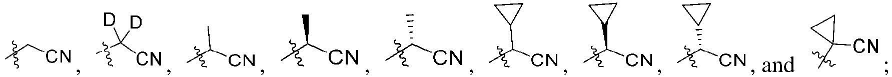 a is selected from a numerical value of 2-8; preferably, a is selected from a numerical value of 4-8;
L is as defined in formula (A).

In some embodiments, provided is the antibody-drug conjugate or the stereoisomer, isotopic derivative, solvate, nitrogen oxide, prodrug, or pharmaceutically acceptable salt thereof, wherein L is selected from

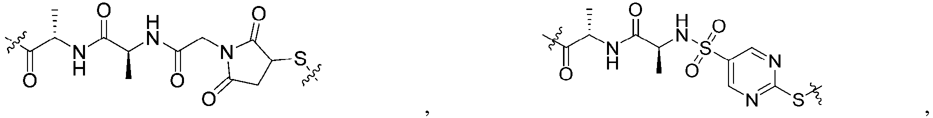

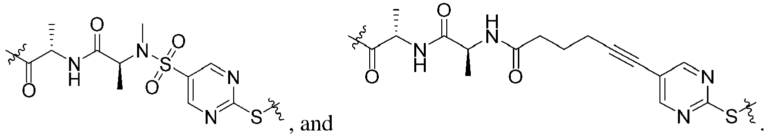

In some embodiments, provided is the antibody-drug conjugate or the stereoisomer, isotopic derivative, solvate, nitrogen oxide, prodrug, or pharmaceutically acceptable salt thereof, wherein L is selected from

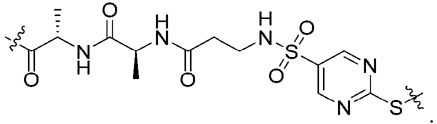

In some embodiments, provided is the antibody-drug conjugate or the stereoisomer, isotopic derivative, solvate, nitrogen oxide, prodrug, or pharmaceutically acceptable salt thereof, wherein a is selected from a numerical value of 2-8; preferably, a is selected from a numerical value of 4-8.

The present disclosure also provides a cytotoxic drug-linker or a stereoisomer, isotopic derivative, solvate, nitrogen oxide, prodrug, or pharmaceutically acceptable salt thereof, wherein the cytotoxic drug-linker has a structure represented by formula (B), 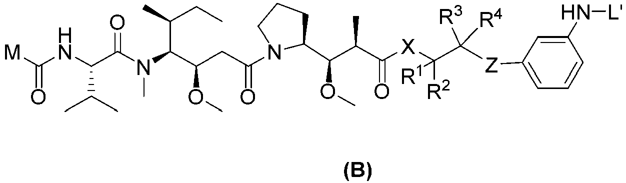
wherein L' is a structure of L before being linked to Ab in formula (A);
preferably, L' is selected from 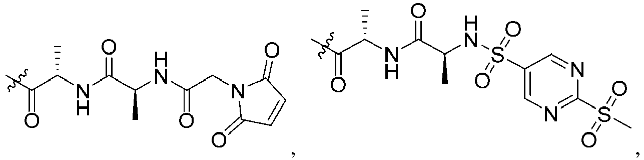 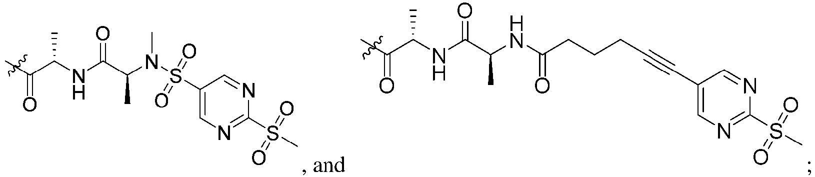
M, R¹, R², R³, R⁴, X, and Z are as defined in formula (I).

In some embodiments, preferably, L' is selected from

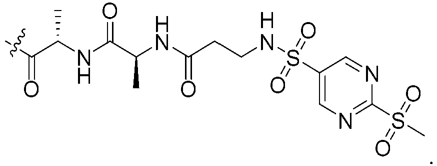

In some embodiments, the cytotoxic drug-linker or the stereoisomer, isotopic derivative, solvate, nitrogen oxide, prodrug, or pharmaceutically acceptable salt thereof is selected from a structure represented by formula (B-1): 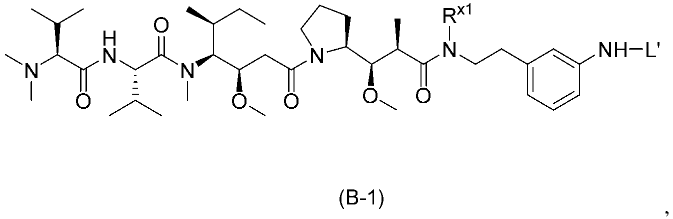
wherein R^{x1} is selected from H, -CH₃, OH, -OCH₃, 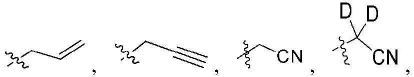 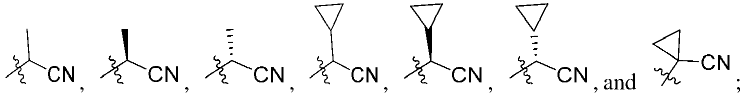 preferably, R^{x1} is selected from -OCH₃, 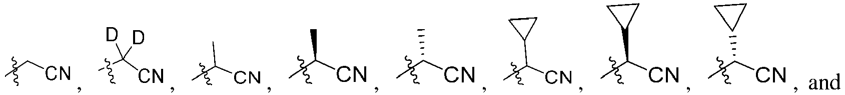 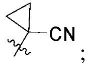
L' is selected from 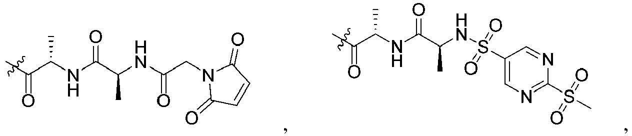 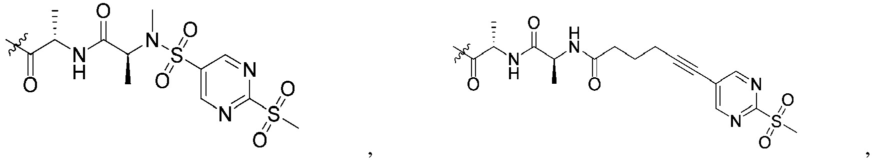 and 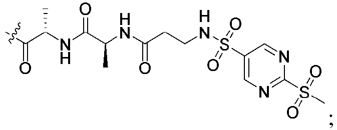 preferably, L' is

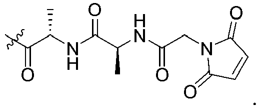

In some embodiments, the cytotoxic drug-linker is selected from: 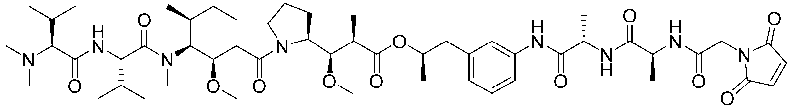 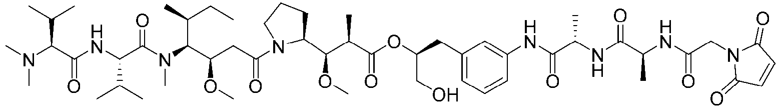 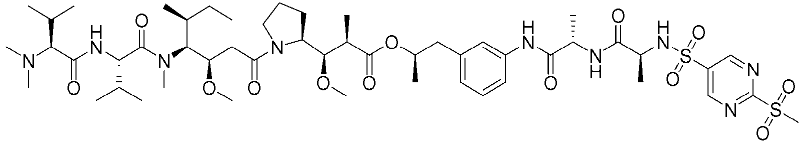 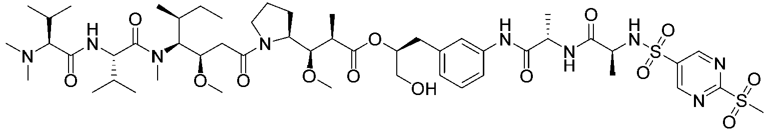 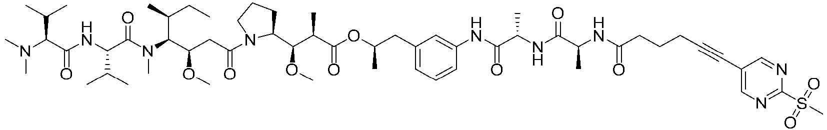 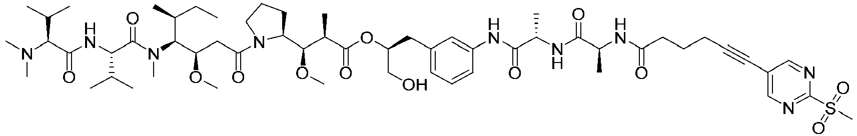 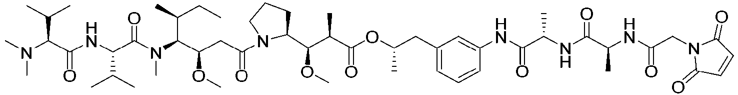 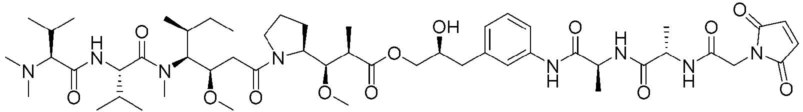 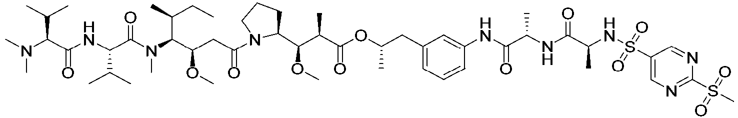 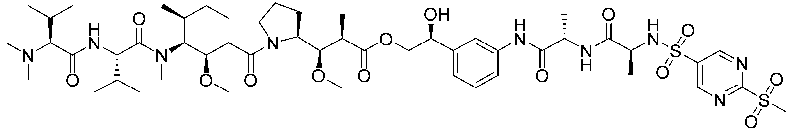 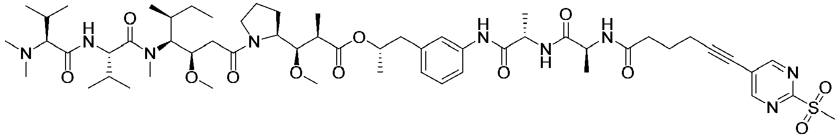 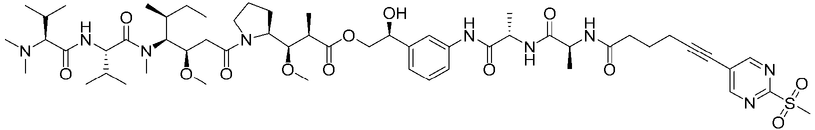 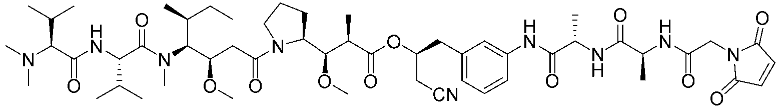 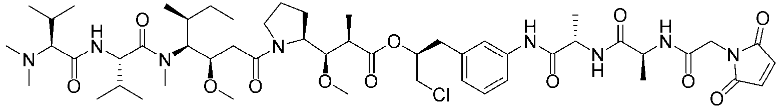 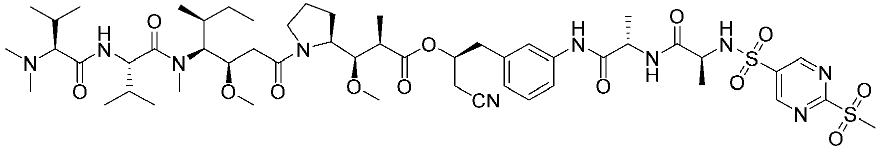 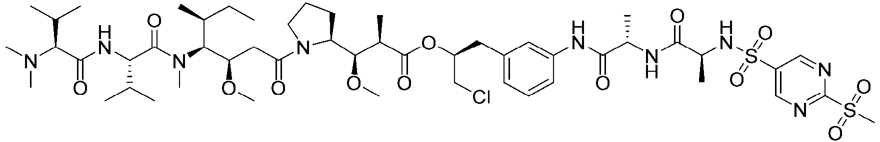 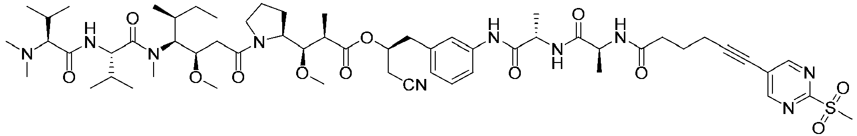 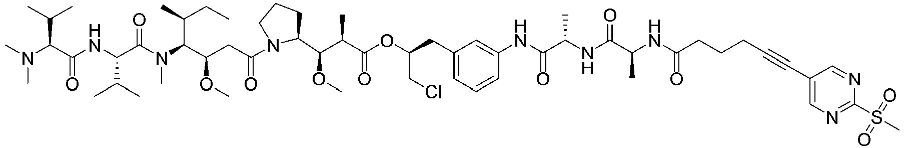 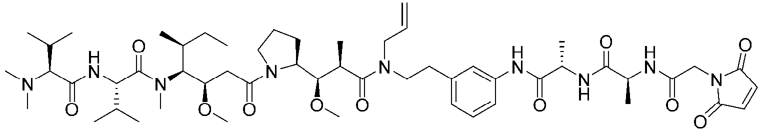 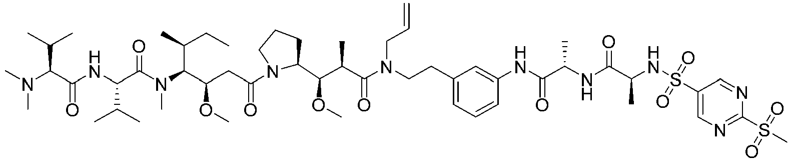 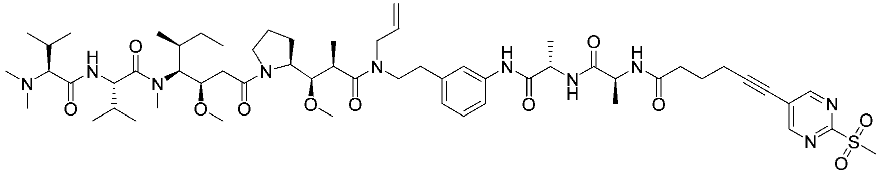 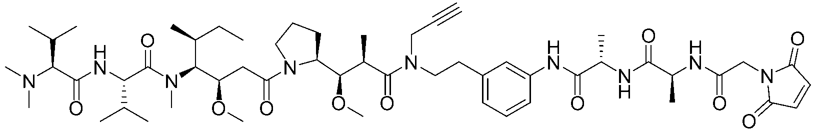 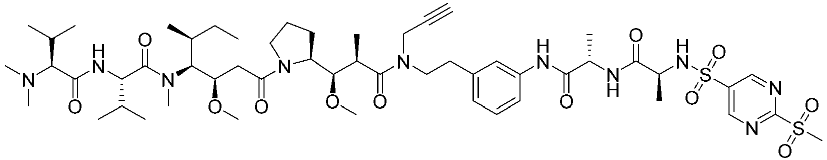 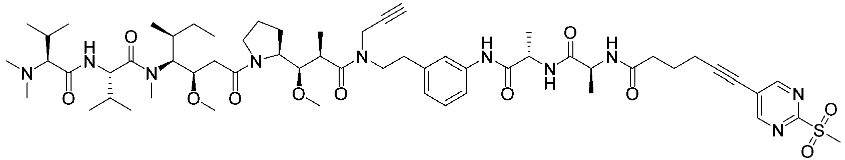 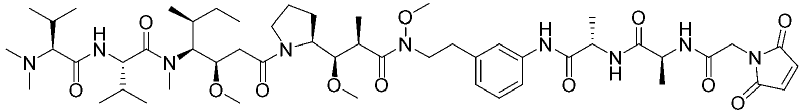 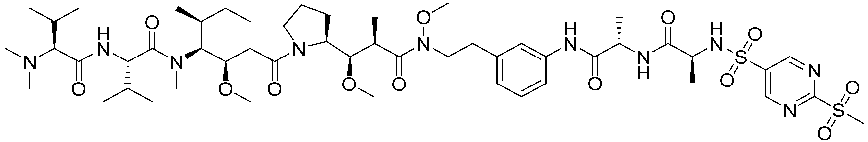 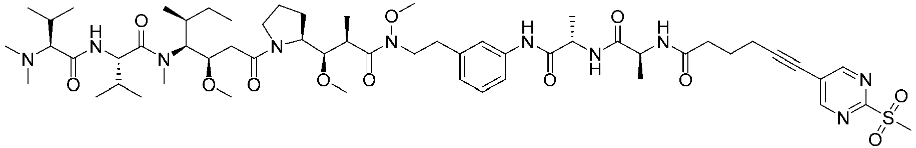 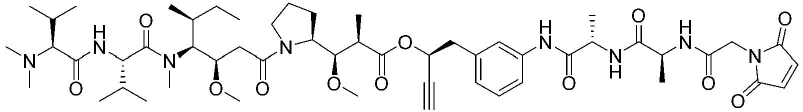 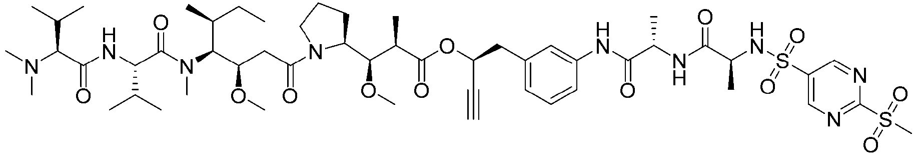 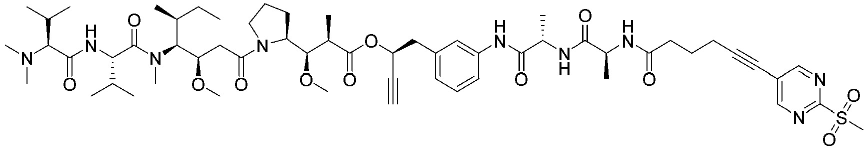 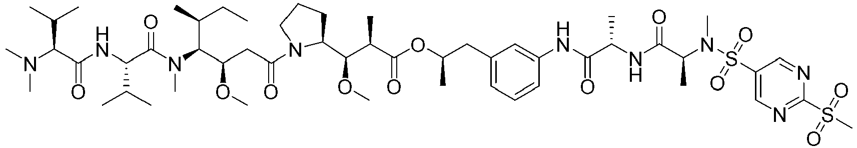 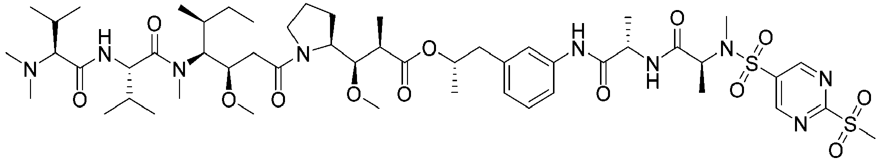 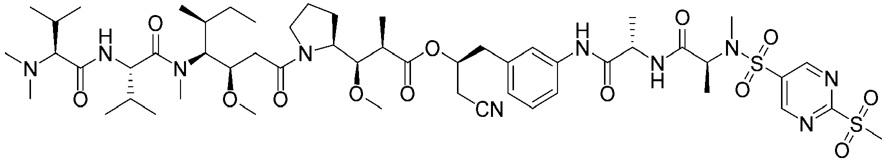 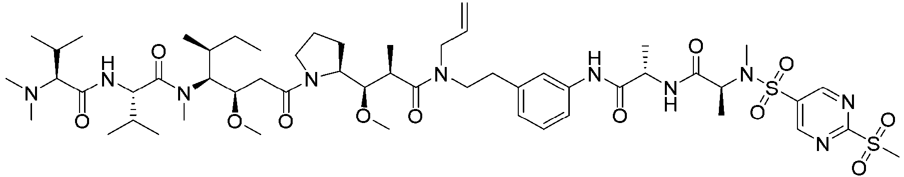 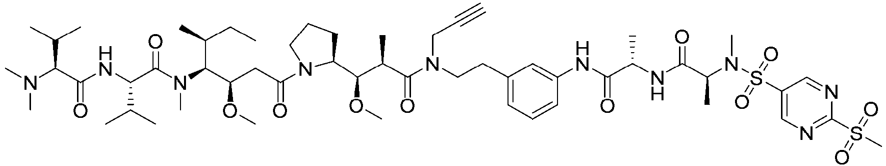 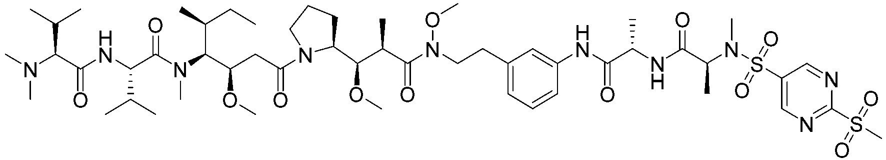 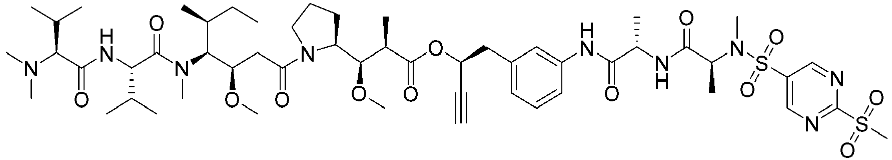 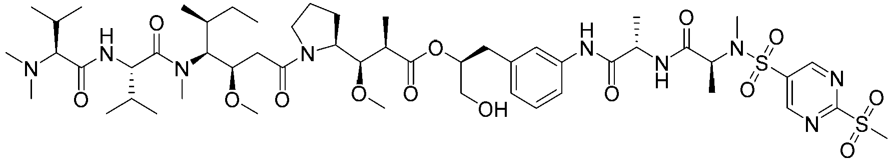 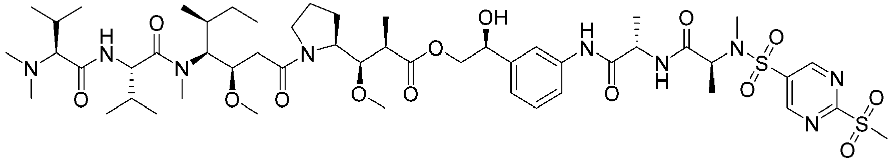 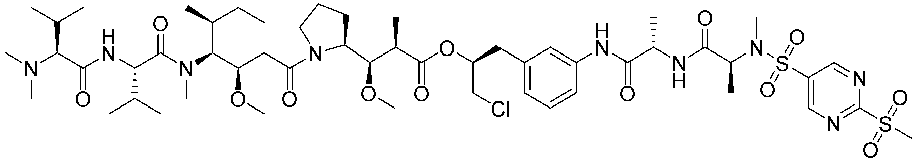 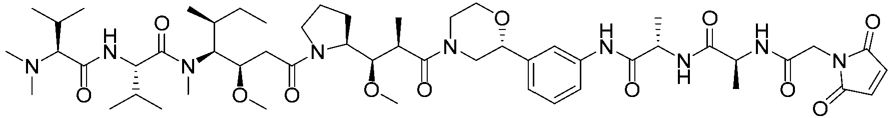 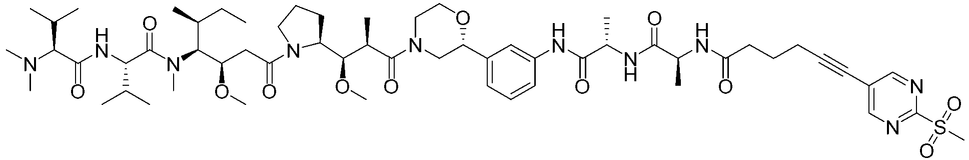 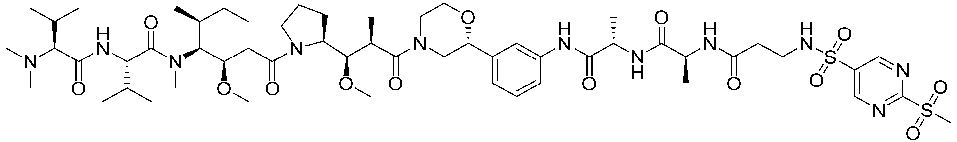 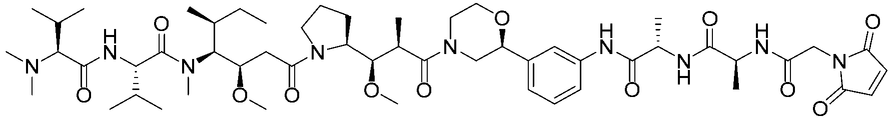 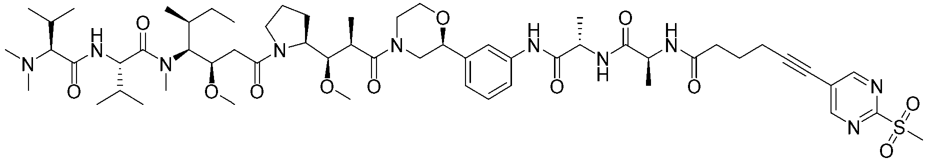 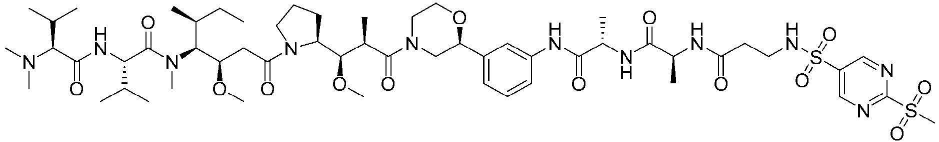 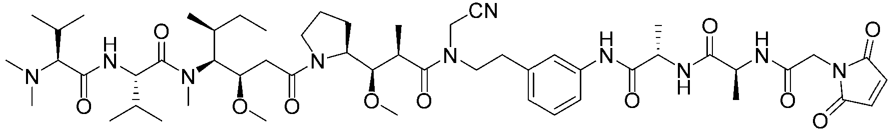 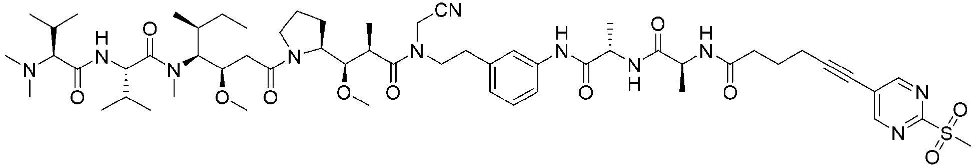 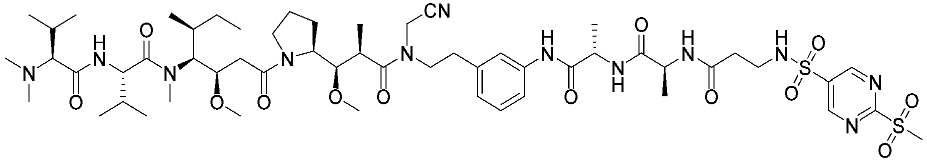 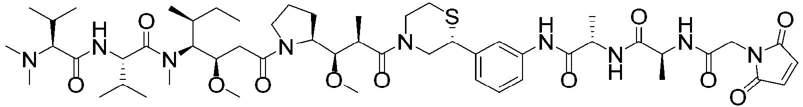 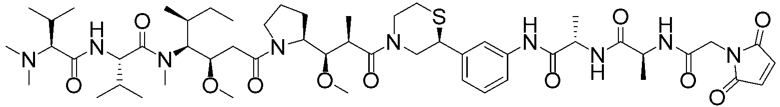 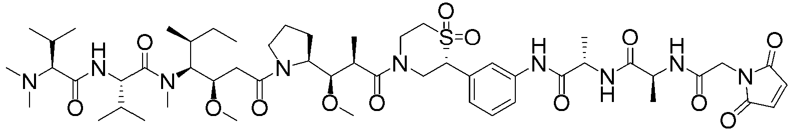 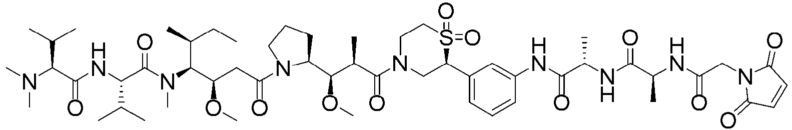 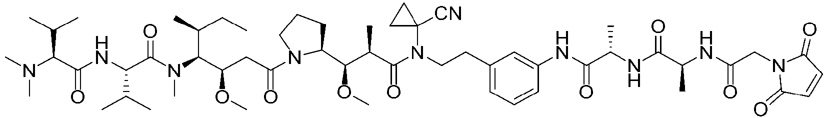 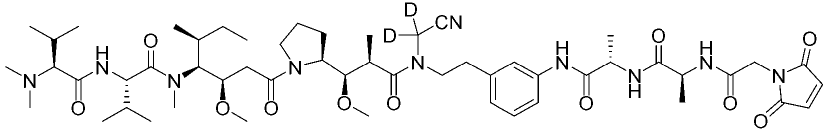 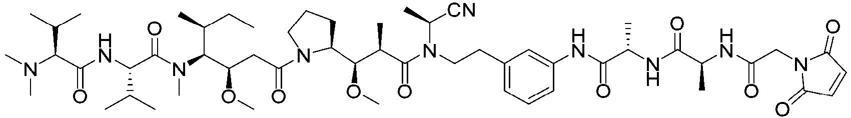 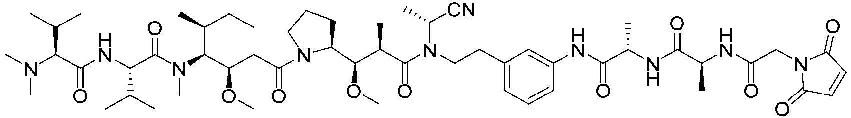 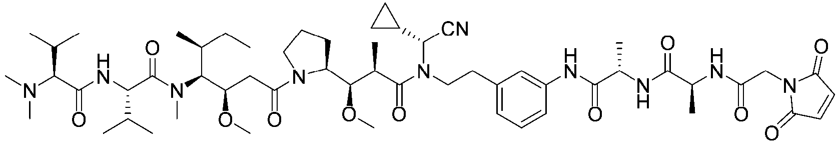 and

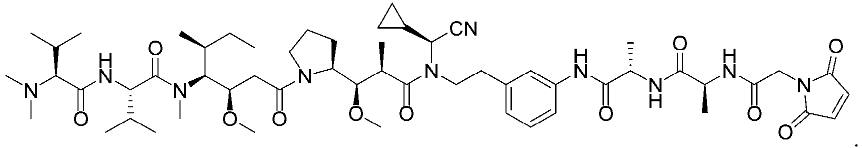

In some embodiments, the antibody-drug conjugate or the stereoisomer, isotopic derivative, solvate, nitrogen oxide, prodrug, or pharmaceutically acceptable salt thereof is selected from: 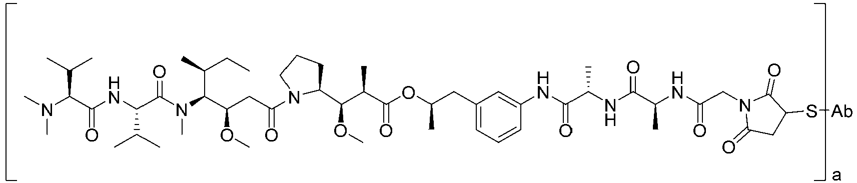 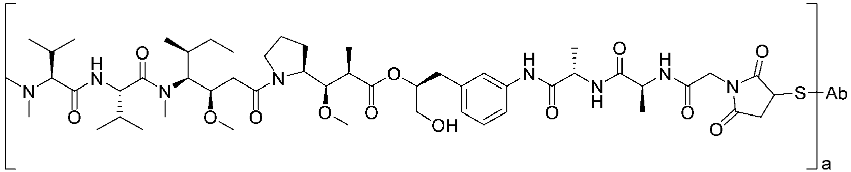 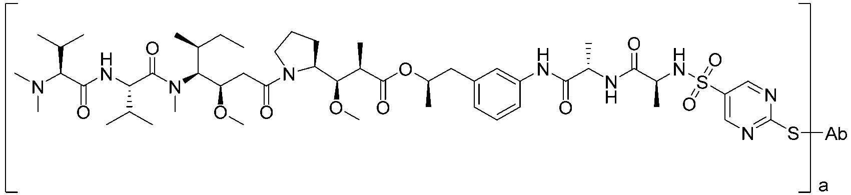 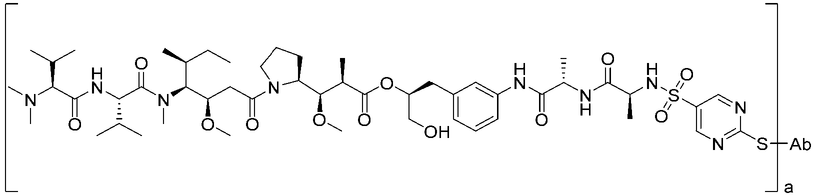 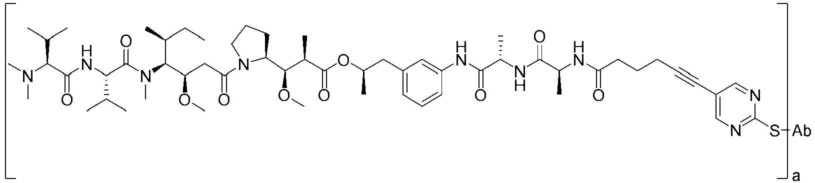 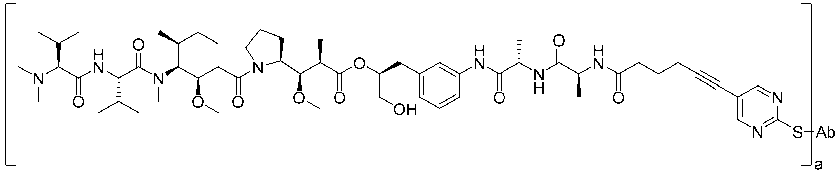 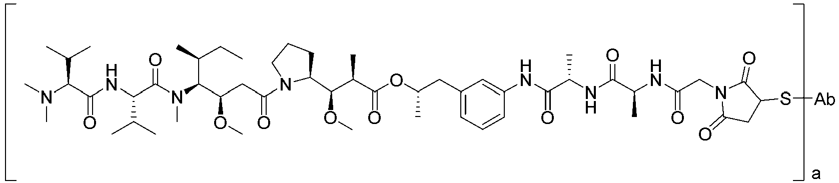 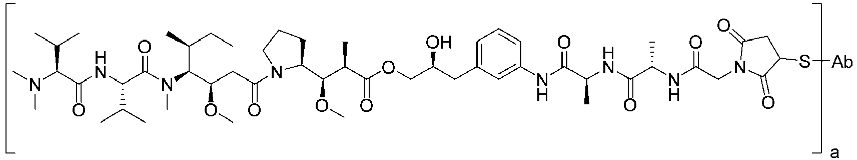 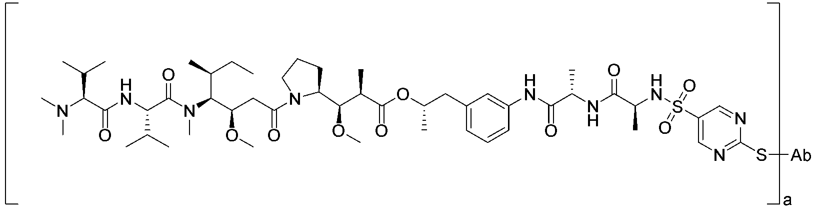 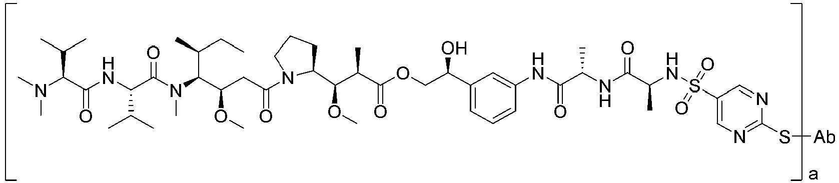 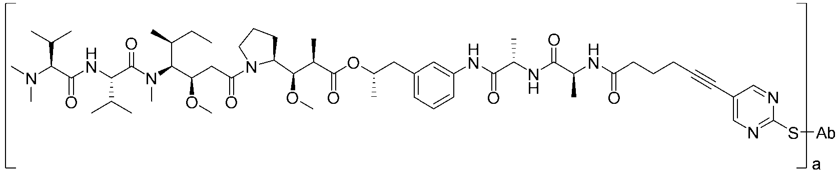 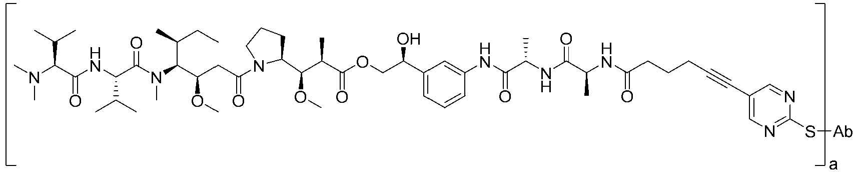 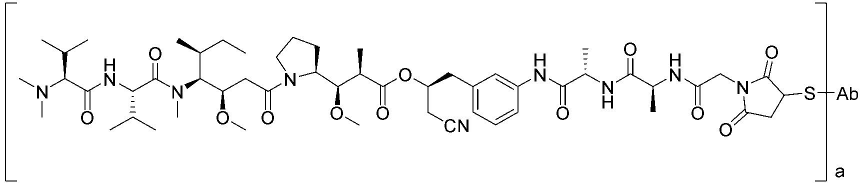 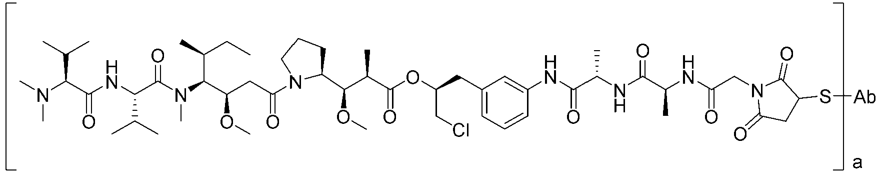 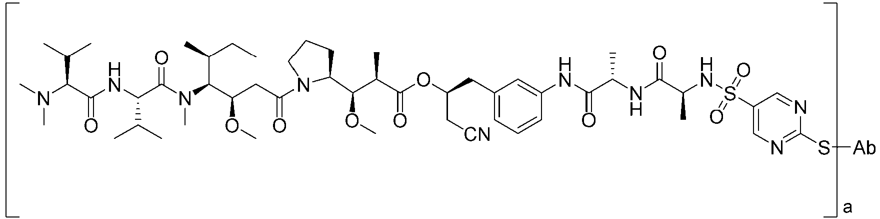 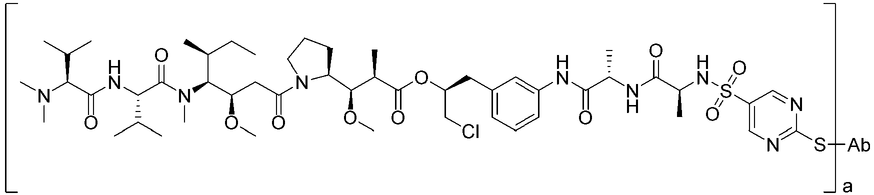 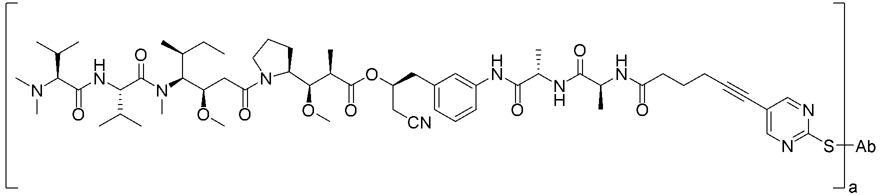 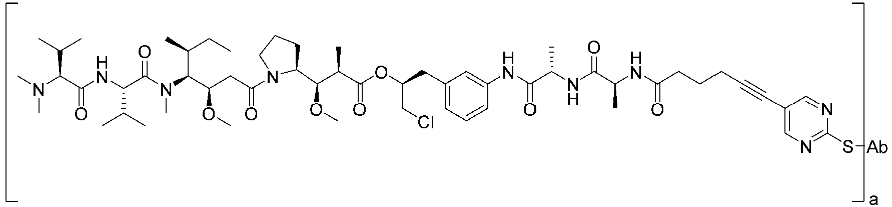 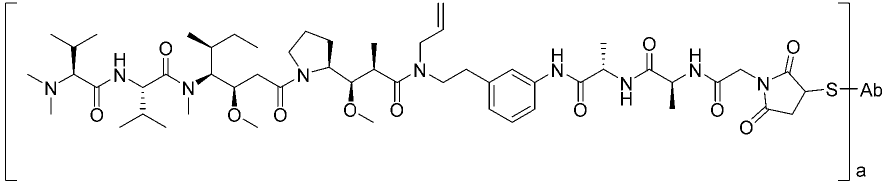 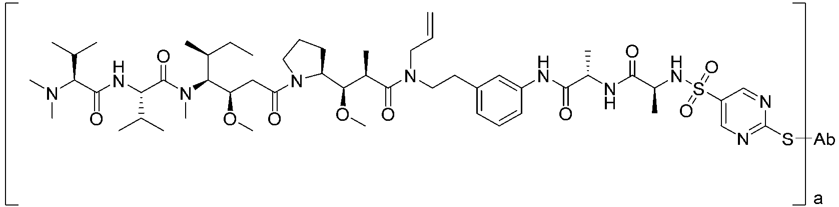 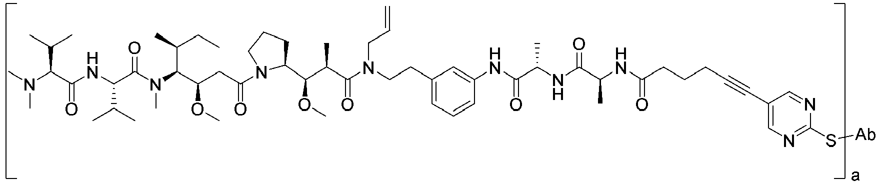 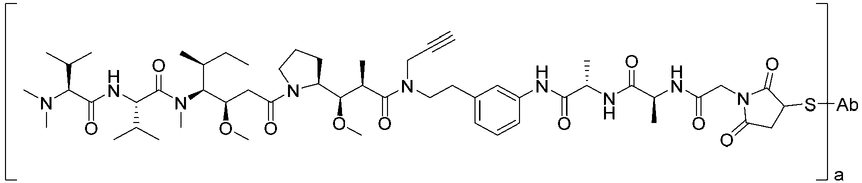 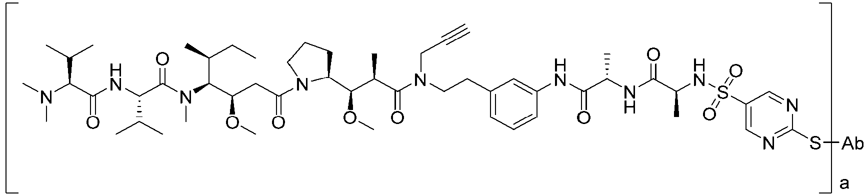 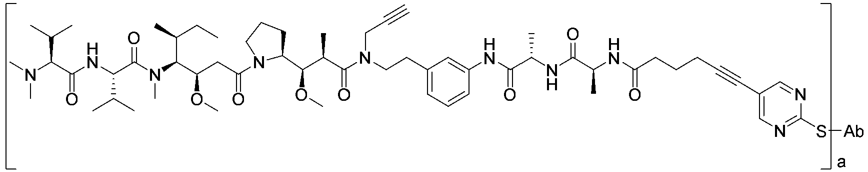 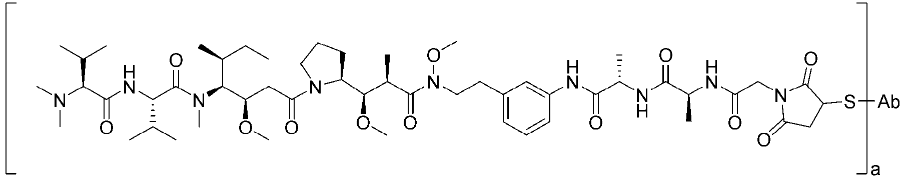 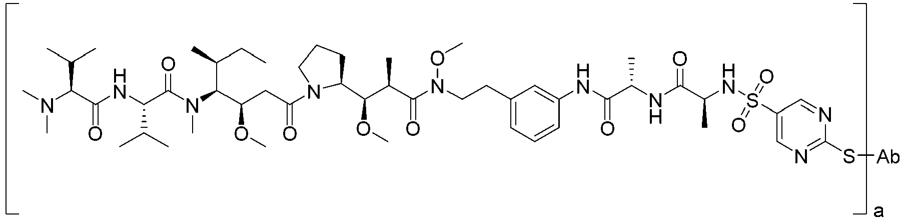 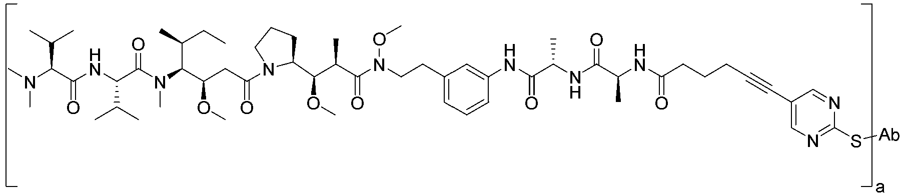 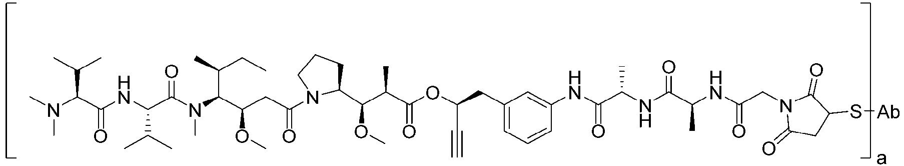 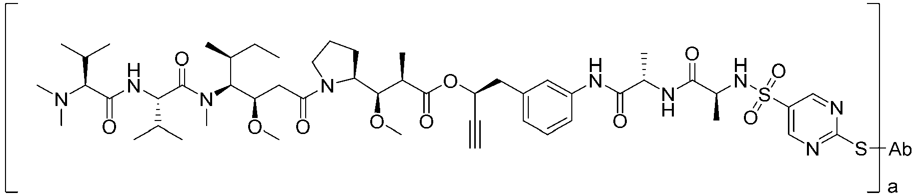 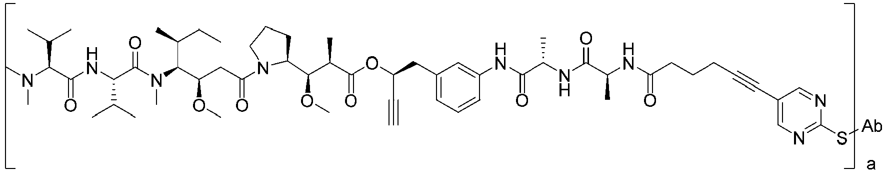 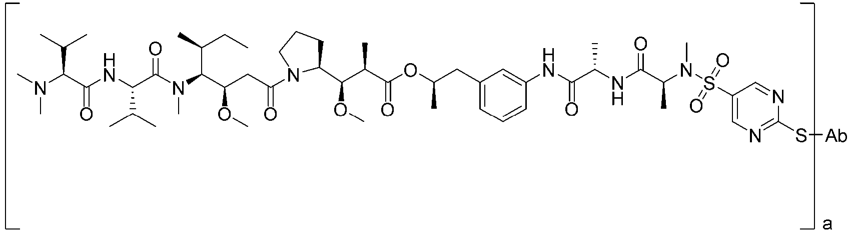 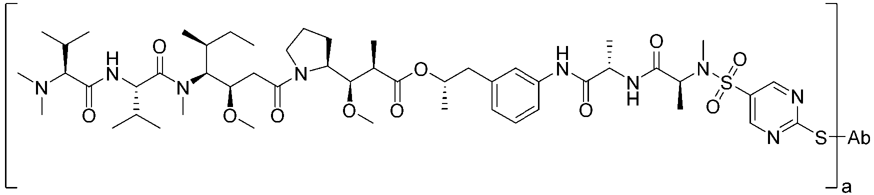 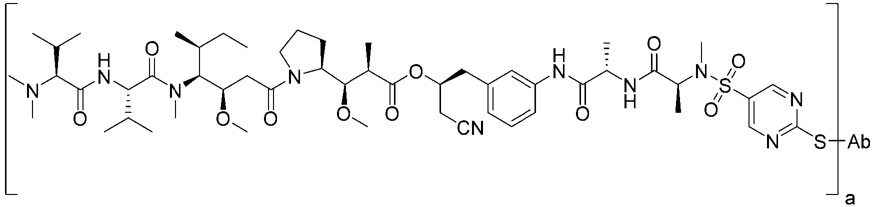 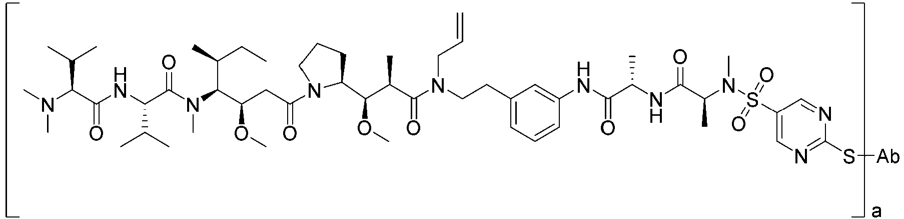 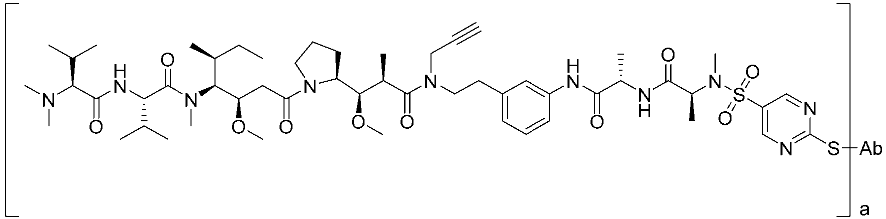 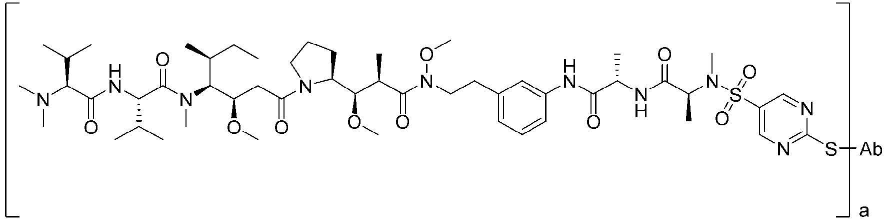 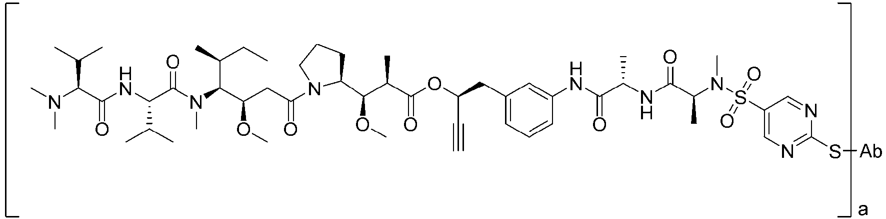 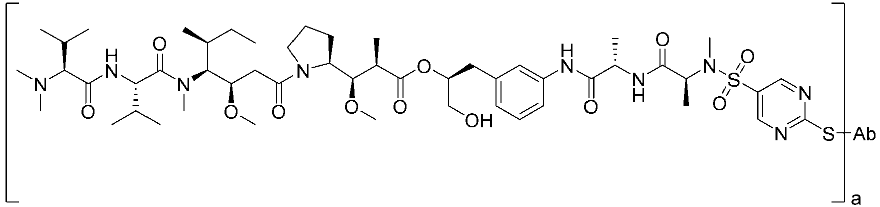 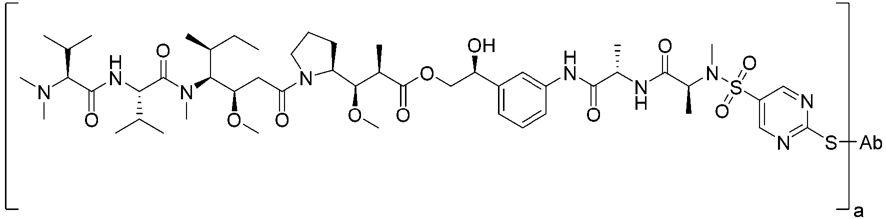 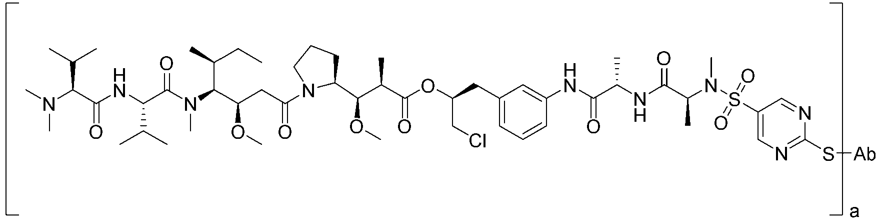 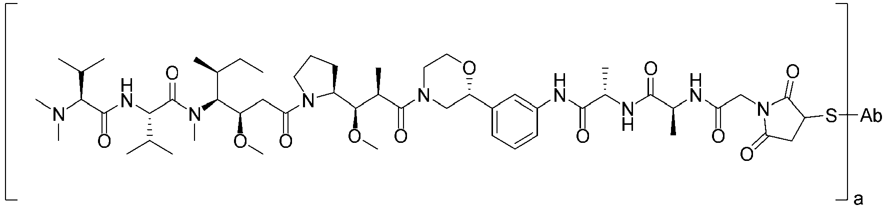 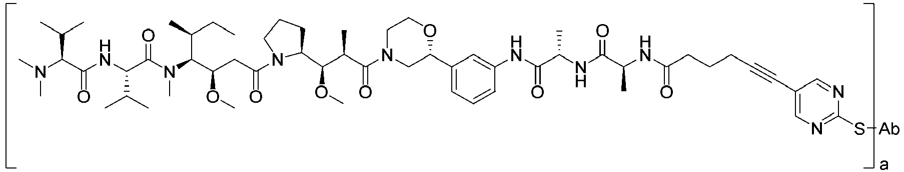 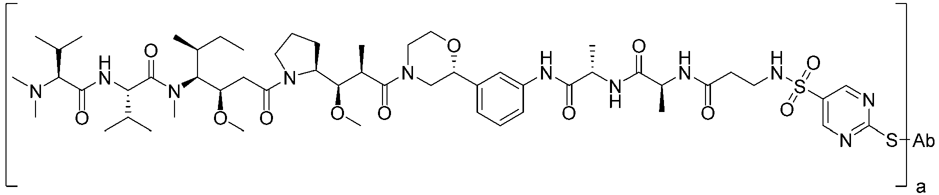 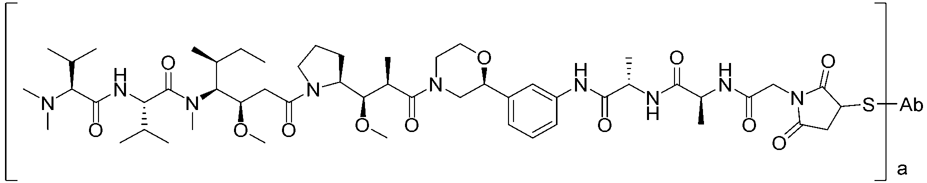 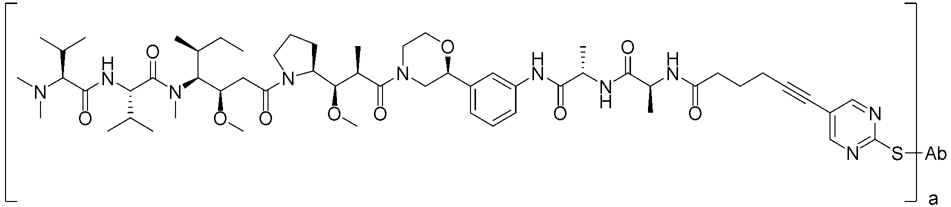 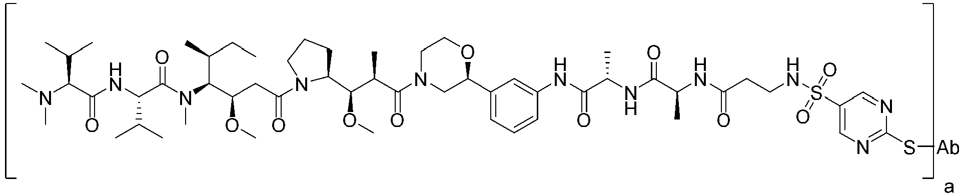 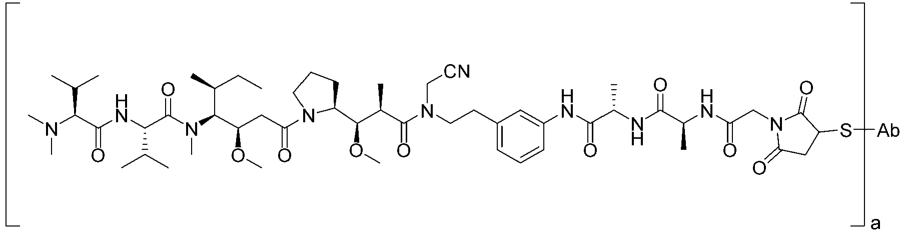 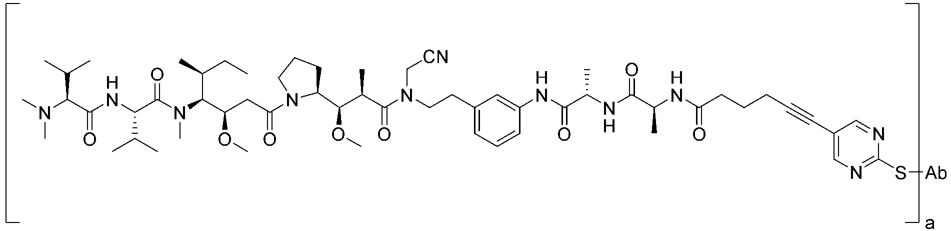 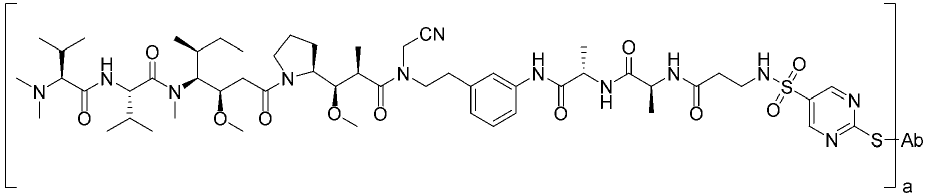 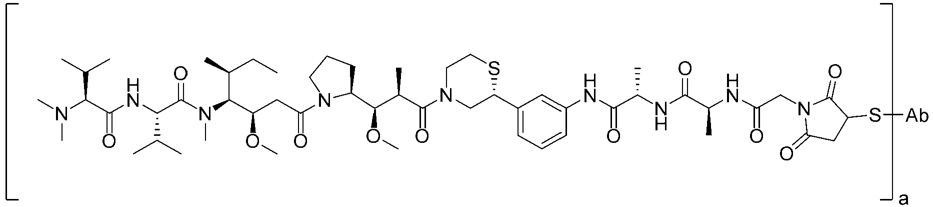 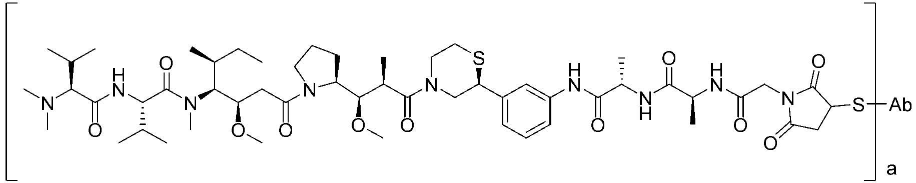 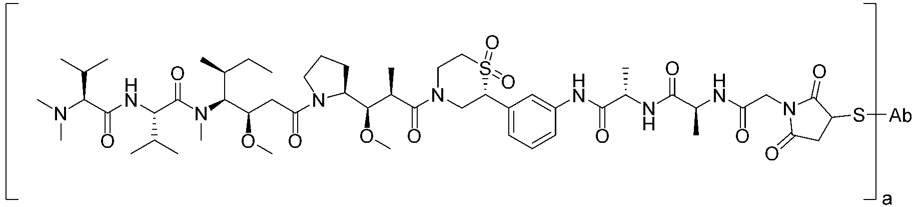 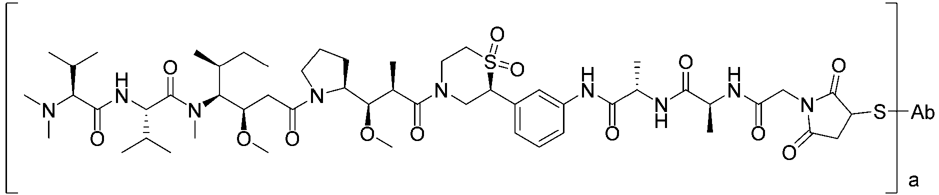 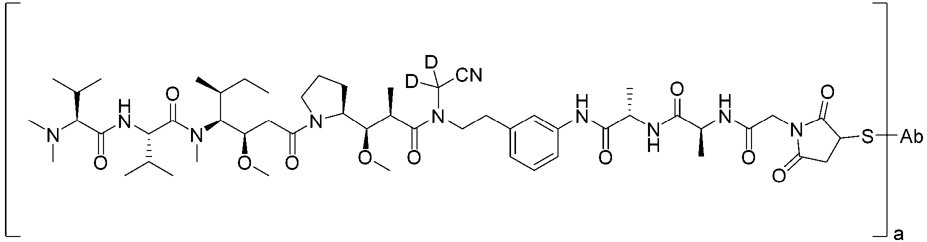 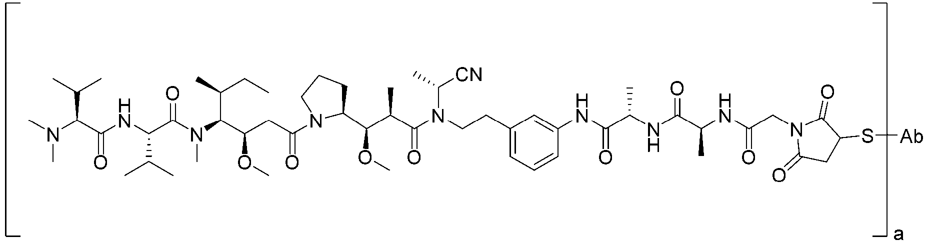 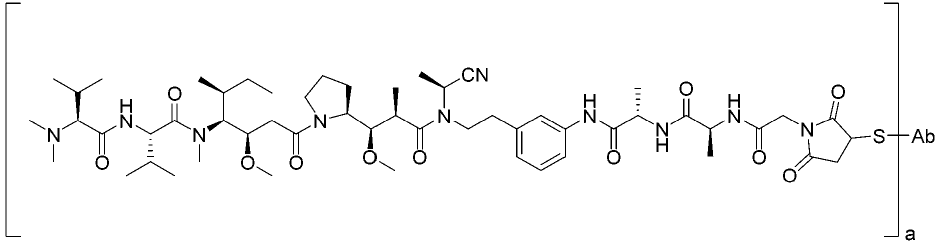 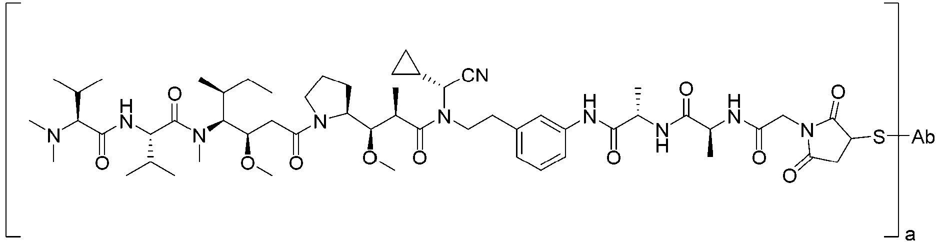 and 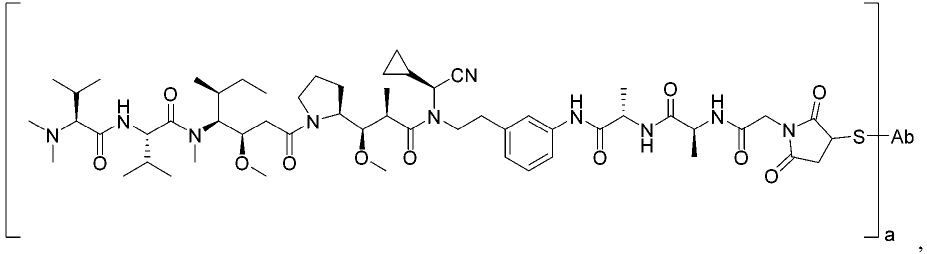
wherein Ab is an antibody, preferably a Her2 antibody, and more preferably trastuzumab;
a is selected from a numerical value of 2-8, preferably a numerical value of 4-8.

In another aspect, the present disclosure provides an antibody-drug conjugate, wherein the drug has a structure represented by the compound described above.

In another aspect, the present disclosure provides a pharmaceutical composition comprising the cytotoxic drug of formula (I) or the stereoisomer, isotopic derivative, solvate, nitrogen oxide, prodrug, or pharmaceutically acceptable salt thereof, or the antibody-drug conjugate of formula (A) or the stereoisomer, isotopic derivative, solvate, nitrogen oxide, prodrug, or pharmaceutically acceptable salt thereof, and a pharmaceutically acceptable carrier.

In some embodiments of the present disclosure, in the pharmaceutical composition, the antibody-drug conjugate or the stereoisomer, isotopic derivative, solvate, nitrogen oxide, prodrug, or pharmaceutically acceptable salt thereof has a content selected from 0.1 mg-1000 mg.

In some embodiments of the present disclosure, in the pharmaceutical composition, the antibody-drug conjugate or the stereoisomer, isotopic derivative, solvate, nitrogen oxide, prodrug, or pharmaceutically acceptable salt thereof has a content of 1%-95%.

In some embodiments of the present disclosure, in the pharmaceutical composition, the pharmaceutically acceptable carrier includes one or more of a filler, a disintegrant, a binder, a glidant, and a lubricant.

In another aspect, the present disclosure provides use of the cytotoxic drug of formula (I) or the stereoisomer, isotopic derivative, solvate, nitrogen oxide, prodrug, or pharmaceutically acceptable salt thereof, the cytotoxic drug-linker of formula (B) or formula (B-1) or the stereoisomer, isotopic derivative, solvate, nitrogen oxide, prodrug, or pharmaceutically acceptable salt thereof, the antibody-drug conjugate of formula (A) or formula (A-1) or the stereoisomer, isotopic derivative, solvate, nitrogen oxide, prodrug, or pharmaceutically acceptable salt thereof, or the pharmaceutical composition thereof as a medicament (i.e., for use in therapy).

In another aspect, the present disclosure also provides use of the cytotoxic drug of formula (I) or the stereoisomer, isotopic derivative, solvate, nitrogen oxide, prodrug, or pharmaceutically acceptable salt thereof, the cytotoxic drug-linker of formula (B) or formula (B-1) or the stereoisomer, isotopic derivative, solvate, nitrogen oxide, prodrug, or pharmaceutically acceptable salt thereof, the antibody-drug conjugate of formula (A) or formula (A-1) or the stereoisomer, isotopic derivative, solvate, nitrogen oxide, prodrug, or pharmaceutically acceptable salt thereof, or the pharmaceutical composition thereof in preparing a medicament for treating a tumor.

In another aspect, the present disclosure also provides a method for preventing or treating a tumor, comprising administering to a patient an effectively prophylactic or therapeutic amount of the cytotoxic drug of formula (I) or the stereoisomer, isotopic derivative, solvate, nitrogen oxide, prodrug, or pharmaceutically acceptable salt thereof, the cytotoxic drug-linker of formula (B) or formula (B-1) or the stereoisomer, isotopic derivative, solvate, nitrogen oxide, prodrug, or pharmaceutically acceptable salt thereof, the antibody-drug conjugate of formula (A) or formula (A-1) or the stereoisomer, isotopic derivative, solvate, nitrogen oxide, prodrug, or pharmaceutically acceptable salt thereof, or the pharmaceutical composition thereof.

In some embodiments of the present disclosure, the tumor includes breast cancer, ovarian cancer, cervical cancer, uterine cancer, prostate cancer, kidney cancer, urethral cancer, bladder cancer, liver cancer, gastric cancer, endometrial cancer, salivary gland cancer, esophageal cancer, melanoma, neuroglioma, neuroblastoma, sarcoma, lung cancer, head and neck cancer, colon cancer, rectal cancer, colorectal cancer, leukemia, skin cancer, thyroid cancer, pancreatic cancer, or lymphoma.

In some embodiments of the present disclosure, the lung cancer is selected from small cell lung cancer and non-small cell lung cancer; the leukemia is selected from acute lymphocytic leukemia, acute myelocytic leukemia, acute promyelocytic leukemia, chronic myelocytic leukemia, and chronic lymphocytic leukemia; the lymphoma is selected from Hodgkin lymphoma, non-Hodgkin lymphoma, and recurrent anaplastic large cell lymphoma.

### Terminology

Unless otherwise stated, the following terms and phrases used herein are intended to have the following meanings. A particular term or phrase, unless otherwise specifically defined, should not be considered uncertain or unclear, but construed according to its common meaning.

The term "cytotoxic drug" or "drug" refers to a small-molecule compound capable of strongly disrupting the normal growth of tumor cells. The cytotoxic drug can kill tumor cells in principle at a sufficiently high concentration; however, due to a lack of specificity, the cytotoxic drug can cause apoptosis of normal cells while killing tumor cells, resulting in serious side effects.

The term "antibody-drug conjugate" means that an antibody is linked to a cytotoxic drug with biological activity via a stable linker.

The term "antibody" refers to an immunoglobulin, which is of a tetrapeptide chain structure formed by linkage between two identical heavy chains and two identical light chains via an interchain disulfide bond.

The term "antigen-binding fragment" refers to one or more fragments of an antibody that retain the ability to specifically bind to an antigen.

The term "linker site" refers to a chemical structural fragment or bond that is linked to Ab.

The term "linker" refers to a chemical structural fragment that links a cytotoxic drug to Ab; in an embodiment of the present disclosure, the linker is -L-.

It should be noted that regarding the sulfur (S) in L of the structural formula 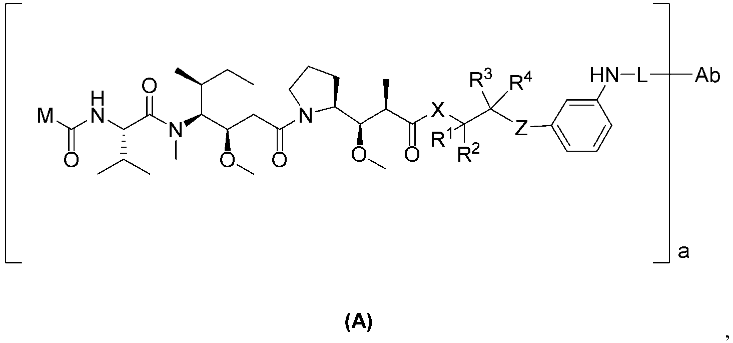 it can be understood by those skilled in the art that L is linked to a sulfhydryl group inherently present in Ab (e.g., an antibody) after the disulfide bond is cleaved (for example, the disulfide bond can be cleaved by reducing the disulfide bond using a reducing agent TCEP, generating the sulfhydryl group (SH)); that is, the S between the right end of L and Ab is not an additionally introduced sulfur atom.

For example,
the -S- in 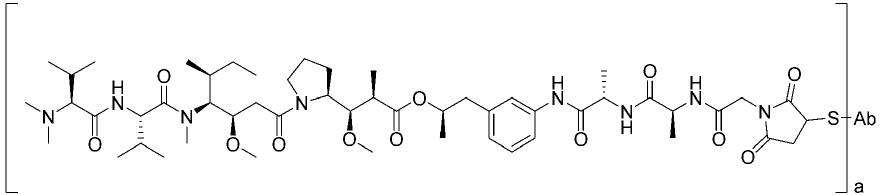 is not an additionally introduced sulfur atom, but rather the -S- formed by linking the sulfhydryl group inherently present in Ab after the disulfide bond is cleaved to the right end of L, such as

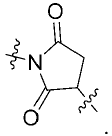

The term "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problems or complications, and are commensurate with a reasonable benefit/risk ratio.

The term "pharmaceutically acceptable salt" refers to a derivative prepared from the compound of the present disclosure with a relatively non-toxic acid or base. Such a salt may be prepared during synthesis, separation, or purification of the compound or by reacting the free form of the purified compound with a suitable acid or base. When the compound contains a relatively acidic functional group (e.g., -COOH, -OH, -SO₃H, etc.), the compound reacts with a suitable inorganic or organic cation (base) to give a base addition salt, including a salt formed with an alkali metal or alkaline earth metal, an ammonium salt formed with amine or a derivative thereof, a salt formed with an amino acid, etc. When the compound contains a relatively basic functional group (e.g., -NH₂), the compound reacts with a suitable inorganic or organic anion (acid) to give an acid addition salt, including a salt formed with an inorganic or organic acid (e.g., carboxylic acid, etc.).

The term "pharmaceutically acceptable carrier" refers to a medium that is generally acceptable in the art for delivering a biologically active agent to animals, particularly mammals, and depending on the route of administration and the nature of the dosage form, includes, for example, an adjuvant or an excipient, such as a diluent, a preservative, a filler, a flow modifier, a disintegrant, a wetting agent, an emulsifier, a suspending agent, a sweetener, a flavoring agent, a fragrance, an antibacterial agent, an antifungal agent, a lubricant, and a dispersing agent. Pharmaceutically acceptable carriers are formulated within the purview of those of ordinary skill in the art based on a large number of factors. These factors include, but are not limited to, the type and nature of the formulated active agent, the subject to which the composition containing the agent is to be administered, the intended route of administration of the composition, and the target therapeutic indication. Pharmaceutically acceptable carriers include both aqueous and non-aqueous media and a variety of solid and semi-solid dosage forms. Such carriers comprise many different ingredients and additives in addition to the active agent, and such additional ingredients comprised in the formula for a variety of reasons (e.g., stabilizing the active agent, binder, etc.) are well known to those of ordinary skill in the art.

The term "effectively prophylactic or therapeutic amount" refers to an amount of the compound, the pharmaceutically acceptable salt thereof, or the isomer thereof of the present disclosure that is sufficient to treat a disorder at a reasonable benefit/risk ratio applicable to any medical treatment and/or prevention. However, it should be appreciated that the total daily amount of the compound represented by formula I or the pharmaceutically acceptable salt thereof and the composition of the present disclosure must be determined by an attending physician within the scope of reliable medical judgment. For any particular patient, the specific therapeutically effective dose level must be determined based on a variety of factors, and these factors include the disorder being treated and the severity of the disorder; the activity of the specific compound employed; the specific composition employed; the age, body weight, general health, sex, and diet of the patient; the time of administration, route of administration, and excretion rate of the specific compound employed; the duration of the treatment; the drugs used in combination or simultaneously with the specific compound employed; and similar factors well known in the medical field.

The "isomer" described in the present disclosure includes a geometric isomer and a stereoisomer, such as an atropisomer, a cis-trans isomer, an enantiomer, a diastereoisomer, a tautomer, and racemic and other mixtures thereof, and all of these mixtures are encompassed within the scope of the present disclosure.

The term "enantiomer" refers to stereoisomers that are mirror images of each other. The term "tautomer" refers to a type of functional isomer in which the point of attachment for hydrogen varies due to the shift of one or more double bonds; for example, a ketone and its enol form represent keto-enol tautomers. The term "diastereoisomer" refers to stereoisomers that are molecules with two or more chiral centers and are not mirror images of each other. The term "cis-trans isomer" refers to different spatial configurations existing due to the inability of double bonds or single bonds of ring-forming carbon atoms to rotate freely in a molecule. The term "atropisomer" refers to stereoisomers that can be separated due to hindered or very slow rotation of a single bond. Stereoisomers of the compounds of the present disclosure can be prepared by chiral synthesis or chiral reagents or other conventional techniques. For example, one enantiomer of a certain compound of the present disclosure can be prepared by asymmetric catalysis techniques or chiral auxiliary derivatization techniques. Alternatively, a compound with a single stereoconfiguration can be obtained from a mixture by chiral resolution techniques. Alternatively, it can be prepared directly using chiral starting materials. The separation of optically pure compounds in the present disclosure is generally accomplished by preparative chromatography using chiral chromatographic columns to achieve the purpose of separating chiral compounds.

The term "isotopic derivative" refers to a compound in which at least one atom is replaced with an atom having the same atomic number but a different atomic mass. Examples of isotopes that can be incorporated into the compounds of the present disclosure include stable and radioactive isotopes of hydrogen, carbon, nitrogen, oxygen, phosphorus, sulfur, fluorine, chlorine, bromine, iodine, etc., such as ²H (deuterium, D), ³H (tritium, T), ¹¹C, ¹³C, ¹⁴C, ¹⁵N, ¹⁷O, ¹⁸O, ³²p, ³³p, ³³S, ³⁴S, ³⁵S, ³⁶S, ¹⁸F, ³⁶Cl, ⁸²Br, ¹²³I, ¹²⁴I, ¹²⁵I, ¹²⁹I, and ¹³¹I, preferably deuterium. Compared with non-deuterated drugs, deuterated drugs have the advantages of reduced toxic and side effects, increased drug stability, enhanced efficacy, prolonged drug biological half-lives, and the like. All transformations of isotopic composition of the compounds of the present disclosure, whether radioactive or not, are encompassed within the scope of the present disclosure. Each available hydrogen atom linked to a carbon atom may be independently replaced with a deuterium atom, wherein the replacement with deuterium may be partial or complete, and partial replacement with deuterium refers to the replacement of at least one hydrogen atom with at least one deuterium atom.

The term "nitrogen oxide" or "N-oxide" refers to a derivative formed by further oxidizing a nitrogen atom in a nitrogen-containing group, and a common N-oxide includes a N-oxide of a tertiary amine or a N-oxide of a nitrogen atom in a nitrogen-containing heterocyclic ring.

The term "prodrug" refers to certain derivatives of the compounds of the present disclosure that have little or no pharmacological activity themselves, which have a cleavable group and decompose through solvolysis or under physiological conditions to become the compounds of the present disclosure. The types of the prodrug include, but are not limited to, amides, esters, anhydrides, salts, and the like. The "ester" refers to a derivative formed by the compound of the present disclosure with a suitable alcohol when the compound of the present disclosure contains an acidic group (e.g., carboxylic acid), and a derivative formed by the compound of the present disclosure with a suitable acid (including an organic acid or an inorganic acid) when the compound of the present disclosure contains a hydroxyl group. Methods for preparing prodrugs are well known to those skilled in the art. The compounds of the present disclosure can exist in unsolvated as well as solvated forms, and the solvated forms include hydrated forms. In general, the solvated forms are equivalent to unsolvated forms and are also encompassed within the scope of the present disclosure. The absolute stereoconfiguration of a compound can be confirmed by conventional technical means in the art. For example, by single-crystal X-ray diffraction, the absolute configuration of a compound can also be confirmed through the chiral structure of the starting material and the reaction mechanism of asymmetric synthesis. Alternatively, after resolution, the stereoconfiguration can be determined by comparison with a product whose absolute configuration is determined. Compounds herein labeled as "absolute configuration unknown/undetermined" are generally obtained by resolving a racemic compound into a single isomer via chiral preparative SFC, followed by characterization and testing.

It is known to those skilled in the art that when a cyclic compound possesses a coplanar delocalized system with a π-electron count of 4n+2, the ring is aromatic; the representation of aromatic structures in compounds may employ either dashed lines indicating electron delocalization or alternating single and double bonds; for example, the structure of a benzene ring may be drawn as 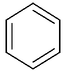 or as

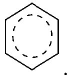

The "optionally substituted" described in the present disclosure refers to both cases where one or more hydrogen atoms of a substituted group may be "substituted" or "not substituted" with one or more substituents.

When 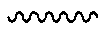 appears in a substituent structure, it indicates that the atom is a bonding atom; for example, 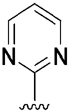 indicates that the C atom on the pyrimidine ring is a bonding atom. When a dash "-" appears in a substituent structure, it indicates the point of attachment for the substituent, e.g., -CH₃ is attached via a C atom.

" 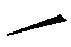 " and " 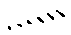 " represent the absolute configuration of a stereocenter, i.e., R or S configuration. " 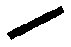 " or " 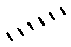 " represents a cis or trans configuration; a pair of solid bonds or a pair of dashed bonds represents a cis configuration, while one solid bond and one dashed bond together represent a trans configuration.

When a bond of a substituent can be cross-linked to a ring, it indicates that the substituent can bond to any atom on the ring. For example, the structural unit 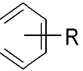 indicates that substitution with the substituent R may occur at any position on the benzene ring.

When a listed substituent does not specify through which atom the substituent is attached to a given group or given structural formula, then the substituent may be attached through any bondable atom thereof.

When any variable (e.g., R_{d}) appears more than once in the constitution or structure of a compound, the variable is independently defined in each case. For example, 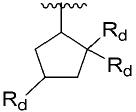 indicates that cyclopentyl is substituted with 3 R_{d}, and each R_{d} has an independent option.

Unless otherwise specified, the term "halogen" refers to a fluorine, chlorine, bromine, or iodine atom.

Unless otherwise specified, the term "alkyl" refers to a group derived by removing one hydrogen atom from a branched or linear saturated aliphatic alkane having a specified number of carbon atoms. For example, "C₁₋₁₀ alkyl" is meant to include C₁, C₂, C₃, C₄, C₅, C₆, C₇, C₈, C₉, and C₁₀ alkyl, "C₁₋₆ alkyl", "C₁₋₄ alkyl", and "C₁₋₃ alkyl"; specific examples include, but are not limited to: methyl, ethyl, n-propyl, isopropyl, sec-butyl, 2-methylbutyl, 1,1-dimethylbutyl, and the like.

Unless otherwise specified, the term "haloalkyl" refers to an alkyl group having one or more hydrogen atoms substituted with a halogen atom. C₁₋₆ haloalkyl is preferred, and C₁₋₄ haloalkyl is more preferred. Examples of haloalkyl include, but are not limited to, monofluoromethyl, difluoromethyl, trifluoromethyl, trichloromethyl, tribromomethyl, 2,2,2-trifluoroethyl, 2,2,2-trichloroethyl, and the like. The alkyl is as defined above. Unless otherwise specified, the term "hydroxyalkyl" refers to a group derived by substituting one or more hydrogen atoms in an alkyl group with hydroxyl; the "hydroxyalkyl" described in the present disclosure includes "C₁₋₆ hydroxyalkyl" and "C₁₋₄ hydroxyalkyl"; specific examples include, but are not limited to, -CH₂OH, -CH₂CH₂OH, -CH(OH)CH₃, -CH₂CH₂CH₂OH, 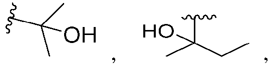 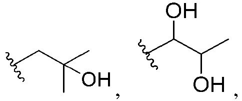 and the like.

Unless otherwise specified, the term "alkoxy" refers to an alkyl group as defined herein attached to another group through an oxygen atom, i.e., "alkyl-O-". It includes "C₁₋₆ alkoxy" (structure: C₁₋₆ alkyl-O-) and "C₁₋₄ alkoxy", and specific examples include, but are not limited to, methoxy, ethoxy, propoxy, 1-methylethoxy, butoxy, 2-methylbutoxy, 3-methylbutoxy, 1,1-dimethylpropoxy, 1,2-dimethylpropoxy, and the like; preferably, the "alkoxy" described in the present disclosure is preferably C₁₋₄ alkoxy, more preferably C₁₋₃ alkoxy, and further preferably C₁₋₂ alkoxy.

Unless otherwise specified, the term "haloalkoxy" refers to a group obtained by substituting one or more hydrogen atoms in an alkoxy group with halogen, and preferably, the "haloalkoxy" described in the present disclosure is preferably "C₁₋₆ haloalkoxy" or "C₁₋₄ haloalkoxy". Specific examples described in the present disclosure include: fluoromethoxy (including monofluoromethoxy, difluoromethoxy, trifluoromethoxy), -OCH₂CF₃, -OCHFCH₃, and the like. The alkoxy is as defined above.

Unless otherwise specified, the term "alkenyl" refers to a group derived by removing one hydrogen atom from a linear or branched alkene (containing at least one carbon-carbon double bond), including "C₂₋₆ alkenyl", "C₂₋₅ alkenyl", "C₂₋₄ alkenyl", and "C₂₋₃ alkenyl", and specific examples include, but are not limited to: -CH=CH₂, -CH=CHCH₃, -C(CH₂)=CH₂, -CH₂CH=CH₂, -CH=CHCH₂CH₃, -CH₂CH=CHCH₃, and the like.

Unless otherwise specified, the term "alkynyl" refers to a group derived by removing one hydrogen atom from a linear or branched alkyne (containing at least one carbon-carbon triple bond), including "C₂₋₅ alkynyl", "C₂₋₄ alkynyl", and "C₂₋₃ alkynyl", and specific examples include, but are not limited to: -C≡CH, -C≡CHCH₃, -CH₂C≡CH, HC≡C-C≡C-, and the like.

Unless otherwise specified, the term "ring" refers to a saturated, partially saturated, or unsaturated monocyclic ring and polycyclic ring, and "polycyclic ring" includes spiro, fused, or bridged rings. A group derived by removing a hydrogen atom from a ring is referred to as a "cyclic group", which includes a monovalent ring, a divalent ring (generally referred to as cyclene), a trivalent ring, a tetravalent ring, and the like, and the specific valence depends on the number of substituents attached to the ring. The description of "cyclic group" in the present disclosure does not specifically distinguish the valence of the ring. Representative "cyclic groups" include substituted or unsubstituted cycloalkyl, heterocyclyl, cycloalkenyl, heterocycloalkenyl, cycloalkynyl, heterocycloalkynyl, aryl, or heteroaryl. The term "hetero" refers to a substituted or unsubstituted heteroatom and an oxidized form of the heteroatom (also referred to as a heteroatom group); the heteroatom is generally selected from N, O, S, and P, the oxidized form generally includes NO, SO, S(O)₂, and P(O), and the nitrogen atom may be substituted, i.e., NR (R is H or another substituent as defined herein); the number of atoms on the ring is generally defined as the number of ring members, and for example, "3- to 6-membered heterocycloalkyl" refers to a ring formed by 3-6 atoms arranged in a cycle; each ring optionally contains 1-3 heteroatoms and/or heteroatom groups, i.e., N, O, S, NO, SO, S(O)₂, P(O), or NR, and each ring is optionally substituted with an R group, R being a group as defined herein.

Unless otherwise specified, "cycloalkenyl" refers to a "cycloalkyl" group in which one or more ring-forming bonds are double bonds, and the cycloalkenyl is not aromatic. The carbon atom in the cycloalkenyl may be further substituted with oxo, i.e., C(O) is formed. The cycloalkenyl includes "3- to 8-membered cycloalkenyl", "3- to 6-membered cycloalkenyl", "3- to 5-membered cycloalkenyl", and "5- to 6-membered cycloalkenyl". Specific examples include, but are not limited to, 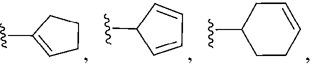 and

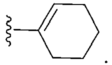

Unless otherwise specified, the term "heterocyclyl" refers to a saturated cyclic group derived by replacing one or more ring carbon atoms in the cycloalkyl with a heteroatom and/or a heteroatom group. The heteroatom and/or the heteroatom group is generally selected from N, O, S, NO, SO, S(O)₂, P(O), and NR, wherein the carbon atom in the heterocyclic ring is optionally substituted with oxo, i.e., -C(O) is formed; preferably, the heteroatom is independently selected from 1-3 N and/or O. The heterocyclyl includes "3- to 8-membered heterocyclyl", "3- to 6-membered heterocyclyl", "3- to 5-membered heterocyclyl", "4- to 6-membered heterocyclyl", and "5- to 6-membered heterocyclyl". Specific examples include, but are not limited to, oxetanyl, azetidinyl, pyrrolidinyl, tetrahydrofuranyl, piperidinyl, piperazinyl, tetrahydropyranyl, morpholinyl, and the like.

Unless otherwise specified, the term "aryl" refers to an unsaturated and generally aromatic hydrocarbon group, which may be a single ring or multiple rings fused together. C₅₋₁₀ aryl is preferred, C₅₋₈ aryl is more preferred, and monocyclic C₅₋₆ aryl is the most preferred; examples of the aryl include, but are not limited to, phenyl and naphthyl.

Unless otherwise specified, the term "fused aryl" refers to a fused ring group formed by two or more aromatic rings sharing two adjacent carbon atoms, including naphthyl, anthryl, phenanthryl, and the like.

The "heteroaryl" described in the present disclosure refers to an aromatic monocyclic group in which at least one ring atom is a heteroatom and/or a heteroatom group, the heteroatom and/or the heteroatom group is generally selected from N, O, S, P, NO, SO, S(O)₂, P(O), and NR, and R is H or any substituent group that may be present, wherein the carbon atom in the heterocyclic ring is optionally substituted with oxo, i.e., -C(O) is formed; preferably, the heteroatom is independently selected from 1-3 N and/or O. The heteroaryl includes "5- to 6-membered heteroaryl"; specific examples include, but are not limited to, pyrrolyl, furanyl, thienyl, pyrazolyl, imidazolyl, pyrazinyl, pyridazinyl, triazinyl, oxazolyl, isoxazolyl, thiazolyl, isothiazolyl, pyridinyl, and pyrimidinyl.

Unless otherwise specified, the term "cycloalkyl" refers to a saturated monocyclic or polycyclic hydrocarbon group. The cycloalkyl is preferably C₃₋₈ cycloalkyl, and more preferably C₃₋₆ cycloalkyl, and examples of the cycloalkyl include, but are not limited to, cyclopropyl, cyclobutyl, cyclopentyl, cyclohexyl, cycloheptyl, and the like.

The compounds of the present disclosure have isomers, such as cis-trans isomers, enantiomers, diastereoisomers, and racemic and other mixtures thereof, and all of these mixtures are encompassed within the scope of the present disclosure.

The term "enantiomer" refers to stereoisomers that are mirror images of each other.

The term "diastereoisomer" refers to stereoisomers that are molecules with two or more chiral centers and are not mirror images of each other.

The term "cis-trans isomer" refers to a configuration existing due to the inability of double bonds or single bonds of ring-forming carbon atoms to rotate freely in a molecule. Unless otherwise stated, a solid wedge bond 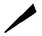 and a dashed wedge bond 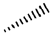 are used to indicate the absolute configuration of a stereocenter.

Stereoisomers of the compounds of the present disclosure can be prepared by chiral synthesis or chiral reagents or other conventional techniques. For example, one enantiomer of a certain compound of the present disclosure can be prepared by asymmetric catalysis techniques or chiral auxiliary derivatization techniques. Alternatively, a compound with a single stereoconfiguration can be obtained from a mixture by chiral resolution techniques. Alternatively, it can be prepared directly using chiral starting materials. The separation of optically pure compounds in the present disclosure is generally accomplished by preparative chromatography using chiral chromatographic columns to achieve the purpose of separating chiral compounds.

It is specifically stated that combinations of substituents and/or variables described in the present disclosure are permissible only if such combinations result in stable compounds or useful synthetic intermediates. A stable compound or stable structure refers to a compound that is sufficiently stable to undergo a chemical reaction, is separated in useful purity, and can be formulated into an effective therapeutic drug.

In the examples of the present disclosure, the naming of the title compounds is performed by converting their chemical structures using Chemdraw. If there is any inconsistency between the compound name and the compound structure, the determination can be assisted by synthesizing relevant information and reaction routes; if confirmation cannot be achieved through other means, the provided compound structural formula shall prevail. The preparation methods of some compounds in the present disclosure refer to the preparation methods of the similar compounds described above. It should be appreciated by those skilled in the art that when using or referring to the preparation methods cited herein, the feed ratio of the reactants, the reaction solvents, the reaction temperatures, and the like may be appropriately adjusted depending on the reactants.

The compounds of the present disclosure can be prepared by a variety of synthetic methods well known to those skilled in the art, including the specific embodiments listed below, embodiments formed by combinations thereof with other chemical synthetic methods, and equivalent alternatives thereof known to those skilled in the art. The preferred embodiments include, but are not limited to, the examples of the present disclosure.

### Summary of experimental instruments:

The compound structures of the present disclosure are determined by nuclear magnetic resonance (NMR) and/or liquid chromatography-mass spectrometry (LC-MS), or ultra performance liquid chromatography-mass spectrometry (UPLC-MS). The NMR chemical shift (δ) is given in parts per million (ppm). NMR determination is performed using a Bruker Neo 400M or Bruker Ascend 400 nuclear magnetic resonance instrument. The determination solvents include deuterated dimethyl sulfoxide (DMSO-d₆), deuterated methanol (CD₃OD), deuterated chloroform (CDCl₃), and heavy water (D₂O), with tetramethylsilane (TMS) as the internal standard.

Liquid chromatography-mass spectrometry (LC-MS) determination is performed using an Agilent 1260-6125B single quadrupole mass spectrometer equipped with a Welch Biomate column (C18, 2.7 µm, 4.6 × 50 mm) or a waters H-Class SQD2 mass spectrometer equipped with a Welch Ultimate column (XB-C18, 1.8 µm, 2.1 × 50 mm) (with electrospray ionization as the ion source).

Ultra performance liquid chromatography-mass spectrometry (UPLC-MS) determination is performed using a waters UPLC H-class SQD mass spectrometer (with electrospray ionization as the ion source).

HPLC determination is performed using waters e2695-2998 or waters ARC and Agilent 1260 or Agilent Poroshell HPH high performance liquid chromatographs.

Preparative HPLC is performed using waters 2555-2489 (10 µm, ODS 250 cm × 5 cm) or GILSON Trilution LC, and the column is a Welch XB-C18 column (5 µm, 21.2 × 150 mm).

The thin-layer chromatography silica gel plate used is a GF254 silica gel plate from Yantai Jiangyou Silicone Development Co., Ltd. or a GF254 silica gel plate from Rushan Sanpont New Materials Co., Ltd. The specifications adopted for TLC range from 0.15 mm to 0.20 mm, with preparative plates measuring 20 × 20 cm. Generally, a 200-300 mesh silica gel from Yucheng Chemical is used as a carrier in column chromatography.

Starting materials in the examples of the present disclosure are known and commercially available, or may be synthesized by using or according to methods known in the art. Unless otherwise specified, all reactions in the present disclosure are performed under a dry nitrogen or argon atmosphere with continuous magnetic stirring, wherein the solvent is a dry solvent, and the reaction temperature is expressed in degrees centigrade or °C.

The compounds of the present disclosure are prepared by referring to WO2023122228A1 and WO2023081230A1, with corresponding reaction substrate substitutions made according to structural differences. Alternatively, the compounds of the present disclosure are prepared using the following routes: 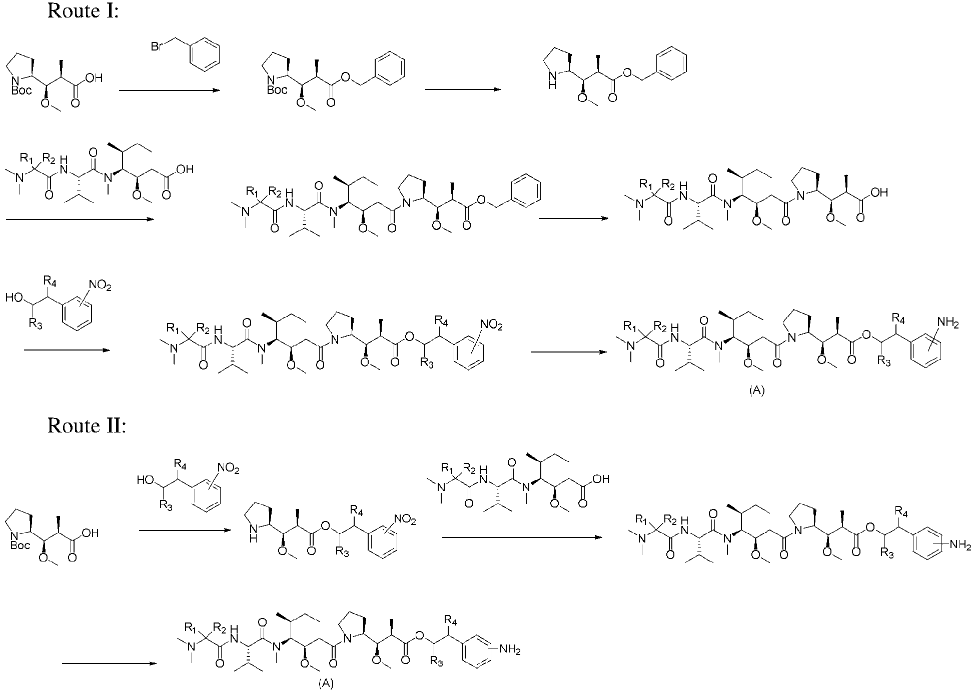
wherein in the route, R¹ and R² are H or C₁₋₆ alkyl, or R¹ and R², together with the carbon atom to which they are attached, form C₃₋₆ cycloalkyl;
R³ is selected from C₂₋₄ alkynyl, C₂₋₄ alkenyl, -COOH, -COO-C₁₋₄ alkyl, C₁₋₄ alkyl, and C₁₋₄ haloalkyl, wherein the C₂₋₄ alkenyl or C₁₋₄ alkyl can be optionally further substituted with -N₃, OH, CN, halogen, or carboxyl.
R⁴ is selected from H and hydroxyl.

The compounds of the present disclosure exhibit inhibition activity against HCC1954 and T47D cells with IC₅₀ < 1 µM, preferably IC₅₀ < 100 nM, more preferably IC₅₀ < 20 nM, and further preferably IC₅₀ < 10 nM.

### BRIEF DESCRIPTION OF THE DRAWINGS

FIG. 1 shows the tumor volume change curves of each group in the NCI-N87 xenograft tumor model at an administration dose of 1 mpk;
FIG. 2 shows the tumor volume change curves of each group in the NCI-N87 xenograft tumor model at an administration dose of 4 mpk.

### DETAILED DESCRIPTION

### Example 1:

### Preparation of (S)-N-((3R,4S,5S)-1-((S)-2-((1R,2R)-3-(((S)-1-(3-aminophenyl)-3-chloropropan-2-yl)amino)-1-methoxy-2-methyl-3-oxopropyl)pyrrolidin-1-yl)-3-methoxy-5-methyl-1-oxoheptan-4-yl)-2-((S)-2-(dimethylamino)-3-methylbutanamido)-N,3-dimethylbutanamide.

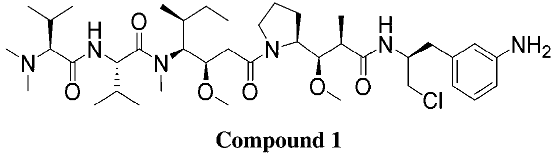

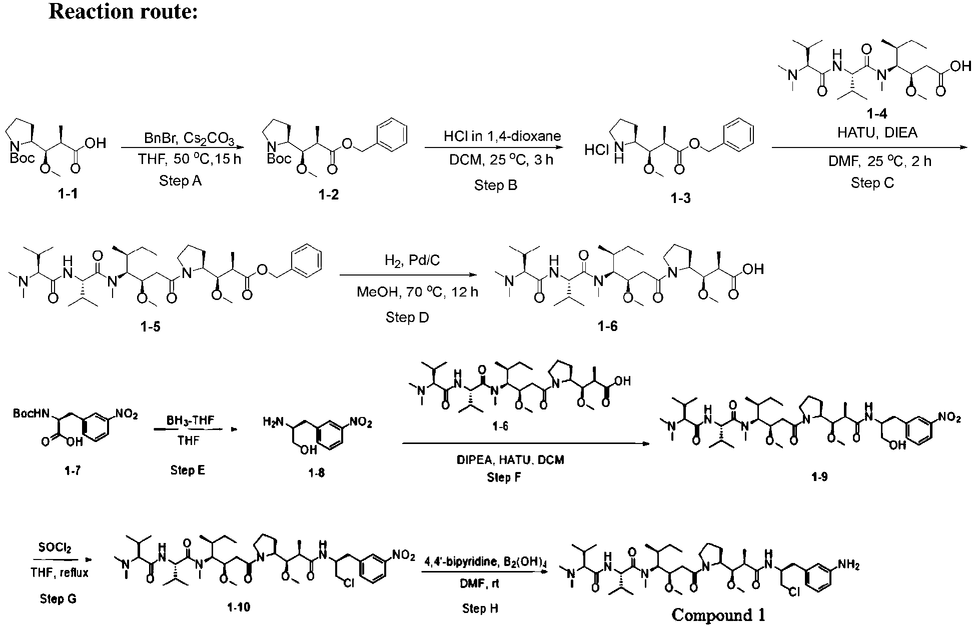

### Procedures:

**Step A:** Compound **1-1** (6.4 g, 22.27 mmol) and cesium carbonate (14.51 g, 44.54 mmol) were added to a single-necked flask, and tetrahydrofuran (80 mL) and benzyl bromide (4.57 g, 26.72 mmol) were added sequentially. The reaction system was reacted at 50°C for 16 h under a nitrogen atmosphere. After completion of the reaction as monitored by LCMS, the reaction liquid was filtered, and the filter cake was washed with tetrahydrofuran (30 mL × 3). The filtrate was collected and concentrated to give a crude product of compound 1-2 (9.2 g, yellow oil), which could be directly used without further purification.

LCMS (ESI) M/Z: 400.2 [M+Na⁺].

**Step B:** Compound **1-2** (9.2 g, crude product) was added to a 250 mL single-necked flask, and dichloromethane (50 mL) and a solution of HCl in 1,4-dioxane (30.5 mL, 122 mmol, 4 M) were added sequentially. The reaction system was reacted at room temperature for 3 h under a nitrogen atmosphere. After completion of the reaction as monitored by LCMS, the reaction liquid was concentrated, and the resulting residue was redissolved in dichloromethane (15 mL), followed by the addition of MTBE (150 mL). The mixture was stirred at 0°C for 0.5 h, and a white solid precipitated. The mixture was filtered, and the filter cake was washed with MTBE:dichloromethane = 10:1 (10 mL × 3) to give compound 1-3 (6.6 g, yield: 86.4%).

LCMS (ESI) M/Z: 278.2 [M+H⁺].

**Step C:** Compound **1-3** (5 g, 11.64 mmol) was added to a 25 mL single-necked flask, and dry N,N-dimethylformamide (15 mL) was added. Compound **1-4** (3.94 g, 12.57 mmol), HATU (7.08 g, 18.62 mmol), and DIEA (5.79 mL, 34.92 mmol) were then added sequentially, and the reaction system was reacted at room temperature for 2 h under a nitrogen atmosphere. After the reaction was completed, the mixture was filtered, and the filtrate was purified by Flash (acetonitrile/0.1% formic acid/water) to give compound 1-5 (6.2 g, yield: 77.4%).

LCMS (ESI) M/Z: 689.5 [M+H⁺].

**Step D:** Compound 1-5 (6.2 g, 9.0 mmol) was added to a 500 mL single-necked flask, and methanol (240 mL) and Pd/C (1.8 g, 10% Pd) were added sequentially. The reaction system was reacted at 70°C for 12 h under a hydrogen atmosphere. After the reaction was completed, the mixture was filtered, and the filtrate was concentrated to give compound **1-6** (5.3 g, yield: 98.48%).

LCMS (ESI) M/Z: 599.9 [M+H⁺].

¹H NMR (400 MHz, DMSO) δ 8.13-7.98 (m, 1H), 4.64 (s, 1H), 4.59-4.45 (m, 1H), 4.06-3.90 (m, 2H), 3.83 (dd, *J* = 7.1, 3.4 Hz, 1H), 3.68-3.45 (m, 2H), 3.40-3.24 (m, 4H), 3.18 (d, *J* = 7.0 Hz, 3H), 3.09-2.94 (m, 3H), 2.68-2.61 (m, 1H), 2.48-2.39 (m, 1H), 2.39-2.27 (m, 2H), 2.20 (d, *J* = 2.4 Hz, 6H), 2.00-1.85 (m, 4H), 1.84-1.63 (m, 3H), 1.37-1.24 (m, 1H), 1.21-1.07 (m, 3H), 0.94-0.85 (m, 12H), 0.79-0.68 (m, 6H).

**Step E:** Tetrahydrofuran (10 mL) was added to a dry reaction flask, followed by the addition of compound **1-7** (2.00 g, 6.45 mmol). The mixture was stirred uniformly. Subsequently, borane tetrahydrofuran complex (2.77 g, 32.25 mmol) was added. The reaction liquid was reacted under stirring at room temperature. After 6 h, the sample was taken for detection and analyzed by LCMS, and the results showed that the starting material spot disappeared, and a target product ion peak was generated. Detection confirmed the completion of the reaction. The reaction liquid was concentrated and then separated by column chromatography (dichloromethane/methanol = 95/5 to 90/10) to give the product **1-8** (900 mg, yield: 71.17%).

LCMS (ESI) M/Z: 197.2 [M+H⁺].

**Step F:** Dichloromethane (5 mL) was added to a dry reaction flask, followed by the addition of compound **1-8** (40 mg, 0.20 mmol) and N,N-diisopropylethylamine (77.54 mg, 0.60 mmol). The mixture was stirred uniformly. Subsequently, compound **1-6** (100 mg, 0.17 mmol) and HATU (114.07 mg, 0.30 mmol) were added. The reaction liquid was reacted under stirring at room temperature. After 4 h, the sample was taken for detection and analyzed by LCMS, and the results showed that the starting material spot disappeared, and a target product ion peak was generated. Detection confirmed the completion of the reaction. The reaction liquid was concentrated and then separated by column chromatography (dichloromethane/methanol = 99/1 to 90/10) to give the product **1-9** (130 mg, yield: 82.07%).

LCMS (ESI) M/Z: 777.5 [M+H⁺].

**Step G:** Tetrahydrofuran (5 mL) was added to a dry reaction flask, followed by the addition of compound **1-9** (60 mg, 0.077 mmol). The mixture was stirred uniformly. Subsequently, thionyl chloride (45.97 mg, 0.39 mmol) was added. The mixture was heated to 80°C in an oil bath and stirred at reflux. After 2 h, the sample was taken for detection and analyzed by LCMS, and the results showed that the starting material spot disappeared, and a target product ion peak was generated. Detection confirmed the completion of the reaction. The reaction liquid was concentrated and then separated by column chromatography (dichloromethane/methanol = 99/1 to 90/10) to give the product **1-10** (40 mg, yield: 65.12%).

LCMS (ESI) M/Z: 795.4 [M+H⁺].

**Step H:** N,N-Dimethylformamide (2 mL) was added to a dry reaction flask, followed by the addition of compound **1-10** (20 mg, 0.025 mmol). The mixture was stirred uniformly. Subsequently, 4,4'-bipyridine (1.95 mg, 0.013 mmol) was added, and after 5 min of stirring, diboronic acid (11.21 mg, 0.13 mmol) was added. The reaction liquid was reacted under stirring at room temperature. After 15 min, the sample was taken for detection and analyzed by LCMS, and the results showed that the starting material spot disappeared, and a target product ion peak was generated. Detection confirmed the completion of the reaction. The reaction liquid was purified by preparative high performance liquid chromatography to give the target compound **1** (3.5 mg, yield: 18.19%).

LCMS (ESI) M/Z: 765.4 [M+H⁺].

¹H NMR (400 MHz, DMSO-*d*₆) δ 7.04-6.93 (m, 2H), 6.64-6.56 (m, 2H), 6.51 (d, *J* = 9.2 Hz, 4H), 4.23-4.14 (m, 1H), 4.10-4.03 (m, 1H), 3.67-3.59 (m, 3H), 3.57-3.51 (m, 1H), 3.44-3.41 (m, 1H), 3.38-3.35 (m, 2H), 3.34 (s, 3H), 3.14 (s, 3H), 2.96-2.92 (m, 2H), 2.86 (d, *J =* 7.9 Hz, 1H), 2.80 (d, *J =* 7.6 Hz, 1H), 2.74-2.65 (m, 3H), 2.53 (d, *J* = 4.2 Hz, 2H), 2.42 (s, 6H), 2.12-2.08 (m, 2H), 1.81-1.77 (m, 2H), 1.45-1.39 (m, 2H), 1.22-1.19 (m, 3H), 1.17-1.13 (m, 3H), 1.04-1.02 (m, 3H), 1.01-1.01 (m, 3H), 1.00-0.99 (m, 3H), 0.91-0.89 (m, 3H), 0.88-0.87 (m, 3H).

### Example 2:

### Preparation of (S)-N-((3R,4S,5S)-1-((S)-2-((1R,2R)-3-(((S)-1-(3-aminophenyl)-but-3-yn-2-yl)amino)-1-methoxy-2-methyl-3-oxopropyl)pyrrolidin-1-yl)-3-methoxy-5-methyl-1-oxoheptan-4-yl)-2-((S)-2-(dimethylamino)-3-methylbutanamido)-N,3-dimethylbutanamide.

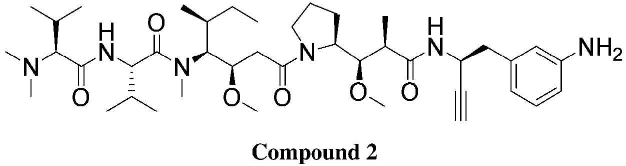

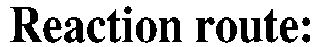

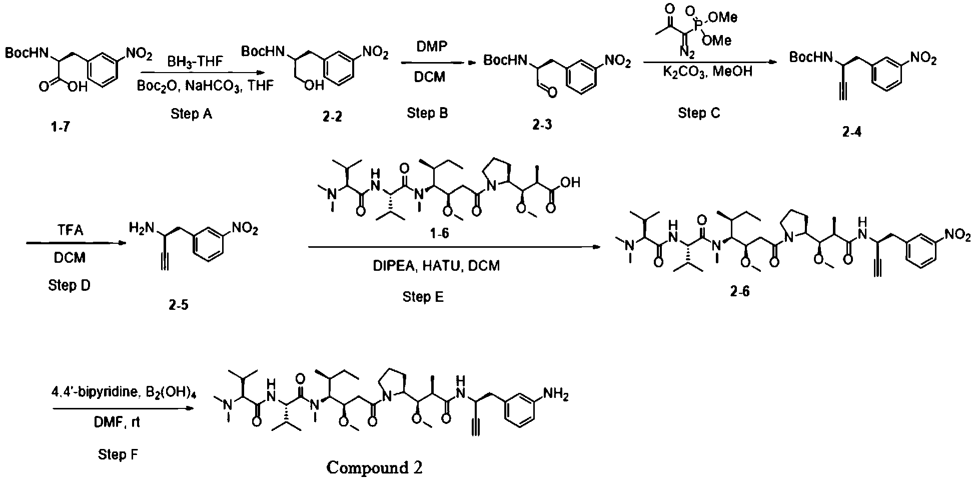

### Procedures:

**Step A:** Tetrahydrofuran (10 mL) was added to a dry reaction flask, followed by the addition of compound **1-7** (1.00 g, 3.22 mmol). The mixture was stirred uniformly. Subsequently, borane tetrahydrofuran complex (830.18 mg, 9.66 mmol) was added. The reaction liquid was reacted under stirring at room temperature. After 6 h, the sample was taken for detection and analyzed by LCMS, and the results showed that the starting material spot disappeared. BOC anhydride (763.88 mg, 3.51 mmol) and sodium bicarbonate (294.88 mg, 3.51 mmol) were added to the reaction liquid, and the reaction was continued under stirring overnight. The sample was taken for monitoring and analyzed by LCMS, and the results showed that a target product ion peak was generated. Detection confirmed the completion of the reaction. The reaction liquid was concentrated and then separated by column chromatography (dichloromethane/methanol = 95/5 to 90/10) to give the product 2-2 (900 mg, yield: 71.17%).

LCMS (ESI) M/Z: 197.2 [M+H⁺-Boc].

**Step B:** Dichloromethane (15 mL) was added to a dry reaction flask, followed by the addition of compound **2-2** (200 mg, 0.67 mmol). The mixture was stirred uniformly. Subsequently, Dess-Martin periodinane (568.35 mg, 1.34 mmol) was added. The reaction liquid was reacted under stirring at room temperature. After 4 h, the sample was taken for detection and analyzed by LCMS, and the results showed that the starting material spot disappeared, and a target product ion peak was generated. Detection confirmed the completion of the reaction. The reaction liquid was concentrated and then separated by preparative column chromatography (dichloromethane/methanol = 99/1 to 98/2) to give the product **2-3** (150 mg, yield: 75.51%).

LCMS (ESI) M/Z: 195.1 [M+H⁺-BOC].

**Step C:** Methanol (5 mL) was added to a dry reaction flask, followed by the addition of compound **2-3** (150 mg, 0.51 mmol). The mixture was stirred uniformly. Subsequently, potassium carbonate (211.46 mg, 1.53 mmol) and dimethyl (1-diazo-2-oxopropyl)phosphonate (293.93 mg, 1.53 mmol) were added. The reaction liquid was reacted under stirring at room temperature. After 4 h, the sample was taken for detection and analyzed by LCMS, and the results showed that the starting material spot disappeared, and a target product ion peak was generated. Detection confirmed the completion of the reaction. The reaction liquid was concentrated and then separated by preparative column chromatography (dichloromethane/methanol = 99/1 to 90/10) to give the product **2-4** (100 mg, yield: 67.58%).

LCMS (ESI) M/Z: 191.1 [M+H⁺-Boc].

**Step D:** Dichloromethane (4 mL) was added to a dry reaction flask, followed by the addition of compound **2-4** (100 mg, 0.34 mmol). The mixture was stirred uniformly. Subsequently, trifluoroacetic acid (1 mL) was added. The reaction liquid was reacted under stirring at room temperature. After 4 h, the sample was taken for detection and analyzed by LCMS, and the results showed that the starting material spot disappeared, and a target product ion peak was generated. Detection confirmed the completion of the reaction. The mixture was filtered and concentrated to give the crude product **2-5** (60 mg, yield: 91.58%), which was directly used in the next step without further purification.

LCMS (ESI) M/Z: 191.1 [M+H⁺].

**Step E:** Dichloromethane (5 mL) was added to a dry reaction flask, followed by the addition of compound **2-5** (40 mg, 0.21 mmol) and N,N-diisopropylethylamine (81.42 mg, 0.63 mmol). The mixture was stirred uniformly. Subsequently, compound **1-6** (100 mg, 0.17 mmol) and HATU (119.77 mg, 0.32 mmol) were added. The reaction liquid was reacted under stirring at room temperature. After 4 h, the sample was taken for detection and analyzed by LCMS, and the results showed that the starting material spot disappeared, and a target product ion peak was generated. Detection confirmed the completion of the reaction. The reaction liquid was concentrated and then separated by preparative column chromatography (dichloromethane/methanol = 99/1 to 90/10) to give the product **2-6** (40 mg, yield: 24.67%).

LCMS (ESI) M/Z: 771.5 [M+H⁺].

**Step F:** N,N-Dimethylformamide (1 mL) was added to a dry reaction flask, followed by the addition of **2-6** (130 mg, 0.17 mmol). The mixture was stirred uniformly. Subsequently, 4,4'-bipyridine (0.013 g, 0.085 mmol) and tetrahydroxydiboron (76.20 mg, 0.85 mmol) were added. The reaction liquid was reacted under stirring at room temperature. After 4 h, the sample was taken for detection and analyzed by LCMS, and the results showed that the starting material spot disappeared, and a target product ion peak was generated. Detection confirmed the completion of the reaction. The reaction liquid was purified by preparative high performance liquid chromatography to give the product compound **2** (20 mg, yield: 16.01%).

LCMS (ESI) M/Z: 741.5 [M+H⁺].

¹H NMR (400 MHz, DMSO-*d*₆) δ 8.49 (d, *J* = 5.5 Hz, 1H), 8.29 (dd, *J* = 12.9, 8.3 Hz, 1H), 8.01 (t, *J* = 9.2 Hz, 2H), 6.96-6.84 (m, 2H), 6.42 (d, *J* = 5.0 Hz, 2H), 6.39-6.33 (m, 3H), 4.95-4.89 (m, 1H), 4.67-4.63 (m, 1H), 4.52 (t, *J* = 8.8 Hz, 1H), 4.05-3.94 (m, 2H), 3.83-3.80 (m, 1H), 3.27 (s, 3H), 3.18 (s, 3H), 3.16-3.15 (m, 2H), 2.99 (s, 3H), 2.66-2.63 (m, 1H), 2.63-2.60 (m, 1H), 2.21 (s, 6H), 2.00-1.87 (m, 7H), 1.76-1.70 (m, 2H), 1.63-1.57 (m, 1H), 1.49-1.43 (m, 1H), 1.10-1.04 (m, 3H), 1.01 (t, *J=* 6.1 Hz, 3H), 0.91-0.90 (m, 3H), 0.89-0.88 (m, 3H), 0.88-0.87 (m, 3H), 0.75-0.73 (m, 3H), 0.72-0.69 (m, 3H).

### Example 3:

### Preparation of (S)-N-((3R,4S,5S)-1-((S)-2-((1R,2R)-3-(((S)-1-amino-3-(3-aminophenyl)-1-oxopropan-2-yl)amino)-1-methoxy-2-methyl-3-oxopropyl)pyrrolidin-1-yl)-3-methoxy-5-methyl-1-oxoheptan-4-yl)-2-((S)-2-(dimethylamino)-3-methylbutanamido)-N,3-dimethylbutanamide.

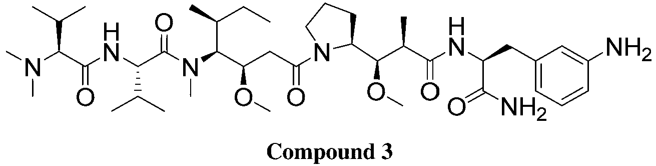

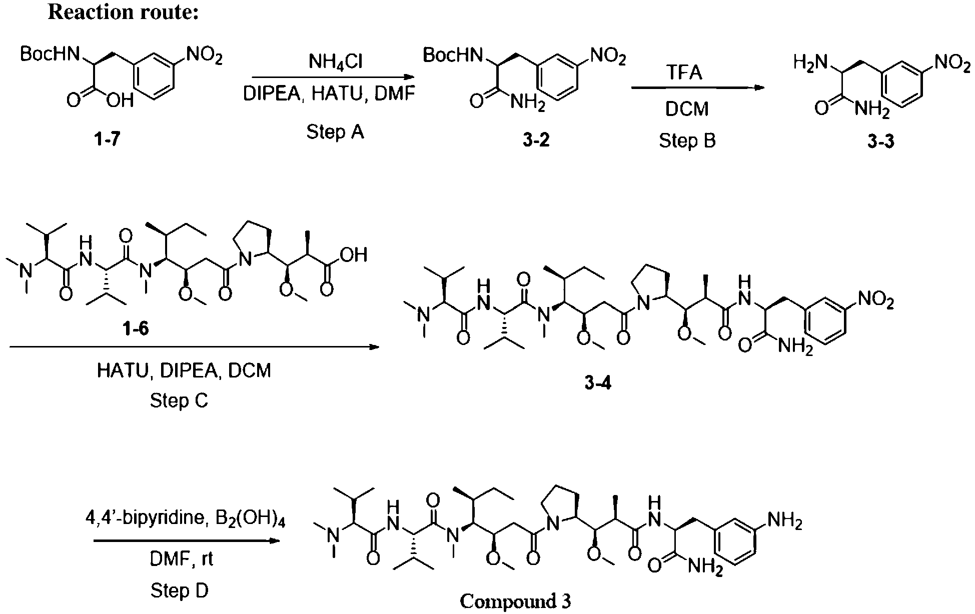

### Procedures:

**Step A:** N,N-Dimethylformamide (4 mL) was added to a dry reaction flask, followed by the addition of compound 1-7 (300 mg, 0.97 mmol), N,N-diisopropylethylamine (376.09 mg, 2.91 mmol), and HATU (553.23 mg, 1.46 mmol). The mixture was stirred uniformly. Subsequently, ammonium chloride (103.77 mg, 1.94 mmol) was added. The reaction liquid was reacted under stirring at room temperature. After that, the sample was taken for detection and analyzed by LCMS, and the results showed that the starting material spot disappeared, and a target product ion peak was generated. Detection confirmed the completion of the reaction. The reaction liquid was diluted with dichloromethane (20 mL), and liquid-liquid extraction was performed with water (30 mL). The oil layer was dried, concentrated, and then separated by preparative column chromatography (dichloromethane/methanol = 99/1 to 90/10) to give the product **3-2** (280 mg, yield: 93.63%).

LCMS (ESI) M/Z: 210.1 [M+H⁺-BOC].

**Step B:** Dichloromethane (4 mL) was added to a dry reaction flask, followed by the addition of compound **3-2** (280 mg, 0.91 mmol). The mixture was stirred uniformly. Subsequently, trifluoroacetic acid (1 mL) was added. The reaction liquid was reacted under stirring at room temperature. After 4 h, the sample was taken for detection and analyzed by LCMS, and the results showed that the starting material spot disappeared, and a target product ion peak was generated. Detection confirmed the completion of the reaction. The mixture was filtered and concentrated to give a crude product of compound **3-3** (200 mg, yield: 105.61%), which was directly used in the next step without further purification.

LCMS (ESI) M/Z: 210.1 [M+H⁺].

**Step C:** Dichloromethane (5 mL) was added to a dry reaction flask, followed by the addition of compound **3-3** (25 mg, 0.12 mmol) and N,N-diisopropylethylamine (46.53 mg, 0.36 mmol). The mixture was stirred uniformly. Subsequently, compound **1-6** (70 mg, 0.12 mmol) and HATU (68.44 mg, 0.18 mmol) were added. The reaction liquid was reacted under stirring at room temperature. After 4 h, the sample was taken for detection and analyzed by LCMS, and the results showed that the starting material spot disappeared, and a target product ion peak was generated. Detection confirmed the completion of the reaction. The reaction liquid was concentrated and then separated by preparative column chromatography (dichloromethane/methanol = 99/1 to 90/10) to give the product **3-4** (70 mg, yield: 74.15%).

LCMS (ESI) M/Z: 790.5 [M+H⁺].

**Step D:** N,N-Dimethylformamide (1 mL) was added to a dry reaction flask, followed by the addition of **3-4** (35 mg, 0.044 mmol). The mixture was stirred uniformly. Subsequently, 4,4'-bipyridine (3.44 mg, 0.022 mmol) and tetrahydroxydiboron (19.72 mg, 0.22 mmol) were added. The reaction liquid was reacted under stirring at room temperature. After 15 min, the sample was taken for detection and analyzed by LCMS, and the results showed that the starting material spot disappeared, and a target product ion peak was generated. Detection confirmed the completion of the reaction. The reaction liquid was purified by preparative high performance liquid chromatography to give the product compound **3** (1.5 mg, yield: 4.45%).

LCMS (ESI) M/Z: 760.5 [M+H⁺].

¹H NMR (400 MHz, Methanol-*d*₄) δ 7.04-6.94 (m, 1H), 6.66-6.47 (m, 3H), 4.67-4.63 (m, 1H), 4.61-4.55 (m, 1H), 4.19-4.03 (m, 1H), 3.88 (d, *J* = 1.8 Hz, 1H), 3.79-3.66 (m, 1H), 3.57-3.47 (m, 1H), 3.45-3.37 (m, 2H), 3.36-3.32 (m, 6H), 3.28-3.25 (m, 2H), 3.20-3.16 (m, 1H), 3.19-3.05 (m, 3H), 2.82-2.70 (m, 2H), 2.53-2.45 (m, 2H), 2.39-2.34 (m, 6H), 2.15-1.97 (m, 3H), 1.96-1.82 (m, 2H), 1.82-1.69 (m, 2H), 1.68-1.56 (m, 1H), 1.20 (d, *J* = 6.8 Hz, 1H), 1.09 (t, *J* = 6.2 Hz, 3H), 1.06-1.01 (m, 6H), 1.00-0.95 (m, 6H), 0.91-0.87 (m, 3H), 0.87-0.82 (m, 3H).

### Example 4

### Preparation of (S)-N-((3R,4S,5S)-1-((S)-2-((1R,2R)-3-(((S)-3-(3-aminophenyl)-1-(methylamino)-1-oxopropan-2-yl)amino)-1-methoxy-2-methyl-3-oxopropyl)pyrrolidin-1-yl)-3-methoxy-5-methyl-1-oxoheptan-4-yl)-2-((S)-2-(dimethylamino)-3-methylbutanamido)-N,3-dimethylbutanamide.

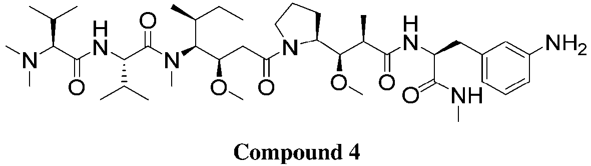

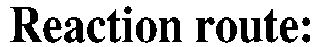

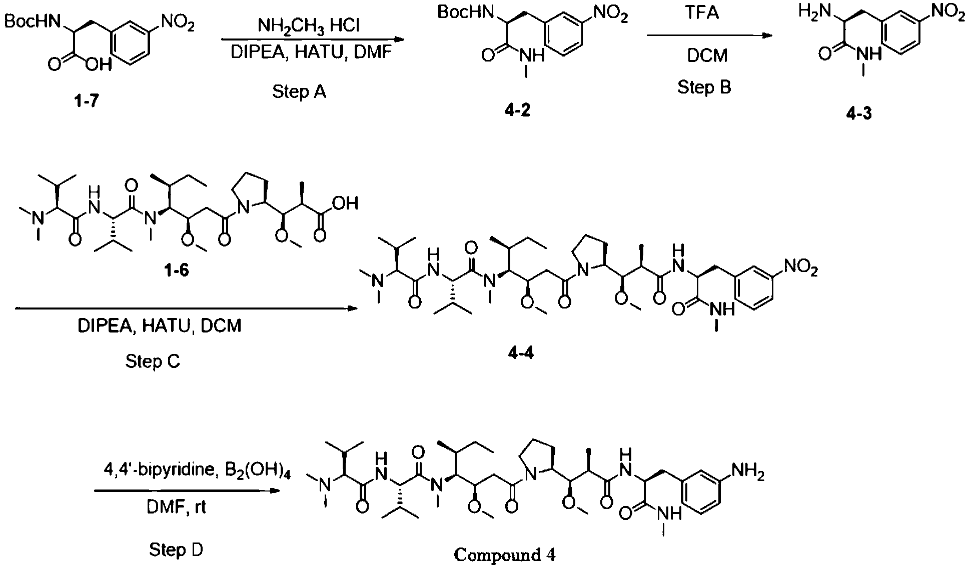

### Procedures:

**Step A:** N,N-Dimethylformamide (4 mL) was added to a dry reaction flask, followed by the addition of compound **1-7** (300 mg, 0.97 mmol), N,N-diisopropylethylamine (376.09 mg, 2.91 mmol), and HATU (553.23 mg, 1.46 mmol). The mixture was stirred uniformly. Subsequently, methylamine hydrochloride (327.47 mg, 4.85 mmol) was added. The reaction liquid was reacted under stirring at room temperature. After that, the sample was taken for detection and analyzed by LCMS, and the results showed that the starting material spot disappeared, and a target product ion peak was generated. Detection confirmed the completion of the reaction. The reaction liquid was diluted with dichloromethane (20 mL), and liquid-liquid extraction was performed with water (30 mL). The oil layer was dried, concentrated, and then separated by preparative column chromatography (dichloromethane/methanol = 99/1 to 90/10) to give the product **4-2** (250 mg, yield: 79.97%).

LCMS (ESI) M/Z: 224.1 [M+H⁺-BOC].

**Step B:** Dichloromethane (4 mL) was added to a dry reaction flask, followed by the addition of compound **4-2** (250 mg, 0.77 mmol). The mixture was stirred uniformly. Subsequently, trifluoroacetic acid (1 mL) was added. The reaction liquid was reacted under stirring at room temperature. After 4 h, the sample was taken for detection and analyzed by LCMS, and the results showed that the starting material spot disappeared, and a target product ion peak was generated. Detection confirmed the completion of the reaction. The mixture was filtered and concentrated to give the crude product **4-3** (230 mg, yield: 133.26%), which was directly used in the next step without further purification.

LCMS (ESI) M/Z: 224.1 [M+H⁺].

**Step C:** Dichloromethane (2 mL) was added to a dry reaction flask, followed by the addition of compound **4-3** (25 mg, 0.12 mmol) and N,N-diisopropylethylamine (42.65 mg, 0.33 mmol). The mixture was stirred uniformly. Subsequently, compound **1-6** (70 mg, 0.12 mmol) and HATU (62.74 mg, 0.17 mmol) were added. The reaction liquid was reacted under stirring at room temperature. After 4 h, the sample was taken for detection and analyzed by LCMS, and the results showed that the starting material spot disappeared, and a target product ion peak was generated. Detection confirmed the completion of the reaction. The reaction liquid was concentrated and then separated by preparative column chromatography (dichloromethane/methanol = 99/1 to 90/10) to give the product **4-4** (18 mg, yield: 19.99%).

LCMS (ESI) M/Z: 804.5 [M+H⁺].

**Step D:** Acetonitrile (1 mL) was added to a dry reaction flask, followed by the addition of compound **4-4** (18.49 mg, 0.023 mmol). The mixture was stirred uniformly. Subsequently, 4,4'-bipyridine (1.80 mg, 0.011 mmol) and tetrahydroxydiboron (10.31 mg, 0.11 mmol) were added. The reaction liquid was reacted under stirring at room temperature. After 15 min, the sample was taken for detection and analyzed by LCMS, and the results showed that the starting material spot disappeared, and a target product ion peak was generated. Detection confirmed the completion of the reaction. The reaction liquid was purified by preparative high performance liquid chromatography to give the product compound **4** (5.1 mg, yield: 28.65%).

LCMS (ESI) M/Z: 774.5 [M+H⁺].

¹H NMR (400 MHz, DMSO-*d*₆) δ 8.18 (d, *J =* 8.7 Hz, 0H), 8.05 (t, *J =* 8.2 Hz, 1H), 7.99 (d, *J =* 8.4 Hz, 1H), 7.87-7.74 (m, 1H), 6.83 (t, *J* = 7.7 Hz, 1H), 6.44-6.28 (m, 3H), 4.73-4.58 (m, 2H), 4.53 (t, *J =* 8.9 Hz, 1H), 4.39 (ddd, *J* = 10.5, 8.4, 4.4 Hz, 1H), 4.02-3.96 (m, 1H), 3.84 (dd, *J* = 9.8, 2.1 Hz, 1H), 3.67-3.62 (m, 3H), 3.37-3.33 (m, 1H), 3.33-3.27 (m, 2H), 3.25 (d, *J* = 5.5 Hz, 3H), 3.19 (d, *J =* 2.8 Hz, 3H), 3.10 (s, 1H), 3.02-2.96 (m, 2H), 2.93-2.86 (m, 1H), 2.72-2.61 (m, 2H), 2.60 (dd, *J* = 4.6, 1.3 Hz, 3H), 2.47-2.36 (m, 1H), 2.34-2.26 (m, 1H), 2.20 (d, *J* = 7.7 Hz, 6H), 2.18-2.12 (m, 1H), 2.05-1.84 (m, 3H), 1.78-1.66 (m, 2H), 1.65-1.42 (m, 2H), 1.02 (d, *J* = 6.7 Hz, 3H), 0.95-0.90 (m, 6H), 0.89-0.85 (m, 6H), 0.80-0.74 (m, 3H), 0.72 (d, *J* = 6.6 Hz, 3H).

### Example 5

### Preparation of (S)-N-((3R,4S,5S)-1-((S)-2-((1R,2R)-3-(allyl(3-aminophenethyl)amino)-1-methoxy-2-methyl-3-oxopropyl)pyrrolidin-1-yl)-3-methoxy-5-methyl-1-oxoheptan-4-yl)-2-((S)-2-(dimethylamino)-3-methylbutanamido)-N,3-dimethylbutanamide.

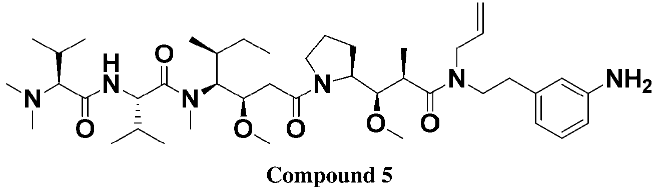

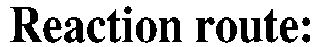

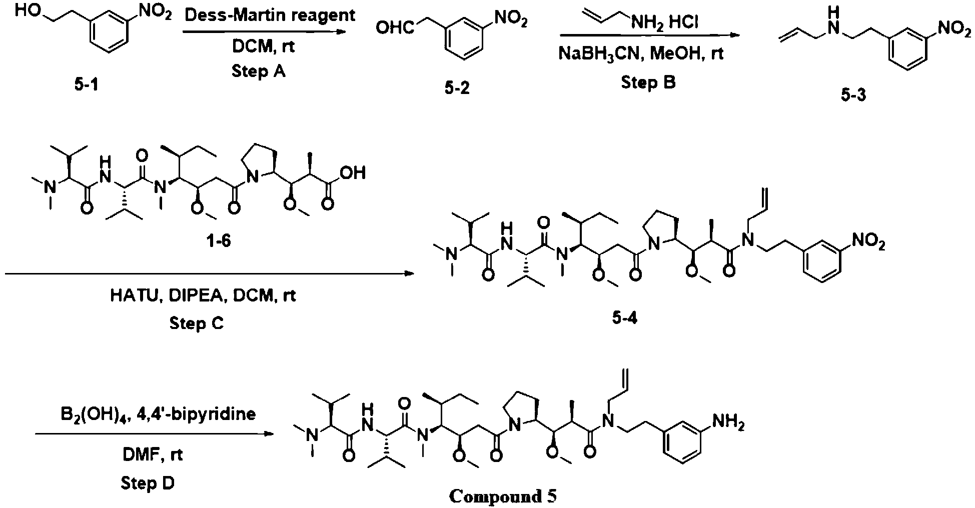

### Procedures:

**Step A:** Dichloromethane (25 mL) was added to a dry reaction flask, followed by the addition of compound **5-1** (1 g, 5.98 mmol). The mixture was stirred uniformly. Subsequently, Dess-Martin periodinane (3.80 g, 8.97 mmol) was added. The reaction liquid was reacted under stirring at room temperature. After 3 h, the sample was taken for detection and analyzed by TLC, and the results showed that the starting material spot disappeared, and a new spot was generated. Detection confirmed the completion of the reaction. The reaction liquid was concentrated and then separated by preparative column chromatography (petroleum ether/ethyl acetate = 95/5 to 80/20) to give the product compound 5-2 (500 mg, yield: 50.61%).

¹H NMR (400 MHz, Chloroform-d) δ 9.84 (t, *J* = 1.7 Hz, 1H), 8.18 (tt, *J* = 5.2, 2.4 Hz, 1H), 8.11 (t, *J* = 1.6 Hz, 1H), 7.58-7.51 (m, 2H), 3.88 (d, *J* = 1.6 Hz, 2H).

**Step B:** Methanol (5 mL) was added to a dry reaction flask, followed by the addition of compound **5-2** (100 mg, 0.61 mmol) and 2-propen-1-amine hydrochloride (100 mg, 1.07 mmol). The mixture was stirred uniformly. After 0.5 h, sodium cyanoborohydride (95.83 mg, 1.52 mmol) was added. The reaction liquid was reacted under stirring at room temperature. After 4 h, the sample was taken for detection and analyzed by LCMS, and the results showed that the starting material spot disappeared, and a target product ion peak was generated. Detection confirmed the completion of the reaction. The reaction liquid was concentrated and then separated by preparative column chromatography (dichloromethane/methanol = 99/1 to 90/10) to give the product **5-3** (46 mg, yield: 36.84%).

LCMS (ESI) M/Z: 207.1 [M+H⁺].

**Step C:** Dichloromethane (3 mL) was added to a dry reaction flask, followed by the addition of compound **1-6** (130 mg, 0.22 mmol), N,N-diisopropylethylamine (85.30 mg, 0.66 mmol), and HATU (125.48 mg, 0.33 mmol). The mixture was stirred uniformly. Subsequently, compound **5-3** (46 mg, 0.22 mmol) was added. The reaction liquid was reacted under stirring at room temperature. After 6 h, the sample was taken for detection and analyzed by LCMS, and the results showed that the starting material spot disappeared, and a target product ion peak was generated. Detection confirmed the completion of the reaction. The reaction liquid was concentrated and then separated by preparative column chromatography (dichloromethane/methanol = 99/1 to 90/10) to give the product 5-4 (54 mg, yield: 30.76%).

LCMS (ESI) M/Z: 787.5 [M+H⁺].

**Step D:** N,N-Dimethylformamide (1 mL) was added to a dry reaction flask, followed by the addition of compound **5-4.** The mixture was stirred uniformly. Subsequently, tetrahydroxydiboron (30.93 mg, 0.35 mmol) and 4,4'-bipyridine (5.39 mg, 0.035 mmol) were added. The reaction liquid was reacted under stirring at room temperature. After 15 min, the sample was taken for detection and analyzed by LCMS, and the results showed that the starting material spot disappeared, and a target product ion peak was generated. Detection confirmed the completion of the reaction. The reaction liquid was purified by preparative high performance liquid chromatography to give the product compound 5 (7.5 mg, yield: 14.44%).

LCMS (ESI) M/Z: 757.5 [M+H⁺].

¹H NMR (400 MHz, Methanol-*d*₄) δ 7.09-6.93 (m, 1H), 6.65-6.49 (m, 3H), 5.88-5.72 (m, 1H), 5.24-5.17 (m, 1H), 5.17-5.09 (m, 1H), 4.73-4.69 (m, 1H), 4.65 (dd, *J* = 8.5, 1.9 Hz, 1H), 4.21-4.01 (m, 2H), 4.00-3.90 (m, 2H), 3.89-3.77 (m, 1H), 3.71-3.60 (m, 2H), 3.55 (t, *J* = 7.3 Hz, 1H), 3.51-3.45 (m, 1H), 3.41 (s, 3H), 3.39-3.36 (m, 1H), 3.34 (s, 3H), 3.29 (s, 3H), 3.28-3.21 (m, 1H), 3.16-3.08 (m, 2H), 2.91-2.83 (m, 1H), 2.81-2.70 (m, 3H), 2.68-2.57 (m, 1H), 2.57-2.48 (m, 1H), 2.44 (s, 6H), 2.21-2.11 (m, 1H), 2.10-1.99 (m, 3H), 1.97-1.90 (m, 1H), 1.84-1.72 (m, 2H), 1.48-1.37 (m, 1H), 1.24-1.13 (m, 3H), 1.06-1.01 (m, 6H), 1.01-0.97 (m, 6H), 0.91 (d, *J* = 6.4 Hz, 3H), 0.87 (d, *J* = 6.4 Hz, 3H).

### Example 6

### Preparation of (S)-N-((3R,4S,5S)-1-((S)-2-((1R,2R)-3-((3-aminophenethyl)(prop-2-yn-1-yl)amino)-1-methoxy-2-methyl-3-oxopropyl)pyrrolidin-1-yl)-3-methoxy-5-methyl-1-oxoheptan-4-yl)-2-((S)-2-(dimethylamino)-3-methylbutanamido)-N,3-dimethylbutanamide.

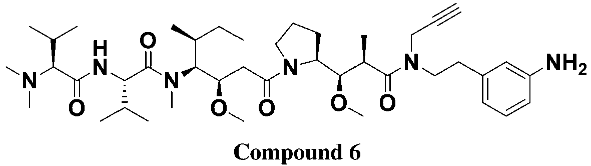

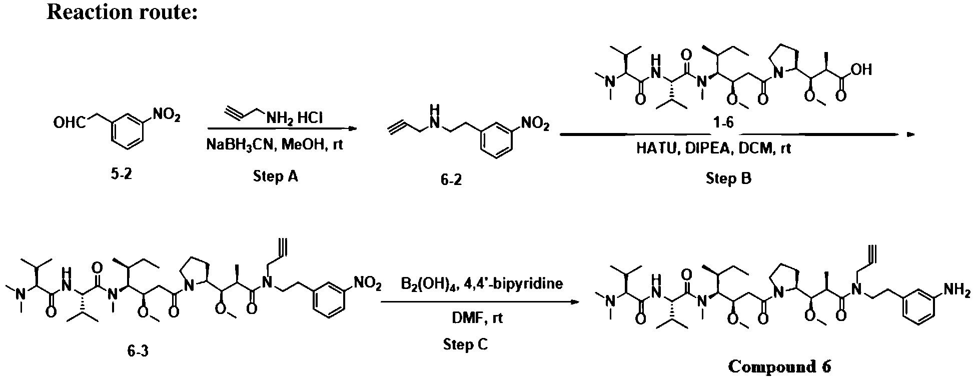

### Procedures:

**Step A:** Methanol (5 mL) was added to a dry reaction flask, followed by the addition of compound **5-2** (100 mg, 0.61 mmol) and propargylamine hydrochloride (111.68 mg, 1.22 mmol). The mixture was stirred uniformly. After 0.5 h, sodium cyanoborohydride (95.83 mg, 1.52 mmol) was added. The reaction liquid was reacted under stirring at room temperature. After 4 h, the sample was taken for detection and analyzed by LCMS, and the results showed that the starting material spot disappeared, and a target product ion peak was generated. Detection confirmed the completion of the reaction. The reaction liquid was concentrated and then separated by preparative column chromatography (dichloromethane/methanol = 99/1 to 90/10) to give the product compound **6-2** (59 mg, yield: 47.71%).

LCMS (ESI) M/Z: 205.1 [M+H⁺].

**Step B:** Dichloromethane (3 mL) was added to a dry reaction flask, followed by the addition of compound **1-6** (76 mg, 0.13 mmol), N,N-diisopropylethylamine (54.28 mg, 0.42 mmol), and HATU (79.85 mg, 0.21 mmol). The mixture was stirred uniformly. Subsequently, compound **6-2** (29 mg, 0.14 mmol) was added. The reaction liquid was reacted under stirring at room temperature. After 6 h, the sample was taken for detection and analyzed by LCMS, and the results showed that the starting material spot disappeared, and a target product ion peak was generated. Detection confirmed the completion of the reaction. The reaction liquid was concentrated and then separated by preparative column chromatography (dichloromethane/methanol = 99/1 to 90/10) to give the product **6-3** (48 mg, yield: 43.06%).

LCMS (ESI) M/Z: 785.5 [M+H⁺].

**Step C:** N,N-Dimethylformamide (1 mL) was added to a dry reaction flask, followed by the addition of compound **6-3** (54 mg, 0.069 mmol). The mixture was stirred uniformly. Subsequently, tetrahydroxydiboron (27.34 mg, 0.30 mmol) and 4,4'-bipyridine (4.76 mg, 0.030 mmol) were added. The reaction liquid was reacted under stirring at room temperature. After 15 min, the sample was taken for detection and analyzed by LCMS, and the results showed that the starting material spot disappeared, and a target product ion peak was generated. Detection confirmed the completion of the reaction. The reaction liquid was purified by preparative high performance liquid chromatography to give the product compound **6** (6.04 mg, yield: 11.52%).

LCMS (ESI) M/Z: 755.5 [M+H⁺].

¹H NMR (400 MHz, Methanol-*d*₄) δ 7.07-6.97 (m, 1H), 6.64-6.49 (m, 3H), 4.74-4.69 (m, 1H), 4.66-4.62 (m, 1H), 4.25-4.12 (m, 1H), 4.11-4.04 (m, 2H), 3.92 (t, *J* = 8.0 Hz, 1H), 3.69 (s, 2H), 3.65-3.56 (m, 1H), 3.43-3.39 (m, 3H), 3.37-3.34 (m, 1H), 3.32 (s, 3H), 3.29 (d, *J =* 2.6 Hz, 3H), 3.26 (s, 1H), 3.14-3.09 (m, 3H), 2.96-2.90 (m, 1H), 2.84-2.81 (m, 2H), 2.80-2.78 (m, 1H), 2.78-2.75 (m, 1H), 2.72-2.61 (m, 2H), 2.51-2.45 (m, 1H), 2.38 (s, 6H), 2.14-2.10 (m, 1H), 2.06-2.00 (m, 3H), 1.84-1.71 (m, 3H), 1.44-1.38 (m, 1H), 1.27-1.14 (m, 3H), 1.02 (d, *J* = 2.3 Hz, 3H), 1.01 (d, *J* = 1.9 Hz, 3H), 0.99-0.99 (m, 3H), 0.97 (d, *J*= 2.5 Hz, 3H), 0.89-0.87 (m, 3H), 0.86 (d, *J =* 1.8 Hz, 3H).

### Example 7

### Preparation of (S)-N-((3R,4S,5S)-1-((S)-2-((1R,2R)-3-((3-aminophenethyl)(methoxy)amino)-1-methoxy-2-methyl-3-oxopropyl)pyrrolidin-1-yl)-3-methoxy-5-methyl-1-oxoheptan-4-yl)-2-((S)-2-(dimethylamino)-3-methylbutanamido)-N,3-dimethylbutanamide.

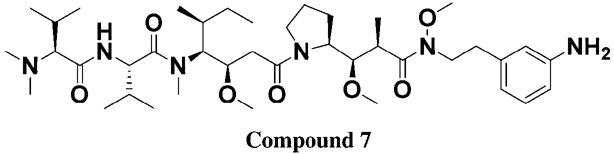

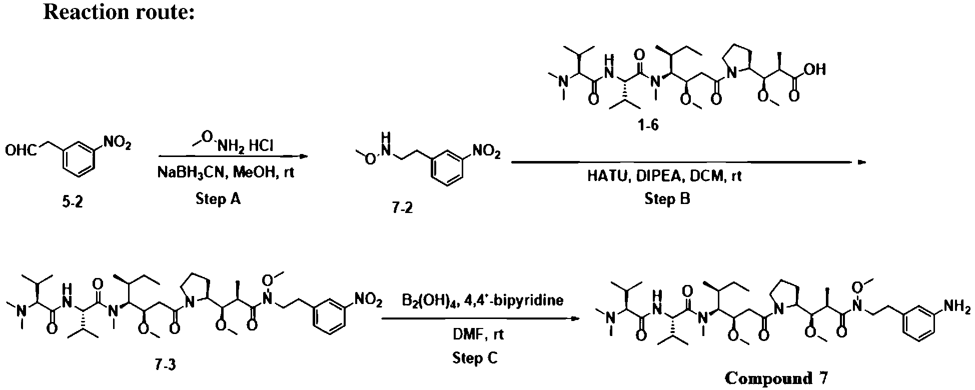

### Procedures:

**Step A:** Methanol (5 mL) was added to a dry reaction flask, followed by the addition of compound **5-2** (100 mg, 0.61 mmol) and methoxyamine hydrochloride (101.89 mg, 1.22 mmol). The mixture was stirred uniformly. After 0.5 h, sodium cyanoborohydride (95.83 mg, 1.52 mmol) was added. The reaction liquid was reacted under stirring at room temperature. After 4 h, the sample was taken for detection and analyzed by LCMS, and the results showed that the starting material spot disappeared, and a target product ion peak was generated. Detection confirmed the completion of the reaction. The reaction liquid was concentrated and then separated by preparative column chromatography (dichloromethane/methanol = 99/1 to 90/10) to give the product **7-2** (46 mg, yield: 38.72%).

LCMS (ESI) M/Z: 197.1 [M+H⁺].

**Step B:** Dichloromethane (3 mL) was added to a dry reaction flask, followed by the addition of compound **1-6** (76 mg, 0.13 mmol), N,N-diisopropylethylamine (46.53 mg, 0.36 mmol), and HATU (68.44 mg, 0.18 mmol). The mixture was stirred uniformly. Subsequently, compound **7-2** (23 mg, 0.12 mmol) was added. The reaction liquid was reacted under stirring at room temperature. After 6 h, the sample was taken for detection and analyzed by LCMS, and the results showed that the starting material spot disappeared, and a target product ion peak was generated. Detection confirmed the completion of the reaction. The reaction liquid was concentrated and then separated by preparative column chromatography (dichloromethane/methanol = 99/1 to 90/10) to give the product 7-3 (12 mg, yield: 13.17%).

LCMS (ESI) M/Z: 777.4 [M+H⁺].

**Step C:** N,N-Dimethylformamide (1 mL) was added to a dry reaction flask, followed by the addition of compound **7-3** (12 mg, 0.015 mmol). The mixture was stirred uniformly. Subsequently, tetrahydroxydiboron (6.72 mg, 0.075 mmol) and 4,4'-bipyridine (1.17 mg, 0.0075 mmol) were added. The reaction liquid was reacted under stirring at room temperature. After 15 min, the sample was taken for detection and analyzed by LCMS, and the results showed that the starting material spot disappeared, and a target product ion peak was generated. Detection confirmed the completion of the reaction. The reaction liquid was purified by preparative high performance liquid chromatography to give the product compound 7 (3.0 mg, yield: 26.00%).

LCMS (ESI) M/Z: 747.5 [M+H⁺].

¹H NMR (400 MHz, DMSO-*d*₆) δ 8.04 (dd, *J* = 15.6, 8.7 Hz, 1H), 6.91 (td, *J* = 7.7, 3.2 Hz, 1H), 6.43-6.33 (m, 3H), 4.94 (s, 1H), 4.76-4.61 (m, 1H), 4.60-4.48 (m, 1H), 4.04-3.88 (m, 2H), 3.82 (dd, *J=* 14.5, 7.4 Hz, 1H), 3.72 (dd, *J* = 7.5, 3.5 Hz, 1H), 3.62 (d, *J* = 11.0 Hz, 3H), 3.26 (s, 3H), 3.18 (d, *J =* 6.2 Hz, 6H), 3.15-3.13 (m, 2H), 3.00 (s, 3H), 2.68-2.65 (m, 1H), 2.65-2.61 (m, 2H), 2.34-2.27 (m, 1H), 2.20 (d, *J* = 9.2 Hz, 6H), 2.03-1.96 (m, 2H), 1.95-1.84 (m, 4H), 1.78-1.61 (m, 3H), 1.51-1.42 (m, 1H), 1.14-1.06 (m, 3H), 0.92-0.88 (m, 6H), 0.87 (d, *J* = 6.3 Hz, 6H), 0.77-0.74 (m, 3H), 0.73-0.69 (m, 3H).

### Example 8

### Preparation of (S)-N-((3R,4S,SS)-1-((S)-2-((1R,2R)-3-((3-aminophenethyl)(hydroxy)amino)-1-methoxy-2-methyl-3-oxopropyl)pyrrolidin-1-yl)-3-methoxy-5-methyl-1-oxoheptan-4-yl)-2-((S)-2-(dimethylamino)-3-methylbutanamido)-N,3-dimethylbutanamide.

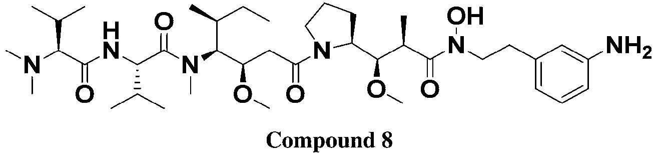

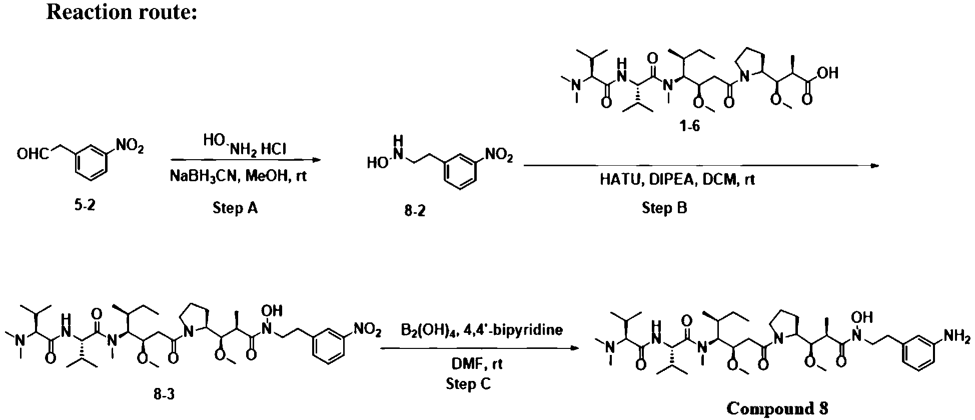

### Procedures:

**Step A:** Methanol (5 mL) was added to a dry reaction flask, followed by the addition of compound **5-2** (100 mg, 0.61 mmol) and hydroxylamine hydrochloride (84.78 mg, 1.22 mmol). The mixture was stirred uniformly. After 0.5 h, sodium cyanoborohydride (95.83 mg, 1.52 mmol) was added. The reaction liquid was reacted under stirring at room temperature. After 4 h, the sample was taken for detection and analyzed by LCMS, and the results showed that the starting material spot disappeared, and a target product ion peak was generated. Detection confirmed the completion of the reaction. The reaction liquid was concentrated and then separated by preparative column chromatography (dichloromethane/methanol = 99/1 to 90/10) to give the product **8-2** (19 mg, yield: 17.22%).

LCMS (ESI) M/Z: 183.1 [M+H⁺].

**Step B:** Dichloromethane (3 mL) was added to a dry reaction flask, followed by the addition of compound **1-6** (76 mg, 0.13 mmol), N,N-diisopropylethylamine (38.77 mg, 0.30 mmol), and HATU (57.03 mg, 0.15 mmol). The mixture was stirred uniformly. Subsequently, compound **8-2** (19 mg, 0.10 mmol) was added. The reaction liquid was reacted under stirring at room temperature. After 6 h, the sample was taken for detection and analyzed by LCMS, and the results showed that the starting material spot disappeared, and a target product ion peak was generated. Detection confirmed the completion of the reaction. The reaction liquid was concentrated and then separated by preparative column chromatography (dichloromethane/methanol = 99/1 to 90/10) to give the product **8-3** (25 mg, yield: 31.42%).

LCMS (ESI) M/Z: 763.4 [M+H⁺].

**Step C:** N,N-Dimethylformamide (1 mL) was added to a dry reaction flask, followed by the addition of compound **8-3** (25 mg, 0.033 mmol). The mixture was stirred uniformly. Subsequently, tetrahydroxydiboron (14.79 mg, 0.17 mmol) and 4,4'-bipyridine (2.58 mg, 0.017 mmol) were added. The reaction liquid was reacted under stirring at room temperature. After 15 min, the sample was taken for detection and analyzed by LCMS, and the results showed that the starting material spot disappeared, and a target product ion peak was generated. Detection confirmed the completion of the reaction. The reaction liquid was purified by preparative high performance liquid chromatography to give the product compound **8** (1.4 mg, yield: 5.83%).

LCMS (ESI) M/Z: 733.5 [M+H⁺].

¹H NMR (400 MHz, DMSO-*d*₆) δ 8.03 (t, *J* = 8.6 Hz, 1H), 6.89 (t, *J* = 7.7 Hz, 1H), 6.49-6.26 (m, 3H), 4.92 (s, 2H), 4.76-4.62 (m, 1H), 4.63-4.46 (m, 2H), 4.04-3.92 (m, 1H), 3.84 (dd, *J* = 18.9, 10.0 Hz, 1H), 3.77-3.68 (m, 1H), 3.30 (s, 3H), 3.28 (s, 3H), 3.20-3.19 (m, 1H), 3.17 (s, 3H), 3.14-3.08 (m, 2H), 2.99 (s, 3H), 2.71-2.61 (m, 3H), 2.34-2.26 (m, 1H), 2.20 (s, 6H), 2.04-1.97 (m, 1H), 1.96-1.89 (m, 2H), 1.88-1.81 (m, 2H), 1.78-1.58 (m, 3H), 1.56-1.36 (m, 1H), 1.13-1.02 (m, 3H), 0.94-0.89 (m, 6H), 0.88-0.85 (m, 6H), 0.78-0.74 (m, 3H), 0.72-0.69 (m, 3H).

### Example 9

### Preparation of (S)-1-(3-aminophenyl)-3-cyanopropan-2-yl (2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanoate.

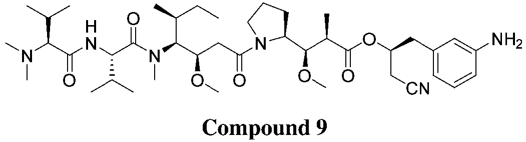

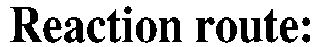

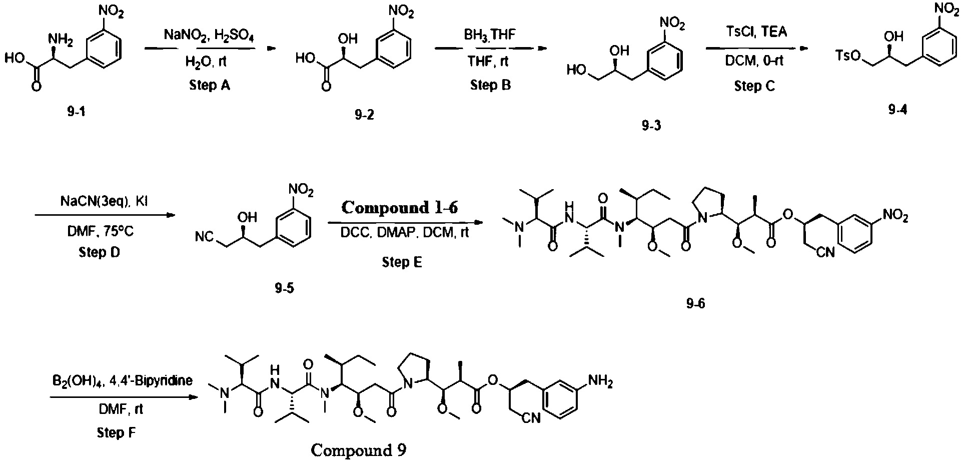

### Procedures:

**Step A:** Compound **9-1** (2.0 g, 9.52 mmol) was dissolved in dilute sulfuric acid (1 M, 20 mL). Subsequently, the solution was cooled to 0°C, and an aqueous sodium nitrite solution (3.9 g/12 mL, 57.31 mmol) was slowly added dropwise. After half an hour, the dropwise addition was completed. The system was naturally warmed to room temperature and stirred overnight. After completion of the reaction as indicated by LCMS, the reaction mixture was extracted with ethyl acetate (100 mL). The organic phase was washed sequentially with water (50 mL) and saturated sodium chloride (50 mL), dried over anhydrous sodium sulfate, and concentrated. The crude product was purified by Flash (acetonitrile/0.1% ammonium bicarbonate/water) to give the product 9-2 (1.2 g, yield: 60%).

LCMS (ESI) M/Z: 212.0 [M+H⁺].

**Step B:** Compound **9-2** (100 mg, 0.47 mmol) was dissolved in anhydrous tetrahydrofuran (3 mL). Subsequently, the solution was cooled to 0°C, and borane tetrahydrofuran (2.8 mL, 2.8 mmol, 1 M tetrahydrofuran) was added dropwise. The reaction system was stirred at room temperature for 16 h. After completion of the reaction as indicated by LCMS, the reaction mixture was quenched with methanol, then diluted with dichloromethane (20 mL), washed sequentially with saturated sodium bicarbonate (10 mL) and saturated sodium chloride (10 mL), dried over anhydrous sodium sulfate, and concentrated to give a crude product. The crude product was purified by a reversed-phase column (acetonitrile/0.1% ammonium bicarbonate/water) to give compound **9-3** (60 mg, yield: 64%).

LCMS (ESI) M/Z: 393.0 [M+H⁺].

**Step C:** Compound **9-3** (100 mg, 0.51 mmol) was dissolved in anhydrous dichloromethane (3 mL). Subsequently, the solution was cooled to 0°C, and p-toluenesulfonyl chloride (145 mg, 0.76 mmol) and triethylamine (0.18 mL, 0.13 mmol) were added. The reaction system was naturally warmed to room temperature and stirred overnight. After completion of the reaction as indicated by LCMS, the reaction liquid was diluted with dichloromethane (10 mL), then washed sequentially with water (10 mL) and saturated sodium chloride (10 mL), dried over anhydrous sodium sulfate, and concentrated to give a crude product. The crude product was purified by a reversed-phase column (acetonitrile/0.1% ammonium bicarbonate/water) to give compound **9-4** (10 mg, yield: 56%).

LCMS (ESI) M/Z: 369.0 [M+H⁺].

**Step D:** Compound **9-4** (80 mg, 0.20 mmol) was dissolved in N,N-dimethylformamide (3 mL), and potassium iodide (38 mg, 0.20 mmol) and sodium cyanide (40 mg, 0.80 mmol) were then added. The reaction system was stirred at 75°C for 16 h. After completion of the reaction as indicated by LCMS, the reaction liquid was diluted with EA (20 mL), then washed sequentially with water (10 mL) and saturated sodium chloride (10 mL), dried over anhydrous sodium sulfate, and concentrated to give a crude product. The crude product was purified by a reversed-phase column (acetonitrile/0.1% ammonium bicarbonate/water) to give the product **9-5** (20 mg, yield: 43%).

LCMS (ESI) M/Z: 411.0 [2M-H]⁻.

**Step E:** Compound **1-6** (200 mg, 0.33 mmol) was dissolved in dry dichloromethane (5 mL), and compound **9-5** (199 mg, 0.96 mmol), N,N'-dicyclohexylcarbodiimide (136 mg, 0.66 mmol), and 4-dimethylaminopyridine (4 mg, 0.03 mmol) were then added sequentially. The reaction system was reacted at room temperature for 16 h under a nitrogen atmosphere. After completion of the reaction as monitored by LCMS, the mixture was filtered, and the filtrate was purified by Flash to give compound **9-6** (140 mg, yield: 53%).

LCMS (ESI) M/Z: 787.2 [M+H⁺].

**Step F:** Tetrahydroxydiboron (58 mg, 0.64 mmol) was dissolved in dry N,N-dimethylformamide (5 mL), and 4,4'-bipyridine (78 mg, 0.37 mmol) and compound **9-6** (120 mg, 0.15 mmol) were then rapidly added sequentially. The reaction system was reacted at room temperature for 5 min under a nitrogen atmosphere. After completion of the reaction as monitored by LCMS, a small amount of methanol was added to quench the reaction, and the mixture was filtered. The filtrate was purified by preparative high performance liquid chromatography to give the target compound **9** (60 mg, yield: 52%).

LCMS (ESI) M/Z: 757.5 [M+H⁺].

¹H NMR (400 MHz, DMSO) δ 8.89 (d, *J =* 7.7 Hz, 1H), 7.25 (m, 1H), 7.00-6.82 (m, 3H), 5.34-5.09 (m, 1H), 4.72-4.50 (m, 2H), 4.00-3.89 (m, 2H), 3.85 (m, 1H), 3.72 (m, 1H), 3.58-3.40 (m, 2H), 3.37-3.21 (m, 4H), 3.17 (m, 3H), 3.03 (m, 3H), 2.97-2.81 (m, 4H), 2.75 (m, 6H), 2.47-2.21 (m, 4H), 2.10-1.63 (m, 6H), 1.38-1.20 (m, 1H), 1.01 (d, *J* = 6.8 Hz, 3H), 0.98-0.82 (m, 15H), 0.81-0.72 (m, 3H).

### Example 10

### Preparation of (S)-N-((3R,4S,5S)-1-((S)-2-((1R,2R)-3-(allyl((S)-1-(3-aminophenyl)-3-cyanopropan-2-yl)amino)-1-methoxy-2-methyl-3-oxopropyl)pyrrolidin-1-yl)-3-methoxy-5-methyl-1-oxoheptan-4-yl)-2-((S)-2-(dimethylamino)-3-methylbutanamido)-N,3-dimethylbutanamide.

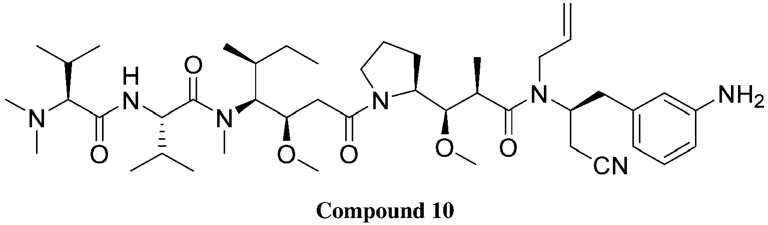

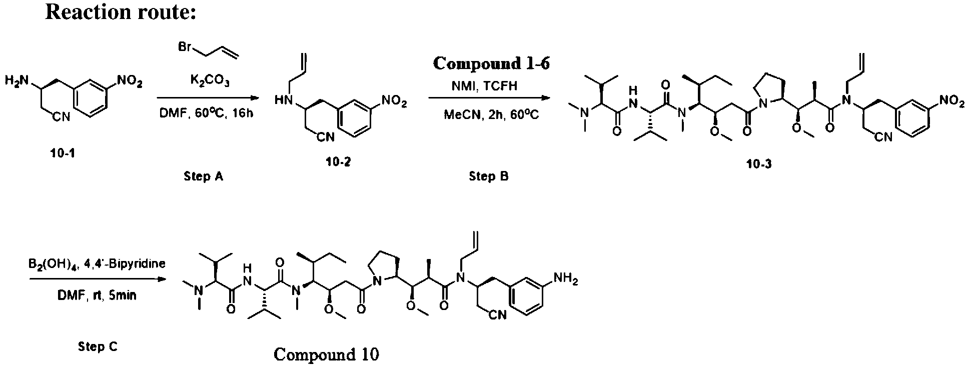

### Procedures:

**Step A:** Compound **10-1** (400 mg, 2 mmol) was dissolved in extra dry N,N-dimethylformamide (15 mL), and allyl bromide (223 mg, 3 mmol) and potassium carbonate (260 mg, 3 mmol) were added. The mixture was stirred at 60°C for 16 h. After completion of the reaction as indicated by LCMS, the mixture was filtered, and the filtrate was purified by preparative high performance liquid chromatography (0.1% FA in water/acetonitrile) to give the product **10-2** (200 mg, yield: 40.01%).

LCMS (ESI) M/Z: 246.1 [M+H⁺].

**Step B:** Compound **10-2** (200 mg, 0.9 mmol), compound **1-6** (400 mg, 0.67 mmol), NMI (0.27 mL), and TCFH (470 mg, 1.68 mmol) were added to 2 mL of anhydrous MeCN (5 mL) under a nitrogen atmosphere, and the resulting mixture was stirred at 60°C overnight. After completion of the reaction as indicated by LCMS, the reaction liquid was concentrated, and 3 mL of tetrahydrofuran was then added. The mixture was filtered, and the filtrate was purified by a reversed-phase column (0.1% FA in water/acetonitrile) to give compound **10-3** (40 mg, yield: 20.63%).

LCMS (ESI) M/Z: 826.4 [M+H⁺].

**Step C:** Tetrahydroxydiboron (80 mg, 0.1 mmol) was added to anhydrous N,N-dimethylformamide (1 mL), and 4,4'-bipyridine (15 mg, 0.1 mmol) and compound **10-3** (40 mg, 0.1 mmol) were then immediately added. The resulting system was stirred at room temperature for 5 min. After completion of the reaction as indicated by LCMS, the mixture was filtered, and the filtrate was purified by high performance liquid chromatography to give the target compound **10** (7 mg, yield: 30.59%).

LCMS (ESI) M/Z: 796.5 [M+H⁺].

¹H NMR (400 MHz, DMSO) δ 8.07-7.96 (m, 1H), 6.95-6.87 (m, 1H), 6.43-6.28 (m, 3H), 5.73-5.59 (m, 1H), 5.35-5.08 (m, 2H), 5.05-4.84 (m, 2H), 4.68-4.47 (m, 2H), 4.03-3.92 (m, 2H), 3.87-3.76 (m, 1H), 3.62-3.48 (m, 2H), 3.29 (d, *J* = 5.5 Hz, 2H), 3.19 (d, *J* = 6.1 Hz, 5H), 3.10 (s, 2H), 3.00 (s, 3H), 2.89-2.76 (m, 2H), 2.71-2.55 (m, 4H), 2.45-2.26 (m, 2H), 2.20 (d, *J* = 10.0 Hz, 6H), 2.01-1.63 (m, 8H), 1.37-1.28 (m, 1H), 0.97-0.85 (m, 15H), 0.78-0.66 (m, 6H).

### Example 11

### Preparation of (R)-1-(3-aminophenyl)propan-2-yl (2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanoate.

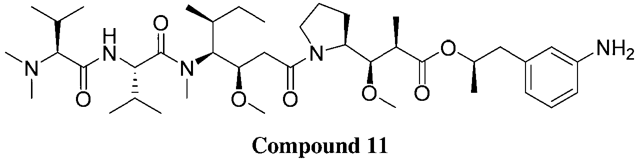

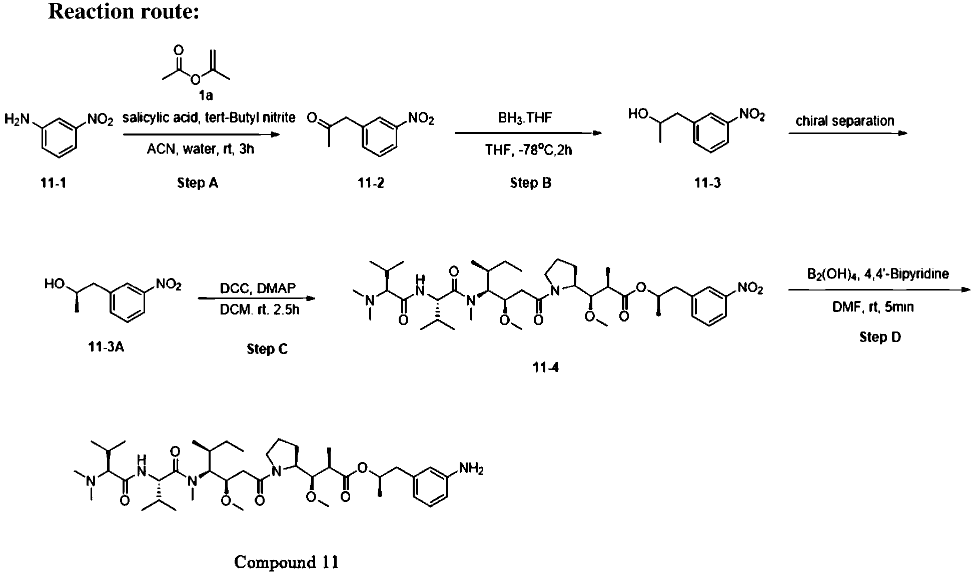

### Procedures:

**Step A:** The starting material **11-1** (2.5 g, 1.81 mmol) was dissolved in acetonitrile (25 mL) and water (2.5 mL), and isopropenyl acetate (1.82 g, 1.81 mmol) and salicylic acid (250 mg, 0.181 mmol) were added. After 5 min of stirring, tert-butyl nitrite (2.8 g, 2.71 mmol) was added. The resulting mixture was reacted at room temperature for 3 h under a nitrogen atmosphere. After completion of the reaction as indicated by TLC, ammonium chloride (20 mL) was added to quench the reaction, and extraction was performed with dichloromethane (50 mL × 3). The organic phases were combined, dried over anhydrous sodium sulfate, filtered, and concentrated. The resulting crude product was purified by column chromatography (PE:EA = 10:1) to give the product **11-2** (0.9 g, yield: 30%).

¹H NMR (400 MHz, CDCl₃) δ 8.32-7.90 (m, 2H), 7.52 (d, *J* = 5.3 Hz, 2H), 3.86 (s, 2H), 2.26 (s, 3H).

**Step B:** Compound **11-2** (600 mg, 2.7 mmol) was dissolved in anhydrous tetrahydrofuran (20 mL), and a solution of borane in tetrahydrofuran (5.6 mL, 5.6 mmol, 1 M in THF) was added dropwise at 0°C. The reaction system was stirred at room temperature for 16 h. After completion of the reaction as indicated by TLC, methanol was added to quench the reaction, and the mixture was diluted with dichloromethane (20 mL), washed sequentially with saturated sodium bicarbonate (10 mL) and saturated sodium chloride (10 mL), dried over anhydrous sodium sulfate, and concentrated to give a crude product. The crude product was purified by column chromatography (PE:EA = 9:1) to give racemate **11-3,** which was then subjected to chiral resolution to give compound **11-3A** (200 mg, yield: 34%) and compound **11-3B** (200 mg, yield: 34%) in a single configuration. Resolution conditions: mobile phase: supercritical carbon dioxide and a solution of 0.1% ammonia in isopropanol; flow rate: 100 mL/min; gradient: maintaining 0.1% ammonia in isopropanol at 20%; detection wavelength: 214 nm; cycle time: 7.5 min.

The retention time (Rt) of compound **11-3A** = 5.6 min; the retention time (Rt) of compound **11-3B** = 8.2 min.

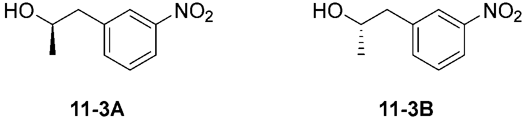

¹H NMR (400 MHz, DMSO) δ 8.19-7.94 (m, 2H), 7.75-7.49 (m, 2H), 4.65 (d, *J =* 4.9 Hz, 1H), 3.87 (m, *J* = 11.4, 5.5 Hz, 1H), 2.90-2.64 (m, 2H), 1.08 (d, *J =* 6.2 Hz, 3H).

**Step C:** Compound **1-6** (132 mg, 0.22 mmol) was dissolved in dry dichloromethane (5 mL), and compound **11-3A** (40 mg, 0.22 mmol), N,N'-dicyclohexylcarbodiimide (91 mg, 0.44 mmol), and 4-dimethylaminopyridine (3 mg, 0.02 mmol) were then added sequentially. The reaction system was reacted at room temperature for 16 h under a nitrogen atmosphere. After completion of the reaction as monitored by LCMS, the mixture was filtered, and the filtrate was purified by Flash (acetonitrile/0.1% ammonium bicarbonate/water) to give compound **11-4** (120 mg, yield: 53%).

LCMS (ESI) M/Z: 762.4 [M+H⁺].

**Step D:** Tetrahydroxydiboron (95 mg, 1.01 mmol) was dissolved in dry N,N-dimethylformamide (5 mL), and 4,4'-bipyridine (20 mg, 0.25 mmol) and compound **11-4** (100 mg, 0.15 mmol) were then rapidly added sequentially. The reaction system was reacted at room temperature for 5 min under a nitrogen atmosphere. After completion of the reaction as monitored by LCMS, a small amount of methanol was added to quench the reaction, and the mixture was filtered. The filtrate was purified by preparative high performance liquid chromatography to give the target compound **11** (30 mg, yield: 52%).

LCMS (ESI) M/Z: 732.3 [M+H⁺].

¹H NMR (400 MHz, DMSO) δ 8.09-7.97 (m, 1H), 6.95-6.79 (m, 1H), 6.44-6.27 (m, 3H), 5.15-4.86 (m, 3H), 4.75-4.43 (m, 2H), 4.05-3.91 (m, 1H), 3.88-3.78 (m, 1H), 3.77-3.68 (m, 1H), 3.64-3.46 (m, 1H), 3.41-3.30 (m, 2H), 3.27-3.10 (m, 6H), 3.09-2.95 (m, 3H), 2.72-2.59 (m, 3H), 2.46-2.29 (m, 3H), 2.24-2.13 (m, 6H), 2.04-1.83 (m, 3H), 1.81-1.53 (m, 4H), 1.36-1.25 (m, 1H), 1.23-1.13 (m, 3H), 1.13-1.04 (m, 3H), 0.95-0.85 (m, 12H), 0.80-0.63 (m, 6H).

### Example 12

### Preparation of (S)-1-(3-aminophenyl)propan-2-yl (2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanoate.

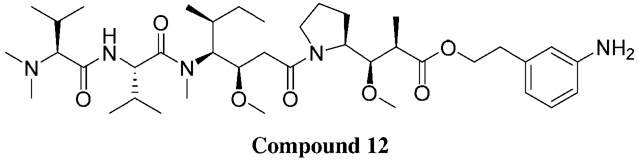

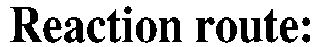

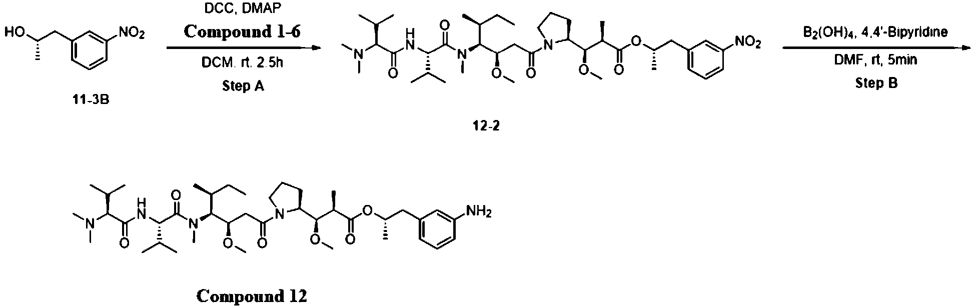

### Procedures:

**Step A:** Compound **1-6** (32 mg, 0.22 mmol) was dissolved in dry dichloromethane (5 mL), and compound **11-3B** (40 mg, 0.22 mmol), N,N'-dicyclohexylcarbodiimide (91 mg, 0.44 mmol), and 4-dimethylaminopyridine (3 mg, 0.02 mmol) were then added sequentially. The reaction system was reacted at room temperature for 16 h under a nitrogen atmosphere. After completion of the reaction as monitored by LCMS, the mixture was filtered, and the filtrate was purified by Flash (acetonitrile/0.1% ammonium bicarbonate/water) to give compound **12-2** (120 mg, yield: 53%).

LCMS (ESI) M/Z: 762.4 [M+H⁺].

**Step B:** Tetrahydroxydiboron (95 mg, 1.01 mmol) was dissolved in dry N,N-dimethylformamide (5 mL), and 4,4'-bipyridine (20 mg, 0.25 mmol) and compound **12-2** (100 mg, 0.15 mmol) were then rapidly added sequentially. The reaction system was reacted at room temperature for 5 min under a nitrogen atmosphere. After completion of the reaction as monitored by LCMS, a small amount of methanol was added. The mixture was filtered, and the filtrate was purified by preparative high performance liquid chromatography (acetonitrile/0.1% ammonium bicarbonate/water) to give compound **12** (30 mg, yield: 52%).

LCMS (ESI) M/Z: 732.3 [M+H⁺].

¹H NMR (400 MHz, DMSO) δ 8.09-7.97 (m, 1H), 6.95-6.79 (m, 1H), 6.44-6.27 (m, 3H), 5.15-4.86 (m, 3H), 4.75-4.43 (m, 2H), 4.05-3.91 (m, 1H), 3.88-3.78 (m, 1H), 3.77-3.68 (m, 1H), 3.64-3.46 (m, 1H), 3.41-3.30 (m, 2H), 3.27-3.10 (m, 6H), 3.09-2.95 (m, 3H), 2.72-2.59 (m, 3H), 2.46-2.29 (m, 3H), 2.24-2.13 (m, 6H), 2.04-1.83 (m, 3H), 1.81-1.53 (m, 4H), 1.36-1.25 (m, 1H), 1.23-1.13 (m, 3H), 1.13-1.04 (m, 3H), 0.95-0.85 (m, 12H), 0.80-0.63 (m, 6H).

The synthetic methods of the above examples were referred to prepare the following target compounds:

| Example | Structural formula | ¹HNMR | MS (ESI) M/Z [M+H]⁺ |
|---|---|---|---|
| 13 | 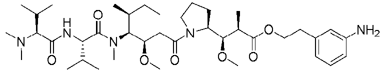 | ¹H NMR (400 MHz, DMSO) δ 8.03 (d, *J* = 7.9 Hz, 1H), 6.91 (dt, *J* = 11.4, 7.7 Hz, 1H), 6.47 - 6.28 (m, 3H), 5.03 - 4.75 (m, 4H), 4.73 - 4.42 (m, 2H), 4.03 - 3.90 (m, 2H), 3.85 (dd, *J* = 7.4, 3.3 Hz, 1H), 3.56 - 3.38 (m, 3H), 3.30 - 3.25 (m, 3H), 3.17 (d, *J* = 6.4 Hz, 3H), 3.07 - 2.95 (m, 3H), 2.75 - 2.57 (m, 3H), 2.49 - 2.26 (m, 4H), 2.20 (d, *J* = 7.4 Hz, 6H), 2.04 - 1.85 (m, 5H), 1.82 - 1.62 (m, 3H), 1.53 - 1.37 (m, 1H), 1.05 (d, *J* = 6.9 Hz, 3H), 0.92 - 0.85 (m, 12H), 0.79 - 0.68 (m, 6H). | 718.2 |
| 14 | 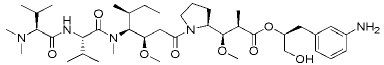 | ¹H NMR (400 MHz, DMSO) δ 8.03 (d, *J* = 7.9 Hz, 1H), 6.91 (dt, *J* = 11.4, 7.7 Hz, 1H), 6.47 - 6.28 (m, 3H), 5.03 - 4.75 (m, 4H), 4.73 - 4.42 (m, 2H), 4.03 - 3.90 (m, 2H), 3.85 (dd, *J* = 7.4, 3.3 Hz, 1H), 3.56 - 3.38 (m, 3H), 3.30 - 3.25 (m, 3H), 3.17 (d, *J* = 6.4 Hz, 3H), 3.07 - 2.95 (m, 3H), 2.75 - 2.57 (m, 3H), 2.49 - 2.26 (m, 4H), 2.20 (d, *J* = 7.4 Hz, 6H), 2.04 - 1.85 (m, 5H), 1.82 - 1.62 (m, 3H), 1.53 - 1.37 (m, 1H), 1.05 (d, *J* = 6.9 Hz, 3H), 0.92 - 0.85 (m, 12H), 0.79 - 0.68 (m, 6H). | 748.2 |
| 15 | 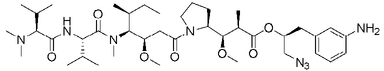 | ¹H NMR (400 MHz, DMSO) δ 8.02 (d, *J =* 9.1 Hz, 1H), 6.91 (t, *J =* 7.8 Hz, 1H), 6.43 - 6.37 (m, 2H), 6.33 (d, *J* = 7.5 Hz, 1H), 5.12 - 4.93 (m, 3H), 4.73 - 4.45 (m, 2H), 3.96 (d, *J* = 4.1 Hz, 2H), 3.90 - 3.78 (m, 1H), 3.67 - 3.42 (m, 3H), 3.30 (s, 2H), 3.26 (s, 3H), 3.19 - 3.15 (m, 3H), 3.07 - 2.97 (m, 3H), 2.70 (d, *J* = 6.7 Hz, 2H), 2.65 - 2.60 (m, 1H), 2.46 (s, 1H), 2.42 (t, *J* = 7.0 Hz, 2H), 2.36 - 2.29 (m, 1H), 2.23 - 2.16 (m, 6H), 2.03 - 1.98 (m, 1H), 1.93 - 1.85 (m, 3H), 1.79 - 1.67 (m, 3H), 1.10 - 1.04 (m, 3H), 0.92 - 0.86 (m, 12H), 0.77 - 0.69 (m, 6H). | 773.6 |
| 16 | 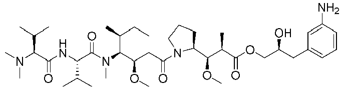 | ¹H NMR (400 MHz, DMSO) δ 8.08 - 7.98 (m, 1H), 6.89 (t, *J* = 7.6 Hz, 1H), 6.44 - 6.32 (m, 3H), 4.91 (s, 3H), 4.72 - 4.46 (m, 2H), 4.17 - 3.77 (m, 6H), 3.56 - 3.46 (m, 1H), 3.31 - 3.23 (m, 3H), 3.22 - 3.11 (m, 3H), 3.08 - 2.92 (m, 3H), 2.68 - 2.61 (m, 1H), 2.57 - 2.53 (m, 2H), 2.47 - 2.40 (m, 2H), 2.39 - 2.28 (m, 1H), 2.26 - 2.12 (m, 6H), 2.07 - 1.83 (m, 5H), 1.83 - 1.66 (m, 3H), 1.36 - 1.22 (m, 2H), 1.21 - 1.10 (m, 3H), 0.94 - 0.83 (m, 12H), 0.80 - 0.67 (m, 6H). | 748.5 |
| 17 | 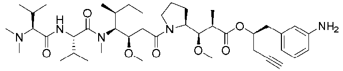 | ¹H NMR (400 MHz, DMSO) δ 8.15 - 7.97 (m, 1H), 6.90 (t, *J* = 7.6 Hz, 1H), 6.57 - 6.31 (m, 3H), 4.98 (s, 3H), 4.78 - 4.47 (m, 2H), 4.02 - 3.93 (m, 1H), 3.87 - 3.80 (m, 1H), 3.76 - 3.70 (m, 1H), 3.60 - 3.47 (m, 3H), 3.30 - 3.24 (m, 2H), 3.22 - 3.13 (m, 5H), 3.01 (s, 2H), 2.95 - 2.89 (m, 1H), 2.88 - 2.76 (m, 1H), 2.76 - 2.61 (m, 2H), 2.48 - 2.44 (m, 2H), 2.43 - 2.28 (m, 3H), 2.25 - 2.15 (m, 6H), 2.08 - 1.53 (m, 8H), 1.35 - 1.29 (m, 1H), 1.17 - 1.06 (m, 3H), 0.94 - 0.86 (m, 12H), 0.78 - 0.69 (m, 6H). | 756.5 |
| 18 | 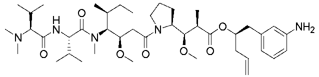 | ¹H NMR (400 MHz, DMSO) δ 8.14 - 7.95 (m, 1H), 6.88 (t, *J* = 7.6 Hz, 1H), 6.48 - 6.24 (m, 3H), 5.92 - 5.65 (m, 1H), 5.19 - 4.89 (m, 5H), 4.74 - 4.44 (m, 2H), 4.05 - 3.93 (m, 1H), 3.80 (dd, *J* = 7.8, 4.0 Hz, 1H), 3.74 (dd, *J* = 6.7, 3.8 Hz, 1H), 3.55 - 3.44 (m, 1H), 3.24 (d, *J* = 5.3 Hz, 1H), 3.21 - 3.11 (m, 5H), 3.07 (d, *J=* 7.2 Hz, 1H), 3.02 - 2.97 (m, 2H), 2.82 - 2.54 (m, 4H), 2.46 - 2.36 (m, 2H), 2.34 - 2.24 (m, 3H), 2.20 (d, *J* = 6.4 Hz, 6H), 2.03 - 1.43 (m, 8H), 1.35 - 1.27 (m, 1H), 1.14 - 1.03 (m, 3H), 0.94 - 0.84 (m, 12H), 0.78 - 0.67 (m, 6H). | 758.3 |
| 19 | 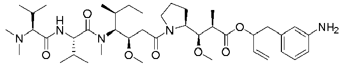 | ¹H NMR (400 MHz, DMSO) δ 8.03 (d, *J* = 9.0 Hz, 1H), 6.94 - 6.83 (m, 1H), 6.44 - 6.30 (m, 3H), 5.97 - 5.77 (m, 1H), 5.50 - 5.29 (m, 1H), 5.24 - 5.08 (m, 2H), 4.94 (s, 2H), 4.73 - 4.58 (m, 1H), 4.58 - 4.47 (m, 1H), 4.02 - 3.93 (m, 1H), 3.92 - 3.70 (m, 2H), 3.56 - 3.44 (m, 1H), 3.30 - 3.27 (m, 1H), 3.24 - 3.22 (m, 1H), 3.20 - 3.14 (m, 3H), 3.13 - 3.06 (m, 2H), 3.04 - 2.95 (m, 3H), 2.80 - 2.60 (m, 3H), 2.46 - 2.28 (m, 4H), 2.20 (s, 6H), 2.02 - 1.83 (m, 4H), 1.81 - 1.66 (m, 3H), 1.35 - 1.27 (m, 1H), 1.14 - 1.03 (m, 3H), 0.93 - 0.85 (m, 12H), 0.78 - 0.69 (m, 6H). | 745.4 |
| 20 and 21 | The structures of compound 20 and compound 21 are as follows: 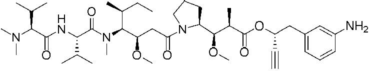 | ¹H NMR (400 MHz, DMSO) δ 8.02 (d, *J =* 7.8 Hz, 1H), 6.91 (t, *J =* 7.6 Hz, 1H), 6.50 - 6.35 (m, 3H), 5.48 - 5.31 (m, 1H), 4.98 (s, 2H), 4.75 - 4.44 (m, 2H), 4.04 - 3.89 (m, 2H), 3.89 - 3.79 (m, 1H), 3.55 - 3.44 (m, 2H), 3.40 - 3.34 (m, 1H), 3.31 - 3.29 (m, 1H), 3.26 (s, 3H), 3.22 - 3.14 (m, 3H), 3.09 - 2.96 (m, 3H), 2.92 - 2.83 (m, 2H), 2.69 - 2.59 (m, 1H), 2.48 - 2.41 (m, 2H), 2.38 - 2.28 (m, 1H), 2.20 (d, *J* = 5.6 Hz, 6H), 2.03 - 1.70 (m, 7H), 1.29 (s, 1H), 1.11 - 1.03 (m, 3H), 0.93 - 0.85 (m, 12H), 0.78 - 0.68 (m, 6H). | 742.5 |
| | OR | | |
| | 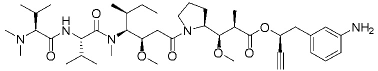 Resolution conditions for compound **20** and compound **21** are as follows: mobile phase: supercritical carbon dioxide and a solution of 0.1% ammonia in isopropanol; flow rate: 100 mL/min; gradient: maintaining 0.1% ammonia in isopropanol at 20%. Detection wavelength: 214 nm. Cycle time: 7.5 min. | | |
| | The retention time (Rt) of compound **20** = 5.5 min; | | |
| | the retention time (Rt) of compound **21** = 8.6 min. | | |
| 22 | 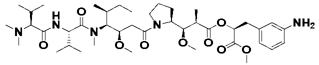 | ¹H NMR (400 MHz, DMSO-*d*₆) δ 8.02 (d, *J* = 8.4 Hz, 1H), 6.90 (t, *J =* 7.6 Hz, 1H), 6.48 - 6.32 (m, 3H), 5.09 (dd, *J* = 7.8, 4.8 Hz, 1H), 4.99 (s, 2H), 4.71 - 4.58 (m, 1H), 4.52 (t, *J* = 8.7 Hz, 1H), 4.03 - 3.93 (m, 2H), 3.91 - 3.78 (m, 1H), 3.67 - 3.63 (m, 1H), 3.62 (s, 3H), 3.61 - 3.57 (m, 1H), 3.25 (s, 3H), 3.17 (s, 3H), 3.00 (s, 3H), 2.96 - 2.85 (m, 2H), 2.69 - 2.58 (m, 2H), 2.47 - 2.39 (m, 2H), 2.36 (s, 1H), 2.20 (s, 6H), 2.00 - 1.84 (m, 5H), 1.75 (dd, *J =* 12.3, 6.5 Hz, 3H), 1.15 - 1.05 (m, 3H), 0.91 (d, *J* = 5.6 Hz, 6H), 0.87 (dd, *J* = 6.7, 2.7 Hz, 6H), 0.75 (t, *J =* 7.1 Hz, 3H), 0.70 (d, *J =* 6.5 Hz, 3H). | 776.5 |
| 23 | 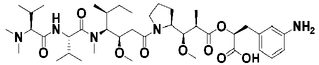 | ¹H NMR (400 MHz, DMSO-*d*₆) δ 8.02 (d, *J* = 9.4 Hz, 1H), 6.91 - 6.81 (m, 1H), 6.49 - 6.29 (m, 3H), 5.04 - 4.78 (m, 2H), 4.69 - 4.60 (m, 1H), 4.60 - 4.48 (m, 2H), 4.10 - 4.02 (m, 1H), 4.01 - 3.95 (m, 1H), 3.89 (dd, *J* = 7.1, 3.7 Hz, 1H), 3.22 (s, 3H), 3.17 (s, 3H), 2.99 (d, *J* = 3.3 Hz, 3H), 2.98 - 2.96 (m, 1H), 2.69 - 2.66 (m, 1H), 2.65 - 2.62 (m, 1H), 2.44 - 2.40 (m, 1H), 2.36 - 2.33 (m, 1H), 2.33 - 2.30 (m, 1H), 2.19 (s, 6H), 1.98 - 1.86 (m, 5H), 1.76 - 1.63 (m, 3H), 1.00 (d, *J =* 7.4 Hz, 3H), 0.92 - 0.89 (m, 6H), 0.87 (d, *J* = 6.4 Hz, 6H), 0.77 - 0.73 (m, 3H), 0.72 - 0.69 (m, 3H). | 762.6 |
| 24 | 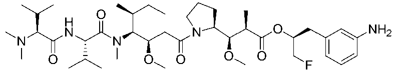 | ¹H NMR (400 MHz, DMSO) δ 8.11 - 7.93 (m, 1H), 7.02 - 6.84 (m, 1H), 6.64 - 6.55 (m, 1H), 6.53 - 6.45 (m, 1H), 6.44 - 6.33 (m, 1H), 6.25 - 6.12 (m, 1H), 5.18 - 4.96 (m, 2H), 4.75 - 4.58 (m, 2H), 4.58 - 4.31 (m, 2H), 4.03 - 3.80 (m, 3H), 3.56 - 3.46 (m, 1H), 3.39 - 3.33 (m, 1H), 3.31 - 3.21 (m, 4H), 3.20 - 3.13 (m, 3H), 3.08 - 2.95 (m, 3H), 2.69 (ddd, *J* = 29.0, 15.9, 7.9 Hz, 2H), 2.57 - 2.51 (m, 1H), 2.47 - 2.39 (m, 2H), 2.32 (dd, *J* = 15.5, 9.4 Hz, 1H), 2.23 - 2.11 (m, 6H), 2.02 - 1.84 (m, 4H), 1.80 - 1.61 (m, 3H), 1.35 - 1.26 (m, 1H), 1.22 - 1.12 (m, 2H), 1.06 (d, *J* = 6.7 Hz, 1H), 0.94 - 0.84 (m, 12H), 0.78 - 0.67 (m, 6H). ¹⁹F NMR (377 MHz, DMSO) δ - 229.23 (s). | 750.2 |
| 25 | 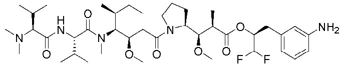 | ¹H NMR (400 MHz, DMSO) δ 8.03 (t, *J* = 7.8 Hz, 1H), 6.99 - 6.81 (m, 1H), 6.49 - 6.31 (m, 3H), 6.29 - 5.96 (m, 1H), 5.33 - 5.15 (m, 1H), 5.00 (d, *J* = 7.5 Hz, 2H), 4.73 - 4.46 (m, 2H), 4.06 - 3.92 (m, 1H), 3.91 - 3.81 (m, 2H), 3.65 - 3.48 (m, 1H), 3.44 (d, *J* = 9.0 Hz, 1H), 3.29 - 3.19 (m, 3H), 3.16 (d, *J* = 8.9 Hz, 3H), 3.00 (d, *J* = 5.7 Hz, 3H), 2.92 - 2.81 (m, 1H), 2.70 - 2.61 (m, 2H), 2.48 - 2.37 (m, 2H), 2.36 - 2.26 (m, 1H), 2.20 (d, *J* = 7.5 Hz, 6H), 2.07 - 1.80 (m, 5H), 1.79 - 1.59 (m, 3H), 1.34 - 1.26 (m, 1H), 0.99 (dd, *J =* 6.7, 4.4 Hz, 3H), 0.93 - 0.86 (m, 12H), 0.78 - 0.69 (m, 6H). ¹⁹F NMR (377 MHz, DMSO) δ - 127.73 (dd, *J* = 284.4, 12.6 Hz), - 131.01 (dd, *J* = 284.8, 95.7 Hz). | 768.5 |
| 26 | 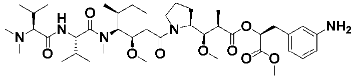 | ¹H NMR (400 MHz, DMSO-*d*₆) δ 8.03 (d, *J* = 6.6 Hz, 1H), 6.95 - 6.86 (m, 1H), 6.48 - 6.37 (m, 3H), 5.68 - 5.50 (m, 1H), 5.00 (s, 2H), 4.70 - 4.58 (m, 1H), 4.57 - 4.43 (m, 1H), 4.04 - 3.91 (m, 1H), 3.84 (d, *J* = 6.2 Hz, 1H), 3.75 - 3.67 (m, 1H), 3.64 (t, *J* = 5.2 Hz, 1H), 3.24 - 3.19 (m, 3H), 3.17 (d, *J* = 2.2 Hz, 3H), 3.00 (s, 3H), 2.81 - 2.73 (m, 1H), 2.69 - 2.60 (m, 2H), 2.46 - 2.38 (m, 2H), 2.35 - 2.29 (m, 1H), 2.20 (s, 6H), 2.02 - 1.82 (m, 5H), 1.80 - 1.65 (m, 3H), 1.07 - 0.96 (m, 3H), 0.94 - 0.89 (m, 6H), 0.89 - 0.85 (m, 6H), 0.75 (t, *J* = 5.3 Hz, 3H), 0.71 (d, *J* = 6.6 Hz, 3H). | 786.5 |
| 27 | 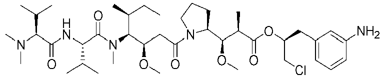 | ¹H NMR (400 MHz, DMSO) δ 8.11 - 7.93 (m, 1H), 7.04 - 6.83 (m, 1H), 6.48 - 6.26 (m, 3H), 5.23 - 5.06 (m, 1H), 5.01 (s, 2H), 4.73 - 4.42 (m, 2H), 4.05 - 3.91 (m, 2H), 3.89 - 3.62 (m, 3H), 3.58 - 3.44 (m, 1H), 3.31 (s, 1H), 3.27 - 3.24 (m, 2H), 3.20 - 3.13 (m, 3H), 3.06 - 2.95 (m, 3H), 2.87 - 2.58 (m, 4H), 2.43 (dd, *J* = 15.9, 11.4 Hz, 3H), 2.32 (dd, *J* = 15.6, 9.5 Hz, 1H), 2.24 - 2.13 (m, 6H), 1.98 - 1.84 (m, 4H), 1.82 - 1.63 (m, 3H), 1.37 - 1.26 (m, 1H), 1.09 - 1.02 (m, 3H), 0.93 - 0.85 (m, 12H), 0.78 - 0.69 (m, 6H). | 766.5 |
| 28 | The structural formulas of compounds 28 and 29 are: 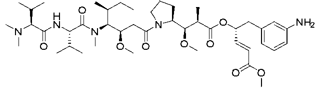 | ¹H NMR (400 MHz, DMSO) δ 8.12 - 7.91 (m, 1H), 7.03 - 6.79 (m, 1H), 6.47 - 6.24 (m, 3H), 4.95 (dd, *J =* 28.2, 9.1 Hz, 2H), 4.79 - 4.45 (m, 2H), 4.17 - 3.81 (m, 2H), 3.79 - 3.56 | 802.5 |
| | or 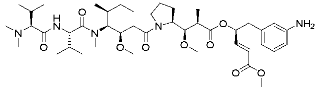 | (m, 2H), 3.55 - 3.41 (m, 2H), 3.39 - 3.33 (m, 1H), 3.30 - 3.25 (m, 2H), 3.22 - 3.09 (m, 5H), 3.05 - 2.71 (m, 6H), 2.69 - 2.53 (m, 4H), 2.47 - 2.37 (m, 1H), 2.28 - 2.14 (m, 6H), 2.08 - 1.55 (m, 8H), 1.38 - 1.25 (m, 1H), 1.11 - 0.96 (m, 3H), 0.93 - 0.85 (m, 12H), 0.79 - 0.67 (m, 6H). | |
| | Resolution conditions for compound **28** and compound **29** are as follows: mobile phase: supercritical carbon dioxide and a solution of 0.1% ammonia in methanol; flow rate: 120 mL/min; gradient: maintaining 0.1% ammonia in isopropanol at 13%. Detection wavelength: 214 nm. Cycle time: 3.1 min. | | |
| 29 | | ¹H NMR (400 MHz, DMSO) δ 8.16 - 7.89 (m, 1H), 6.99 - 6.83 (m, 2H), 6.48 - 6.42 (m, 1H), 6.38 (t, *J* = 9.0 Hz, 2H), 5.99 - 5.86 (m, 1H), 5.69 - 5.43 (m, 1H), 4.97 (s, 2H), 4.73 - 4.46 (m, 2H), 4.04 - 3.93 (m, 1H), 3.83 - 3.77 (m, 1H), 3.76 - 3.71 (m, 1H), 3.69 - 3.62 (m, 3H), 3.53 - 3.45 (m, 1H), 3.25 - 3.15 (m, 4H), 3.11 (s, 2H), 3.07 - 2.98 (m, 3H), 2.92 - 2.83 (m, 1H), 2.78 - 2.57 (m, 3H), 2.47 - 2.28 (m, 4H), 2.20 (s, 6H), 2.01 - 1.84 (m, 3H), 1.82 - 1.66 (m, 3H), 1.63 - 1.53 (m, 1H), 1.35 - 1.27 (m, 1H), 1.16 - 1.04 (m, 3H), 0.94 - 0.84 (m, 12H), 0.79 - 0.68 (m, 6H). | 802.5 |
| | The retention time (Rt) of compound **28** = 5.20 min; | | |
| | the retention time (Rt) of compound **29** = 6.25 min. | | |
| 30 | 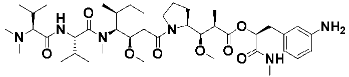 | ¹H NMR (400 MHz, DMSO-*d*₆) δ 8.08 - 7.99 (m, 1H), 7.93 (q, *J* = 4.6 Hz, 1H), 6.89 (t, *J* = 7.7 Hz, 1H), 6.46 - 6.29 (m, 3H), 5.09 - 4.84 (m, 3H), 4.70 - 4.57 (m, 1H), 4.57 - 4.48 (m, 1H), 4.05 - 3.92 (m, 2H), 3.91 - 3.84 (m, 1H), 3.56 - 3.47 (m, 2H), 3.22 (s, 3H), 3.17 (s, 3H), 3.00 (s, 3H), 2.91 - 2.85 (m, 1H), 2.80 - 2.72 (m, 1H), 2.68 - 2.61 (m, 2H), 2.58 (d, *J* = 4.6 Hz, 3H), 2.46 (d, *J* = 2.9 Hz, 1H), 2.45 - 2.40 (m, 1H), 2.37 - 2.28 (m, 1H), 2.20 (s, 6H), 2.04 - 1.82 (m, 5H), 1.81 - 1.60 (m, 3H), 1.04 (d, *J* = 6.7 Hz, 3H), 0.94 - 0.89 (m, 6H), 0.89 - 0.85 (m, 6H), 0.75 (t, *J* = 7.1 Hz, 3H), 0.71 (d, *J* = 6.5 Hz, 3H). | 775.5 |
| 31 | 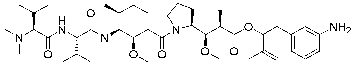 | ¹H NMR (400 MHz, DMSO) δ 8.11 - 7.96 (m, 1H), 6.96 - 6.82 (m, 1H), 6.45 - 6.30 (m, 3H), 5.37 - 5.16 (m, 1H), 5.00 - 4.79 (m, 4H), 4.73 - 4.45 (m, 2H), 4.02 - 3.91 (m, 1H), 3.91 - 3.80 (m, 1H), 3.80 - 3.65 (m, 1H), 3.56 - 3.42 (m, 1H), 3.39 - 3.32 (m, 4H), 3.30 - 3.15 (m, 5H), 3.13 - 2.93 (m, 4H), 2.78 - 2.61 (m, 2H), 2.46 - 2.29 (m, 3H), 2.25 - 2.13 (m, 6H), 2.01 - 1.64 (m, 9H), 1.34 - 1.22 (m, 1H), 1.07 - 0.96 (m, 3H), 0.93 - 0.84 (m, 12H), 0.78 - 0.69 (m, 6H). | 758.5 |
| 32 | 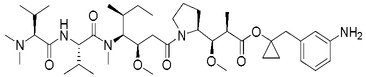 | ¹H NMR (400 MHz, DMSO) δ 8.04 (t, *J* = 8.8 Hz, 1H), 6.89 (t, *J* = 7.6 Hz, 1H), 6.49 - 6.29 (m, 3H), 5.02 - 4.85 (m, 2H), 4.62 (s, 1H), 4.52 (t, *J =* 8.7 Hz, 1H), 4.07 - 3.89 (m, 1H), 3.84 - 3.68 (m, 2H), 3.55 - 3.39 (m, 1H), 3.29 (s, 1H), 3.24 (s, 2H), 3.22 - 3.16 (m, 3H), 3.12 (s, 1H), 3.01 - 2.93 (m, 3H), 2.86 - 2.78 (m, 1H), 2.69 - 2.60 (m, 1H), 2.45 - 2.38 (m, 1H), 2.35 - 2.23 (m, 2H), 2.20 (d, *J* = 7.3 Hz, 6H), 2.04 - 1.83 (m, 4H), 1.81 - 1.59 (m, 4H), 1.54 - 1.40 (m, 1H), 1.35 - 1.28 (m, 1H), 1.09 - 1.01 (m, 3H), 0.92 - 0.86 (m, 12H), 0.82 - 0.68 (m, 10H). | 744.3 |

### Example 33

### Preparation of (S)-N-((3R,4S,5S)-1-((S)-2-((1R,2R)-3-(allyl(3-((S)-2-((S)-2-(2-(2,5-dioxo-2,5-dihydro-1H-pyrrol-1-yl)acetamido)propanamido)propanamido)phenethyl)amino)-1-methoxy-2-methyl-3-oxopropyl)pyrrolidin-1-yl)-3-methoxy-5-methyl-1-oxoheptan-4-yl)-2-((S)-2-(dimethylamino)-3-methylbutanamido)-N,3-dimethylbutanamide.

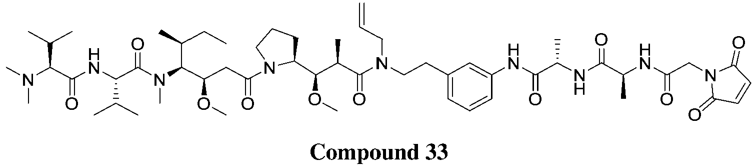

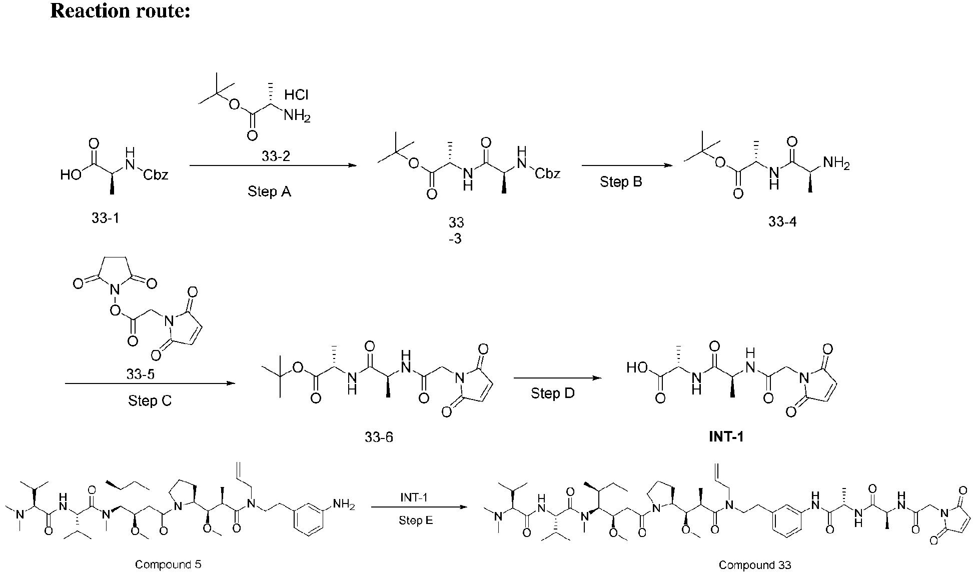

### Procedures:

**Step A:** Compound **33-1** (20 g, 89.59 mmol) was added to a 500 mL single-necked flask, and N,N-dimethylformamide (150 mL), compound **33-2** (17.9 g, 98.55 mmol), 1-(3-dimethylaminopropyl)-3-ethylcarbodiimide hydrochloride (18.89 g, 98.55 mmol), and HOBt (12.11 g, 89.59 mmol) were added sequentially. The reaction system was reacted at 25°C for 12 h under a nitrogen atmosphere. After completion of the reaction as monitored by LCMS, the reaction liquid was poured into water, and the mixture was extracted with ethyl acetate (800 mL). The organic phase was washed with a saturated sodium chloride solution (300 mL × 3), dried over anhydrous sodium sulfate, and filtered. The filtrate was concentrated, and the resulting crude product was purified by a silica gel column (PE/EA = 3:1) to give compound **33-3** (31 g, yield: 98.7%).

LCMS (ESI) M/Z: 373.1 [M+H⁺].

¹H NMR (400 MHz, DMSO) δ 8.15 (d, *J* = 7.0 Hz, 1H), 7.46-7.24 (m, 6H), 5.01 (s, 2H), 4.08 (dt, *J* = 12.1, 7.3 Hz, 2H), 1.38 (s, 9H), 1.23 (dd, *J* = 12.8, 7.2 Hz, 6H).

**Step B:** Compound **33-3** (15 g, 42.81 mmol) was added to a 500 mL single-necked flask, and methanol (300 mL), water (15 mL), and 10% Pd/C (3 g) were added sequentially. The reaction system was reacted at 25°C for 2 h under a hydrogen atmosphere. After completion of the reaction as monitored by LCMS, the mixture was filtered, and the filtrate was concentrated and lyophilized to give compound **33-4** (9 g, yield: 97.2%).

LCMS (ESI) M/Z: 217.1 [M+H⁺].

¹H NMR (400 MHz, DMSO) δ 8.05 (d, *J* = 7.0 Hz, 1H), 4.12 (p, *J* = 7.2 Hz, 1H), 3.26 (q, *J* = 6.9 Hz, 1H), 1.39 (s, 9H), 1.25 (d, *J* = 7.3 Hz, 3H), 1.12 (d, *J* = 6.9 Hz, 3H).

**Step C:** Compound **33-4** (8 g, 36.99 mmol) was added to a 250 mL single-necked flask, and dry N,N-dimethylformamide (80 mL) was added. Compound **33-5** (11.19 g, 44.39 mmol) and 4-methylmorpholine (7.48 mL, 8.13 mL) were then added sequentially, and the reaction system was reacted at 25°C for 12 h under a nitrogen atmosphere. After completion of the reaction as monitored by LCMS, the mixture was filtered, and the filtrate was purified by column chromatography (acetonitrile/0.1% aqueous formic acid solution) to give compound **33-6** (9 g, yield: 68.8%).

LCMS (ESI) M/Z: 376.1 [M+H⁺].

¹H NMR (400 MHz, DMSO) δ 8.39 (d, *J =* 7.7 Hz, 1H), 8.24 (d, *J =* 6.9 Hz, 1H), 7.09 (s, 2H), 4.31 (p, *J* = 7.0 Hz, 1H), 4.15-3.98 (m, 3H), 1.38 (s, 9H), 1.23 (dd, *J* = 11.9, 7.2 Hz, 6H).

**Step D:** Compound **33-6** (3 g, 9.0 mmol) was added to a 500 mL single-necked flask, and dichloromethane (150 mL) and TFA (30 mL) were added sequentially. The reaction system was reacted at 25°C for 12 h under a nitrogen atmosphere. After completion of the reaction as monitored by LCMS, the reaction liquid was concentrated under reduced pressure at a low temperature, and the crude product was purified by column chromatography (acetonitrile/water) to give intermediate INT-1 (2.3 g, yield: 91.1%).

LCMS (ESI) M/Z: 298.2 [M+H⁺].

¹H NMR (400 MHz, DMSO) δ 12.50 (s, 1H), 8.38 (d, *J* = 7.7 Hz, 1H), 8.21 (d, *J* = 7.3 Hz, 1H), 7.09 (s, 2H), 4.32 (p, *J* = 7.1 Hz, 1H), 4.18 (p, *J* = 7.3 Hz, 1H), 4.13-3.99 (m, 2H), 1.27 (d, *J* = 7.3 Hz, 3H), 1.20 (d, *J* = 7.0 Hz, 3H).

**Step E:** Compound **5** (30 mg, 0.04 mmol) was added to a 50 mL single-necked flask, and dry N,N-dimethylformamide (2 mL) was added. Intermediate INT-1 (15 mg, 0.04 mmol) and 2-ethoxy-1-ethoxycarbonyl-1,2-dihydroquinoline (25 mg, 0.06 mmol) were then added sequentially, and the reaction system was reacted at room temperature for 2 h under a nitrogen atmosphere. After completion of the reaction as monitored by LCMS, the reaction liquid was purified by column chromatography to give compound **33** (2.18 mg, yield: 20.51%).

LCMS (ESI) M/Z: 1034.7 [M+H⁺].

¹H NMR (400 MHz, DMSO) δ 9.90-9.76 (m, 1H), 9.58 (s, 1H), 8.91 (s, 1H), 8.48-8.37 (m, 1H), 8.25-8.10 (m, 1H), 7.54-7.37 (m, 2H), 7.27-7.17 (m, 1H), 7.09 (s, 2H), 6.98-6.86 (m, 1H), 5.87-5.61 (m, 1H), 5.24-5.03 (m, 2H), 4.78-4.55 (m, 2H), 4.44-4.26 (m, 2H), 4.13-3.70 (m, 8H), 3.59-3.47 (m, 3H), 3.33-3.28 (m, 4H), 3.22-3.09 (m, 6H), 3.00 (s, 1H), 2.80-2.65 (m, 9H), 2.47-2.41 (m, 1H), 2.35-2.23 (m, 2H), 2.04-1.63 (m, 7H), 1.32-1.28 (m, 3H), 1.23-1.20 (m, 3H), 1.11-1.05 (m, 2H), 0.98-0.84 (m, 16H), 0.80-0.75 (m, 3H).

### Example 34

### Preparation of N-((S)-1-(((S)-1-((3-(2-((2R,3R)-N-allyl-3-((S)-1-((3R,4S,5S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)ethyl)phenyl)amino)-1-oxopropan-2-yl)amino)-1-oxopropan-2-yl)-6-(2-(methylsulfonyl)pyrimidin-5-yl)hex-5-ynamide.

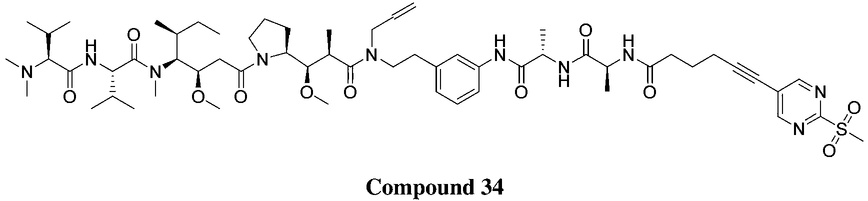

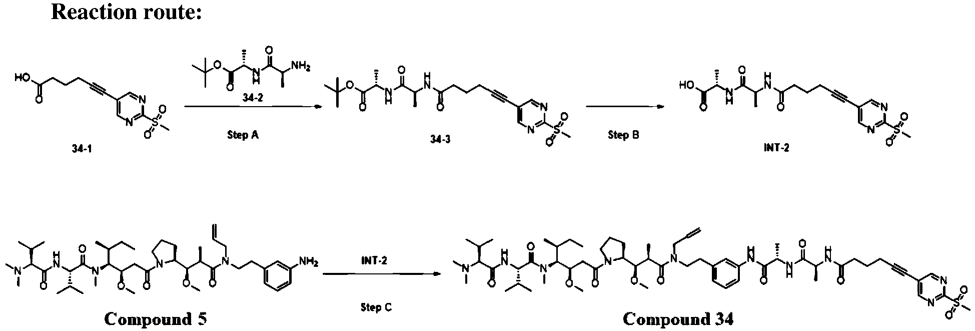

### Procedures:

Step A: Compound **34-1** (1.5 g, 5.60 mmol) was dissolved in dry N,N-dimethylformamide (20 mL), and HATU (2.34 g, 6.15 mmol), compound **34-2** (1.66 g, 5.60 mmol), and N,N-diisopropylethylamine (1.52 g, 11.74 mmol) were then added sequentially. The reaction system was reacted at room temperature for 2 h under a nitrogen atmosphere. After completion of the reaction as monitored by LCMS, the reaction liquid was purified by column chromatography (acetonitrile/0.1% aqueous trifluoroacetic acid solution) to give compound **34-3** (2.4 g, yield: 92.01%).

LCMS (ESI) M/Z: 489.0 [M+H⁺].

**Step B:** Compound **34-3** (500 mg, 1.07 mmol) was dissolved in dichloromethane (50 mL), and TFA (15 mL) was then added dropwise. The reaction system was stirred for 3 h under an argon atmosphere. After completion of the reaction as monitored by LCMS, the reaction liquid was concentrated, and the crude product was slurried with diethyl ether to give intermediate INT-2 (400 mg, yield: 90.94%).

LCMS (ESI) M/Z: 411.1 [M+H⁺].

¹H NMR (400 MHz, DMSO) δ 9.12 (s, 2H), 8.14 (d, *J =* 7.3 Hz, 1H), 8.03 (d, *J =* 7.7 Hz, 1H), 4.46-4.26 (m, 1H), 4.18 (t, *J =* 7.3 Hz, 1H), 3.41 (s, 3H), 2.56 (t, *J =* 7.1 Hz, 2H), 2.30 (t, *J =* 7.3 Hz, 2H), 1.86-1.73 (m, 2H), 1.27 (d, *J =* 7.3 Hz, 3H), 1.19 (d, *J =* 7.1 Hz, 3H).

**Step C:** Compound **5** (200 mg, 0.26 mmol) was added to a 25 mL single-necked flask, and dry dichloromethane (15 mL) was added. Intermediate INT-2 (113.12 mg, 0.28 mmol) and 2-ethoxy-1-ethoxycarbonyl-1,2-dihydroquinoline (60 mg, 0.34 mmol) were then added sequentially, and the reaction system was reacted at room temperature for 20 min under a nitrogen atmosphere. After completion of the reaction as monitored by LCMS, the mixture was concentrated by rotary evaporation under reduced pressure at a low temperature to remove the solvent. The resulting residue was purified by column chromatography (acetonitrile/0.1% aqueous trifluoroacetic acid solution) to give compound **34** (30 mg, yield: 32.25%).

LCMS (ESI) M/Z: 1149.8 [M+H⁺].

¹H NMR (400 MHz, DMSO) δ 9.91-9.65 (m, 1H), 9.57 (s, 1H), 9.15-9.04 (m, 2H), 8.97-8.83 (m, 1H), 8.39-8.07 (m, 2H), 7.58-7.40 (m, 2H), 7.27-7.14 (m, 1H), 6.97-6.86 (m, 1H), 5.86-5.64 (m, 1H), 5.21-5.03 (m, 2H), 4.78-4.54 (m, 2H), 4.42-4.23 (m, 2H), 4.04-3.91 (m, 3H), 3.90-3.81 (m, 3H), 3.79-3.75 (m, 2H), 3.56-3.45 (m, 3H), 3.41 (s, 3H), 3.35-3.25 (m, 4H), 3.21-3.15 (m, 4H), 3.11-2.99 (m, 2H), 2.82-2.70 (m, 8H), 2.59-2.55 (m, 2H), 2.38-2.22 (m, 4H), 2.05-1.98 (m, 1H), 1.93-1.76 (m, 5H), 1.73-1.59 (m, 2H), 1.32-1.28 (m, 3H), 1.23-1.18 (m, 3H), 1.09-1.04 (m, 2H), 0.99-0.74 (m, 21H).

### Example 36

### Preparation of (S)-N-((3R,4S,SS)-1-((S)-2-((1R,2R)-3-(allyl(3-((S)-2-((S)-2-(3-((2-(methylsulfonyl)pyrimidine)-5-sulfonamido)propanamido)propanamido)propanamido)phenethyl)amino)-1-methoxy-2-methyl-3-oxopropyl)pyrrolidin-1-yl)-3-methoxy-5-methyl-1-oxoheptan-4-yl)-2-((S)-2-(dimethylamino)-3-methylbutanamido)-N,3-dimethylbutanamide.

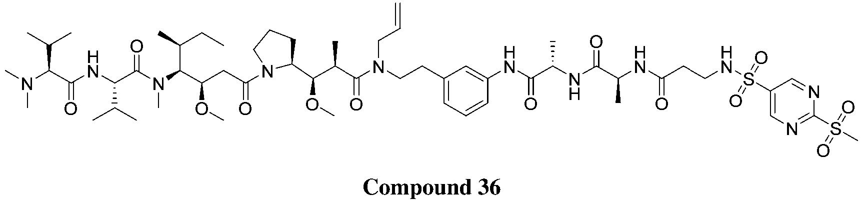

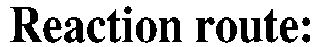

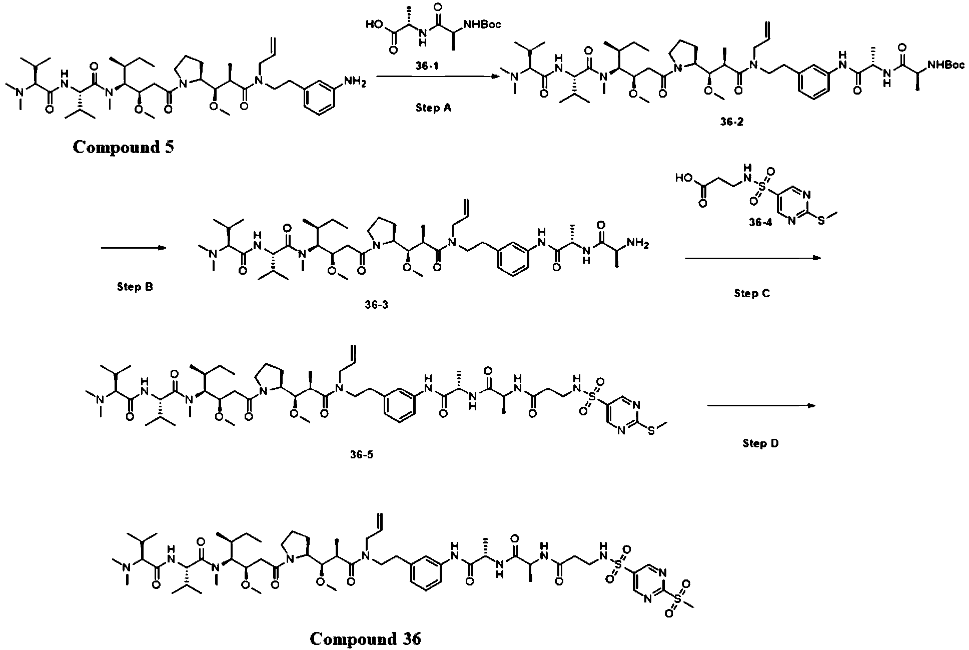

### Procedures:

**Step A:** Compound **5** (78 mg, 0.10 mmol) was added to a 25 mL single-necked flask, and dry dichloromethane (2 mL) was added. Compound **36-1** (30 mg, 0.12 mmol) and 2-ethoxy-1-ethoxycarbonyl-1,2-dihydroquinoline (31 mg, 0.13 mmol) were **then** added sequentially, and the reaction system was reacted at room temperature for 2 h under a nitrogen atmosphere. After completion of the reaction as monitored by LCMS, the reaction liquid was purified by column chromatography (dichloromethane:methanol = 10:1) to give compound **36-2** (100 mg, yield: 97%).

LCMS (ESI) M/Z: 999.7 [M+H⁺].

**Step B:** Compound **36-2** (60 mg, 0.06 mmol) was added to a 25 mL single-necked flask, and dichloromethane (5 mL) and trifluoroacetic acid (2.5 mL) were added. The mixture was stirred at room temperature for 1 h. After completion of the reaction as monitored by LCMS, the pH of the reaction liquid was adjusted to about 8 with ammonium bicarbonate. The organic phase was dried over anhydrous sodium sulfate and concentrated to dryness by rotary evaporation to give compound **36-3** (50 mg, yield: 92.63%).

LCMS (ESI) M/Z: 899.5 [M+H⁺].

**Step C:** Compound **36-3** (50 mg, 0.067 mmol) was added to a 25 mL single-necked flask, and dry dichloromethane (5 mL) was added. Compound **36-4** (37 mg, 0.134 mmol), 1-(3-dimethylaminopropyl)-3-ethylcarbodiimide hydrochloride (25 mg, 0.131 mmol), and N,N-diisopropylethylamine (25 mg, 0.194 mmol) were then added sequentially, and the reaction system was reacted at room temperature for 1 h under a nitrogen atmosphere. After completion of the reaction as monitored by LCMS, the reaction liquid was purified by column chromatography (dichloromethane:methanol = 10:1) to give compound **36-5** (40 mg, yield: 62.10%).

LCMS (ESI) M/Z: 1158.5 [M+H⁺].

**Step D:** Compound **36-5** (15 mg, 0.013 mmol) was added to a 25 mL single-necked flask, and dry 2-methyltetrahydrofuran (2 mL) and water (0.5 mL) were added, followed by the addition of potassium peroxymonosulfate (11 mg, 0.032 mmol). The reaction system was reacted at room temperature for 1 h under a nitrogen atmosphere. After completion of the reaction as monitored by LCMS, the reaction liquid was purified by column chromatography (dichloromethane:methanol = 10:1) to give compound **36** (7 mg, yield: 62.10%).

LCMS (ESI) M/Z: 1188.5 [M+H⁺].

¹H NMR (400 MHz, DMSO) δ 9.92-9.72 (m, 1H), 9.53 (s, 1H), 9.39 (s, 2H), 8.90 (s, 1H), 8.34 (s, 1H), 8.30-8.05 (m, 2H), 7.61-7.35 (m, 2H), 7.26-7.16 (m, 1H), 7.00-6.83 (m, 1H), 5.94-5.59 (m, 1H), 5.20-5.00 (m, 2H), 4.76-4.55 (m, 2H), 4.42-4.20 (m, 3H), 4.05-3.92 (m, 3H), 3.89-3.77 (m, 3H), 3.75-3.68 (m, 2H), 3.65-3.51 (m, 7H), 3.20-3.10 (m, 6H), 3.00 (s, 2H), 2.84-2.67 (m, 8H), 2.40-2.21 (m, 4H), 2.05-1.83 (m, 5H), 1.72-1.60 (m, 2H), 1.51-1.43 (m, 1H), 1.35-1.27 (m, 5H), 1.21-1.16 (m, 3H), 1.12-1.03 (m, 3H), 0.97-0.83 (m, 15H), 0.81-0.74 (m, 3H).

### Example 37

### Preparation of (S)-2-((S)-2-(dimethylamino)-3-methylbutanamido)-N-((3R,4S,SS)-1-((S)-2-((1R,2R)-3-((3-((S)-2-((S)-2-(2-(2,5-dioxo-2,5-dihydro-1H-pyrrol-1-yl)acetamido)propanamido)propanamido)phenethyl)(prop-2-yn-1-yl)amino)-1-methoxy-2-methyl-3-oxopropyl)pyrrolidin-1-yl)-3-methoxy-5-methyl-1-oxoheptan-4-yl)-N,3-dimethylbutanamide.

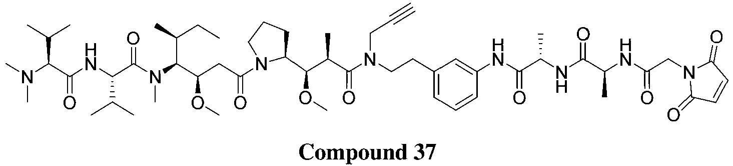

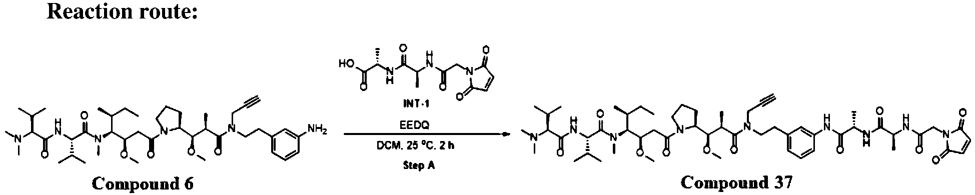

### Procedures:

**Step A:** Compound **6** (1 g, 1.32 mmol) was dissolved in dry N,N-dimethylformamide (30 mL), and **intermediate INT-1** (394 mg, 1.32 mmol) and 2-ethoxy-1-ethoxycarbonyl-1,2-dihydroquinoline (489 mg, 1.98 mmol) were then added sequentially. The reaction system was reacted at room temperature for 2 h under a nitrogen atmosphere. After completion of the reaction as monitored by LCMS, the reaction liquid was directly purified by column chromatography (acetonitrile/0.1% aqueous trifluoroacetic acid solution) to give compound **37** (210 mg, yield: 15.4%).

LCMS (ESI) M/Z: 1032.6 [M+H⁺].

¹H NMR (400 MHz, DMSO) δ 10.01-9.77 (m, 1H), 9.56 (s, 1H), 8.90 (d, *J =* 5.1 Hz, 1H), 8.44 (d, *J* = 6.3 Hz, 1H), 8.17 (d, *J =* 6.9 Hz, 1H), 7.65-7.35 (m, 2H), 7.32-7.16 (m, 1H), 7.11 (s, 2H), 7.03-6.86 (m, 1H), 4.77-4.48 (m, 4H), 4.41-4.23 (m, 4H), 4.19-3.83 (m, 12H), 3.73-3.69 (m, 2H), 3.22-3.09 (m, 5H), 3.04-2.92 (m, 2H), 2.89-2.69 (m, 8H), 2.36-2.25 (m, 1H), 2.16-1.53 (m, 6H), 1.31 (d, *J =* 6.5 Hz, 3H), 1.26-1.07 (m, 5H), 1.00-0.82 (m, 18H), 0.80-0.74 (m, 3H).

### Example 38

### Preparation of N-((S)-1-(((S)-1-((3-(2-((2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methyl-N-(prop-2-yn-1-yl)propanamido)ethyl)phenyl)amino)-1-oxopropan-2-yl)amino)-1-oxopropan-2-yl)-6-(2-(methylsulfonyl)pyrimidin-5-yl)hex-5-ynamide.

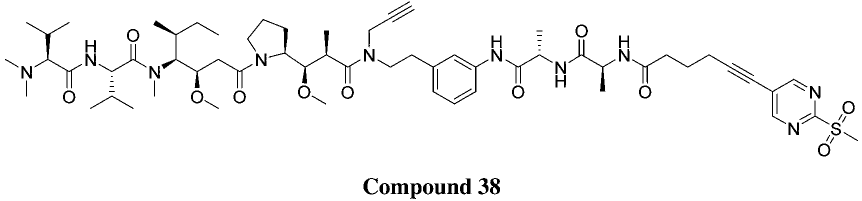

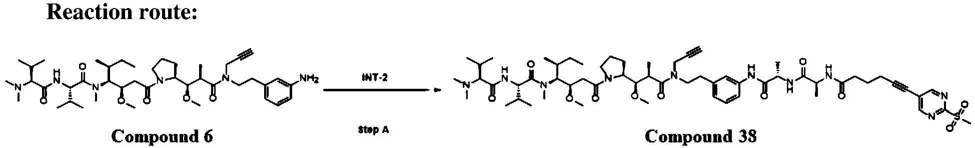

### Procedures:

**Step A:** Compound **6** (600 mg, 0.794 mmol) was dissolved in dry dichloromethane (30 mL), and intermediate INT-2 (113.12 mg, 0.794 mmol) and 2-ethoxy-1-ethoxycarbonyl-1,2-dihydroquinoline (294 mg, 1.19 mmol) were then added sequentially. The reaction system was reacted at room temperature for 30 min under a nitrogen atmosphere. After completion of the reaction as monitored by LCMS, the mixture was concentrated by rotary evaporation under reduced pressure at a low temperature to remove the solvent. The residue was purified by column chromatography (acetonitrile/0.1% aqueous trifluoroacetic acid solution) to give compound **38** (230 mg, yield: 25.40%).

LCMS (ESI) M/Z: 1147.5 [M+H⁺].

¹H NMR (400 MHz, DMSO) δ 9.90-9.80 (m, 1H), 9.60 (s, 1H), 9.11 (s, 2H), 8.91 (d, *J =* 7.6 Hz, 1H), 8.11 (d, *J =* 5.1 Hz, 2H), 7.60-7.40 (m, 2H), 7.28-7.16 (m, 1H), 7.01-6.86 (m, 1H), 4.77-4.62 (m, 1H), 4.61-4.53 (m, 1H), 4.43-4.36 (m, 1H), 4.34-4.27 (m, 1H), 4.23-4.08 (m, 3H), 4.06-3.88 (m, 4H), 3.84-3.67 (m, 3H), 3.64-3.45 (m, 3H), 3.41 (s, 3H), 3.37-3.33 (m, 1H), 3.30-3.25 (m, 1H), 3.20-3.10 (m, 5H), 3.00 (s, 2H), 2.86-2.70 (m, 8H), 2.61-2.53 (m, 2H), 2.47-2.41 (m, 1H), 2.37-2.23 (m, 4H), 2.05-1.90 (m, 2H), 1.88-1.79 (m, 3H), 1.75-1.58 (m, 2H), 1.30 (d, *J =* 6.9 Hz, 3H), 1.22 (d, *J =* 7.0 Hz, 3H), 1.11 (dd, *J =* 12.9, 6.6 Hz, 1H), 1.04-0.73 (m, 22H).

### Example 40

### Preparation of (S)-2-((S)-2-(dimethylamino)-3-methylbutanamido)-N-((3R,4S,SS)-1-((S)-2-((1R,2R)-3-((3-((S)-2-((S)-2-(2-(2,5-dioxo-2,5-dihydro-1H-pyrrol-1-yl)acetamido)propanamido)propanamido)phenethyl)(methoxy)amino)-1-methoxy-2-methyl-3-oxopropyl)pyrrolidin-1-yl)-3-methoxy-5-methyl-1-oxoheptan-4-yl)-N,3-dimethylbutanamide.

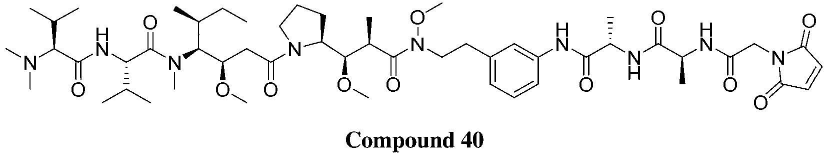

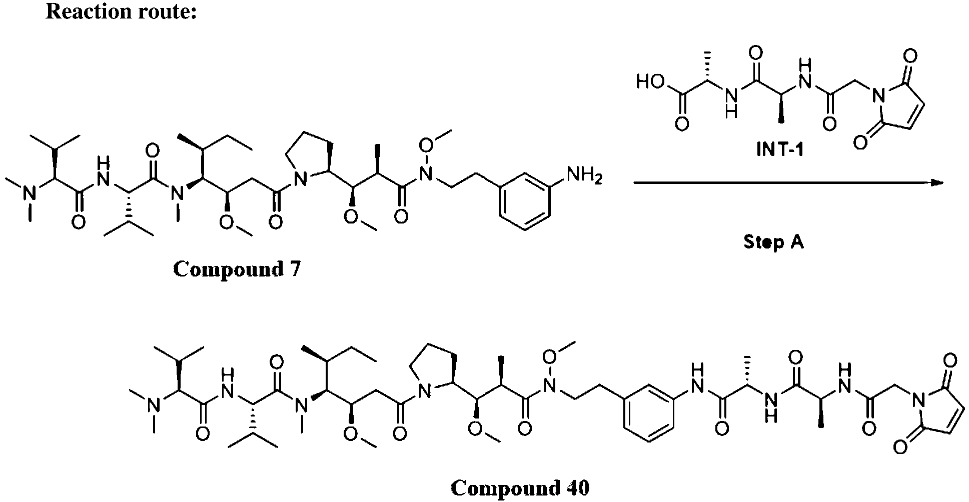

### Procedures:

**Step A:** Compound 7 (500 mg, 67 mmol) was added to a 50 mL single-necked flask, and dry N,N-dimethylformamide (10 mL) was added. **Intermediate INT-1** (211.11 mg, 0.71 mmol), HATU (331.18 mg, 0.87 mmol), and 2-6-lutidine (215.37 mg, 2.01 mmol) were then added sequentially, and the reaction system was reacted at room temperature for 15 min under a nitrogen atmosphere. After completion of the reaction as monitored by LCMS, the reaction liquid was purified by column chromatography (tetrahydrofuran/0.1% aqueous trifluoroacetic acid solution) to give compound **40** (470 mg, yield: 68.41%).

LCMS (ESI) M/Z: 1026.7 [M+H⁺].

¹H NMR (400 MHz, DMSO) δ 9.81 (s, 1H), 9.67 (s, 1H), 8.93 (d, *J =* 7.8 Hz, 1H), 8.45 (d, *J =* 7.2 Hz, 1H), 8.22-8.12 (m, 1H), 7.49 (s, 1H), 7.41 (t, *J* = 6.8 Hz, 1H), 7.19 (t, *J* = 7.8 Hz, 1H), 7.09 (s, 2H), 6.92 (d, *J* = 7.3 Hz, 1H), 4.76-4.54 (m, 2H), 4.43-4.27 (m, 2H), 4.15-4.04 (m, 2H), 3.99 (dd, *J* = 2.2, 1.6 Hz, 1H), 3.94-3.82 (m, 2H), 3.77-3.68 (m, 2H), 3.66-3.59 (m, 4H), 3.53-3.45 (m, 1H), 3.29 (d, *J =* 6.2 Hz, 1H), 3.24 (s, 2H), 3.19 (d, *J =* 2.3 Hz, 3H), 3.13 (s, 1H), 3.01 (s, 2H), 2.78 (s, 9H), 2.49-2.42 (m, 1H), 2.39-2.20 (m, 2H), 2.06-1.96 (m, 1H), 1.95-1.79 (m, 3H), 1.79-1.55 (m, 3H), 1.30 (dd, *J =* 6.9, 3.9 Hz, 4H), 1.22 (d, *J =* 7.0 Hz, 3H), 1.14-1.04 (m, 3H), 0.98-0.84 (m, 16H), 0.81-0.74 (m, 3H).

### Example 41

### Preparation of N-((S)-1-(((S)-1-((3-(2-((2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl)pyrrolidin-2-yl)-N,3-dimethoxy-2-methylpropanamido)ethyl)phenyl)amino)-1-oxopropan-2-yl)amino)-1-oxopropan-2-yl)-6-(2-(methylsulfonyl)pyrimidin-5-yl)hex-5-ynamide.

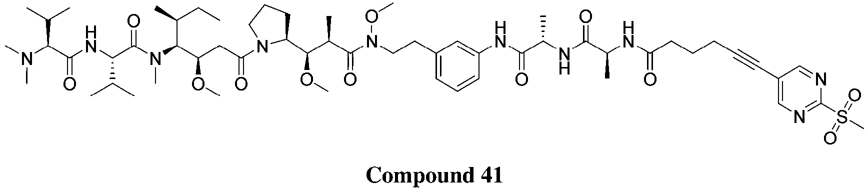

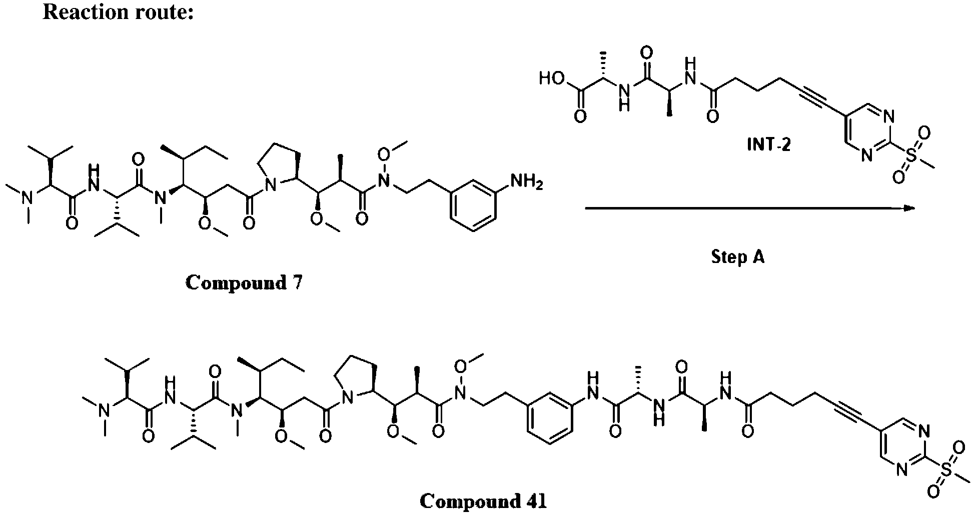

### Procedures:

**Step A:** Compound 7 (600 mg, 0.80 mmol) was added to a 25 mL single-necked flask, and dry N,N-dimethylformamide (12 mL) was added. Intermediate INT-2 (350 mg, 0.82 mmol), HATU (395.44 mg, 1.04 mmol), and 2,6-lutidine (257.16 mg, 2.4 mmol) were then added sequentially, and the reaction system was reacted at room temperature for 15 min under a nitrogen atmosphere. After completion of the reaction as monitored by LCMS, the mixture was purified by column chromatography (tetrahydrofuran/0.1% aqueous trifluoroacetic acid solution) to give compound 41 (780 mg, yield: 85.23%).

LCMS (ESI) M/Z: 1139.8 [M+H⁺].

¹H NMR (400 MHz, DMSO) δ 9.82 (s, 1H), 9.66 (s, 1H), 9.14-9.07 (m, 2H), 8.93 (d, *J =* 7.8 Hz, 1H), 8.12 (t, *J =* 5.6 Hz, 2H), 7.46 (dd, *J =* 19.7, 11.3 Hz, 2H), 7.19 (t*, J* = 7.8 Hz, 1H), 6.91 (d, *J =* 7.4 Hz, 1H), 4.76-4.52 (m, 2H), 4.44-4.24 (m, 2H), 4.06-3.94 (m, 1H), 3.90-3.84 (m, 1H), 3.80-3.68 (m, 2H), 3.60 (dd, *J =* 22.7, 9.5 Hz, 4H), 3.53-3.43 (m, 1H), 3.41 (s, 3H), 3.37-3.27 (m, 2H), 3.24 (s, 2H), 3.21-3.05 (m, 5H), 3.01 (s, 2H), 2.79 (t, *J =* 13.8 Hz, 9H), 2.57 (t, *J =* 7.1 Hz, 2H), 2.46 (d, *J =* 8.2 Hz, 1H), 2.39-2.21 (m, 4H), 2.09-1.96 (m, 1H), 1.92-1.57 (m, 7H), 1.30 (dd, *J* = 7.0, 4.2 Hz, 4H), 1.22 (dd, *J =* 7.1, 2.3 Hz, 3H), 1.07 (dd, *J =* 11.7, 6.6 Hz, 3H), 0.99-0.83 (m, 16H), 0.81-0.74 (m, 3H).

### Example 42

### Preparation of (S)-1-cyano-3-(3-((S)-2-((S)-2-(2-(2,5-dioxo-2,5-dihydro-1H-pyrrol-1-yl)acetamido)propanamido)propanamido)phenyl)propan-2-yl (2R,3R)-3-((S)-1-((3R,4S,SS)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanoate.

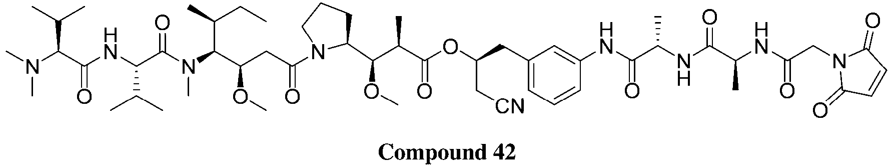

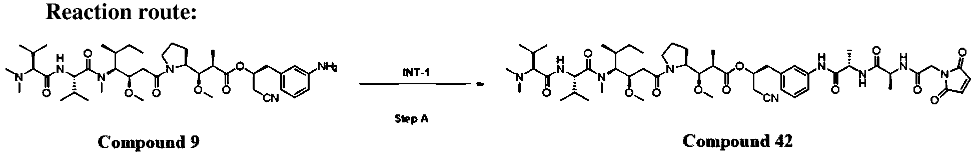

**Step A:** Compound **9** (40 mg, 0.04 mmol) was added to a 25 mL single-necked flask, and dry dichloromethane (2.5 mL) was added. **Intermediate INT-1** (12 mg, 0.04 mmol) and 2-ethoxy-1-ethoxycarbonyl-1,2-dihydroquinoline (12.35 mg, 0.05 mmol) were then added sequentially, and the reaction system was reacted at room temperature for 15 min under a nitrogen atmosphere. After completion of the reaction as monitored by LCMS, the reaction liquid was purified by column chromatography (acetonitrile/0.1% aqueous trifluoroacetic acid solution) to give compound **42** (9 mg, yield: 16.4%).

LCMS (ESI) M/Z: 1034.5 [M+H⁺].

¹H NMR (400 MHz, DMSO) δ 9.98-9.80 (m, 1H), 8.48-8.33 (m, 1H), 8.27-8.18 (m, 1H), 8.09-7.95 (m, 1H), 7.59-7.37 (m, 2H), 7.29-7.14 (m, 1H), 7.08 (s, 2H), 6.91 (d, *J =* 7.1 Hz, 1H), 5.27-5.07 (m, 1H), 4.71-4.55 (m, 1H), 4.51 (t, *J =* 8.6 Hz, 1H), 4.43-4.24 (m, 2H), 4.15-3.79 (m, 7H), 3.29-3.22 (m, 3H), 3.19-3.15 (m, 3H), 3.05-2.78 (m, 8H), 2.70-2.56 (m, 2H), 2.46-2.28 (m, 3H), 2.21-2.14 (m, 6H), 2.00-1.66 (m, 7H), 1.31-1.26 (m, 3H), 1.24-1.17 (m, 3H), 1.05-0.96 (m, 3H), 0.94-0.85 (m, 12H), 0.79-0.67 (m, 6H).

### Example 43

### Preparation of (R)-1-(3-((S)-2-((S)-2-(2-(2,5-dioxo-2,5-dihydro-1H-pyrrol-1-yl)acetamido)propanamido)propanamido)phenyl)propan-2-yl (2R,3R)-3-((S)-1-((3R,4S,SS)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanoate.

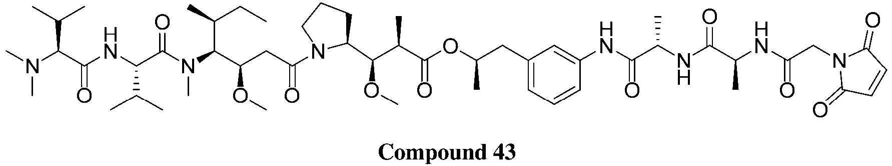

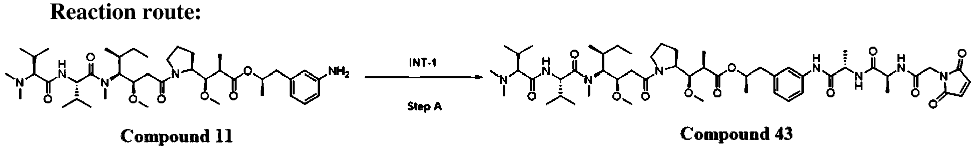

### Procedures:

### Step A:

Compound **11** (20 mg, 0.03 mmol) was added to a 50 mL single-necked flask, and dry N,N-dimethylformamide (2 mL) was added. **Intermediate INT-1** (15 mg, 0.04 mmol) and 2-ethoxy-1-ethoxycarbonyl-1,2-dihydroquinoline (25 mg, 0.06 mmol) were then added sequentially, and the reaction system was reacted at room temperature for 2 h under a nitrogen atmosphere. After completion of the reaction as monitored by LCMS, the reaction liquid was purified by column chromatography (tetrahydrofuran/0.1% aqueous trifluoroacetic acid solution) to give compound **43** (2.12 mg, yield: 35.31%).

LCMS (ESI) M/Z: 1011.5 [M+H⁺].

¹H NMR (400 MHz, DMSO) δ 9.81 (s, 1H), 9.53 (s, 1H), 8.92 (s, 1H), 8.43 (d, *J =* 7.2 Hz, 1H), 8.16 (d, *J* = 7.2 Hz, 1H), 7.51-7.39 (m, 2H), 7.22-7.15 (m, 1H), 7.09 (s, 2H), 6.91 (d, *J =* 7.4 Hz, 1H), 5.08-4.93 (m, 1H), 4.68-4.54 (m, 2H), 4.41-4.28 (m, 2H), 4.15-3.90 (m, 4H), 3.78-3.67 (m, 3H), 3.52-3.48 (m, 1H), 3.23-3.17 (m, 3H), 3.14-3.13 (m, 2H), 3.07-2.99 (m, 3H), 2.81-2.73 (m, 8H), 2.45-2.26 (m, 5H), 2.17-1.88 (m, 2H), 1.88-1.49 (m, 6H), 1.31-1.28 (m, 3H), 1.23-1.18 (m, 6H), 1.07-1.01 (m, 3H), 0.96-0.88 (m, 12H), 0.86-0.82 (m, 3H), 0.79-0.74 (m, 3H).

### Example 44

### Preparation of (S)-1-(3-((S)-2-((S)-2-(2-(2,5-dioxo-2,5-dihydro-1H-pyrrol-1-yl)acetamido)propanamido)propanamido)phenyl)propan-2-yl (2R,3R)-3-((S)-1-((3R,4S,SS)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanoate.

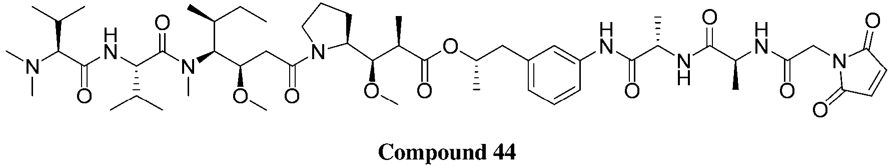

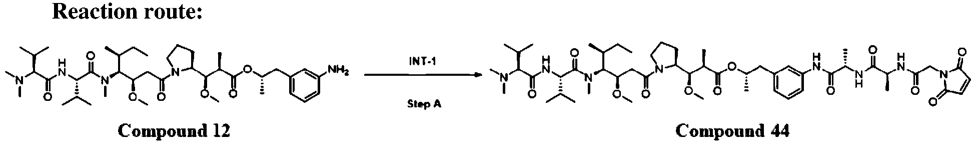

### Procedures:

### Step A:

Compound **12** (20 mg, 0.03 mmol) was added to a 50 mL single-necked flask, and dry N,N-dimethylformamide (2 mL) was added. **Intermediate INT-1** (15 mg, 0.04 mmol) and 2-ethoxy-1-ethoxycarbonyl-1,2-dihydroquinoline (25 mg, 0.06 mmol) were then added sequentially, and the reaction system was reacted at room temperature for 2 h under a nitrogen atmosphere. After completion of the reaction as monitored by LCMS, the reaction liquid was purified by column chromatography (tetrahydrofuran/0.1% aqueous trifluoroacetic acid solution) to give compound **44** (2.39 mg, yield: 35.37%).

LCMS (ESI) M/Z: 1011.5 [M+H⁺].

¹H NMR (400 MHz, DMSO) δ 9.83 (s, 1H), 8.43 (d, J = 7.3 Hz, 1H), 8.18 (d, J = 6.8 Hz, 1H), 8.03 (d, J = 8.1 Hz, 1H), 7.56-7.37 (m, 2H), 7.20 (t, J = 7.9 Hz, 1H), 7.10 (s, 2H), 6.91 (d, J = 7.4 Hz, 1H), 5.08-4.93 (m, 1H), 4.69-4.49 (m, 2H), 4.41-4.30 (m, 2H), 4.09 (d, J = 4.1 Hz, 2H), 4.02-3.75 (m, 4H), 3.54-3.47 (m, 2H), 3.25 (s, 3H), 3.19 (s, 2H), 3.03 (d, J = 19.8 Hz, 3H), 2.84-2.75 (m, 2H), 2.64 (d, J = 10.0 Hz, 1H), 2.47-2.29 (m, 4H), 2.20 (d, J = 6.1 Hz, 6H), 1.98-1.67 (m, 8H), 1.31 (d, J = 7.0 Hz, 3H), 1.22 (d, J = 7.0 Hz, 3H), 1.15 (d, J = 6.1 Hz, 3H), 1.03 (d, J = 6.8 Hz, 3H), 0.93-0.86 (m, 12H), 0.78-0.69 (m, 6H).

### Example 45

### Preparation of (S)-N-((3R,4S,5S)-1-((S)-2-((1R,2R)-3-((3-aminophenethyl)(cyanomethyl)amino)-1-methoxy-2-methyl-3-oxopropyl)pyrrolidin-1-yl)-3-methoxy-5-methyl-1-oxoheptan-4-yl)-2-((S)-2-(dimethylamino)-3-methylbutanamido)-N,3-dimethylbutanamide.

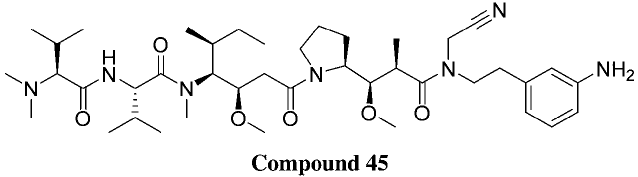

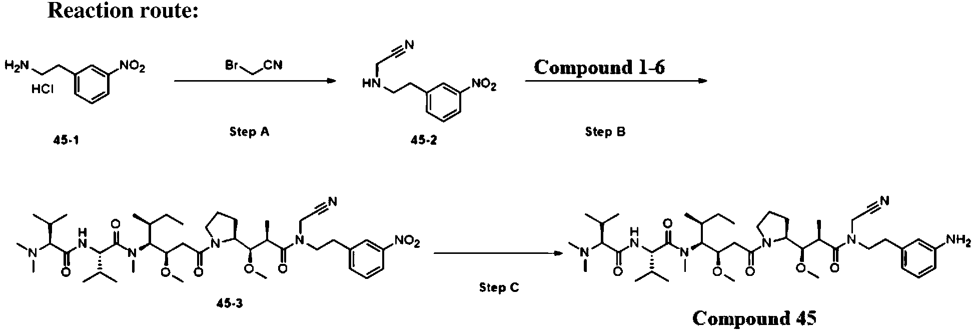

### Procedures:

**Step A:** Compound **45-1** (500 mg, 2.48 mmol) and N,N-diisopropylethylamine (960 mg, 7.44 mmol) were dissolved in acetonitrile (10 mL). The mixture was stirred at room temperature for 10 min, and bromoacetonitrile (320 mg, 2.69 mmol) was slowly added dropwise. The mixture was then stirred at room temperature for another 1 h. After completion of the reaction as indicated by LCMS, the reaction liquid was concentrated, and the crude product was purified by a reversed-phase column to give compound **45-2** (310 mg, yield: 61.29%).

LCMS (ESI) M/Z: 206.2 [M+H]⁺.

**Step B:** Compound **1-6** (883 mg, 1.48 mmol) was dissolved in anhydrous dichloromethane (10 mL), and oxalyl chloride (0.16 mL) and N,N-dimethylformamide (1 drop) were slowly added dropwise at 0°C. The resulting mixture was stirred at 0°C for 1 h, and the reaction system was then concentrated to dryness by rotary evaporation at 0°C. The residue was dissolved in 2 mL of dichloromethane for later use. Compound **45-2** (250 mg, 1.21 mmol) was dissolved in dichloromethane (10 mL), and triethylamine (0.37 mL) was added. The solution of acyl chloride in dichloromethane described above was slowly added dropwise at 0°C, and the mixture was then stirred at room temperature for 1 h. After completion of the reaction as indicated by LCMS, the reaction liquid was concentrated, and the crude product was purified by a reversed-phase column to give compound **45-3** (408 mg, yield: 42.77%).

LCMS (ESI) M/Z: 786.8 [M+H]⁺.

**Step C:** Tetrahydroxydiboron (158 mg, 1.78 mmol) was dissolved in anhydrous N,N-dimethylformamide (5 mL), and 4,4-bipyridine (35 mg, 0.22 mmol) and compound **45-3** (400 mg, 0.51 mmol) were added under a nitrogen atmosphere. The resulting mixture was stirred at room temperature for 5 min. After completion of the reaction as indicated by LCMS, the reaction liquid was purified by a reversed-phase column to give the target compound **45** (350 mg, yield: 85.76%).

LCMS (ESI) M/Z: 756.4 [M+H]⁺.

¹H NMR (400 MHz, DMSO) δ 8.18-8.00 (m, 1H), 6.99-6.88 (m, 1H), 6.52 (s, 1H), 6.46-6.34 (m, 3H), 5.01 (s, 1H), 4.73-4.25 (m, 4H), 4.00-3.91 (m, 1H), 3.80-3.41 (m, 5H), 3.28-3.24 (m, 2H), 3.21-3.09 (m, 5H), 2.99 (s, 2H), 2.79-2.60 (m, 4H), 2.46-2.40 (m, 1H), 2.33-2.19 (m, 6H), 2.01-1.56 (m, 8H), 1.35-1.28 (m, 1H), 1.18-1.07 (m, 1H), 1.00 (d, *J =* 6.6 Hz, 1H), 0.95-0.84 (m, 15H), 0.79-0.70 (m, 6H).

### Example 46

### Preparation of (S)-N-((3R,4S,55)-1-((S)-2-((1R,2R)-3-((cyanomethyl)(3-((S)-2-((S)-2-(2-(2,5-dioxo-2,5-dihydro-1H-pyrrol-1-yl)acetamido)propanamido)propanamido)phenethyl)amino)-1-methoxy-2-methyl-3-oxopropyl)pyrrolidin-1-yl)-3-methoxy-5-methyl-1-oxoheptan-4-yl)-2-((S)-2-(dimethylamino)-3-methylbutanamido)-N,3-dimethylbutanamide.

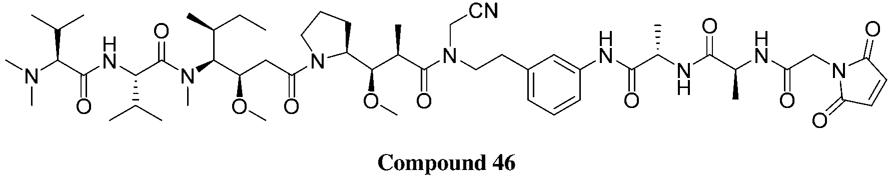

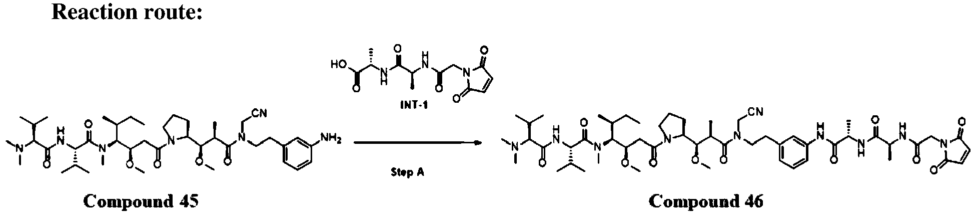

### Procedures:

**Step A:** Compound **45** (800 mg, 1.05 mmol) was added to a 25 mL single-necked flask, and dry dichloromethane (20 mL) was added. Intermediate INT-1 (350 mg, 1.1 mmol), HATU (500 mg, 1.3 mmol), and 2,6-lutidine (0.45 mL) were then added sequentially, and the reaction system was reacted at room temperature for 20 min under a nitrogen atmosphere. After the reaction was completed, the reaction liquid was concentrated under reduced pressure at a low temperature. The residue was purified by column chromatography to give compound **46** (900 mg, yield: 85.67%).

LCMS (ESI) M/Z: 1035.8 [M+H]⁺.

¹H NMR (400 MHz, DMSO) δ 9.93-9.79 (m, 1H), 9.60 (brs, 1H), 8.89 (brs, 1H), 8.43 (d, *J* = 7.3 Hz, 1H), 8.17 (d, *J =* 7.1 Hz, 1H), 7.57-7.36 (m, 2H), 7.29-7.18 (m, 1H), 7.09 (s, 2H), 7.03-6.88 (m, 1H), 4.80-4.50 (m, 3H), 4.49-4.25 (m, 4H), 4.17-3.92 (m, 4H), 3.79-3.68 (m, 2H), 3.59-3.49 (m, 2H), 3.31-3.25 (m, 3H), 3.22-3.10 (m, 5H), 2.99 (s, 2H), 2.90-2.70 (m, 9H), 2.36-2.19 (m, 2H), 2.06-1.84 (m, 4H), 1.81-1.60 (m, 3H), 1.30 (d, *J =* 6.7 Hz, 3H), 1.21 (d, *J* = 7.0 Hz, 3H), 1.11 (dd, *J =* 12.6, 6.2 Hz, 1H), 1.04-0.83 (m, 19H), 0.81-0.74 (m, 3H).

### Example 47

### Preparation of N-((S)-1-(((S)-1-((3-(2-((2R,3R)-N-(cyanomethyl)-3-((S)-1-((3R,4S,SS)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)ethyl)phenyl)amino)-1-oxopropan-2-yl)amino)-1-oxopropan-2-yl)-6-(2-(methylsulfonyl)pyrimidin-5-yl)hex-5-ynamide.

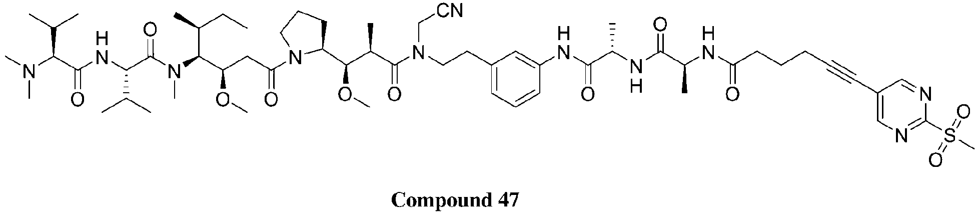

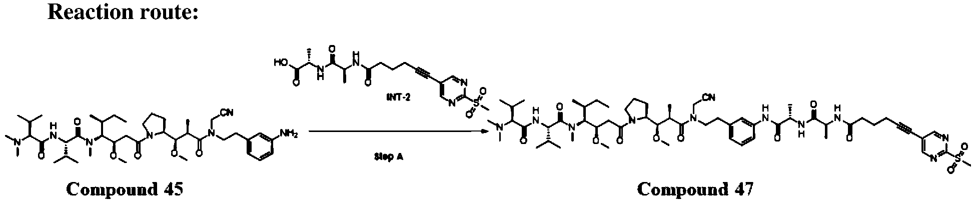

### Procedures:

**Step A:** Compound **45** (300 mg, 0.33 mmol) was added to a 25 mL single-necked flask, and dry dichloromethane (10 mL) was added. Intermediate INT-2 (150 mg, 0.35 mmol), HATU (195 mg, 0.42 mmol), and 2,6-lutidine (0.15 mL) were then added sequentially, and the reaction system was reacted at room temperature for 20 min under a nitrogen atmosphere. After the reaction was completed, the mixture was concentrated by rotary evaporation under reduced pressure at a low temperature to remove the solvent. The residue was dissolved in N,N-dimethylformamide and purified by column chromatography to give compound **47** (300 mg, yield: 76.75%).

LCMS (ESI) M/Z: 1148.6 [M+H⁺].

¹H NMR (400 MHz, DMSO) δ 9.90-9.82 (m, 1H), 9.12-9.09 (m, 2H), 8.17-7.97 (m, 4H), 7.56-7.44 (m, 2H), 7.29-7.20 (m, 1H), 7.03-6.91 (m, 1H), 4.75-4.55 (m, 2H), 4.54-4.47 (m, 1H), 4.46-4.28 (m, 4H), 4.07-3.87 (m, 2H), 3.78-3.75 (m, 1H), 3.69-3.59 (m, 1H), 3.59-3.44 (m, 3H), 3.34-3.30 (m, 4H), 3.27 (s, 2H), 3.20 (s, 1H), 3.17-3.15 (m, 3H), 3.12 (s, 1H), 2.99 (s, 2H), 2.90-2.61 (m, 5H), 2.59-2.55 (m, 2H), 2.48-2.40 (m, 1H), 2.34-2.30 (m, 2H), 2.22-2.20 (m, 2H), 2.19 (s, 3H), 1.96-1.77 (m, 7H), 1.74-1.64 (m, 2H), 1.32-1.29 (m, 3H), 1.21 (d, *J =* 7.1 Hz, 3H), 1.12 (dd, *J =* 13.8, 6.6 Hz, 1H), 1.01 (d, *J =* 6.7 Hz, 1H), 0.94-0.84 (m, 15H), 0.76-0.69 (m, 6H).

### Example 48

### Preparation of (S)-N-((3R,4S,55)-1-((S)-2-((1R,2R)-3-((R)-2-(3-aminophenyl)morpholino)-1-methoxy-2-methyl-3-oxopropyl)pyrrolidin-1-yl)-3-methoxy-5-methyl-1-oxoheptan-4-yl)-2-((S)-2-(dimethylamino)-3-methylbutanamido)-N,3-dimethylbutanamide.

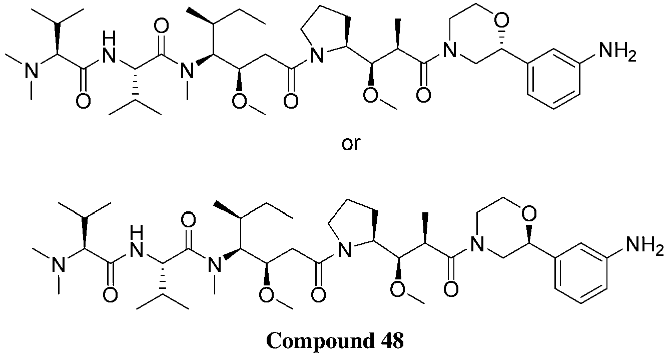

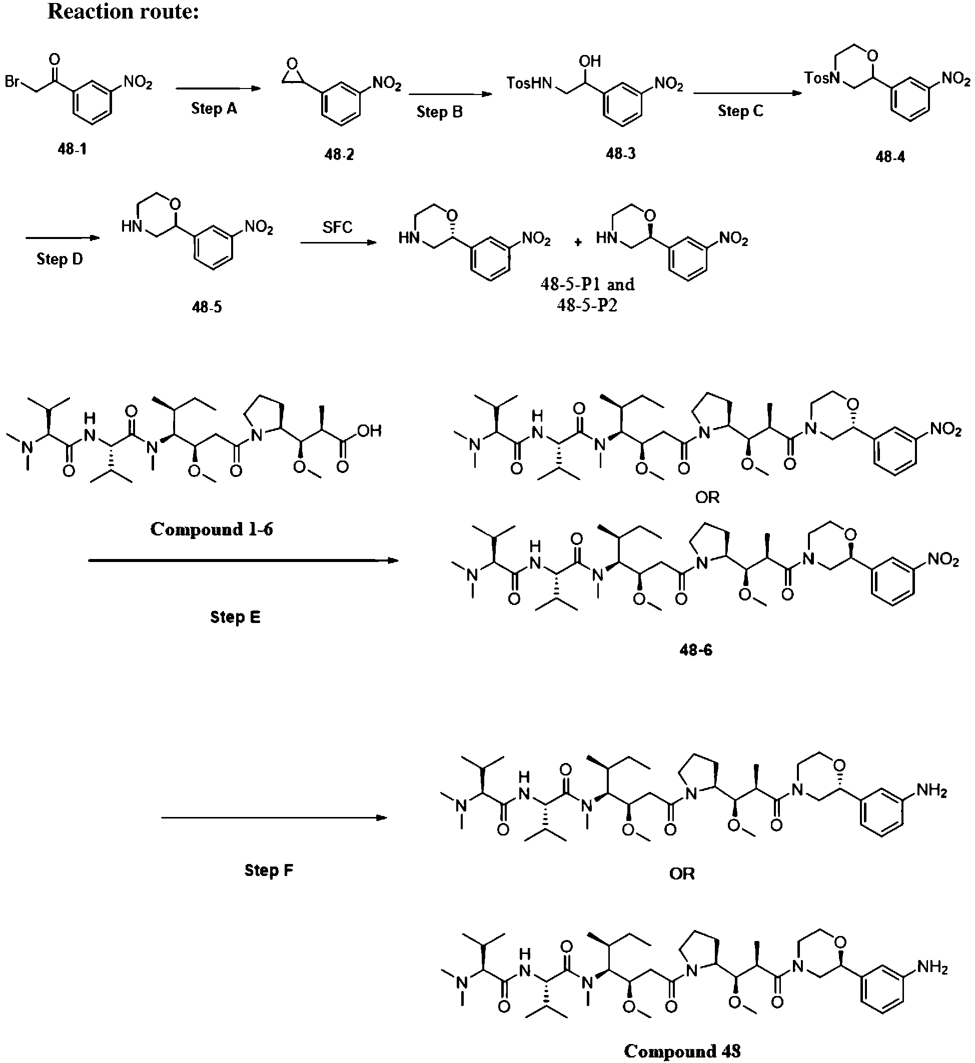

### Procedures:

**Step A:** Compound **48-1** (10 g, 41.15 mmol) was added to a 500 mL single-necked flask, and ethanol (200 mL) was added for dissolution. Sodium borohydride (1.51 g, 39.92 mmol) was then added, and the mixture was stirred at room temperature for 1 h. Subsequently, potassium hydroxide (2.3 g, 41.07 mmol) was added, and the mixture was stirred at room temperature for another 2 h. After the reaction was completed, ethanol was removed under reduced pressure, and water was added to the residue. The mixture was extracted with dichloromethane, and the organic phase was dried over anhydrous sodium sulfate, filtered, and concentrated. The crude product was purified by column chromatography (petroleum ether:ethyl acetate = 40:1) to give compound **48-2** (5 g, yield: 73.5%).

¹H NMR (400 MHz, CDCl₃) δ 8.19-8.02 (m, 2H), 7.61 (d, *J =* 7.7 Hz, 1H), 7.52 (t, *J* = 8.0 Hz, 1H), 4.05-3.77 (m, 1H), 3.36-3.09 (m, 1H), 2.98-2.57 (m, 1H).

**Step B:** Compound **48-2** (5.54 g, 33.55 mmol), TosNH₂ (11.49 g, 67.1 mmol), TEBA (760 mg, 3.35 mmol), and potassium carbonate (460 mg, 3.35 mmol) were added sequentially to a 250 mL single-necked flask, and 1,4-dioxane (70 mL) was added for dissolution. The mixture was warmed to 90°C and reacted for 4 h. After completion of the reaction as monitored by LCMS, the mixture was distilled under reduced pressure to remove 1,4-dioxane, and the residue was purified by column chromatography (acetonitrile/0.1% aqueous ammonium bicarbonate solution) to give compound **48-3** (6.93 g, yield: 61.42%).

LCMS (ESI) M/Z: 359.1 [M+H⁺].

**Step C:** Compound **48-3** (7.18 g, 5.95 mmol) was added to a single-necked flask, and anhydrous dichloromethane (270 mL) was added for dissolution. Sodium hydride (2.56 g, 64.05 mmol) was added in an ice bath. The mixture was stirred at this temperature for another half an hour, and (2-bromoethyl)diphenylsulfonium trifluoromethanesulfonate (14.20 g, 32.03 mmol) was then added. The system was warmed to room temperature and reacted for 2 h. After completion of the reaction as monitored by LCMS, water was added to quench the reaction, and extraction was performed with dichloromethane. The organic phase was dried over anhydrous sodium sulfate, filtered, and concentrated. The residue was purified by Flash (acetonitrile/0.1% aqueous formic acid solution) to give compound **48-4** (1.86 g, yield: 20.04%).

LCMS (ESI) M/Z: 385.1 [M+H⁺].

**Step D:** Compound **48-4** (2.6 g, 7.17 mmol) was added to a single-necked flask, and phenol (4.04 g, 43.03 mmol) and HBr (29 mL, 33% in AcOH) were then added. The mixture was reacted at 75°C for 2 h. After completion of the reaction as monitored by LCMS, the reaction liquid was purified by column chromatography (acetonitrile/0.1% aqueous formic acid solution) to give compound **48-8** (1.2 g, yield: 80%), which was then subjected to chiral resolution to give **48-5-P1** (550 mg) and **48-5-P2** (610 mg). Resolution conditions: mobile phase: supercritical carbon dioxide and a solution of 0.1% ammonia in ethanol; flow rate: 50 mL/min; gradient: maintaining 0.1% ammonia in isopropanol at 35%; detection wavelength: 214 nm; cycle time: 4.91 min.

The retention time (Rt) of compound **48-5-P1** = 6.27 min; the retention time (Rt) of compound **48-5-P2** = 8.84 min.

LCMS (ESI) M/Z: 209.1 [M+H⁺].

**Step E:** Compound **48-5-P1** (500 mg, 2.40 mmol) was added to a 50 mL single-necked flask, and dry N,N-dimethylformamide (20 mL) was added. Compound 1-6 (1.44 g, 2.4 mmol), HATU (1.37 g, 3.6 mmol), and N,N-diisopropylethylamine (1.19 mL, 7.2 mmol) were then added sequentially, and the reaction system was reacted at room temperature for 2 h under a nitrogen atmosphere. After the reaction was completed, the mixture was filtered, and the filtrate was purified by column chromatography (acetonitrile/0.1% aqueous formic acid solution) to give compound **48-6** (1.05 g, yield: 55.42%).

LCMS (ESI) M/Z: 789.5 [M+H⁺].

**Step F:** Tetrahydroxydiboron (408.8 mg, 4.56 mmol) was added to a 25 mL single-necked flask, and dry N,N-dimethylformamide (12 mL) was added. 4,4'-Bipyridine (178.05 mg, 1.14 mmol) and compound **48-6** (900 mg, 1.14 mmol) were then rapidly added sequentially, and the reaction system was reacted at room temperature for 5 min under a nitrogen atmosphere. After completion of the reaction as monitored by LCMS, a small amount of methanol was added to help dissolution, and the mixture was purified by column chromatography (acetonitrile/0.1% aqueous formic acid solution) to give compound **48** (650 mg, yield: 75.07%).

LCMS (ESI) M/Z: 759.6 [M+H⁺].

¹H NMR (400 MHz, DMSO) δ 8.07-7.93 (m, 1H), 7.04-6.92 (m, 1H), 6.67-6.42 (m, 3H), 5.12-4.96 (m, 2H), 4.78-4.43 (m, 2H), 4.36-4.12 (m, 2H), 4.09-3.70 (m, 5H), 3.65-3.40 (m, 3H), 3.35 (s, 1H), 3.30-3.27 (m, 1H), 3.20 (d, *J =* 12.0 Hz, 2H), 3.16-3.04 (m, 3H), 3.03-2.86 (m, 3H), 2.83-2.68 (m, 1H), 2.67-2.55 (m, 2H), 2.47-2.39 (m, 1H), 2.35-2.13 (m, 7H), 1.98-1.63 (m, 7H), 1.34-1.26 (m, 1H), 1.20-1.09 (m, 2H), 1.08-1.03 (m, 1H), 0.94-0.85 (m, 11H), 0.81-0.67 (m, 8H).

### Example 49

### Preparation of (S)-2-((S)-2-(dimethylamino)-3-methylbutanamido)-N-((3R,45,55)-1-((S)-2-((1R,2R)-3-((R)-2-(3-((S)-2-((S)-2-(2-(2,5-dioxo-2,5-dihydro-1H-pyrrol-1-yl)acetamido)propanamido)propanamido)phenyl)morpholino)-1-methoxy-2-methyl-3-oxopropyl)pyrrolidin-1-yl)-3-methoxy-5-methyl-1-oxoheptan-4-yl)-N,3-dimethylbutanamide.

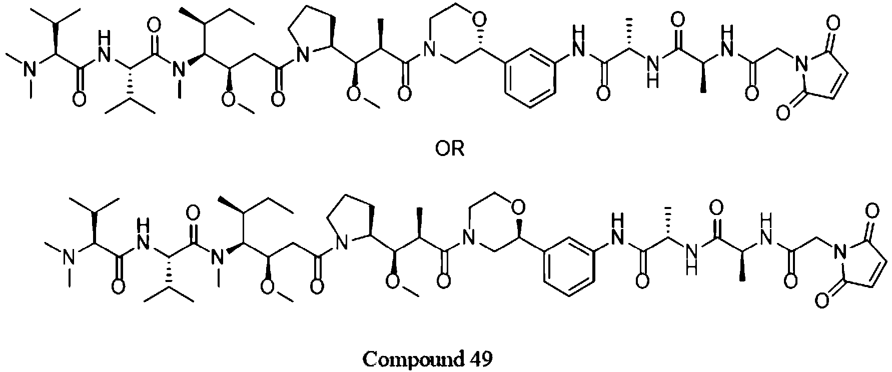

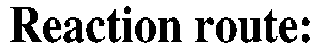

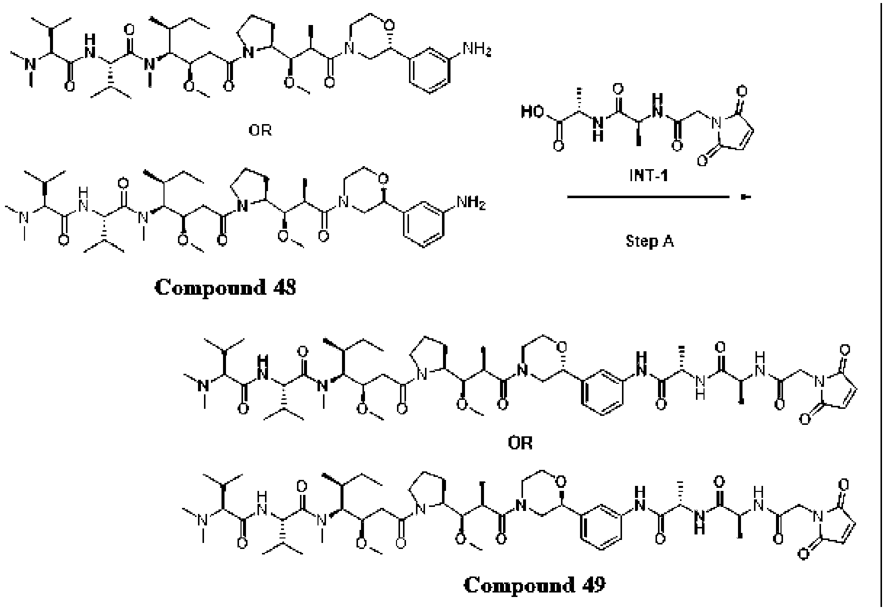

### Procedures:

**Step A:** Compound **48** (300 mg, 0.40 mmol) was added to a 25 mL single-necked flask, and dry dichloromethane (15 mL) was added. Intermediate INT-1 (126.04 mg, 0.42 mmol), HATU (197.72 mg, 0.52 mmol), and 2,6-lutidine (128.58 mg, 1.20 mmol) were then added sequentially, and the reaction system was reacted at room temperature for 20 min under a nitrogen atmosphere. After the reaction was completed, the mixture was distilled under reduced pressure at a low temperature. The residue was dissolved in N,N-dimethylformamide and then purified by column chromatography (acetonitrile/0.1% aqueous trifluoroacetic acid solution) to give compound **49** (195 mg, yield: 47.52%).

LCMS (ESI) M/Z: 1038.7 [M+H⁺].

¹H NMR (400 MHz, DMSO) δ 10.00-9.82 (m, 1H), 9.59 (s, 1H), 8.88 (dd, *J =* 30.5, 7.7 Hz, 1H), 8.44 (dd, *J =* 7.0, 3.9 Hz, 1H), 8.16 (dd, *J =* 10.8, 7.3 Hz, 1H), 7.76-7.63 (m, 1H), 7.52 (d, *J =* 8.1 Hz, 1H), 7.32-7.20 (m, 1H), 7.18-6.99 (m, 3H), 4.78-4.50 (m, 2H), 4.46-4.28 (m, 4H), 4.14-3.97 (m, 5H), 3.91-3.82 (m, 1H), 3.62-3.46 (m, 4H), 3.38-3.27 (m, 4H), 3.20 (d, *J* = 10.7 Hz, 2H), 3.16-3.04 (m, 3H), 2.99 (d, *J =* 6.7 Hz, 2H), 2.78 (t, *J =* 14.9 Hz, 7H), 2.62 (dd, *J =* 28.1, 11.8 Hz, 1H), 2.44-2.16 (m, 2H), 2.14-1.56 (m, 7H), 1.30 (t, *J =* 6.3 Hz, 3H), 1.20 (dd, *J =* 13.2, 6.8 Hz, 4H), 1.12 (d, *J =* 6.6 Hz, 1H), 1.05 (t, *J* = 6.5 Hz, 1H), 0.99-0.82 (m, 15H), 0.81-0.68 (m, 5H).

### Example 50

### Preparation of N-((S)-1-(((S)-1-((3-((R)-4-((2R,3R)-3-((S)-1-((3R,4S,SS)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanoyl)morpholin-2-yl)phenyl)amino)-1-oxopropan-2-yl)amino)-1-oxopropan-2-yl)-6-(2-(methylsulfonyl)pyrimidin-5-yl)hex-5-ynamide.

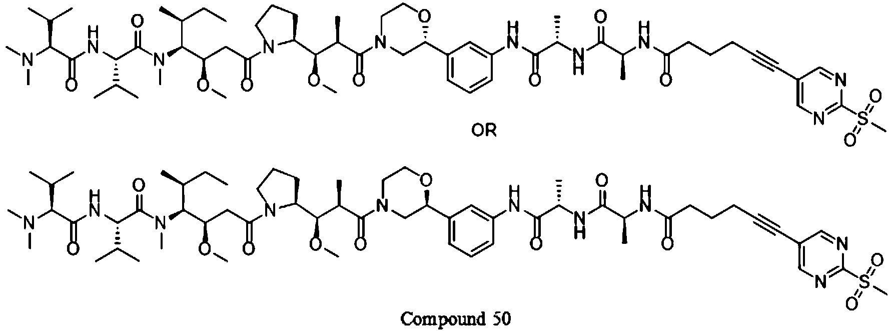

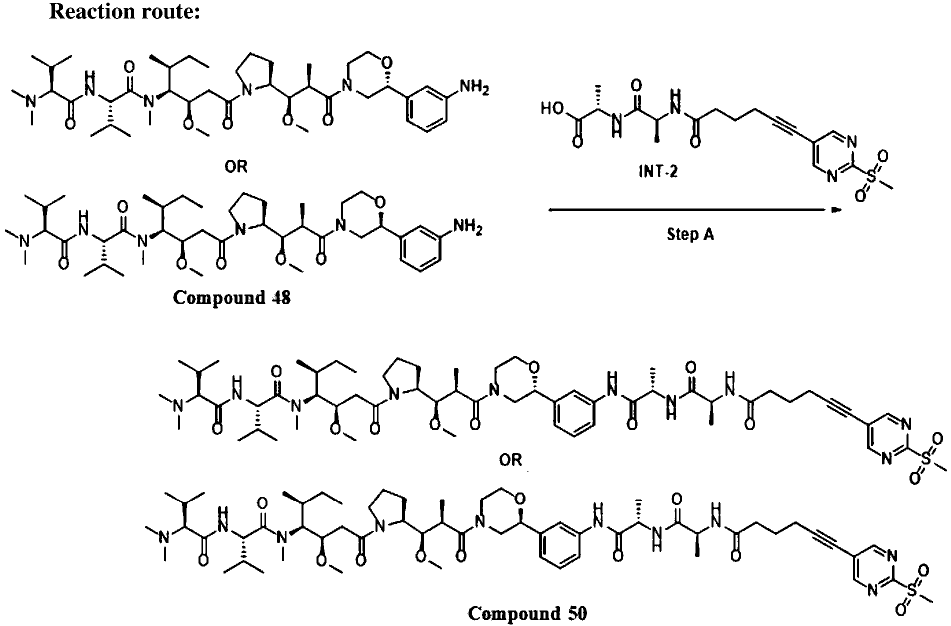

### Procedures:

**Step A:** Compound **48** (200 mg, 0.26 mmol) was added to a 25 mL single-necked flask, and dry dichloromethane (15 mL) was added. Intermediate INT-2 (113.12 mg, 0.28 mmol), HATU (128.52 mg, 0.34 mmol), and 2,6-lutidine (83.58 mg, 0.78 mmol) were then added sequentially, and the reaction system was reacted at room temperature for 20 min under a nitrogen atmosphere. After completion of the reaction as monitored by LCMS, the mixture was concentrated under reduced pressure at a low temperature. The residue was dissolved in N,N-dimethylformamide and then purified by column chromatography (acetonitrile/0.1% aqueous trifluoroacetic acid solution) to give compound **50** (280 mg, yield: 92.25%).

LCMS (ESI) M/Z: 1151.8 [M+H⁺].

¹H NMR (400 MHz, DMSO) δ 9.97-9.86 (m, 1H), 9.60 (s, 1H), 9.10 (d, *J* = 8.1 Hz, 2H), 8.97-8.81 (m, 1H), 8.11 (t, *J* = 7.6 Hz, 2H), 7.70 (d, *J =* 17.5 Hz, 1H), 7.56 (d, *J =* 5.7 Hz, 1H), 7.34-7.20 (m, 1H), 7.18-6.99 (m, 1H), 4.69-4.53 (m, 2H), 4.41-4.26 (m, 4H), 4.10-3.88 (m, 4H), 3.84-3.67 (m, 2H), 3.61-3.47 (m, 2H), 3.41 (s, 3H), 3.38-3.27 (m, 4H), 3.26-3.15 (m, 3H), 3.12 (t, *J* = 6.2 Hz, 2H), 3.08-2.88 (m, 3H), 2.85-2.69 (m, 7H), 2.57 (t, *J =* 7.1 Hz, 2H), 2.43 (d, *J =* 21.3 Hz, 1H), 2.36-2.25 (m, 3H), 2.14-1.57 (m, 9H), 1.30 (t, *J =* 6.4 Hz, 3H), 1.20 (dd, *J =* 15.2, 6.8 Hz, 4H), 1.12 (d, *J =* 6.7 Hz, 1H), 1.05 (t, *J =* 6.5 Hz, 1H), 0.99-0.82 (m, 15H), 0.81-0.68 (m, 5H).

### Example 51

### Preparation of (S)-N-((3R,4S,SS)-1-((S)-2-((1R,2R)-3-((S)-2-(3-aminophenyl)morpholino)-1-methoxy-2-methyl-3-oxopropyl)pyrrolidin-1-yl)-3-methoxy-5-methyl-1-oxoheptan-4-yl)-2-((S)-2-(dimethylamino)-3-methylbutanamido)-N,3-dimethylbutanamide.

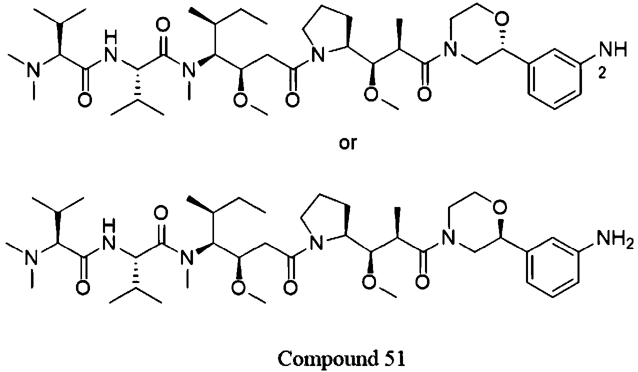

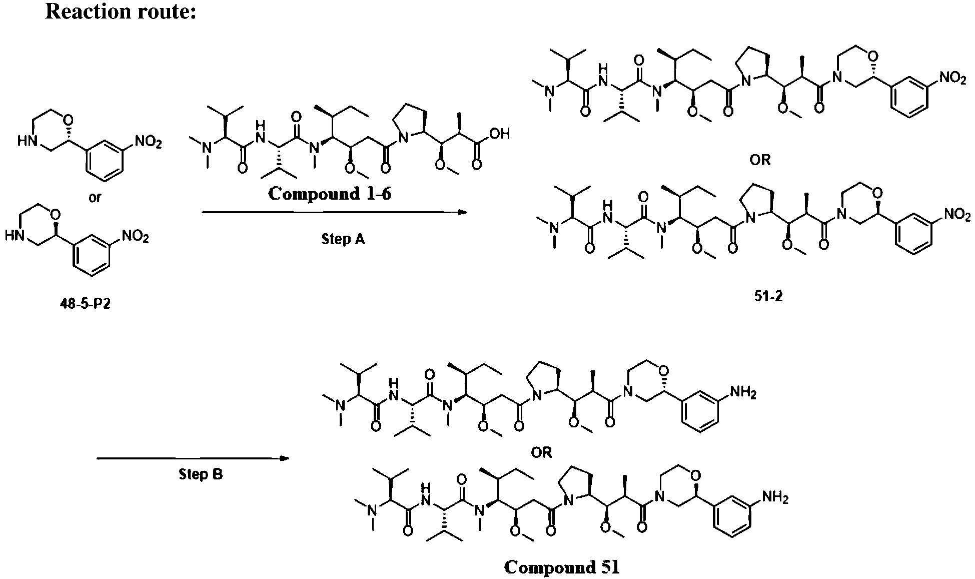

### Procedures:

**Step A:** Compound **48-5-P2** (550 mg, 2.62 mmol) was added to a 50 mL single-necked flask, and dry N,N-dimethylformamide (20 mL) was added. Compound **1-6** (1.60 g, 2.62 mmol), HATU (1.50 g, 3.93 mmol), and N,N-diisopropylethylamine (1.32 mL, 7.92 mmol) were then added sequentially, and the reaction system was reacted at room temperature for 2 h under a nitrogen atmosphere. After the reaction was completed, the mixture was filtered, and the filtrate was purified by column chromatography (acetonitrile/0.1% aqueous formic acid solution) to give compound **51-2** (1.20 g, yield: 56.52%).

LCMS (ESI) M/Z: 789.3 [M+H⁺].

**Step B:** Tetrahydroxydiboron (408.8 mg, 4.56 mmol) was added to a 25 mL single-necked flask, and dry N,N-dimethylformamide (12 mL) was added. 4,4'-Bipyridine (178.05 mg, 1.14 mmol) and compound **51-2** (900 mg, 1.14 mmol) were then rapidly added sequentially, and the reaction system was reacted at room temperature for 5 min under a nitrogen atmosphere. After completion of the reaction as monitored by LCMS, a small amount of methanol was added to help dissolution, and the mixture was purified by column chromatography (acetonitrile/0.1% aqueous formic acid solution) to give compound **51** (800 mg, yield: 90.12%).

LCMS (ESI) M/Z: 759.6 [M+H⁺].

¹H NMR (400 MHz, DMSO) δ 8.02 (d, *J =* 8.6 Hz, 1H), 7.05-6.92 (m, 1H), 6.67-6.40 (m, 3H), 5.05 (s, 2H), 4.78-4.47 (m, 2H), 4.41-4.10 (m, 2H), 4.07-3.89 (m, 4H), 3.86-3.71 (m, 1H), 3.64-3.40 (m, 3H), 3.31-3.29 (m, 2H), 3.22-3.11 (m, 5H), 3.08-2.96 (m, 2H), 2.93-2.71 (m, 2H), 2.69-2.55 (m, 2H), 2.38-2.11 (m, 7H), 2.03-1.57 (m, 8H), 1.21 (d, *J =* 6.7 Hz, 1H), 1.12 (dd, *J* = 14.5, 6.6 Hz, 2H), 1.05 (d, *J =* 6.6 Hz, 1H), 0.96-0.82 (m, 13H), 0.78-0.68 (m, 6H).

### Example 52

### Preparation of (S)-2-((S)-2-(dimethylamino)-3-methylbutanamido)-N-((3R,4S,SS)-1-((S)-2-((1R,2R)-3-((S)-2-(3-((S)-2-((S)-2-(2-(2,5-dioxo-2,5-dihydro-1H-pyrrol-1-yl)acetamido)propanamido)propanamido)phenyl)morpholino)-1-methoxy-2-methyl-3-oxopropyl)pyrrolidin-1-yl)-3-methoxy-5-methyl-1-oxoheptan-4-yl)-N,3-dimethylbutanamide.

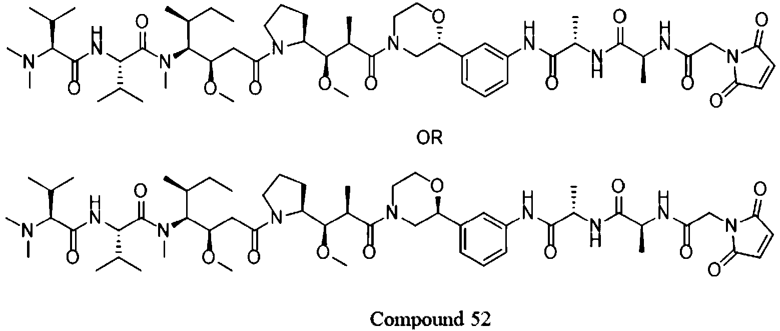

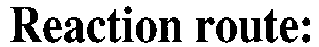

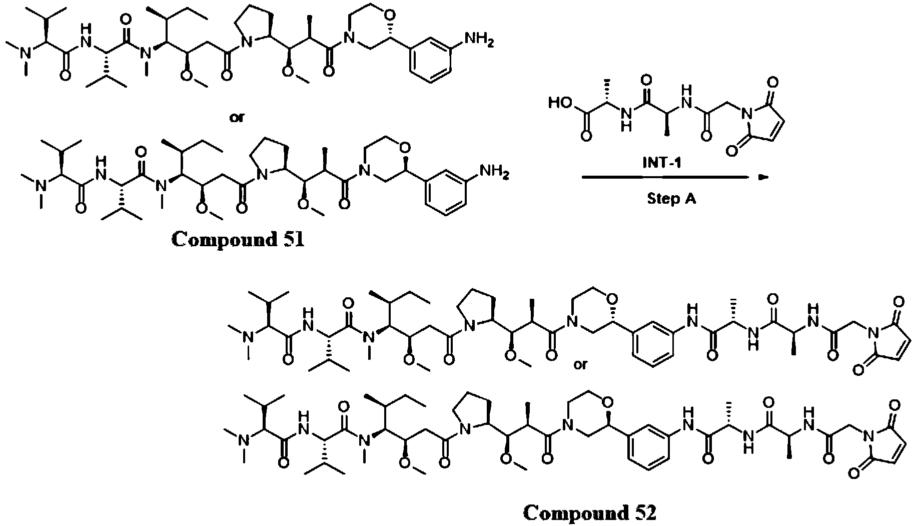

### Procedures:

**Step A:** Compound **51** (300 mg, 0.40 mmol) was added to a 25 mL single-necked flask, and dry dichloromethane (15 mL) was added. Intermediate INT-1 (126.04 mg, 0.42 mmol), HATU (197.72 mg, 0.52 mmol), and 2,6-lutidine (128.58 mg, 1.20 mmol) were then added sequentially, and the reaction system was reacted at room temperature for 20 min under a nitrogen atmosphere. After the reaction was completed, the mixture was concentrated under reduced pressure at a low temperature. The residue was purified by column chromatography (acetonitrile/0.1% aqueous trifluoroacetic acid solution) to give compound **52** (255 mg, yield: 55.47%).

LCMS (ESI) M/Z: 1038.7 [M+H⁺].

¹H NMR (400 MHz, DMSO) δ 9.91 (s, 1H), 9.67 (brs, 1H), 9.00-8.87 (m, 1H), 8.44 (d, J = 7.2 Hz, 1H), 8.17 (d, J = 6.6 Hz, 1H), 7.69 (d, J = 7.1 Hz, 1H), 7.59-7.46 (m, 1H), 7.34-7.21 (m, 1H), 7.19-6.95 (m, 3H), 4.78-4.45 (m, 2H), 4.41-4.27 (m, 4H), 4.09-3.86 (m, 6H), 3.80-3.70 (m, 1H), 3.67-3.40 (m, 3H), 3.33 (dd, J = 15.1, 7.8 Hz, 4H), 3.24-3.12 (m, 6H), 3.01 (d, J = 11.7 Hz, 2H), 2.95-2.84 (m, 1H), 2.79-2.56 (m, 8H), 2.39-2.19 (m, 1H), 2.08-1.58 (m, 6H), 1.32-1.05 (m, 11H), 0.94-0.73 (m, 18H).

### Example 53

### Preparation of N-((S)-1-(((S)-1-((3-((S)-4-((2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanoyl)morpholin-2-yl)phenyl)amino)-1-oxopropan-2-yl)amino)-1-oxopropan-2-yl)-6-(2-(methylsulfonyl)pyrimidin-5-yl)hex-5-ynamide.

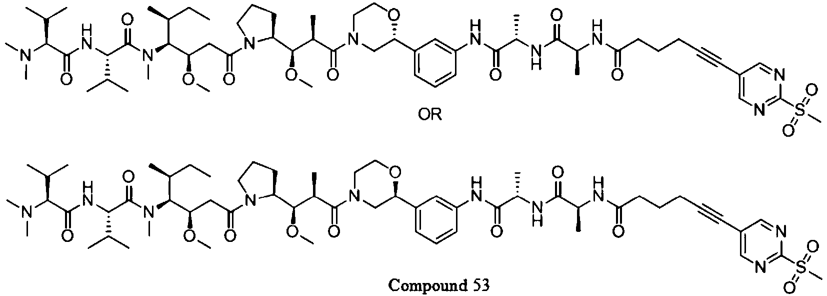

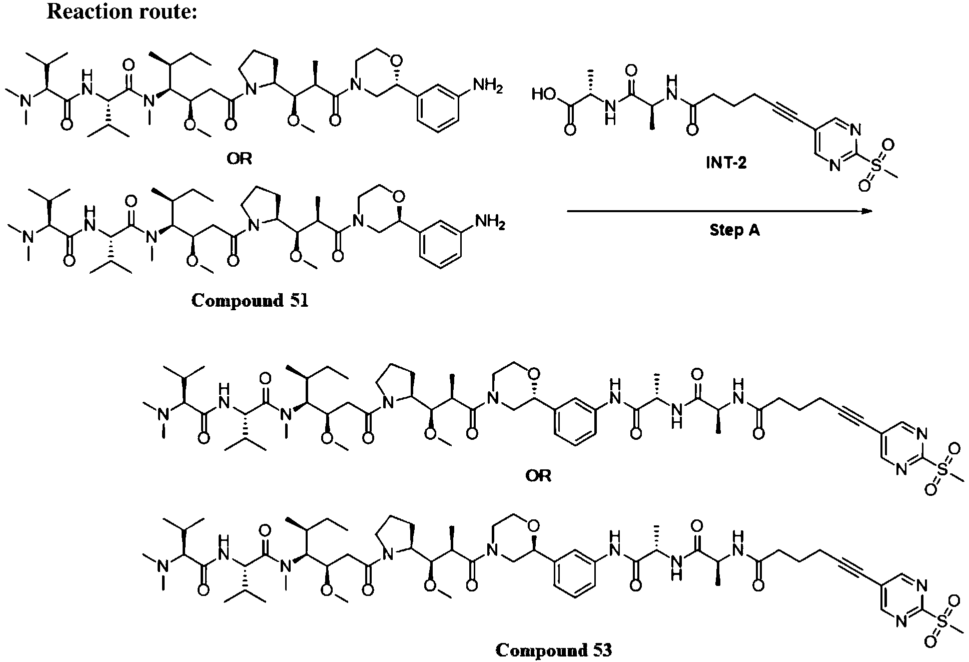

### Procedures:

**Step A:** Compound **51** (250 mg, 0.33 mmol) was added to a 25 mL single-necked flask, and dry dichloromethane (10 mL) was added. Intermediate INT-2 (142 mg, 0.35 mmol), HATU (160 mg, 0.42 mmol), and 2,6-lutidine (105 mg, 0.98 mmol) were then added sequentially, and the reaction system was reacted at room temperature for 20 min under a nitrogen atmosphere. After the reaction was completed, the mixture was concentrated by rotary evaporation under reduced pressure at a low temperature to remove the solvent. The residue was dissolved in N,N-dimethylformamide and then purified by column chromatography (acetonitrile/0.1% aqueous trifluoroacetic acid solution) to give compound **53** (320 mg, yield: 76.78%).

LCMS (ESI) M/Z: 1151.7 [M+H⁺].

¹H NMR (400 MHz, DMSO) δ 9.97-9.87 (m, 1H), 9.62 (s, 1H), 9.11 (s, 2H), 8.97-8.84 (m, 1H), 8.12 (d, *J* = 6.9 Hz, 2H), 7.70 (d, *J* = 9.6 Hz, 1H), 7.61-7.52 (m, 1H), 7.35-7.22 (m, 1H), 7.14-6.96 (m, 1H), 468-4.55 (m, 2H), 4.41-4.28 (m, 4H), 4.09-3.87 (m, 4H), 3.83-3.65 (m, 2H), 3.62-3.51 (m, 2H), 3.41 (s, 3H), 3.37-3.29 (m, 3H), 3.24-3.10 (m, 6H), 3.06-2.96 (m, 2H), 2.94-2.87 (m, 1H), 2.82-2.70 (m, 7H), 2.59-2.55 (m, 2H), 2.48-2.43 (m, 1H), 2.36-2.26 (m, 3H), 2.07-1.56 (m, 9H), 1.31 (d, *J =* 7.0 Hz, 4H), 1.22 (d, *J =* 7.0 Hz, 4H), 1.16 (d, *J =* 6.6 Hz, 1H), 1.11 (d, *J =* 6.5 Hz, 1H), 1.06 (d, *J =* 6.4 z, 1H), 0.99-0.74 (m, 20H).

The synthetic methods of the above examples were referred to prepare the following target compounds:

| Example | Structural formula | ¹HNMR | MS (ESI) M/Z [M+H]⁺ |
|---|---|---|---|
| 54 | 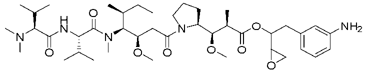 | ¹H NMR (400 MHz, DMSO) δ 8.94 (d, *J* = 6.7 Hz, 1H), 7.31 - 7.04 (m, 1H), 6.99 - 6.70 (m, 3H), 5.16 - 4.83 (m, 1H), 4.76 - 4.53 (m, 2H), 4.05 - 3.88 (m, 1H), 3.88 - 3.68 (m, 2H), 3.64 - 3.45 (m, 1H), 3.45 - 3.09 (m, 8H), 3.09 - 2.93 (m, 4H), 2.92 - 2.57 (m, 9H), 2.55 - 2.15 (m, 6H), 2.11 - 1.97 (m, 1H), 1.94 - 1.50 (m, 5H), 1.30 (s, 1H), 1.17 - 0.70 (m, 23H). | 760.5 |
| 55 | 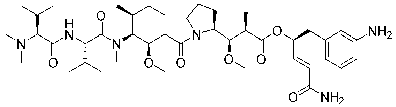 | ¹H NMR (400 MHz, DMSO) δ 8.92 (s, 1H), 7.64 - 7.42 (m, 1H), 7.35 - 6.92 (m, 3H), 6.86 - 6.50 (m, 4H), 6.23 - 5.89 (m, 1H), 5.70 - 5.43 (m, 1H), 4.65 (s, 1H), 4.61 - 4.52 (m, 1H), 4.00 - 3.91 (m, 2H), 3.88 - 3.83 (m, 1H), 3.82 - 3.72 (m, 2H), 3.58 - 3.49 (m, 1H), 3.43 - 3.31 (m, 1H), 3.29 - 3.14 (m, 6H), 3.10 - 2.83 (m, 5H), 2.76 (s, 6H), 2.47 - 2.20 (m, 4H), 2.07 - 1.97 (m, 1H), 1.93 - 1.85 (m, 1H), 1.80 - 1.65 (m, 3H), 1.21 - 1.11 (m, 1H), 1.09 - 1.04 (m, 2H), 1.01 - 0.60 (m, 21H). | 787.5 |
| 56 | 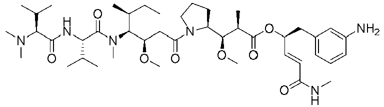 | ¹H NMR (400 MHz, DMSO) δ 8.09 - 7.92 (m, 2H), 6.95 - 6.85 (m, 1H), 6.65 - 6.50 (m, 1H), 6.43 - 6.33 (m, 3H), 6.15 - 5.94 (m, 1H), 5.57 - 5.38 (m, 1H), 5.02 - 4.92 (m, 2H), 4.73 - 4.46 (m, 2H), 3.98 - 3.74 (m, 3H), 3.56 - 3.48 (m, 1H), 3.24 (s, 2H), 3.20 - 3.15 (m, 4H), 3.06 - 2.95 (m, 3H), 2.78 (d, *J* = 7.4 Hz, 1H), 2.66 - 2.61 (m, 4H), 2.48 - 2.27 (m, 4H), 2.22 - 2.17 (m, 6H), 1.98 - 1.83 (m, 4H), 1.81 - 1.68 (m, 3H), 1.31 - 1.25 (m, 1H), 1.08 (d, *J* = 6.9 Hz, 3H), 1.05 - 0.81 (m, 15H), 0.77 - 0.68 (m, 6H). | 801.5 |
| 57 | 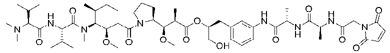 | ¹H NMR (400 MHz, DMSO) δ 9.91 - 9.76 (m, 1H), 9.57 (s, 1H), 8.90 (s, 1H), 8.42 (d, *J* = 7.3 Hz, 1H), 8.17 (d, *J* = 7.4 Hz, 1H), 7.51 (s, 1H), 7.44 - 7.35 (m, 1H), 7.23 - 7.14 (m, 1H), 7.09 (s, 2H), 6.89 (d, *J =* 7.2 Hz, 1H), 4.97 - 4.82 (m, 2H), 4.73 - 4.49 (m, 2H), 4.41 - 4.24 (m, 2H), 4.15 - 4.03 (m, 2H), 4.00 - 3.87 (m, 2H), 3.84 - 3.78 (m, 1H), 3.73 - 3.67 (m, 1H), 3.55 - 3.41 (m, 3H), 3.30 - 3.15 (m, 7H), 3.06 - 2.97 (m, 3H), 2.90 - 2.62 (m, 8H), 2.44 - 2.22 (m, 5H), 2.08 - 1.60 (m, 7H), 1.31 - 1.27 (m, 3H), 1.25 - 1.18 (m, 3H), 1.09 - 0.98 (m, 3H), 0.98 - 0.80 (m, 15H), 0.79 - 0.73 (m, 3H). | 1027.5 |
| 58 | 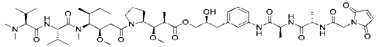 | ¹H NMR (400 MHz, DMSO) δ 9.90 - 9.78 (m, 1H), 8.48 - 8.38 (m, 1H), 8.27 - 8.16 (m, 1H), 8.10 - 7.99 (m, 1H), 7.56 - 7.33 (m, 2H), 7.24 - 7.12 (m, 1H), 7.10 - 7.04 (m, 2H), 6.95 - 6.84 (m, 1H), 5.10 - 4.88 (m, 1H), 4.69 - 4.59 (m, 1H), 4.57 - 4.46 (m, 1H), 4.44 - 4.24 (m, 2H), 4.16 - 4.01 (m, 2H), 4.00 - 3.77 (m, 6H), 3.57 - 3.47 (m, 1H), 3.40 - 3.31 (m, 4H), 3.24 - 3.10 (m, 3H), 3.08 - 2.94 (m, 3H), 2.79 - 2.53 (m, 4H), 2.47 - 2.38 (m, 2H), 2.36 - 2.25 (m, 1H), 2.22 - 2.12 (m, 6H), 2.00 - 1.65 (m, 8H), 1.38 - 1.25 (m, 3H), 1.24 - 1.12 (m, 6H), 0.93 - 0.82 (m, 12H), 0.77 - 0.65 (m, 6H). | 1027.5 |
| 59 | 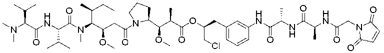 | ¹H NMR (400 MHz, DMSO) δ 9.86 (s, 1H), 9.69 (s, 1H), 8.91 (s, 1H), 8.44 (d, *J* = 7.1 Hz, 1H), 8.19 (d, *J =* 7.0 Hz, 1H), 7.56 (d, *J =* 9.5 Hz, 1H), 7.41 (t, *J* = 9.4 Hz, 1H), 7.21 (t, *J =* 7.7 Hz, 1H), 7.09 (s, 2H), 6.92 (d, *J =* 7.4 Hz, 1H), 5.17 (s, 1H), 4.71 - 4.52 (m, 2H), 4.41 - 4.28 (m, 2H), 4.15 - 4.02 (m, 2H), 4.00 - 3.92 (m, 2H), 3.87 - 3.66 (m, 4H), 3.56 - 3.50 (m, 1H), 3.31 - 3.23 (m, 4H), 3.22 - 3.14 (m, 3H), 3.05 - 2.97 (m, 3H), 2.98 - 2.81 (m, 3H), 2.80 - 2.64 (m, 5H), 2.46 - 2.38 (m, 2H), 2.36 - 2.26 (m, 2H), 2.11 - 1.64 (m, 7H), 1.37 - 1.14 (m, 6H), 1.06 - 0.67 (m, 21H). | 1045.4 |

### Example 60

### Preparation of (S)-N-((3R,4S,5S)-1-((S)-2-((1R,2R)-3-((R)-2-(3-aminophenyl)thiomorpholino)-1-methoxy-2-methyl-3-oxopropyl)pyrrolidin-1-yl)-3-methoxy-5-methyl-1-oxoheptan-4-yl)-2-((S)-2-(dimethylamino)-3-methylbutanamido)-N,3-dimethylbutanamide (Compound 60-P1 or Compound 60-P2) and (S)-N-((3R,4S,55)-1-((S)-2-((1R,2R)-3-((S)-2-(3-aminophenyl)thiomorpholino)-1-methoxy-2-methyl-3-oxopropyl)pyrrolidin-1-yl)-3-methoxy-5-methyl-1-oxoheptan-4-yl)-2-((S)-2-(dimethylamino)-3-methylbutanamido)-N,3-dimethylbutanamide (Compound 60-P2 or Compound 60-P1).

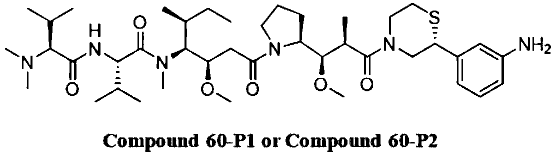

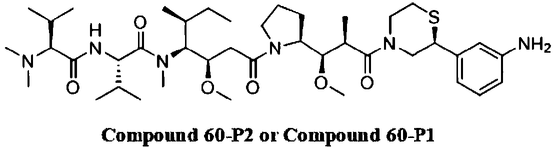

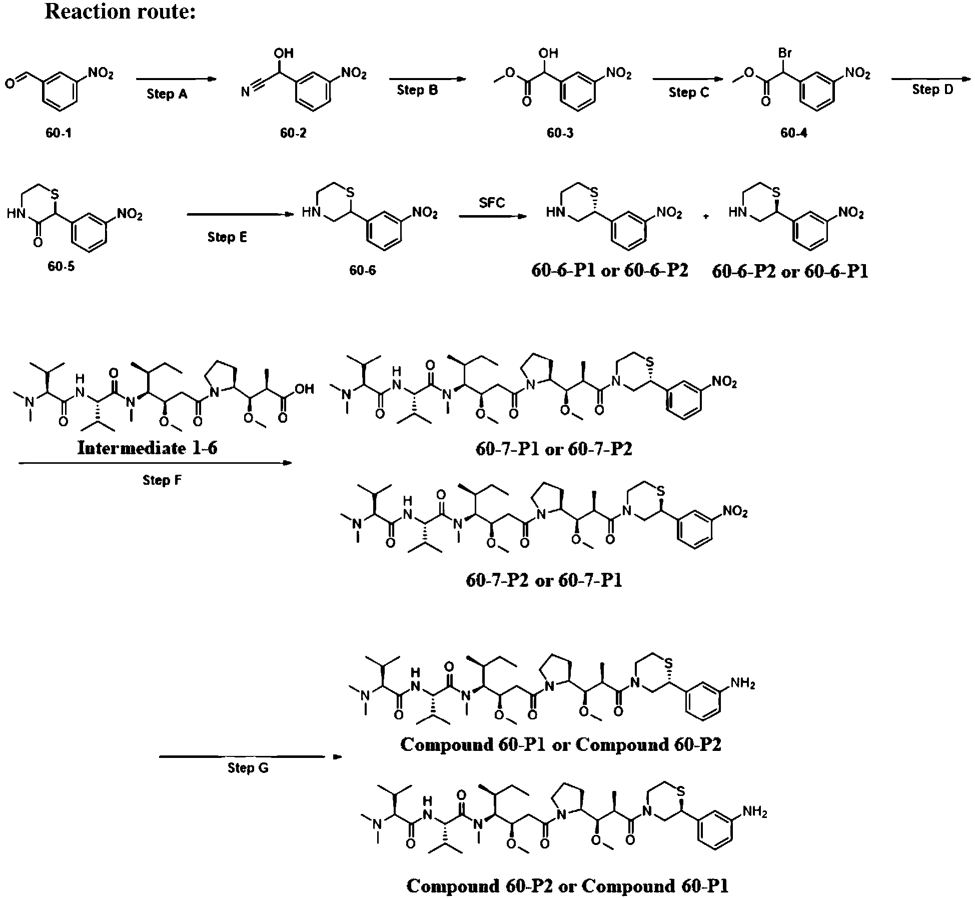

### Procedures:

**Step A:** Compound **60-1** (5.0 g, 33.1 mmol) was added to a 100 mL single-necked flask, dry dichloromethane (30 mL) was added, and trimethylsilyl cyanide (4.6 mL, 37.1 mmol) was added at 0°C. The mixture was stirred at room temperature for 6 h. Zinc iodide (0.21 g, 0.66 mmol) was then added, and the mixture was reacted at reflux at 50°C for 30 min. Subsequently, 2 M hydrochloric acid (100 mL) and diethyl ether (150 mL) were added, and the mixture was stirred for 16 h. After completion of the reaction as indicated by LCMS, the reaction liquid was extracted with diethyl ether. The organic phase was collected, dried over anhydrous sodium sulfate, and concentrated to give a residue, which was purified by normal phase (petroleum ether:ethyl acetate = 1:1) to give compound **60-2** (5.26 g, yield: 89.24%).

¹H NMR (400 MHz, CDCl₃) δ 8.35 (t, *J =* 1.9 Hz, 1H), 8.15 (ddd, *J =* 8.2, 2.2, 0.9 Hz, 1H), 7.84 (d, *J* = 7.8 Hz, 1H), 7.51 (t, *J =* 8.0 Hz, 1H), 5.34 (s, 1H), 3.76 (s, 3H).

**Step B:** Compound **60-2** (5.0 g, 28.08 mmol) was added to a 100 mL single-necked flask, and a solution of hydrochloric acid in methanol (30 mL, 6 M in MeOH) was added. The mixture was stirred at reflux for 3 h. After completion of the reaction as indicated by LCMS, the reaction liquid was extracted with ethyl acetate. The organic phase was collected, dried over anhydrous sodium sulfate, and concentrated to give a residue, which was purified by normal phase (petroleum ether:ethyl acetate = 1:1) to give compound **60-3** (5.35 g, yield: 90.26%).

¹H NMR (400 MHz, CDCl₃) δ 8.34 (t, *J* = 1.7 Hz, 1H), 8.23-8.14 (m, 1H), 7.82 (d, *J* = 7.7 Hz, 1H), 7.56 (t, *J* = 8.0 Hz, 1H), 5.33 (d, *J* = 5.0 Hz, 1H), 3.84 (d, *J =* 5.0 Hz, 1H), 3.81 (s, 3H).

**Step C:** Compound **60-3** (5.0 g, 23.68 mmol) was added to a 100 mL single-necked flask, and dry dichloromethane (5 mL) was added. Carbon tetrabromide (11.70 g, 35.28 mmol) and triphenylphosphine (9.30 g, 35.46 mmol) were added sequentially at 0°C, and the reaction system was reacted at room temperature for 2 h under a nitrogen atmosphere. After completion of the reaction as indicated by LCMS, the reaction liquid was concentrated and purified by normal phase (petroleum ether:ethyl acetate = 10:1) to give compound **60-4** (5.56 g, yield: 85.98%).

¹H NMR (400 MHz, CDCl₃) δ 8.35 (t, *J =* 1.9 Hz, 1H), 8.15 (ddd, *J =* 8.2, 2.2, 0.9 Hz, 1H), 7.84 (d, *J* = 7.8 Hz, 1H), 7.51 (t, *J =* 8.0 Hz, 1H), 5.34 (s, 1H), 3.76 (s, 3H).

**Step D:** Compound **60-4** (5.06 g, 18.46 mmol) was added to a 500 mL single-necked flask, ethanol (150 mL) was added, and mercaptoethylamine hydrochloride (2.11 g, 18.57 mmol) and potassium carbonate (2.56 g, 18.55 mmol) were added sequentially. The reaction system was reacted at 85°C for 10 h under a nitrogen atmosphere. After completion of the reaction as indicated by LCMS, the reaction liquid was concentrated, and the system was slurried with petroleum ether and filtered. The filter cake was collected to give compound **60-5** (2.06 g, yield: 46.83%).

LCMS (ESI) M/Z: 239.1 [M+H⁺].

**Step E:** Compound **60-5** (0.5 g, 2.10 mmol) was added to a 100 mL single-necked flask, and dry tetrahydrofuran (10 mL) was added. Borane dimethyl sulfide complex (6.2 mL, 1 M in THF) was added at 0°C. The reaction system was reacted at 70°C for 2 h under a nitrogen atmosphere. After the reaction was completed, p-toluenesulfonic acid monohydrate (0.40 g, 2.10 mmol) was added, and the mixture was stirred for another 1 h. After completion of the reaction as indicated by LCMS, methanol was slowly added at 0°C to quench the reaction. Subsequently, the reaction liquid was concentrated, and the crude product was purified by column chromatography (acetonitrile/0.1% aqueous formic acid solution) to give compound **60-6** (0.319 g, yield: 67.78%), which was then subjected to chiral resolution to give **60-6-P1** (119 mg) and **60-6-P2** (129 mg). Resolution conditions: mobile phase: supercritical carbon dioxide and a solution of 0.1 % ammonia in methanol; flow rate: 140 mL/min; gradient: maintaining the solution of 0.1% ammonia in methanol at 30%; detection wavelength: 214 nm; cycle time: 8 min.

The retention time (Rt) of compound **60-6-P1** = 6.5-9 min; the retention time (Rt) of compound **60-6-P2** = 9.8-14 min.

LCMS (ESI) M/Z: 225.1 [M+H⁺].

**Step F:** Compound **60-6-P1** (50 mg, 0.22 mmol) was added to a 25 mL single-necked flask, and dry dichloromethane (5 mL) was added. Compound **1-6** (200 mg, 0.33 mmol), HATU (170 mg, 0.447 mmol), and N,N-diisopropylethylamine (86 mg, 0.67 mmol) were then added sequentially, and the reaction system was reacted at room temperature for 2 h under a nitrogen atmosphere. After the reaction was completed, the mixture was filtered, and the filtrate was purified by column chromatography (acetonitrile/0.1% aqueous formic acid solution) to give compound **60-7-P1** (150 mg, yield: 83.57%).

Similarly, compound **60-6-P2** (20 mg, 0.089 mmol) was converted to compound **60-7-P2** (75 mg, yield: 86.57%) under the same conditions.

LCMS (ESI) M/Z: 805.9 [M+H⁺].

**Step G:** Compound **60-7-P1** (150 mg, 0.19 mmol) and tetrahydroxydiboron (89 mg, 0.99 mmol) were added to a 25 mL single-necked flask, and dry N,N-dimethylformamide (5 mL) was added, followed by the addition of 4,4'-bipyridine (19 mg, 0.12 mmol). The reaction system was reacted at room temperature for 5 min under a nitrogen atmosphere. After completion of the reaction as monitored by LCMS, a small amount of methanol was added to help dissolution, and the mixture was purified by column chromatography (acetonitrile/0.1% aqueous formic acid solution) to give compound **60-P1** (73 mg, yield: 50.55%).

LCMS (ESI) M/Z: 775.5 [M+H⁺].

¹H NMR (400 MHz, DMSO) δ 8.97-8.79 (m, 1H), 7.19-7.03 (m, 1H), 6.83-6.59 (m, 3H), 4.79-4.53 (m, 3H), 4.44-4.24 (m, 2H), 3.93-3.79 (m, 6H), 3.74-3.63 (m, 4H), 3.62-3.50 (m, 3H), 3.36-3.28 (m, 4H), 3.22-3.15 (m, 4H), 3.12-3.07 (m, 1H), 3.04-2.97 (m, 2H), 2.78-2.76 (m, 3H), 2.74 (s, 3H), 2.36-2.20 (m, 2H), 2.08-1.98 (m, 1H), 1.96-1.84 (m, 3H), 1.80-1.64 (m, 3H), 1.35-1.16 (m, 1H), 1.14-1.05 (m, 2H), 0.97-0.93 (m, 6H), 0.90-0.81 (m, 8H), 0.79-0.74 (m, 4H).

Similarly, compound **60-7-P2** (75 mg, 0.09 mmol) was converted to compound **60-P2** (35 mg, yield: 52.75%) under the same conditions.

LCMS (ESI) M/Z: 775.6 [M+H⁺].

¹H NMR (400 MHz, DMSO) δ 8.11-7.87 (m, 1H), 7.07-6.88 (m, 1H), 6.63-6.41 (m, 3H), 5.20-5.01 (m, 2H), 4.79-4.42 (m, 3H), 4.33-4.14 (m, 1H), 4.06-3.91 (m, 2H), 3.80-3.54 (m, 5H), 3.27-3.19 (m, 5H), 3.15-3.07 (m, 3H), 3.00 (s, 2H), 2.92-2.76 (m, 2H), 2.71-2.59 (m, 3H), 2.46-2.39 (m, 1H), 2.20 (s, 6H), 1.98-1.74 (m, 6H), 1.24 (s, 1H), 1.22-1.03 (m, 3H), 0.94-0.83 (m, 12H), 0.79-0.66 (m, 8H).

### Example 61

### Preparation of (S)-N-((3R,4S,5S)-1-((S)-2-((1R,2R)-3-((R)-2-(3-aminophenyl)-1,1-dioxidothiomorpholino)-1-methoxy-2-methyl-3-oxopropyl)pyrrolidin-1-yl)-3-methoxy-5-methyl-1-oxoheptan-4-yl)-2-((S)-2-(dimethylamino)-3-methylbutanamido)-N,3-dimethylbutanamide (Compound 61-P1 or Compound 61-P2) and (S)-N-((3R,4S,5S)-1-((S)-2-((1R,2R)-3-((S)-2-(3-aminophenyl)-1,1-dioxidothiomorpholino)-1-methoxy-2-methyl-3-oxopropyl)pyrrolidin-1-yl)-3-methoxy-5-methyl-1-oxoheptan-4-yl)-2-((S)-2-(dimethylamino)-3-methylbutanamido)-N,3-dimethylbutanamide (Compound 61-P2 or Compound 61-P1).

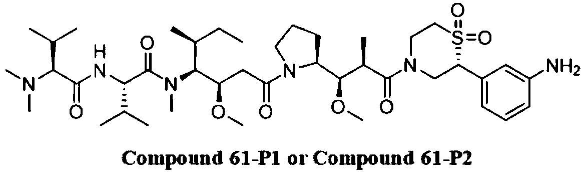

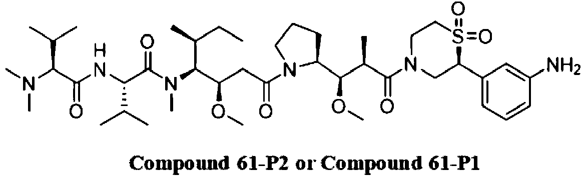

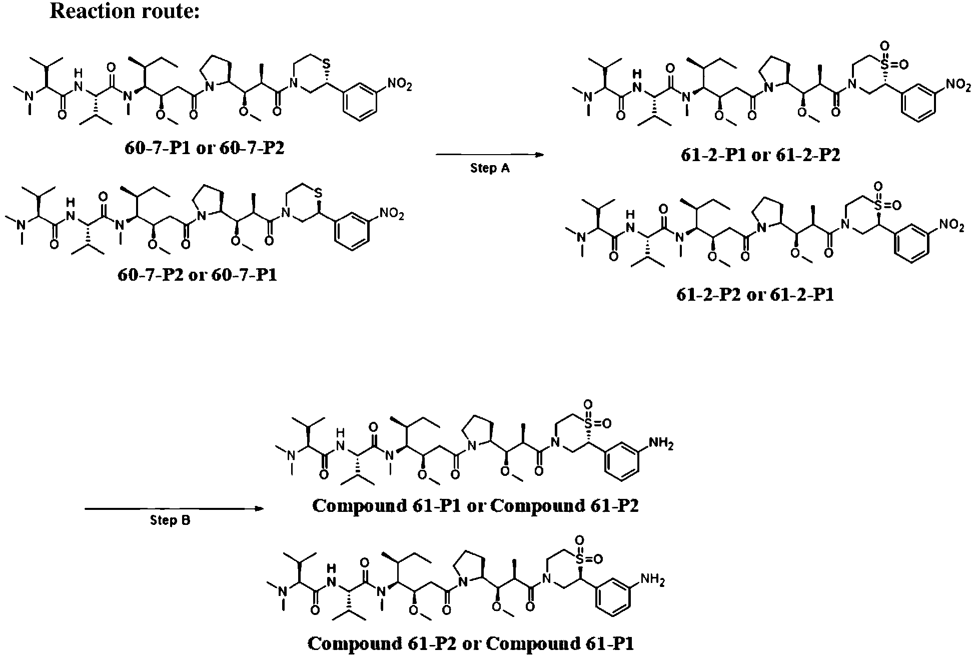

### Procedures:

**Step A:** Compound **60-7-P1** (50 mg, 0.22 mmol) was added to a 25 mL single-necked flask, and 2-methyltetrahydrofuran/water (2:1 = 2 mL/0.5 mL) was added, followed by the addition of potassium peroxymonosulfate (273 mg, 0.446 mmol). The reaction system was reacted at room temperature for 2 h under a nitrogen atmosphere. After the reaction was completed, the mixture was filtered, and the filtrate was purified by column chromatography (acetonitrile/0.1% aqueous formic acid solution) to give compound **61-2-P1** (40 mg, yield: 80.26%).

Similarly, compound **60-7-P2** (50 mg, 0.22 mmol) was converted to compound **61-2-P2** (40 mg, yield: 80.26%) under the same conditions.

LCMS (ESI) M/Z: 837.3 [M+H⁺].

**Step B:** Compound **61-2-P1** (40 mg, 0.062 mmol) and tetrahydroxydiboron (25 mg, 0.227 mmol) were added to a 25 mL single-necked flask, and dry N,N-dimethylformamide (2 mL) was added, followed by the addition of 4,4'-bipyridine (8 mg, 0.40 mmol). The reaction system was reacted at room temperature for 5 min under a nitrogen atmosphere. After completion of the reaction as monitored by LCMS, a small amount of methanol was added to help dissolution, and the mixture was purified by column chromatography (acetonitrile/0.1% aqueous formic acid solution) to give compound **61-P1** (25 mg, yield: 60.31%).

LCMS (ESI) M/Z: 807.6 [M+H⁺].

1H NMR (400 MHz, DMSO-d6) δ 8.98-8.75 (m, 1H), 7.19-7.04 (m, 1H), 6.80-6.57 (m, 3H), 4.85-4.66 (m, 2H), 4.60-4.47 (m, 2H), 4.45-4.30 (m, 2H), 4.27-4.18 (m, 2H), 4.06-4.03 (m, 1H), 3.98-3.93 (m, 2H), 3.64-3.59 (m, 2H), 3.34-3.30 (m, 3H), 3.28-3.24 (m, 2H), 3.21-3.16 (m, 3H), 3.02-2.96 (m, 3H), 2.78-2.68 (m, 6H), 2.36-2.12 (m, 3H), 2.05-1.63 (m, 8H), 1.31-1.22 (m, 1H), 1.18-1.05 (m, 3H), 0.97-0.84 (m, 12H), 0.81-0.68 (m, 8H).

Similarly, compound **61-2-P2** (40 mg, 0.062 mmol) was converted to compound **61-P2** (25 mg, yield: 60.31%) under the same conditions.

LCMS (ESI) M/Z: 807.6 [M+H⁺].

¹H NMR (400 MHz, DMSO-d6) δ 8.97-8.77 (m, 1H), 7.17-7.02 (m, 1H), 6.76-6.56 (m, 3H), 4.87-4.52 (m, 4H), 4.43-4.20 (m, 4H), 4.07-3.94 (m, 5H), 3.35-3.30 (m, 3H), 3.24-3.20 (m, 3H), 3.16-3.11 (m, 2H), 3.06-2.99 (m, 3H), 2.79-2.73 (m, 5H), 2.46-2.40 (m, 1H), 2.35-2.17 (m, 3H), 2.07-1.66 (m, 8H),1.23-1.18 (m, 1H), 1.14-1.04 (m, 3H), 1.00-0.83 (m, 16H), 0.80-0.73 (m, 4H).

The trastuzumabab was purchased from Sanyou Biopharmaceuticals (Shanghai) Co., Ltd., and the sequences of the antibody are as follows:
The amino acid sequence of the light chain is shown in SEQ ID NO: 1: 
DIQMTQSPSSLSASVGDRVTITCRASQDVNTAVAWYQQKPGKAPKLLIYSASFLYSGVPS
RFSGSRSGTDFTLTISSLQPEDFATYYCQQHYTTPPTFGQGTKVEIKRTVAAPSVFIFPP
SDEQLKSGTASVVCLLNNFYPREAKVQWKVDNALQSGNSQESVTEQDSKDSTYSLSSTLT
LSKADYEKHKVYACEVTHQGLSSPVTKSFNRGEC
The amino acid sequence of the heavy chain is shown in SEQ ID NO: 2:

EVQLVESGGGLVQPGGSLRLSCAASGFNIKDTYIHWVRQAPGKGLEWVARIYPTNGYTRY
ADSVKGRFTISADTSKNTAYLQMNSLRAEDTAVYYCSRWGGDGFYAMDYWGQGTLVTVSS
ASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPELLGG
PSVFLFPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSREE

MTKNQVSLTCLVKGFYPSDIAVEWESNGQPENNYKTTPPVLDSDGSFFLYSKLTVDKSRW
QQGNVFSCSVMHEALHNHYTQKSLSLSPGK

The method for coupling the payload-linker conjugate of the present disclosure to the trastuzumabab is as follows:

### Coupling Example 1: ADC-1

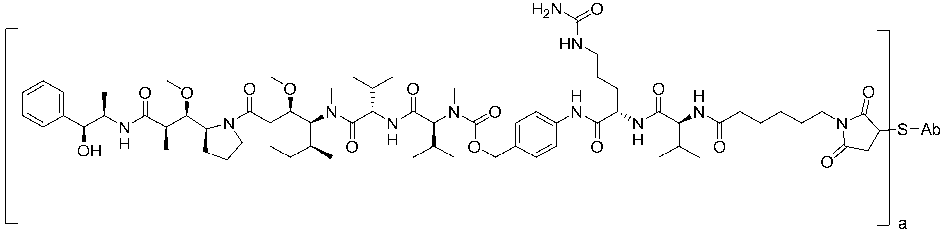

The trastuzumabab was buffer-exchanged into a 10 mM phosphate buffer at pH 7.0 by desalting chromatography or ultrafiltration. The antibody concentration was determined using the UV method. A 10 mM TCEP solution (3.2 eq.) was added to the antibody solution (10 mg/mL, 3 mL). After uniform mixing, the mixture was reduced at 8°C for 3.5 h. Subsequently, a 10 mM solution of mc-vc-PAB-MMAE (MCE, Cat. No.: HY-15575) in dimethyl sulfoxide (6.5 eq.) was added. After uniform mixing, the mixture was reacted at 8°C for 2.0 h. After completion of the coupling reaction, a 100 mM N-acetyl-cysteine (Merck, Cat. No.: 1009005) solution was added at a quenching ratio of 20:1 (quenching agent: antibody). The mixture was shaken uniformly and then quenched at room temperature for 30 min to terminate the coupling reaction. The reaction liquid was desalted and purified using a Sephadex G25 gel column (elution phase: a solution containing 0.02 M His-HAc at pH 5.5), concentrated by ultrafiltration, and then filtered through a 0.2 µm filter to obtain ADC-1 in a PBS buffer (12.13 mg/mL, 2.2 mL), which was stored frozen at 4°C. The average number of toxins linked per antibody molecule (DAR) calculated by RP-HPLC was: a = 4.14. The SEC purity was: 98.8%.

### Coupling Example 2: ADC-2

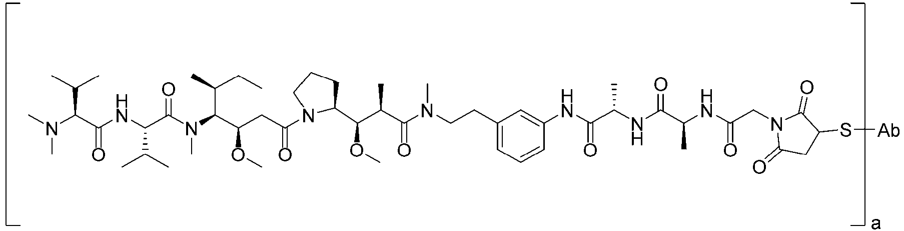

The reference linker-payload was synthesized by referring to the method for compound 28 in the patent WO2023081230A1.

The trastuzumabab was buffer-exchanged into a 10 mM phosphate buffer at pH 7.0 by desalting chromatography or ultrafiltration. The antibody concentration was determined using the UV method. A 10 mM TCEP solution (10 eq.) was added to the antibody solution (10 mg/mL, 3 mL). After uniform mixing, the mixture was reduced at 37°C for 3 h. Subsequently, a 10 mM solution of the aforementioned reference linker-payload in dimethyl sulfoxide (20 eq.) was added. After uniform mixing, the mixture was reacted at 25°C for 2.5 h. After completion of the coupling reaction, the mixture was desalted and purified using a Sephadex G25 gel column (elution phase: a solution containing 0.02 M His-HAc at pH 5.5), concentrated by ultrafiltration, and then filtered through a 0.2 µm filter to obtain ADC-2 in a PBS buffer (11.62 mg/mL, 2.5 mL), which was stored frozen at 4°C. The average number of toxins linked per antibody molecule (DAR) calculated by RP-HPLC was: a = 7.78. The SEC purity was: 99.3%.

### Coupling Example 3: ADC-3

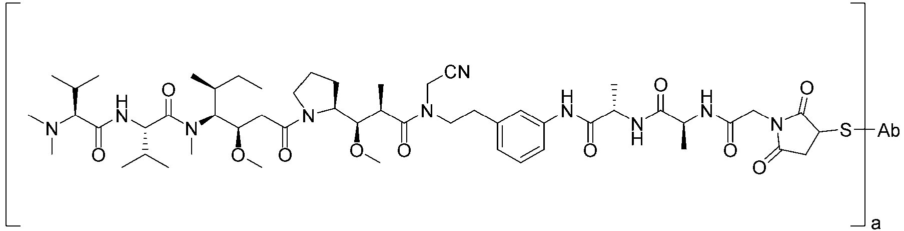

The trastuzumabab was buffer-exchanged into a 10 mM phosphate buffer at pH 7.0 by desalting chromatography or ultrafiltration. The antibody concentration was determined using the UV method. A 10 mM TCEP solution (10 eq.) was added to the antibody solution (10 mg/mL, 3 mL). After uniform mixing, the mixture was reduced at 37°C for 3 h. Subsequently, a 10 mM solution of compound 46 in dimethyl sulfoxide (20 eq.) was added. After uniform mixing, the mixture was reacted at 25°C for 2.5 h. After completion of the coupling reaction, the mixture was desalted and purified using a Sephadex G25 gel column (elution phase: a solution containing 0.02 M His-HAc at pH 5.5), concentrated by ultrafiltration, and then filtered through a 0.2 µm filter to obtain ADC-3 in a PBS buffer (15.3 mg/mL, 1.7 mL), which was stored frozen at 4°C. The average number of toxins linked per antibody molecule (DAR) calculated by RP-HPLC was: a = 7.82. The SEC purity was: 99.0%.

### Coupling Example 4: ADC-4

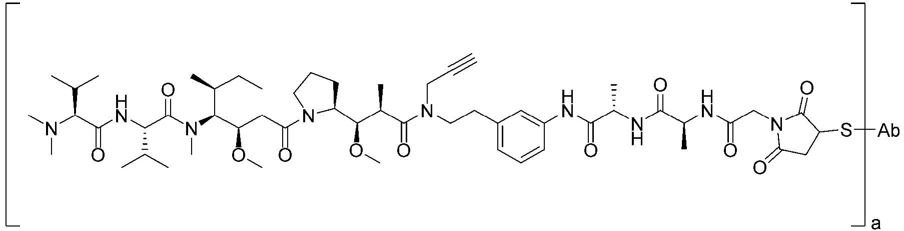

The trastuzumabab was buffer-exchanged into a 10 mM phosphate buffer at pH 7.0 by desalting chromatography or ultrafiltration. The antibody concentration was determined using the UV method. A 10 mM TCEP solution (10 eq.) was added to the antibody solution (10 mg/mL, 3 mL). After uniform mixing, the mixture was reduced at 37°C for 3 h. Subsequently, a 10 mM solution of compound 37 in dimethyl sulfoxide (20 eq.) was added. After uniform mixing, the mixture was reacted at 25°C for 2.5 h. After completion of the coupling reaction, the mixture was desalted and purified using a Sephadex G25 gel column (elution phase: a solution containing 0.02 M His-HAc at pH 5.5), concentrated by ultrafiltration, and then filtered through a 0.2 µm filter to obtain ADC-4 in a PBS buffer (4.67 mg/mL, 6.2 mL), which was stored frozen at 4°C. The average number of toxins linked per antibody molecule (DAR) calculated by RP-HPLC was: a = 7.78. The SEC purity was: 99.2%.

### Coupling Example 5: ADC-5

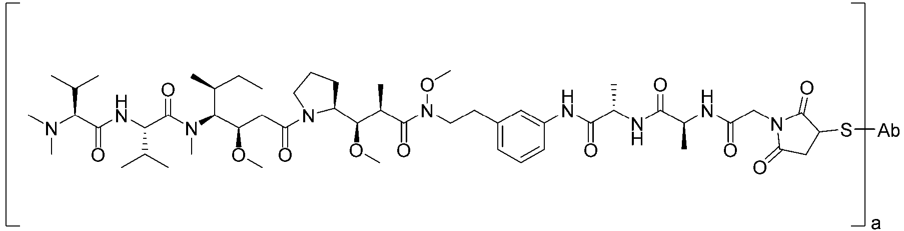

The trastuzumabab was buffer-exchanged into a 10 mM phosphate buffer at pH 7.0 by desalting chromatography or ultrafiltration. The antibody concentration was determined using the UV method. A 10 mM TCEP solution (10 eq.) was added to the antibody solution (10 mg/mL, 3 mL). After uniform mixing, the mixture was reduced at 37°C for 3 h. Subsequently, a 10 mM solution of compound 40 in dimethyl sulfoxide (20 eq.) was added. After uniform mixing, the mixture was reacted at 25°C for 2.5 h. After completion of the coupling reaction, the mixture was desalted and purified using a Sephadex G25 gel column (elution phase: a solution containing 0.02 M His-HAc at pH 5.5), concentrated by ultrafiltration, and then filtered through a 0.2 µm filter to obtain ADC-5 in a PBS buffer (12.67 mg/mL, 2.2 mL), which was stored frozen at 4°C. The average number of toxins linked per antibody molecule (DAR) calculated by RP-HPLC was: a = 7.65. The SEC purity was: 98.8%.

### Coupling Example 6: ADC-6

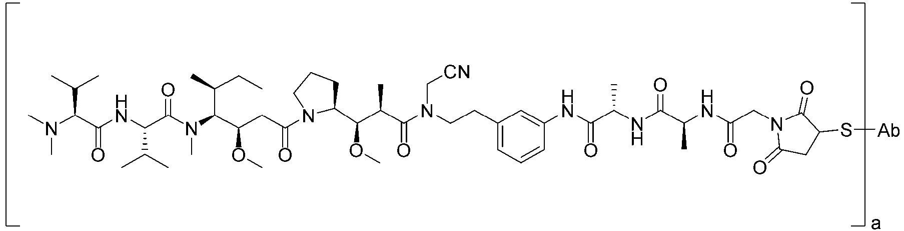

The trastuzumabab was buffer-exchanged into a 10 mM phosphate buffer at pH 7.0 by desalting chromatography or ultrafiltration. The antibody concentration was determined using the UV method. A 10 mM TCEP solution (3.0 eq.) was added to the antibody solution (10 mg/mL, 3 mL). After uniform mixing, the mixture was reduced at 8°C for 3.5 h. Subsequently, a 10 mM solution of compound 46 in dimethyl sulfoxide (6 eq.) was added. After uniform mixing, the mixture was reacted at 8°C for 2 h. After completion of the coupling reaction, the reaction liquid was desalted and purified using a Sephadex G25 gel column (elution phase: a solution containing 0.02 M His-HAc at pH 5.5) and filtered through a 0.2 µm filter to obtain ADC-6 in a PBS buffer (4.52 mg/mL, 5.6 mL), which was stored frozen at 4°C. The average number of toxins linked per antibody molecule (DAR) calculated by RP-HPLC was: a = 4.34. The SEC purity was: 98.8%.

### Biological Test Example 1: Cytotoxicity Test for Compounds

96-well clear flat-bottom white plate (Corning, #3610)
RPMI1640 culture medium (Gibco, #A10491-01)
Fetal Bovine Serum (Sigma-Aldrich, #F8687-500ML)
CellTiter-Glo^{®} Luminescent Cell Viability Assay (Promega, # G7572/G7571)

### Experimental method:

Human gastric cancer cells NCI-N87 (ATCC, Cat. No.: CRL-5822), human breast cancer cells MDA-MB-468 (Cell Bank of the Committee for Type Culture Collection of the Chinese Academy of Sciences, Cat. No.: TCHu136), and human breast cancer cells KPL-4 (Nanjing Cobioer Biosciences Co., Ltd., Cat. No.: CBP60379) were washed with PBS and digested with 0.25% Trypsin-EDTA trypsin for about 3-10 min. The digestion was terminated with a complete cell culture medium. The cells were centrifuged at 1000 rpm for 5 min, and the supernatant was discarded. The cells were resuspended in a complete cell culture medium. The cell number was counted using a cell counter, and the cells were adjusted to the desired density. 90.0 µL of the cell suspension was added to each well at 3000 cells/well. The cell plate was incubated in a cell incubator at 37°C with 5% CO₂ overnight.

In the experiment, a DMSO control group and test sample groups were set. The cell plate was removed from the incubator to observe cell adherence. After the cells adhered, 10.00 µL of the sample (starting from a final concentration of 10 µM, 5-fold dilution, 9 concentrations) was added to each well. After the mixture was gently shaken uniformly, the plate was incubated in the incubator.

After 3 days of incubation, 100.0 µL/well of CellTiter-Glo^{™} (Promega, Cat. No.: G7572/G7571) working solution was added. The plate was shaken on a thermostatic shaker to lyse the cells. After 10 min, the plate was read on a microplate reader.

Formula for calculating the cell proliferation inhibition rate: cell proliferation inhibition rate = (1 - RLU_{test sample well}/RLU_{DMSO control well}) × 100%.

Data analysis: GraphPad Prism 8.0 software was used to plot a graph with the Log value of the sample concentration as the abscissa and the Inhibition% as the ordinate. The data were analyzed by Nonlinear regression (curve fit) to obtain the IC₅₀ value of each test sample.

**Table 1. Inhibition activity of compounds of the present disclosure on cell proliferation**

| Example compound | KPL-4/IC₅₀ (nM) | NCI-87/IC₅₀ (nM) | MDA-MD-468/IC₅₀ (nM) |
|---|---|---|---|
| 1 | 0.41 | 0.74 | 0.63 |
| 2 | 0.21 | 0.51 | 0.36 |
| 3 | 18.9 | 38.39 | 25.13 |
| 4 | 5.26 | 14.66 | 6.12 |
| 5 | 3.56 | 4.28 | 8.81 |
| 6 | 2.96 | 3.93 | 12.61 |
| 7 | 0.45 | 0.89 | 13.40 |
| 8 | 0.14 | 0.66 | 0.56 |
| 9 | 0.74 | 3.84 | 3.34 |
| 10 | 1.25 | 3.35 | 1.97 |
| 11 | 0.10 | 0.15 | 3.00 |
| 12 | 1.42 | 4.05 | 11.79 |
| 13 | 0.18 | 0.58 | 0.56 |
| 14 | 1.13 | 5.13 | 3.82 |
| 15 | 2.00 | 11.30 | 2.74 |
| 16 | 0.91 | 3.72 | 3.18 |
| 17 | <0.0256 | <0.0256 | <0.0256 |
| 18 | <0.0256 | <0.0256 | <0.0256 |
| 19 | <0.0256 | <0.0256 | <0.0256 |
| 20 | <0.0256 | 0.21 | 0.12 |
| 21 | <0.0256 | <0.0256 | <0.0256 |
| 22 | 0.37 | 3.85 | 0.34 |
| 24 | <0.0256 | <0.0256 | <0.0256 |
| 25 | <0.0256 | <0.0256 | <0.0256 |
| 27 | 0.50 | 7.10 | 4.58 |
| 28 | <0.0256 | 3.33 | 3.25 |
| 29 | <0.0256 | 3.73 | 3.17 |
| 30 | 2.42 | 5.62 | 0.72 |
| 31 | <0.0256 | <0.0256 | 0.11 |
| 32 | <0.0256 | <0.0256 | <0.0256 |
| 45 | 0.65 | 2.21 | NT |
| 48 | 2.06 | 4.73 | NT |
| 51 | 6.03 | 16.06 | NT |
| 60-P1 | 5.81 | 9.63 | 8.02 |
| 60-P2 | 0.6 | 0.71 | 1.01 |
| 61-P1 | 49.53 | 93.94 | 34.63 |
| 61-P2 | 24.27 | 56.3 | 13.32 |

| | | | |
|---|---|---|---|
| Note: NT denotes not tested. | | | |

Conclusion: The compounds of the present disclosure have relatively strong inhibitory effects on the proliferation of KPL-4, NCI-87, and MDA-MD-468 cells.

### Biological Test Example 2: Hematologic Toxicity of Compounds

Experimental objective: The effects of cytotoxic drugs on the differentiation and development of CD34⁺ hematopoietic stem/progenitor cells into the megakaryocytic lineage were investigated, so as to evaluate their potential hematologic toxicity of causing thrombocytopenia.

Experimental design: After resuscitation, bone marrow-mobilized peripheral blood-derived HSCs were seeded at 2000 cells/well/180 µL in an ultra-low attachment 96-well round-bottom plate and stabilized for 4 h. The cells were treated with compounds by performing a 5-fold gradient dilution starting from the highest dose of 1 µM, resulting in a total of 9 concentration points, and this treatment was maintained continuously for 10 days. Cell viability was detected using the CTG reagent (CellTiter-Glo luminescent cell viability detection kit, manufacturer: Promega, Cat. No.: G7571).

**Table 2. Hematologic toxicity test results of cytotoxic drugs**

| No. | CD34⁺/IC₅₀(nM) |
|---|---|
| Example 6 | 3.962 |
| Example 7 | 0.144 |
| Example 45 | 0.235 |
| WO2023081230A1 Compound 1 | 0.017 |

The structure of the control compound is as follows:

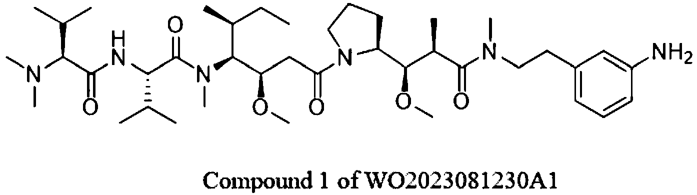

Conclusion: Compared with compound 1 of WO2023081230A1, the compounds of the present disclosure have weaker inhibitory effects on CD34⁺ cells, indicating their lower potential hematologic toxicity.

### Biological Test Example 3: In Vitro Plasma Stability

Experimental objective: To detect the stability of the HER2-ADC drugs of the present disclosure in *in vitro* plasma from various species.

Experimental method: Under sterile conditions, the ADC samples, healthy human plasma (donated by colleagues from the department of Qilu Pharmaceutical Staff Hospital), SD rat plasma (Shandong Xinbo Pharmaceutical Research Co., Ltd.), and CD1 mouse plasma (Beijing IPhase Pharma Services Co., Ltd.) were separately filtered and sterilized using a 0.22 µm filter. The drug stock solution was diluted to 100 µg/mL using plasma from different species and incubated in a cell incubator at 37°C. The day of incubation initiation was recorded as day 0. Samples were then taken out on day 21 for free toxin detection.

**Free toxin detection method:** 300 µL of acetonitrile solution containing an internal standard (internal standard: 0.1 ng/mL aprepitant) was added to a prepared biological sample or a biological sample with unknown concentration (50 µL). The mixture was vortexed for uniform mixing for 5 min and centrifuged at 4000 rpm at a low temperature (4°C) for 10 min. 100 µL of the supernatant was added to 100 µL of aqueous solution containing 0.2% formic acid, and the mixture was vortexed for 3 min and transferred to a 96-well sample feeding plate for LC-MS/MS analysis (Shimadzu Exion LC-AB Triple Quad^{®}5500+). The release rates of free toxin for the Her2-ADC compounds of the examples of the present disclosure in plasma are shown in Table 3.

**DAR value detection method:** Human plasma samples were captured using human her2 protein beads. PBST (EZ-Buffers E 10X PBST Buffer, manufacturer: Sangon, Cat. No.: C520004-0500) was added, followed by incubation with shaking at 1000 rpm for 1 h. After sequential washing with 200 µL each of PBST, PBS (pH 7.4, manufacturer: BasalMedia, Cat. No.: B320KJ), ultrapure water, and 10% acetonitrile, elution was performed with 100 µL of 20% acetonitrile (containing 0.5% formic acid) for 5 min. The entire supernatant was collected. 20 µL of 100 mM TCEP (manufacturer: Sigma, Cat. No.: C4706-2G) was added, followed by reaction with shaking at 1000 rpm at room temperature for 1 h. The samples were then analyzed (QTOF-MS). The test results for DAR value loss rate in *in vitro* human plasma are shown in Table 4.

**Table 3. In vitro plasma stability test results of ADCs**

| Example No. | Free toxin release rate (%) of ADC samples on day 21 | | |
|---|---|---|---|
| | Human plasma | Rat plasma | Mouse Plasma |
| ADC-1 | 0.76% | 3.13% | 25.05% |
| ADC-2 | 0.12% | 0.87% | 1.36% |
| ADC-3 | 0.14% | 0.41% | 1.05% |
| ADC-4 | 0.34% | 1.11% | 1.25% |
| ADC-5 | 0.27% | 0.02% | 1.34% |

As can be seen from Table 3, the *in vitro* plasma stability of the ADCs of the present disclosure is superior to that of the control group ADC-1, with the free toxin detachment rates in various species all being lower than 2%.

**Table 4. DAR value loss rates of ADCs in in vitro human plasma**

| Example No. | DAR value decrease rate (%) in human plasma | | | |
|---|---|---|---|---|
| | Day 3 | Day 7 | Day 14 | Day 21 |
| ADC-1 | 59.8% | 64.8% | 68.0% | 80.4% |
| ADC-2 | 5.3% | 6.1% | 7.1% | 8.1% |
| ADC-3 | 0 | 0 | 0 | 0 |

As can be seen from Table 4, the DAR value of ADC-3 of the present disclosure does not decrease in human plasma, indicating that the stability of its cytotoxic drug-linker is superior to that of other ADCs.

### Biological Test Example 4: Hematologic Toxicity of ADCs

Experimental objective: The effects of ADC molecules on the differentiation and development of CD34⁺ hematopoietic stem/progenitor cells into the megakaryocytic lineage were investigated, so as to evaluate their potential hematologic toxicity of causing thrombocytopenia.

Experimental design: After resuscitation, bone marrow-mobilized peripheral blood-derived HSCs were seeded at 2000 cells/well/180 µL in an ultra-low attachment 96-well round-bottom plate and stabilized for 4 h. The cells were treated with compounds by performing a 3-fold gradient dilution starting from the highest dose of 1 µM, resulting in a total of 9 concentration points, and this treatment was maintained continuously for 10 days. Cell viability was detected using the CTG reagent. The experimental results are shown in Table 5.

**Table 5. Hematologic toxicity test results of ADCs**

| No. | CD34⁺/IC₅₀(nM) |
|---|---|
| ADC-4 | 423.7 |
| ADC-2 | 135.8 |
| ADC-3 | 346.7 |
| ADC-1 | 96.61 |

Conclusion: The ADCs of the present disclosure have relatively weak inhibitory effects on CD³⁴⁺ cells, relatively low hematologic toxicity, and a potentially beneficial effect of reducing hematologic adverse reactions.

### Biological Test Example 5: In Vitro Neurotoxicity Evaluation

Experimental objective: To investigate the inhibitory effects of ADCs on mouse dorsal root ganglion cells.

Experimental design: After resuscitation, mouse dorsal root ganglion cells (Wuhan Procell) were seeded into a 96-well plate at about 7500 cells/well. The cells were treated at a dose of 200 nM for a continuous duration of 5 days. Cell viability was detected using the CTG reagent. The test results are shown in Table 6.

**Table 6. Inhibition rates of ADCs on mouse dorsal root ganglion cells**

| Example No. | DRG Neuron inhibition rate (%) @200 nM |
|---|---|
| ADC-1 | 46.74 |
| ADC-3 | 22.81 |
| ADC-5 | 28.90 |
| ADC-4 | 3.84 |
| ADC-6 | 5.14 |

Conclusion: At the same concentration, the ADCs of the present disclosure exhibit significantly weaker inhibitory effects on mouse dorsal root ganglion cells compared with the ADC-1 control group, indicating higher safety.

### Biological Test Example 6: In Vitro Bystander Effects

The bystander effect refers to the additional killing ability exhibited by an ADC against cells with low or no antigen expression in the presence of antigen-expressing cells. The objective of this experiment is to evaluate the *in vitro* bystander effects of the ADC drugs in the present disclosure.

The method used in this test was the medium transfer method.

Her2-positive cell seeding: SKBR3 cells (Cell Resource Center, Shanghai Institutes for Biological Sciences, Chinese Academy of Sciences) were collected and centrifuged at 1000 rpm for 5 min. The supernatant was discarded. The cells were resuspended in 1-2 mL of complete culture medium (complete culture medium: DMEM + 10% FBS). The cell suspension was mixed with an equal volume of trypan blue staining solution. The cells were counted using a Countstar counter. The cell density was adjusted to 5 × 10⁴ cells/mL with a complete culture medium. According to the "sample layout", the adjusted cells were added to the corresponding 96-well plate at 100.0 µL/well. The plate was cultured overnight. The plate edges were sealed with PBS at 200 µL/well.

Co-incubation with Her2-positive cells for 6 days: The test sample was diluted into a 2× working solution with a complete culture medium (a starting concentration of 100 nM, 3-fold gradient, 10 concentrations). The prepared test sample was added to the corresponding 96-well plate at 100.0 µL/well. After the addition was completed, the culture plate was gently tapped to mix uniformly. The 96-well plate was incubated in an incubator for 6 d.

Her2-negative cell seeding: MDA-MB-468 cells were collected and centrifuged at 1000 rpm for 5 min. The supernatant was discarded. The cells were resuspended in 1-2 mL of complete culture medium (complete culture medium: RPMI1640 + 10% FBS). The cell suspension was mixed with an equal volume of trypan blue staining solution. The cells were counted using a Countstar counter. The cell density was adjusted to 2 × 10⁴ cells/mL with a complete culture medium. According to the "sample layout", the adjusted cells were added to the corresponding 96-well plate at 100.0 µL/well. The plate was cultured overnight. An MDA-MB-468 administration plate (with 100 µL of SKBR3 cell supernatant) was set up. The plate edges were sealed with PBS at 200 µL/well.

Medium transfer method: The supernatant from the Her2-positive cell administration plate was transferred to the MDA-MB-468 administration plate at 100 µL/well. The plate was incubated in an incubator for 6 d. After the incubation was completed, the 96-well cell culture plate was taken out and left to stand at room temperature for 30 min. 50.0 µL of CellTiter-Glo luminescent dye was added to each well, and the plate was shaken on a microplate thermostatic oscillator at 200 rpm at room temperature for 10 min. The above operations were performed in the dark.

Data processing: The data from this experiment were processed using the data processing software GraphPad prism8.0.

**Table 7. Bystander effects of ADCs**

| Cell line | MDA-MB-468 (bystander effect) | |
|---|---|---|
| Sample name | IC₅₀ (nM) | Maximum inhibition rate (%) |
| ADC-2 | 0.53 | 86.6 |
| ADC-1 | 0.15 | 84.2 |
| ADC-5 | 0.04 | 92.77 |

Conclusion: As can be seen from Table 7, the bystander effect of ADC-5 is stronger than that of the control ADC.

### Biological Test Example 7: Evaluation of In Vivo Efficacy of Test Substances in NCI-N87 Human Breast Cancer Model

BALB/c nude mice were used as experimental animals to evaluate the *in vivo* efficacy of the ADCs of the present disclosure and the control ADC against NCI-N87 xenograft tumors.

Female BALB/c nude mice, purchased from Beijing Vital River Laboratory Animal Technology Co., Ltd., were acclimatized and reared in an SPF animal facility for 7 days for subsequent studies. NCI-N87 cells were inoculated subcutaneously at the right axilla of the nude mice at 5 × 10⁶ cells/0.1 mL. When the mean tumor volume reached about 150 mm³, 66 mice with moderate tumor volumes were selected and divided into 11 groups, with 6 mice per group. A single intravenous injection was administered on the day of grouping, which was day 10 after cell inoculation, and the Vehicle group received 0.9% sodium chloride injection. Tumor size was measured and body weight was recorded twice weekly. The long and short diameters of the tumor were measured using a vernier caliper. Tumor volume (mm³) = 0.5 × long diameter × short diameter. Tumor inhibition rate (%) = (Crtv - Trtv)/Crtv (%), where Crtv and Trtv are the relative tumor volumes of the blank control group (Vehicle, PBS) and the experimental group, respectively, at the end of the experiment.

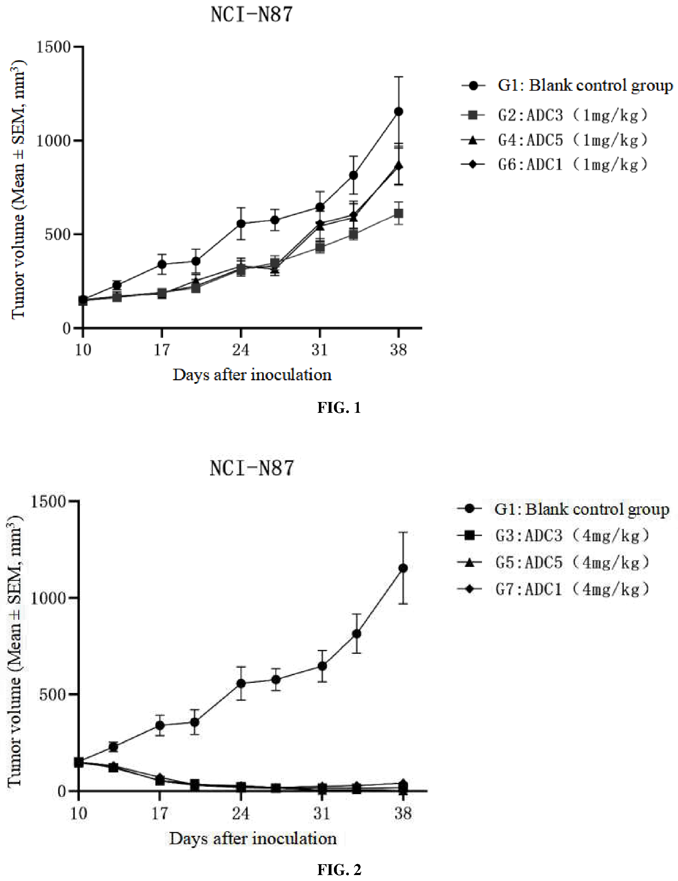

The experimental results are shown in Table 8 and FIGs. 1-2. Compared with the Vehicle group, all test molecules can significantly inhibit the growth of NCI-N87 tumors. In the low-dose groups (1 mpk), the efficacy of ADC-3 of the present disclosure was slightly better than that of the ADC-1 control group, and the efficacy of ADC-4 and ADC-5 was substantially comparable to that of the ADC-1 control group. In the high-dose groups (4 mpk), the anti-tumor activity of ADC-3 and ADC-5 of the present disclosure was slightly better than that of the ADC-1 control group. The body weight of mice in each administration group showed an increasing trend, and the drugs were well tolerated.

In conclusion, the drugs described in the present disclosure exhibit good efficacy and safety, possessing great research and application value.

**Table 8. Evaluation indicators for anti-tumor efficacy of test substances**

| Group | Tumor volume on day 38^{a} | Tumor inhibition rate (%)^{b} |
|---|---|---|
| G1: blank control group | 1155.1±185.6 | / |
| G2: ADC-3(1 mg/kg) | 613.4±60.1 | 53.5 |
| G3: ADC-3(4 mg/kg) | 18.9±8.0 | 113.3 |
| G4: ADC-5(1 mg/kg) | 877.4±108.7 | 27.0 |
| G5: ADC-5(4 mg/kg) | 4.5±1.5 | 114.3 |
| G6: ADC-1(1 mg/kg) | 862.4±98.2 | 29.2 |
| G7: ADC-1(4 mg/kg) | 42.1±9.9 | 110.9 |

| | | |
|---|---|---|
| Note: a, mean ± standard error; b, tumor inhibition rate (%) = [1 - (T₃₈ - T₁₀)/(V₃₈ - V₁₀)] × 100%. | | |

### Biological Test Example 8: Rat Tolerance Experiment

Experimental protocol: Tail vein injection was administered once every 3 weeks. Each group consisted of 3 male rats (SD rats, SPF grade, purchased from Hunan SJA Laboratory Animal Co., Ltd.). The administration doses were: 10 mpk, 20 mpk, 30 mpk, 40 mpk, and 60 mpk, respectively. Changes in rat body weight were observed, and blood routine and blood biochemical indicators were determined.

Clinical examination and observation: Body weight indicators and mortality were observed, and abnormal indicators were recorded.

**Table 9. Changes in rat physical signs after administration in each dose group**

| Example No. | DAR value | 10mpk | 20mpk | 30mpk | 40mpk | 60mpk |
|---|---|---|---|---|---|---|
| ADC-4 | 8 | No abnormalities | No abnormalities | No abnormalities | No abnormalities | No abnormalities |
| ADC-3 | 8 | No abnormalities | No abnormalities | No abnormalities | No abnormalities | No abnormalities |
| ADC-2 | 8 | No abnormalities | Perigenital hair loss, testicular swelling | Perigenital hair loss, testicular swelling | Perigenital hair loss, testicular swelling | Perigenital hair loss, testicular swelling |
| ADC-1 | 4 | No abnormalities | No abnormalities | Body weight decreased by 2% | D5, one rat died, and one was moribund | D3, all died |

Conclusion: As can be seen from Table 9, in the SD rat model, the maximum tolerated dose of the ADC-1 control group was 30 mpk, while the tolerated doses of the other groups were higher than 60 mpk. Compared with the control group ADC-1 and ADC-2, ADC-3 and ADC-4 of the present disclosure showed no abnormalities at any dose level, indicating good safety. In contrast, the control group ADC-1 and ADC-2 exhibited reproductive toxicity, body weight decrease, or mortality at medium and high doses.

**Table 10. Blood routine data on day 4 after administration in healthy rats and rats in each administration group at 10 mpk dose**

| **Test item** | Unit | Healthy rat | ADC-2 | ADC-4 | ADC-3 | ADC-1 |
|---|---|---|---|---|---|---|
| White blood cell count | ×10^9/L | 11.04±2.38 | 9.07±1.92 | 12.26±1.71 | 10.32±1.56 | 5.96±0.52 |
| Neutrophil count | ×10^9/L | 1.52±0.23 | 1.11±0.25 | 1.4±0.17 | 1.75±0.52 | 0.52±0.07 |
| Reticulocyte count | K/µL | 783.8±40.5 | 715.9±44.1 | 710.2±30.4 | 725.8±78.2 | 60.8±14.9 |
| Platelet | K/µL | 803±127.7 | 809±22.7 | 915.7±50.6 | 789.3±104.6 | 911.7±57.4 |
| Lymphocyte count | ×10^9/L | 8.34±1.88 | 7.07±1.58 | 9.6±1.59 | 7.44±1.01 | 4.65±0.5 |
| Monocyte count | ×10^9/L | 1.06±0.4 | 0.76±0.14 | 1.12±0.05 | 0.93±0.23 | 0.75±0.02 |

Conclusion: At the 10 mpk dose, the ADC-1 control group showed significant reductions in white blood cell count, neutrophil count, lymphocyte count, monocyte count, and reticulocyte count on day 4 after administration, while the ADCs disclosed in the present disclosure showed no abnormalities in blood routine indicators. White blood cells are further divided into lymphocytes, neutrophils, eosinophils, basophils, monocytes, etc., with lymphocytes and neutrophils being the most important. A decrease in white blood cells indicates that the drug has a myelosuppressive effect and can easily lead to secondary severe infections. Reticulocyte count is an important indicator reflecting bone marrow hematopoietic function. A decrease in reticulocyte indicates reduced bone marrow hematopoietic function, commonly seen in aplastic anemia and myelophthisic anemia. The above data indicate that under the 10 mpk condition, the ADC-1 control group exhibited obvious hematologic toxicity, while the ADCs disclosed in the present disclosure showed no obvious hematologic toxicity.

**Table 11. Liver function on day 4 after administration in healthy rats and rats in each administration group at 40 mpk dose**

| **Test item** | Unit | Healthy rat | ADC-2 | ADC-4 | ADC-3 | ADC-1 |
|---|---|---|---|---|---|---|
| **Alanine transaminase** | U/L | 37.8±5 | 31±11 | 39.1±5.3 | 33.2±5 | 235±118.6 |
| **Aspartate transaminase** | U/L | 137±20.6 | 144.3±37.7 | 146.4±35.9 | 114.5±23.9 | 748.9±480.8 |

Conclusion: At the 40 mpk dose, the ADC-1 control group mainly showed increased alanine transaminase and aspartate transaminase after administration, indicating abnormal liver function and potential hepatotoxicity. No abnormalities in these two enzyme indicators were observed in the other groups.

## Claims

1. A cytotoxic drug represented by formula (I) or a stereoisomer, isotopic derivative, solvate, nitrogen oxide, prodrug, or pharmaceutically acceptable salt thereof: 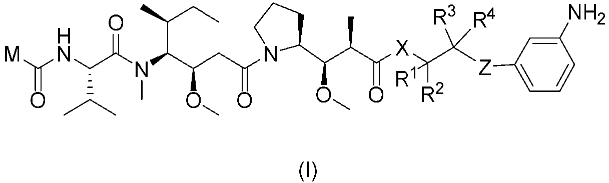
wherein M is selected from C₁₋₆ alkyl, C₃₋₆ cycloalkyl, and 3- to 8-membered heterocyclyl; the C₁₋₆ alkyl, C₃₋₆ cycloalkyl, or 3- to 8-membered heterocyclyl is optionally further substituted with a substituent selected from C₁₋₄ alkyl and NR^{m1}R^{m2}, and the 3- to 8-membered heterocyclyl contains 1-3 heteroatoms selected from N, O, and S;
R^{m1} and R^{m2} are each independently selected from H and C₁₋₄ alkyl;
X is selected from O and -N(R^{x1})-;
R^{x1} is selected from H, OH, C₁₋₄ alkyl, C₁₋₄ alkoxy, C₂₋₄ alkenyl, C₂₋₄ alkynyl, and C₃₋₆ cycloalkyl; the C₁₋₄ alkyl or C₃₋₆ cycloalkyl is optionally further substituted with 1-3 substituents selected from halogen, CN, deuterium, and C₃₋₆ cycloalkyl;
R³ and R⁴ are each independently selected from H and OH;
or, X and R³, together with the atoms to which they are attached, form a 4- to 8-membered heterocyclic ring, and the 4- to 8-membered heterocyclic ring contains 1-3 heteroatoms or groups selected from N, O, S, and S(O)₂;
or, X and R⁴, together with the atoms to which they are attached, form a 4- to 8-membered heterocyclic ring, and the 4- to 8-membered heterocyclic ring contains 1-3 heteroatoms or groups selected from N, O, S, and S(O)₂;
R¹ and R² are each independently selected from H, OH, C₁₋₄ alkyl, C₂₋₄ alkynyl, C₂₋₄ alkenyl, C₃₋₆ cycloalkyl, 3- to 8-membered heterocyclyl, -COOR^{1a}, and -CONR^{1b}R^{1c}; the C₁₋₄ alkyl, C₂₋₄ alkenyl, C₃₋₆ cycloalkyl, or 3- to 8-membered heterocyclyl is optionally further substituted with 1-3 substituents selected from CN, OH, N₃, halogen, -COOR^{1a}, -CONR^{1b}R^{1c}, and C₁₋₄ alkyl, and the 3- to 8-membered heterocyclyl contains 1-3 heteroatoms selected from N, O, and S;
R^{1a}, R^{1b}, and R^{1c} are each independently selected from H and C₁₋₄ alkyl;
or, R¹ and R², together with the carbon atom to which they are attached, form C₃₋₆ cycloalkyl or 3- to 8-membered heterocyclyl;
Z is selected from a chemical bond and -(CH₂)ₙ-, and n is selected from 1 and 2.

2. The cytotoxic drug or the stereoisomer, isotopic derivative, solvate, nitrogen oxide, prodrug, or pharmaceutically acceptable salt thereof according to claim 1:
wherein M is selected from C₁₋₆ alkyl, C₃₋₆ cycloalkyl, and 3- to 8-membered heterocyclyl; the C₁₋₆ alkyl, C₃₋₆ cycloalkyl, or 3- to 8-membered heterocyclyl is optionally further substituted with a substituent selected from C₁₋₄ alkyl and NR^{m1}R^{m2}, and the 3- to 8-membered heterocyclyl contains 1-3 heteroatoms selected from N, O, and S;
R^{m1} and R^{m2} are each independently selected from H and C₁₋₄ alkyl;
X is selected from O and -N(R^{x1})-;
R^{x1} is selected from H, OH, C₁₋₄ alkyl, C₁₋₄ alkoxy, C₂₋₄ alkenyl, and C₂₋₄ alkynyl; the C₁₋₄ alkyl is optionally further substituted with 1-3 substituents selected from halogen and CN;
R³ and R⁴ are each independently selected from H and OH;
or, X and R³, together with the atoms to which they are attached, form a 4- to 8-membered heterocyclic ring, and the 4- to 8-membered heterocyclic ring contains 1-3 heteroatoms or groups selected from N, O, S, and S(O)₂;
or, X and R⁴, together with the atoms to which they are attached, form a 4- to 8-membered heterocyclic ring, and the 4- to 8-membered heterocyclic ring contains 1-3 heteroatoms or groups selected from N, O, S, and S(O)₂;
R¹ and R² are each independently selected from H, OH, C₁₋₄ alkyl, C₂₋₄ alkynyl, C₂₋₄ alkenyl, C₃₋₆ cycloalkyl, 3- to 8-membered heterocyclyl, -COOR^{1a}, and -CONR^{1b}R^{1c}; the C₁₋₄ alkyl, C₂₋₄ alkenyl, C₃₋₆ cycloalkyl, or 3- to 8-membered heterocyclyl is optionally further substituted with 1-3 substituents selected from CN, OH, N₃, halogen, -COOR^{1a}, and -CONR^{1b}R^{1c}, and the 3- to 8-membered heterocyclyl contains 1-3 heteroatoms selected from N, O, and S;
R^{1a}, R^{1b}, and R^{1c} are each independently selected from H and C₁₋₄ alkyl;
or, R¹ and R², together with the carbon atom to which they are attached, form C₃₋₆ cycloalkyl or 3- to 8-membered heterocyclyl;
Z is selected from a chemical bond and -(CH₂)ₙ-, and n is selected from 1 and 2.

3. The cytotoxic drug or the stereoisomer, isotopic derivative, solvate, nitrogen oxide, prodrug, or pharmaceutically acceptable salt thereof according to claim 1 or 2:
wherein M is selected from C₁₋₆ alkyl, C₃₋₆ cycloalkyl, and 3- to 8-membered heterocyclyl; the C₁₋₆ alkyl, C₃₋₆ cycloalkyl, or 3- to 8-membered heterocyclyl is optionally further substituted with a substituent selected from C₁₋₄ alkyl and NR^{m1}R^{m2}, and the 3- to 8-membered heterocyclyl contains 1-3 heteroatoms selected from N, O, and S;
R^{m1} and R^{m2} are each independently selected from H and C₁₋₄ alkyl;
X is selected from O and -N(R^{x1})-;
R^{x1} is selected from H, OH, C₁₋₄ alkyl, C₁₋₄ alkoxy, C₂₋₄ alkenyl, and C₂₋₄ alkynyl; the C₁₋₄ alkyl is optionally further substituted with 1-3 substituents selected from halogen and CN;
R¹ and R² are each independently selected from H, OH, C₁₋₄ alkyl, C₂₋₄ alkynyl, C₂₋₄ alkenyl, C₃₋₆ cycloalkyl, 3- to 8-membered heterocyclyl, -COOR^{1a}, and -CONR^{1b}R^{1c}; the C₁₋₄ alkyl, C₂₋₄ alkenyl, C₃₋₆ cycloalkyl, or 3- to 8-membered heterocyclyl is optionally further substituted with 1-3 substituents selected from CN, OH, N₃, halogen, -COOR^{1a}, and -CONR^{1b}R^{1c}, and the 3- to 8-membered heterocyclyl contains 1-3 heteroatoms selected from N, O, and S;
R^{1a}, R^{1b}, and R^{1c} are each independently selected from H and C₁₋₄ alkyl;
or, R¹ and R², together with the carbon atom to which they are attached, form C₃₋₆ cycloalkyl or 3- to 8-membered heterocyclyl;
R³ and R⁴ are each independently selected from H and OH;
Z is selected from a chemical bond and -(CH₂)ₙ-, and n is selected from 1 and 2.

4. The cytotoxic drug or the stereoisomer, isotopic derivative, solvate, nitrogen oxide, prodrug, or pharmaceutically acceptable salt thereof according to any one of claims 1-3, wherein M is selected from C₁₋₄ alkyl, C₃₋₆ cycloalkyl, and 3- to 6-membered heterocyclyl; the C₁₋₄ alkyl, C₃₋₆ cycloalkyl, or 3- to 6-membered heterocyclyl is optionally further substituted with a substituent selected from C₁₋₄ alkyl and NR^{m1}R^{m2}, and the 3- to 6-membered heterocyclyl contains 1-3 heteroatoms selected from N, O, and S; R^{m1} and R^{m2} are each independently selected from H and C₁₋₂ alkyl.

5. The cytotoxic drug or the stereoisomer, isotopic derivative, solvate, nitrogen oxide, prodrug, or pharmaceutically acceptable salt thereof according to any one of claims 1-4, wherein M is selected from

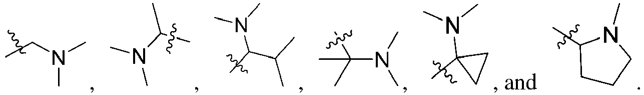

6. The cytotoxic drug or the stereoisomer, isotopic derivative, solvate, nitrogen oxide, prodrug, or pharmaceutically acceptable salt thereof according to any one of claims 1-5, wherein R^{x1} is selected from H, -CH₃, OH, -OCH₃,

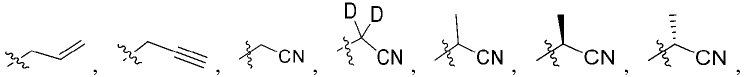

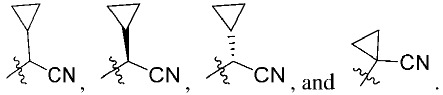

7. The cytotoxic drug or the stereoisomer, isotopic derivative, solvate, nitrogen oxide, prodrug, or pharmaceutically acceptable salt thereof according to any one of claims 1-6, wherein X is selected from O, -NH-, -N(CH₃)-,

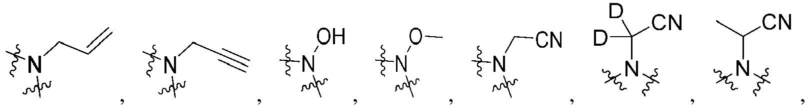

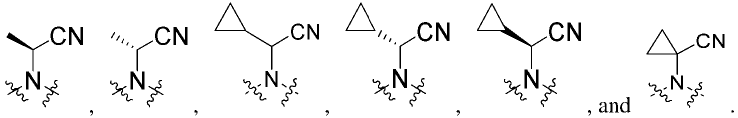

8. The cytotoxic drug or the stereoisomer, isotopic derivative, solvate, nitrogen oxide, prodrug, or pharmaceutically acceptable salt thereof according to any one of claims 1-7, wherein R¹ and R² are each independently selected from H, -CH₃, -CH₂OH, -CH₂F, -CHF₂, -CF₃, -CH₂Cl, 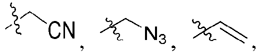 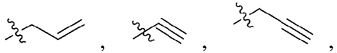 -COOH, -COOCH₃, -CONH₂, -CONHCH₃, 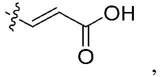 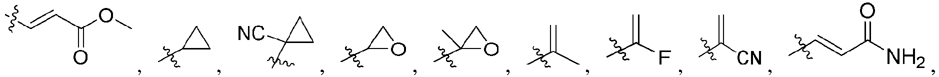 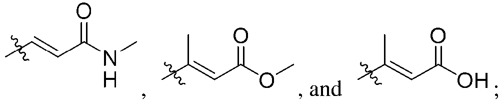 or, R¹ and R², together with the carbon atom to which they are attached, form 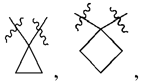 or

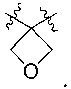

9. The cytotoxic drug or the stereoisomer, isotopic derivative, solvate, nitrogen oxide, prodrug, or pharmaceutically acceptable salt thereof according to any one of claims 1-8, wherein R³ and R⁴ are each independently selected from H and OH; preferably, R³ and R⁴ are each independently H.

10. The cytotoxic drug or the stereoisomer, isotopic derivative, solvate, nitrogen oxide, prodrug, or pharmaceutically acceptable salt thereof according to any one of claims 1-9, wherein X and R³, together with the atoms to which they are attached, form a 5- to 6-membered heterocyclic ring, and the 5- to 6-membered heterocyclic ring contains 1-3 heteroatoms or groups selected from N, O, S, and S(O)₂;
or, X and R⁴, together with the atoms to which they are attached, form a 5- to 6-membered heterocyclic ring, and the 5- to 6-membered heterocyclic ring contains 1-3 heteroatoms or groups selected from N, O, S, and S(O)₂.

11. The cytotoxic drug or the stereoisomer, isotopic derivative, solvate, nitrogen oxide, prodrug, or pharmaceutically acceptable salt thereof according to any one of claims 1-10, wherein X and R³, together with the atoms to which they are attached, form 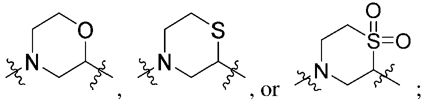 or, X and R⁴, together with the atoms to which they are attached, form 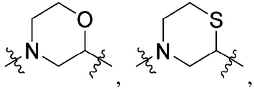 or

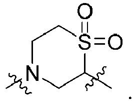

12. The cytotoxic drug or the stereoisomer, isotopic derivative, solvate, nitrogen oxide, prodrug, or pharmaceutically acceptable salt thereof according to any one of claims 1-11, wherein Z is selected from a chemical bond and -CH₂-.

13. The cytotoxic drug or the stereoisomer, isotopic derivative, solvate, nitrogen oxide, prodrug, or pharmaceutically acceptable salt thereof according to any one of claims 1-12, wherein the cytotoxic drug or the stereoisomer, isotopic derivative, solvate, nitrogen oxide, prodrug, or pharmaceutically acceptable salt thereof is selected from a compound of general formula (Ia), (Ib), or (Ic): 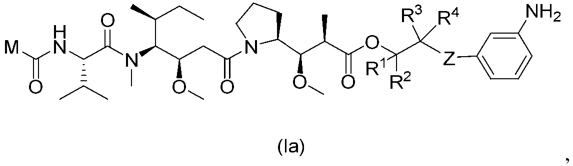 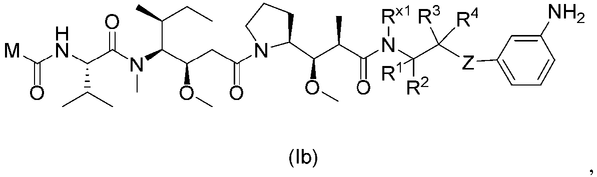 or 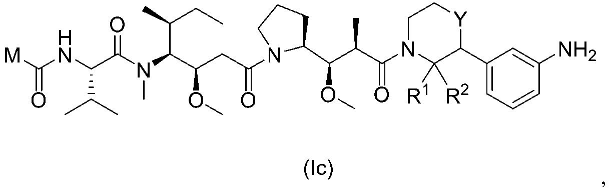 wherein Y is selected from O, S, and S(O)₂, and M, R¹, R², R³, R⁴, R^{x1}, and Z are as defined in any one of claims 1-12.

14. The cytotoxic drug or the stereoisomer, isotopic derivative, solvate, nitrogen oxide, prodrug, or pharmaceutically acceptable salt thereof according to any one of claims 1-13, wherein the cytotoxic drug or the stereoisomer, isotopic derivative, solvate, nitrogen oxide, prodrug, or pharmaceutically acceptable salt thereof is selected from a compound of general formula (IIa), (IIb), or (IIc): 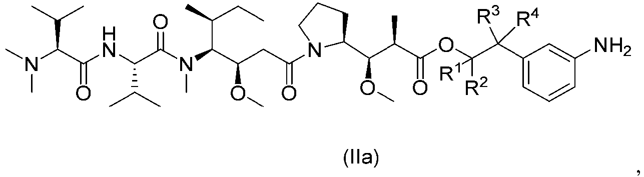 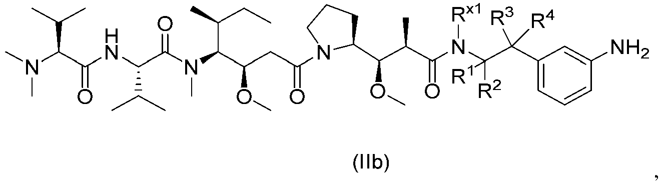 or 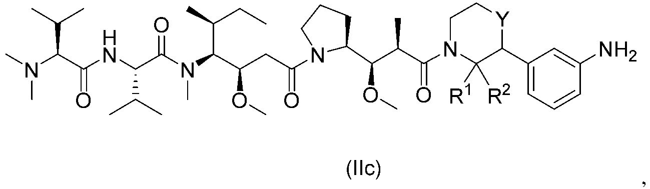 wherein Y is selected from O, S, and S(O)₂, and R¹, R², R³, R⁴, and R^{x1} are as defined in any one of claims 1-13.

15. The cytotoxic drug or the stereoisomer, isotopic derivative, solvate, nitrogen oxide, prodrug, or pharmaceutically acceptable salt thereof according to any one of claims 1-14, wherein the cytotoxic drug or the stereoisomer, isotopic derivative, solvate, nitrogen oxide, prodrug, or pharmaceutically acceptable salt thereof is selected from a compound of general formula (IIIa): 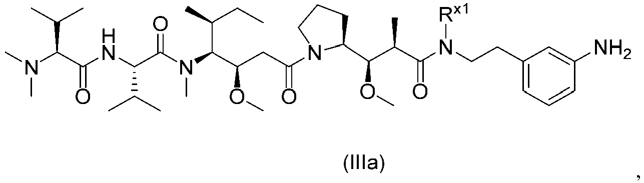 wherein R^{x1} is selected from OH, C₁₋₄ alkyl, C₁₋₄ alkoxy, and C₃₋₆ cycloalkyl, and the C₁₋₄ alkyl or C₃₋₆ cycloalkyl is further substituted with 1-3 substituents selected from deuterium, methyl, CN, and cyclopropyl; preferably, R^{x1} is selected from -OCH₃,

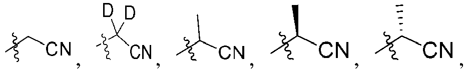

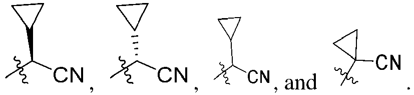

16. The cytotoxic drug or the stereoisomer, isotopic derivative, solvate, nitrogen oxide, prodrug, or pharmaceutically acceptable salt thereof according to claim 1, wherein the cytotoxic drug or the stereoisomer, isotopic derivative, solvate, nitrogen oxide, prodrug, or pharmaceutically acceptable salt thereof is selected from: 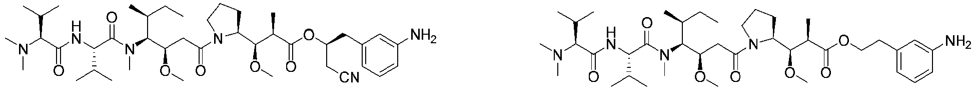 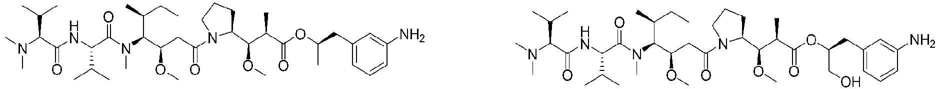 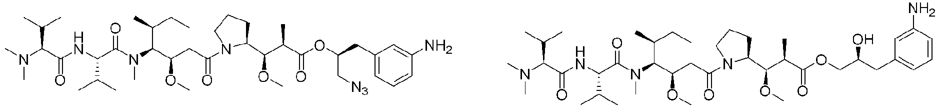 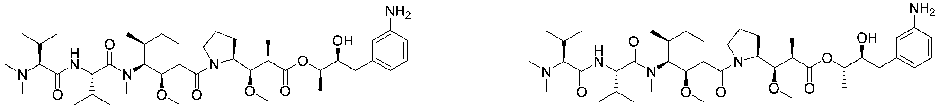 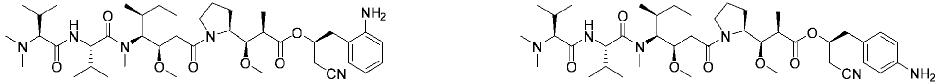 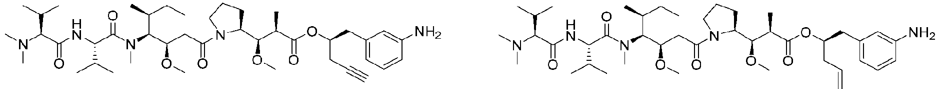 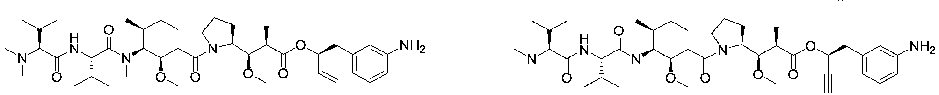 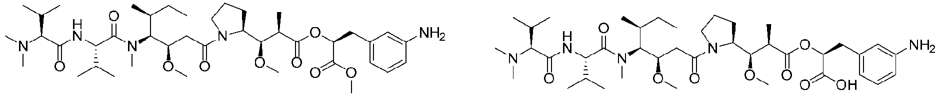 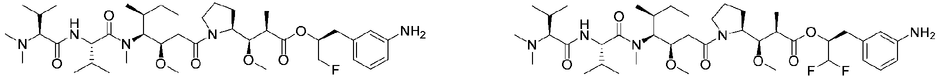 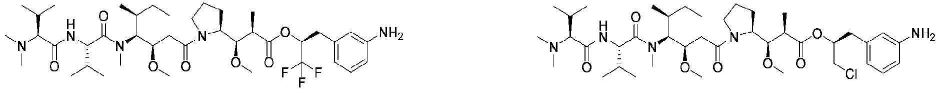 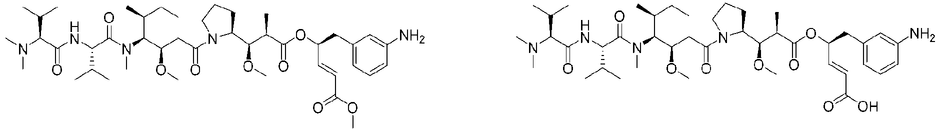 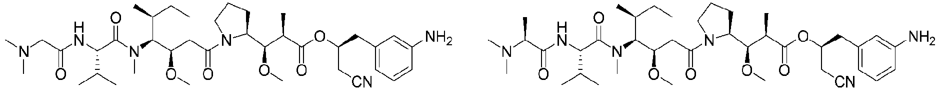 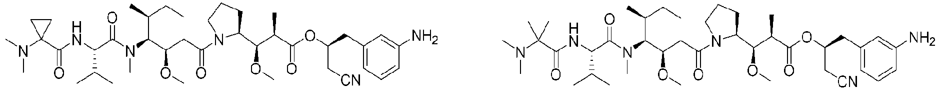 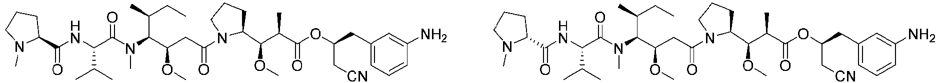 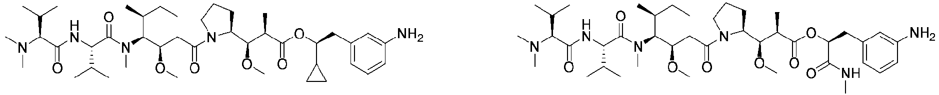 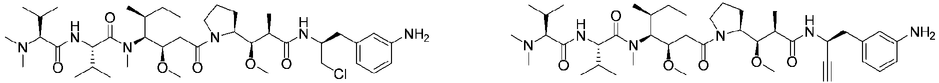 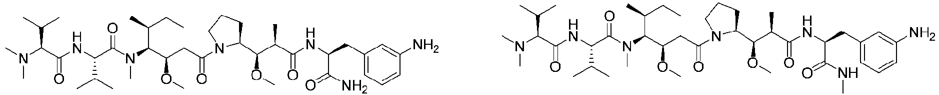 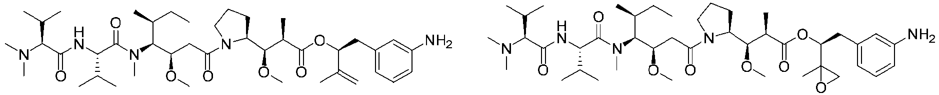 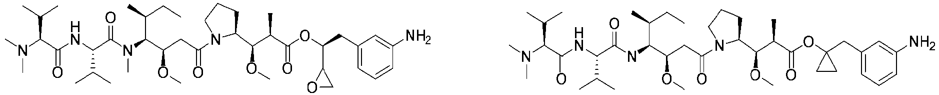 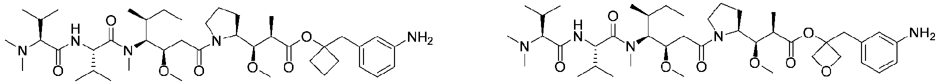 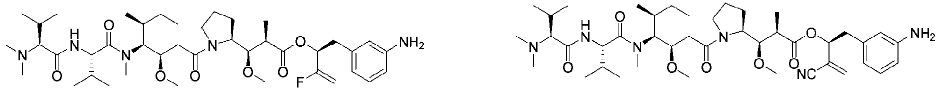 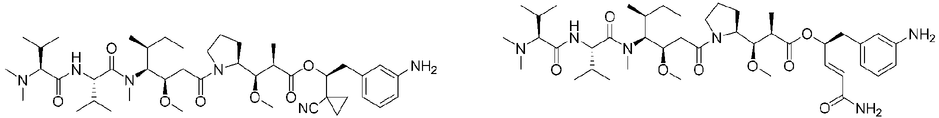 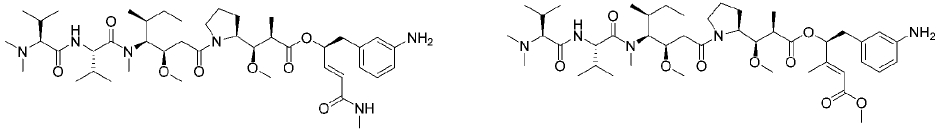 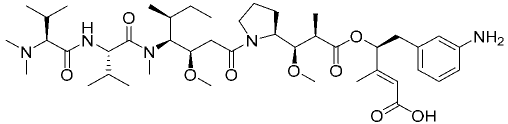 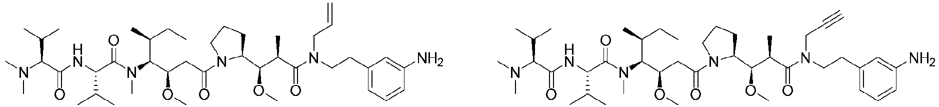 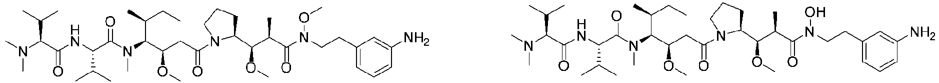 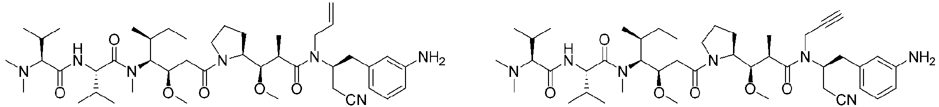 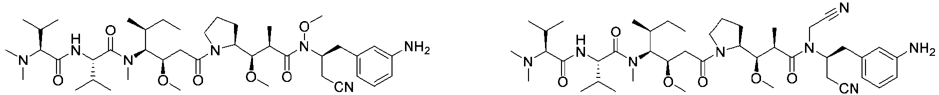 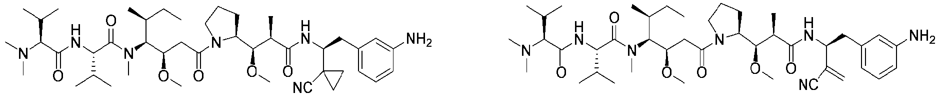 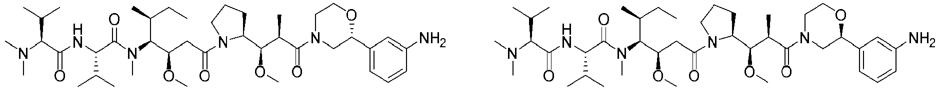 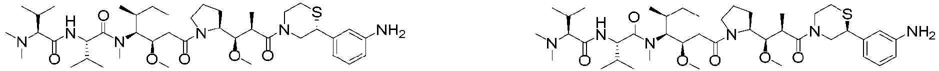 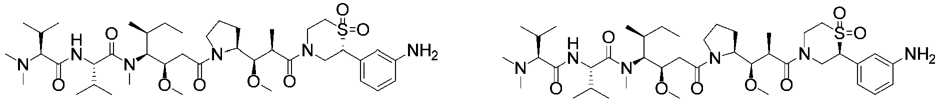 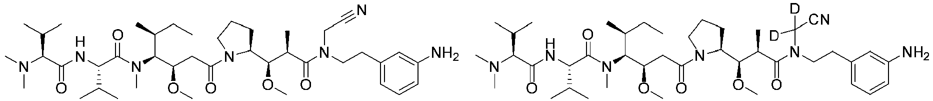 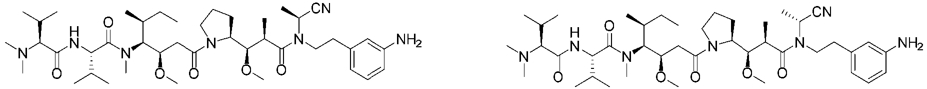 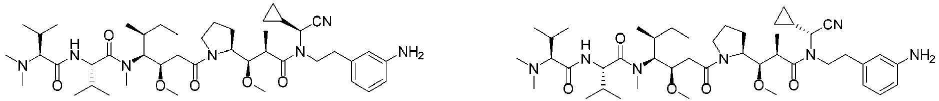 and

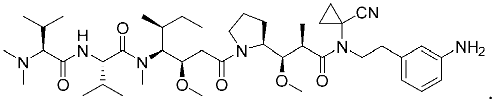

17. An antibody-drug conjugate or a stereoisomer, isotopic derivative, solvate, nitrogen oxide, prodrug, or pharmaceutically acceptable salt thereof, wherein the antibody-drug conjugate or the stereoisomer, isotopic derivative, solvate, nitrogen oxide, prodrug, or pharmaceutically acceptable salt thereof is selected from a structure represented by formula (A): 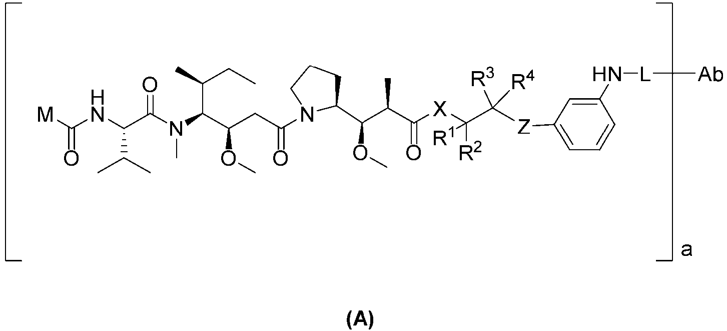
wherein Ab is an antibody or an antigen-binding fragment;
L is a linking group, with the left end thereof linked to NH on the cytotoxic drug and the right end thereof linked to Ab;
a is selected from a numerical value of 1-10;
M, R¹, R², R³, R⁴, X, and Z are as defined in any one of claims 1-16.

18. The antibody-drug conjugate or the stereoisomer, isotopic derivative, solvate, nitrogen oxide, prodrug, or pharmaceutically acceptable salt thereof according to claim 17, wherein the antibody-drug conjugate or the stereoisomer, isotopic derivative, solvate, nitrogen oxide, prodrug, or pharmaceutically acceptable salt thereof is selected from a structure represented by formula (A-1): 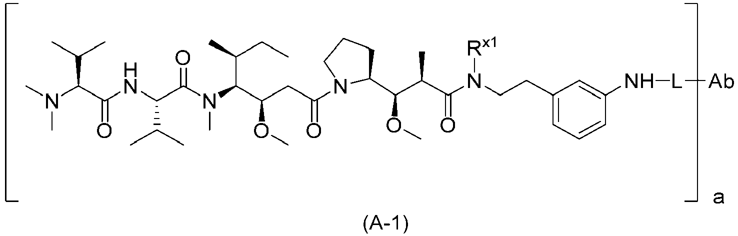
wherein Ab is a Her2 antibody; preferably, Ab is trastuzumab;
R^{x1} is selected from H, -CH₃, OH, -OCH₃, 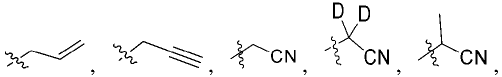 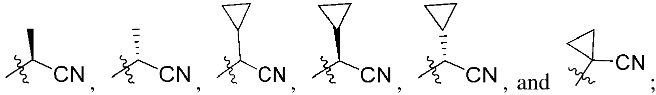 preferably, R^{x1} is selected from - OCH₃, 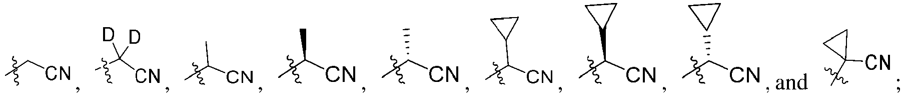
a is selected from a numerical value of 2-8; preferably, a is selected from a numerical value of 4-8;
L is as defined in claim 17.

19. The antibody-drug conjugate or the stereoisomer, isotopic derivative, solvate, nitrogen oxide, prodrug, or pharmaceutically acceptable salt thereof according to claim 17 or 18, wherein L is selected from: 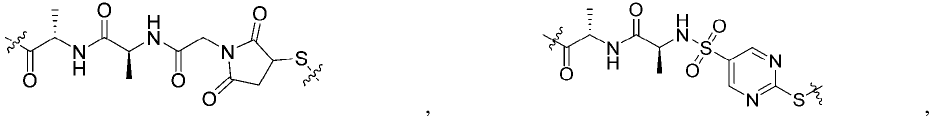 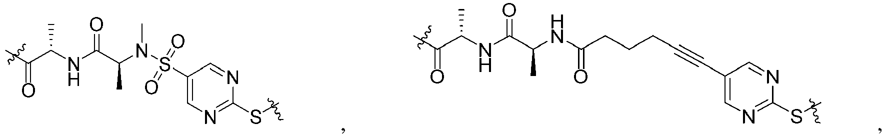 and

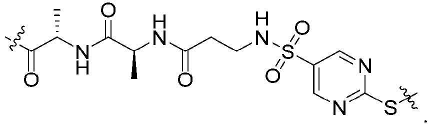

20. The antibody-drug conjugate or the stereoisomer, isotopic derivative, solvate, nitrogen oxide, prodrug, or pharmaceutically acceptable salt thereof according to any one of claims 17-19, wherein a is selected from a numerical value of 2-8; preferably, a is selected from a numerical value of 4-8.

21. A cytotoxic drug-linker or a stereoisomer, isotopic derivative, solvate, nitrogen oxide, prodrug, or pharmaceutically acceptable salt thereof, wherein the cytotoxic drug-linker has a structure represented by formula (B), 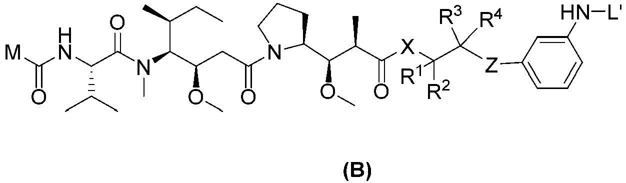
wherein L' is a structure of L before being linked to Ab according to any one of claims 17-20;
preferably, L' is selected from 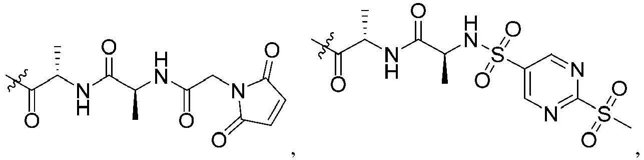 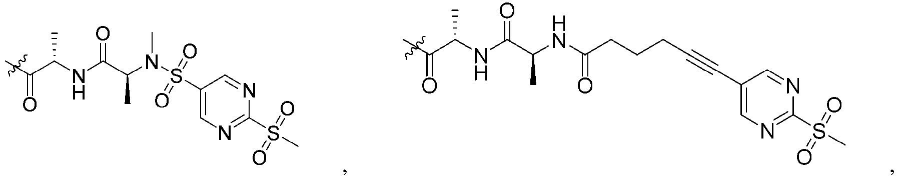 and 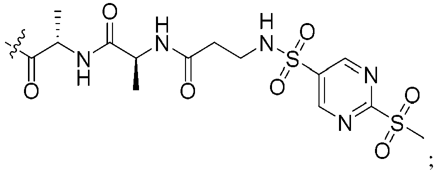
M, R¹, R², R³, R⁴, X, and Z are as defined in any one of claims 1-16.

22. The cytotoxic drug-linker or the stereoisomer, isotopic derivative, solvate, nitrogen oxide, prodrug, or pharmaceutically acceptable salt thereof according to claim 21, wherein the cytotoxic drug-linker or the stereoisomer, isotopic derivative, solvate, nitrogen oxide, prodrug, or pharmaceutically acceptable salt thereof is selected from a structure represented by formula (B-1): 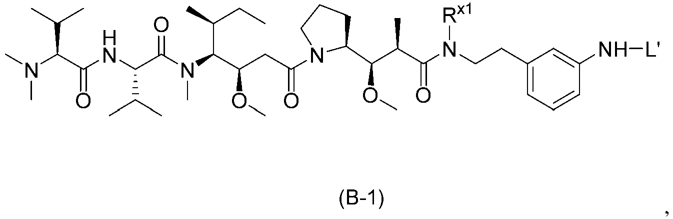
wherein R^{x1} is selected from H, -CH₃, OH, -OCH₃, 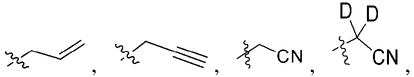 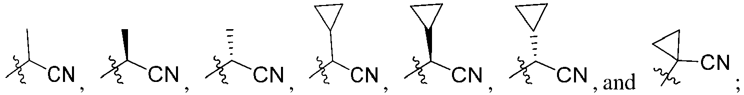 preferably, R^{x1} is selected from -OCH₃, 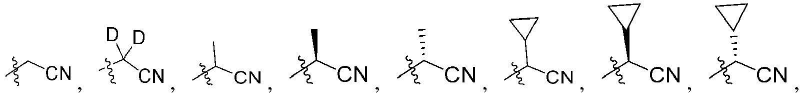
and 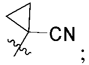
L' is selected from 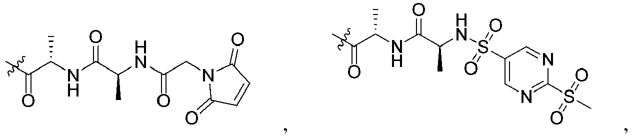 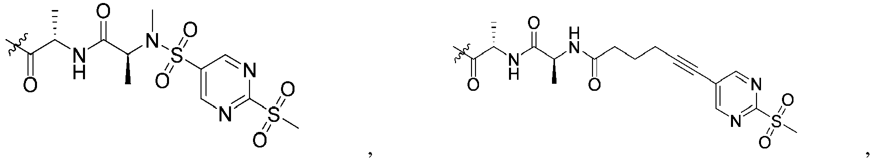 and 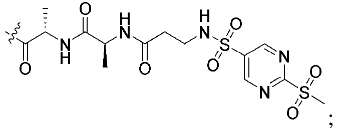 preferably, L' is

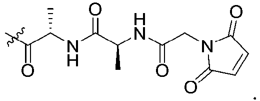

23. The cytotoxic drug-linker or the stereoisomer, isotopic derivative, solvate, nitrogen oxide, prodrug, or pharmaceutically acceptable salt thereof according to claim 21 or 22, wherein the cytotoxic drug-linker is selected from: 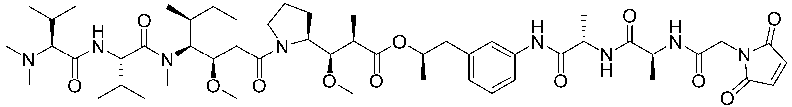 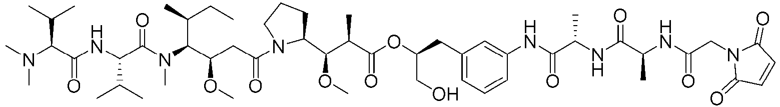 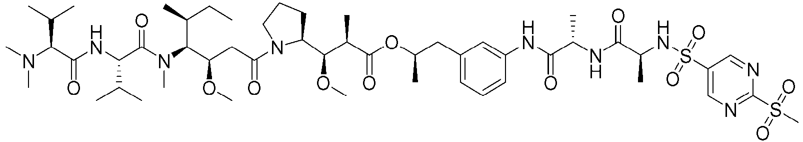 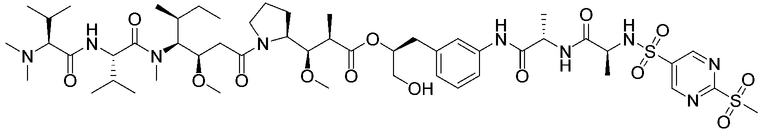 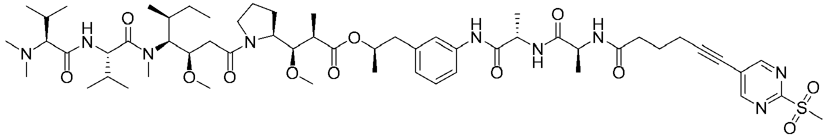 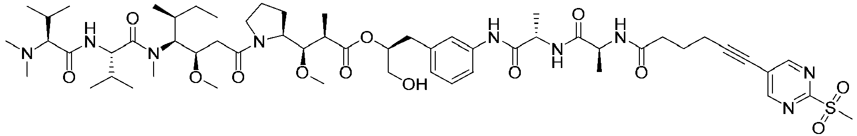 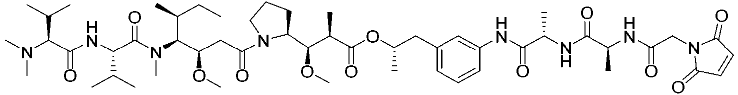 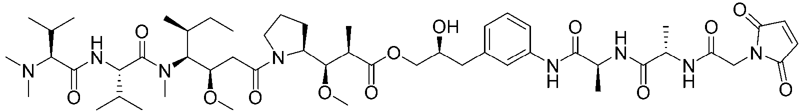 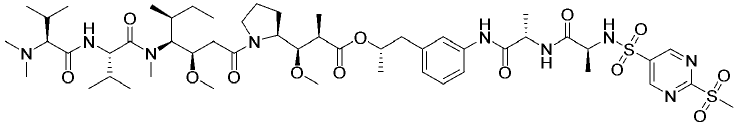 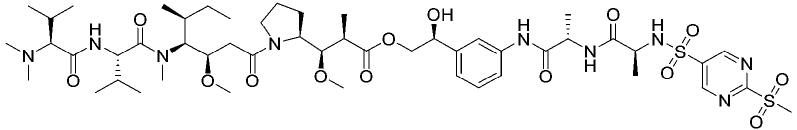 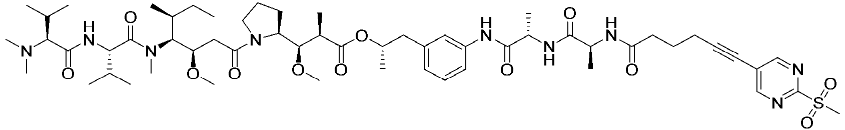 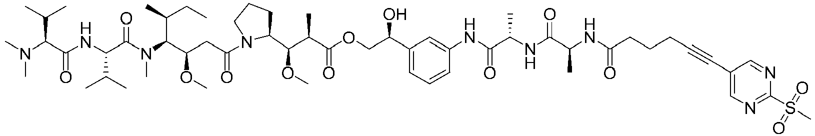 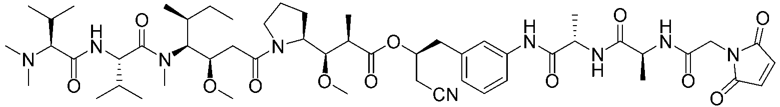 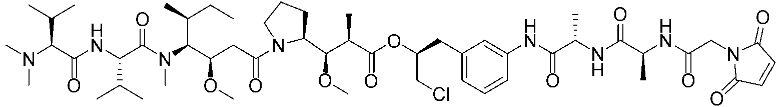 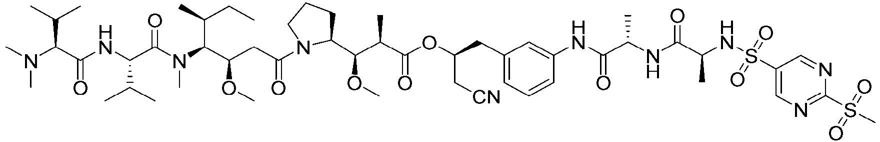 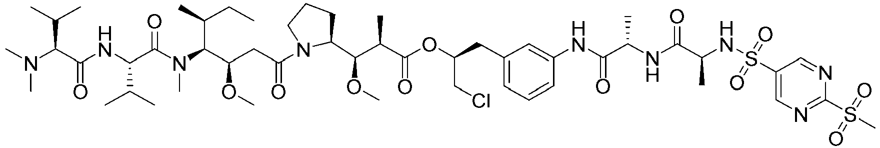 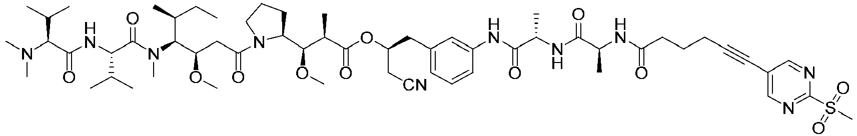 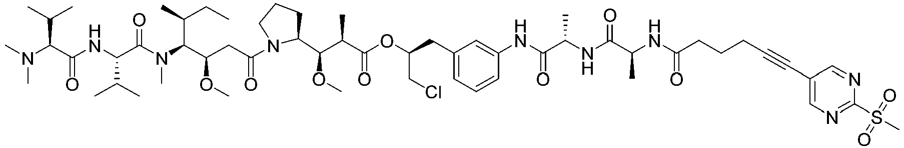 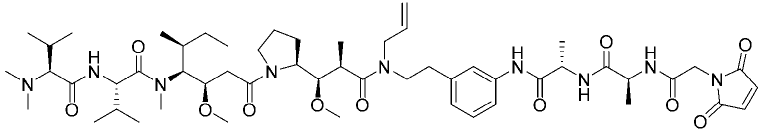 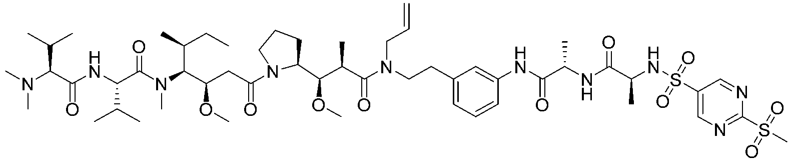 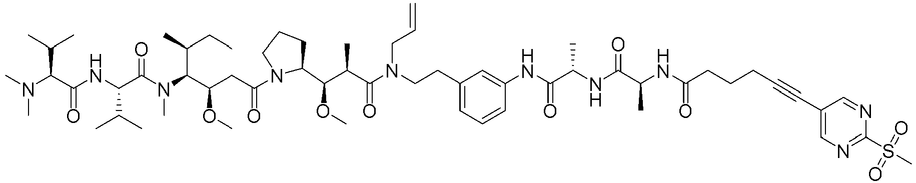 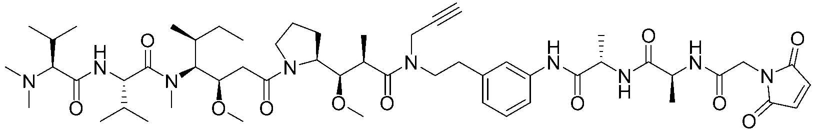 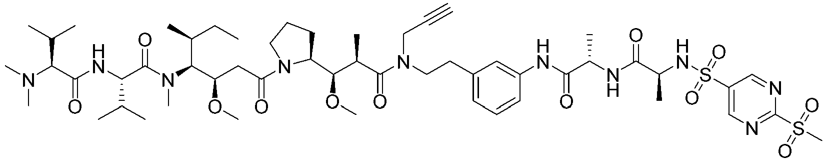 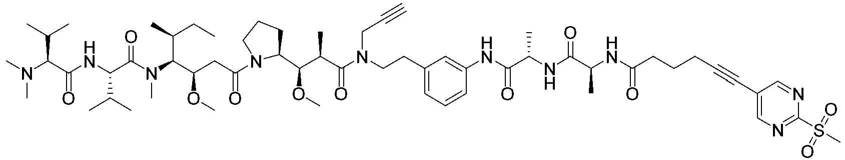 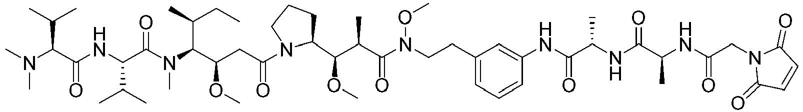 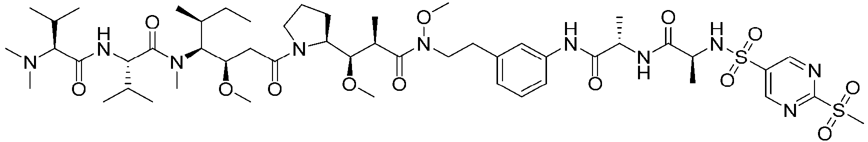 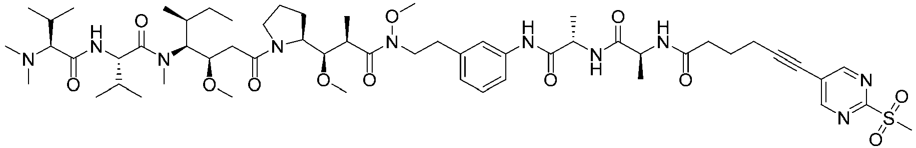 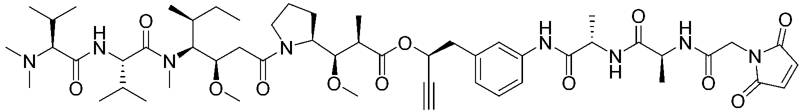 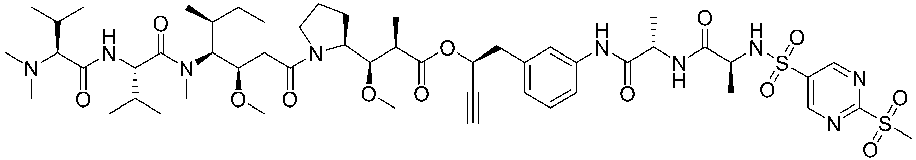 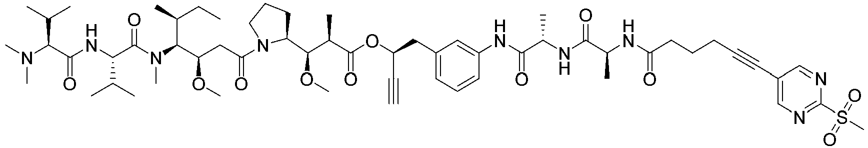 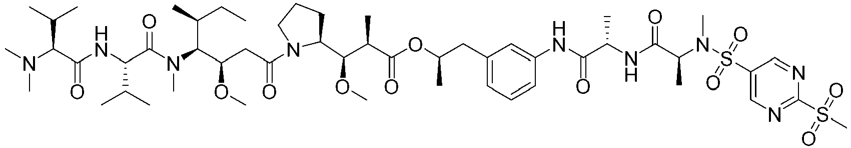 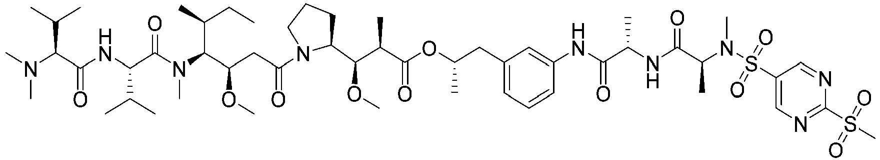 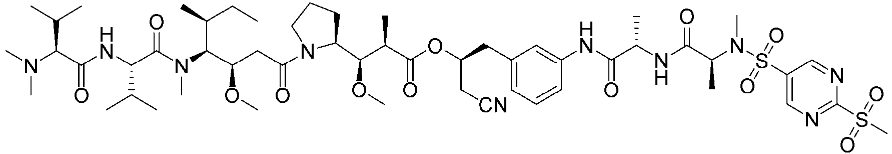 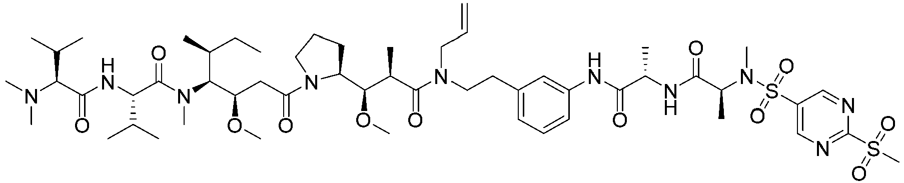 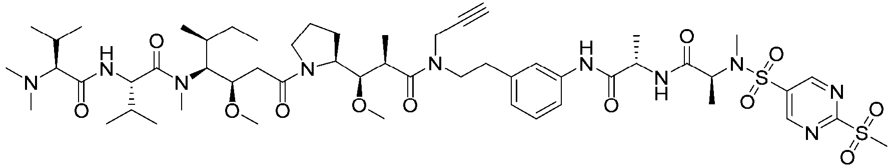 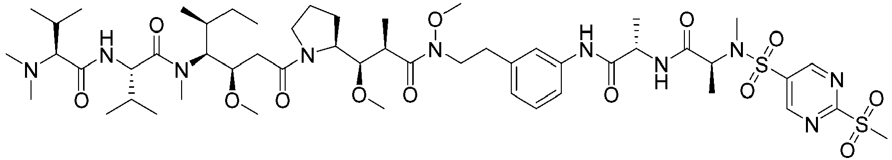 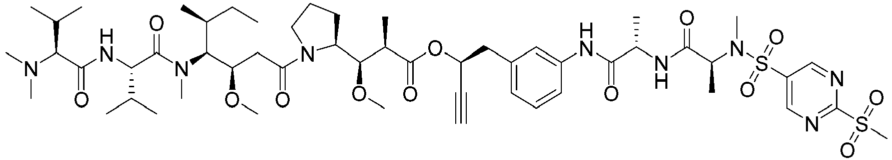 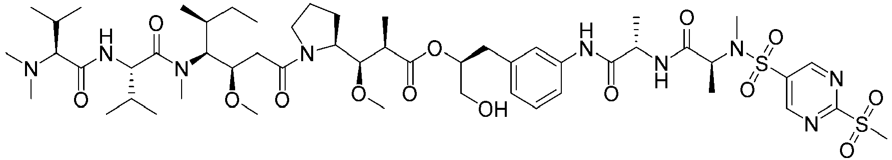 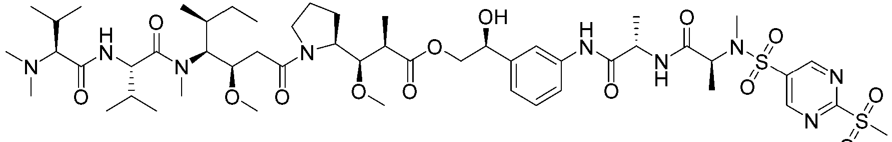 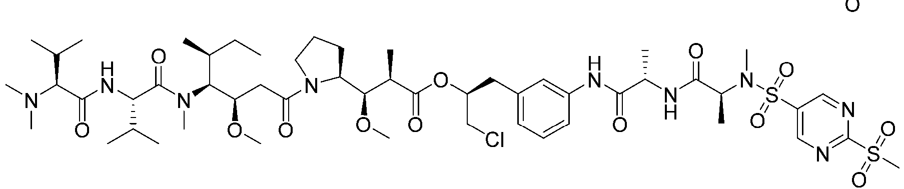 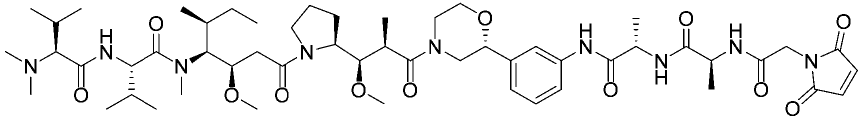 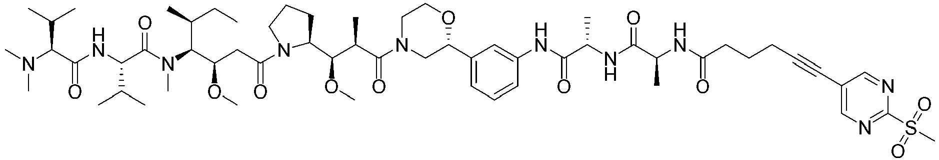 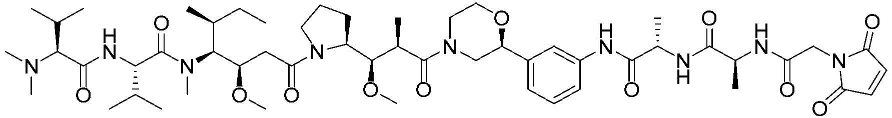 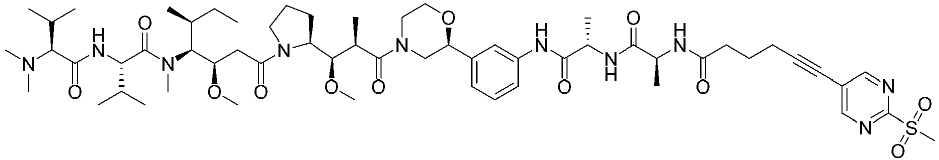 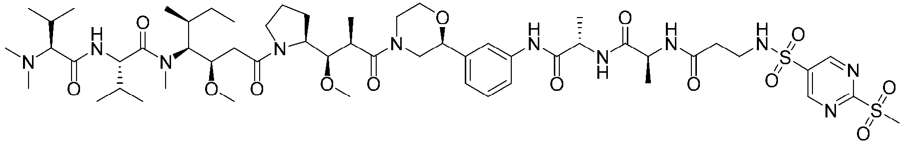 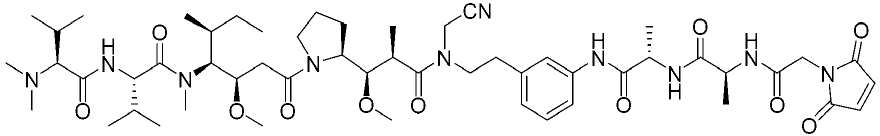 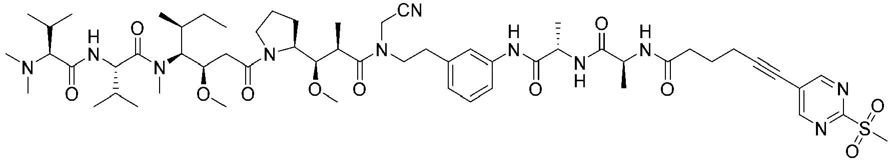 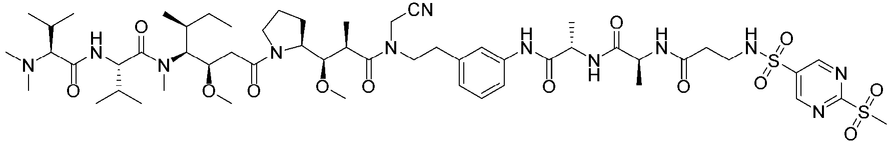 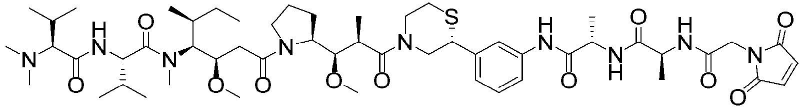 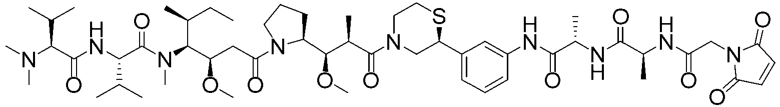 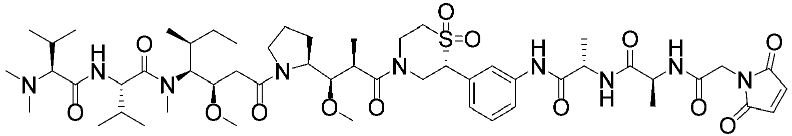 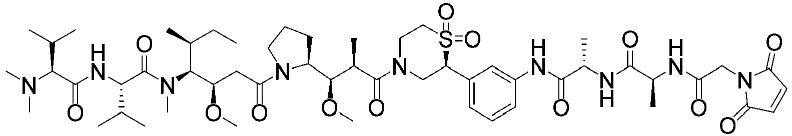 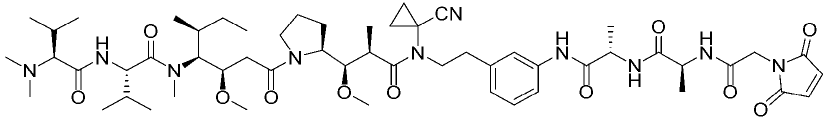 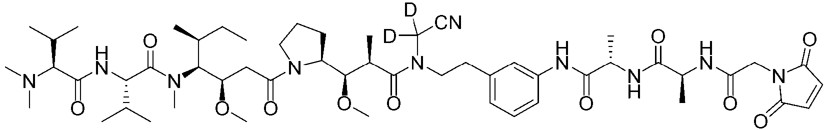 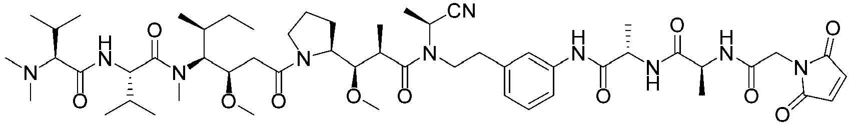 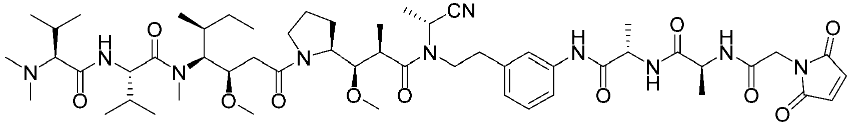 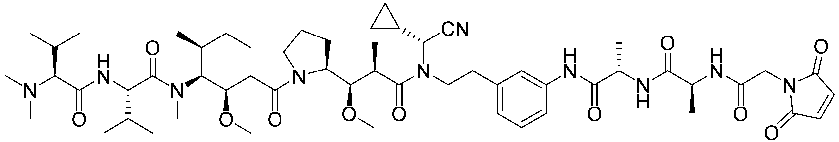 and

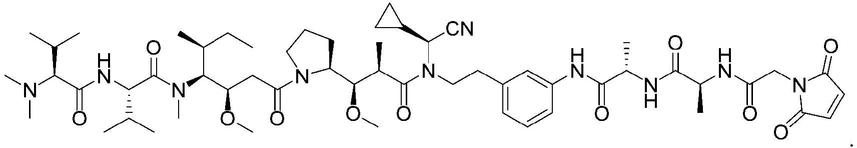

24. The antibody-drug conjugate or the stereoisomer, isotopic derivative, solvate, nitrogen oxide, prodrug, or pharmaceutically acceptable salt thereof according to any one of claims 17-20, wherein the antibody-drug conjugate or the stereoisomer, isotopic derivative, solvate, nitrogen oxide, prodrug, or pharmaceutically acceptable salt thereof is selected from: 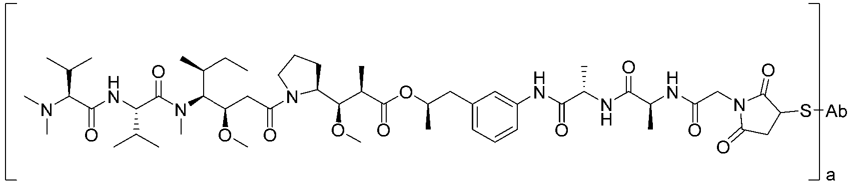 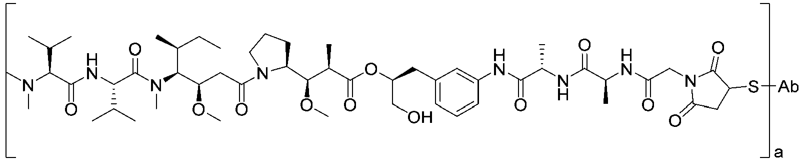 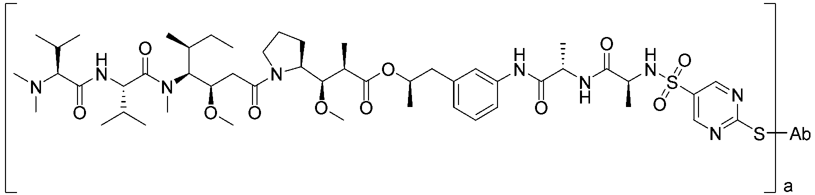 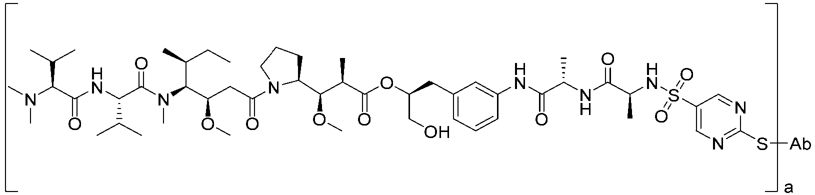 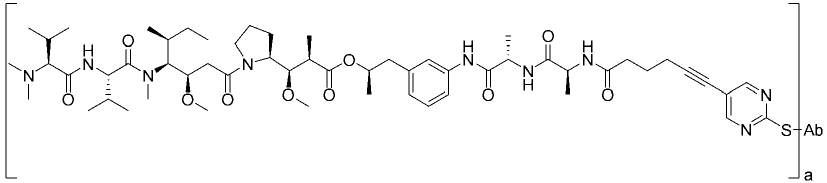 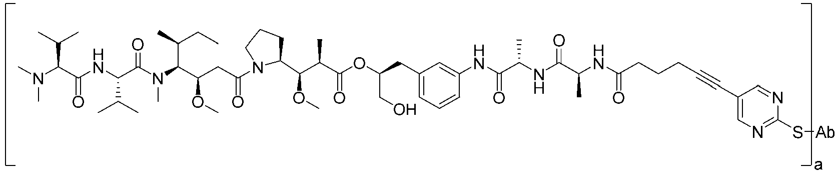 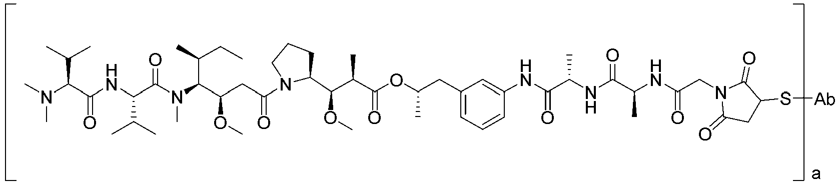 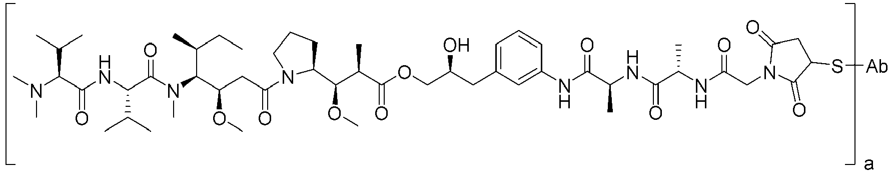 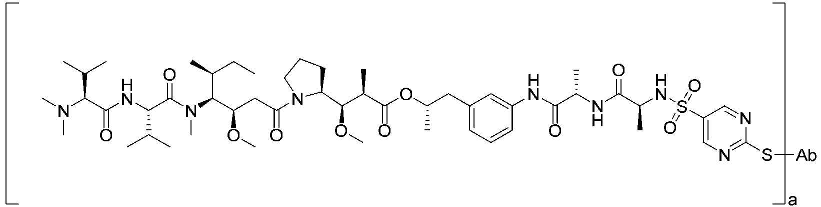 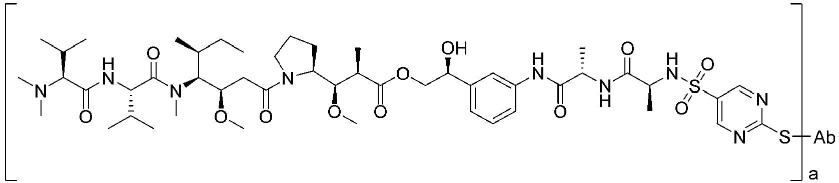 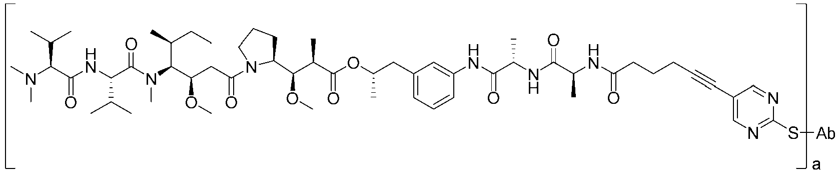 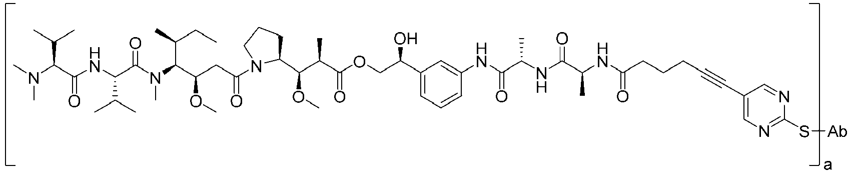 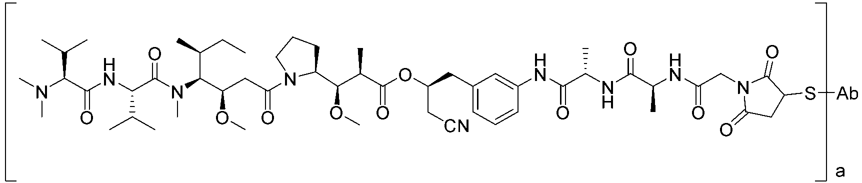 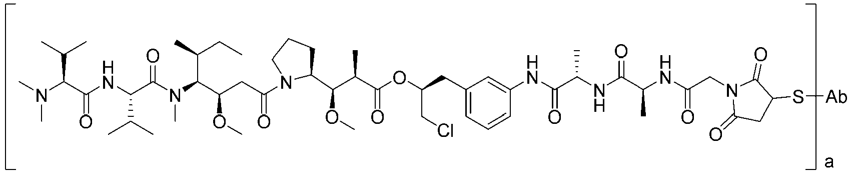 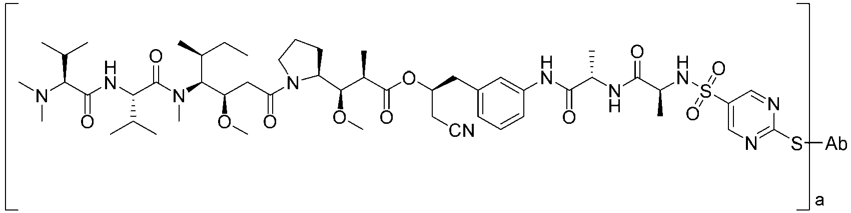 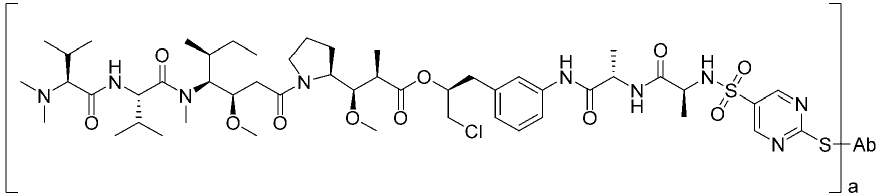 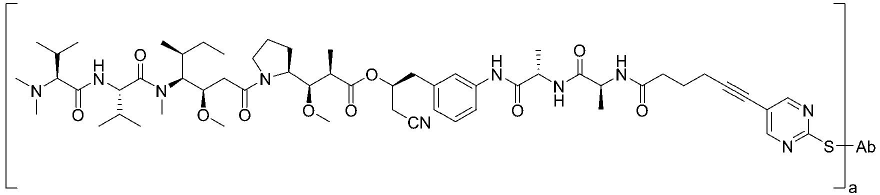 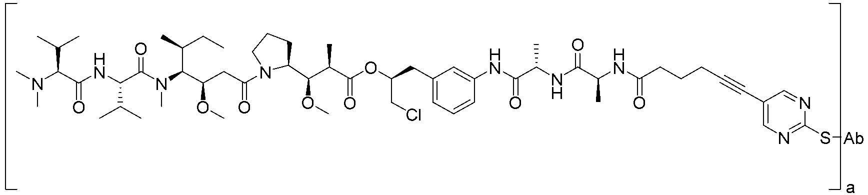 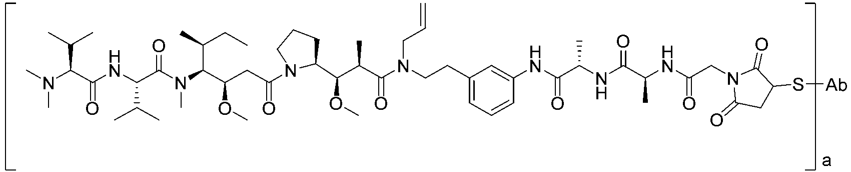 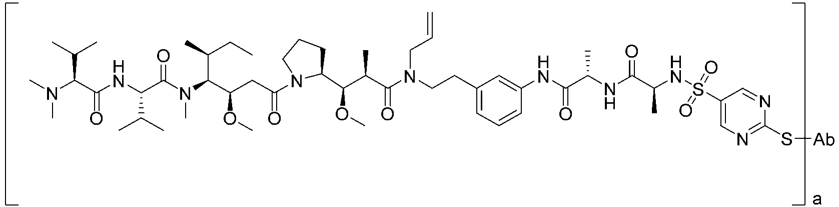 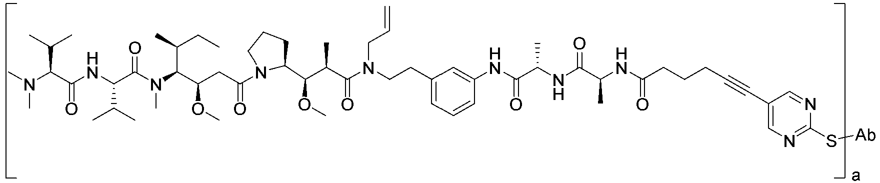 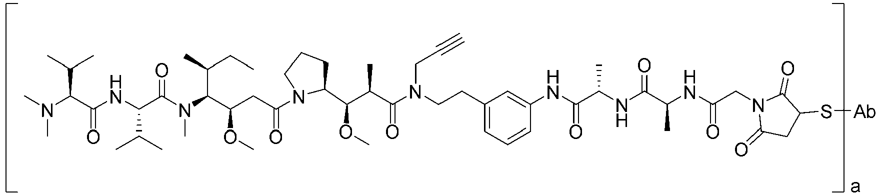 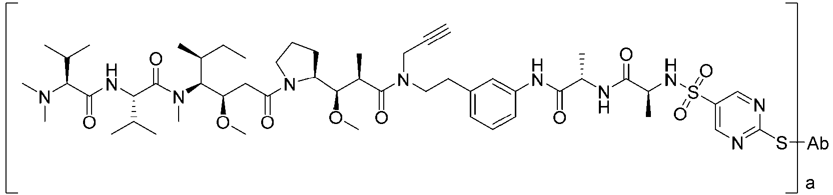 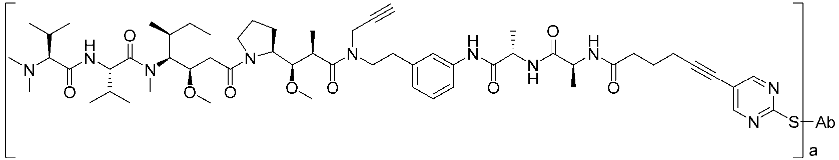 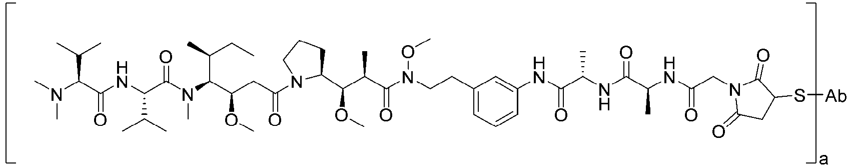 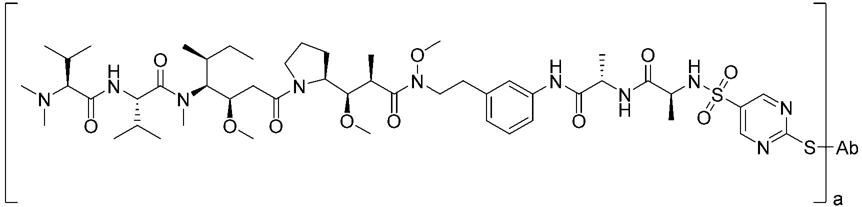 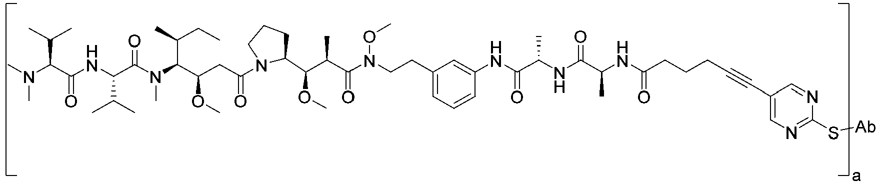 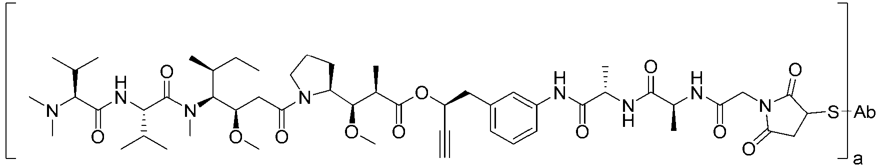 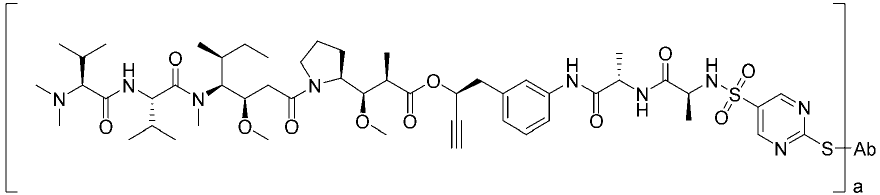 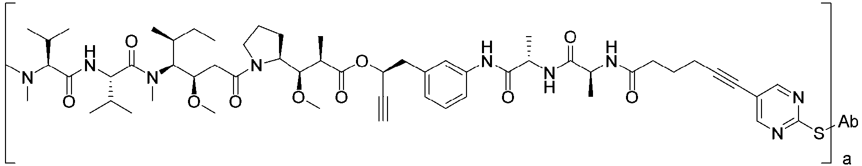 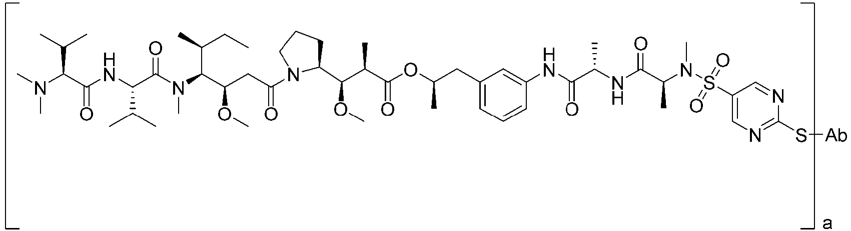 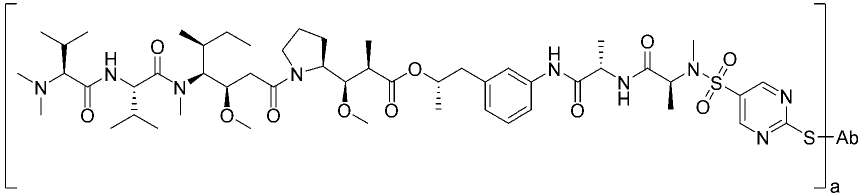 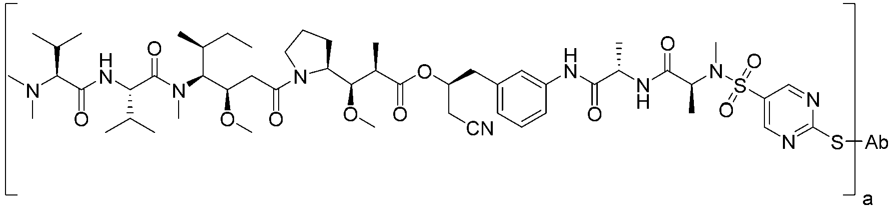 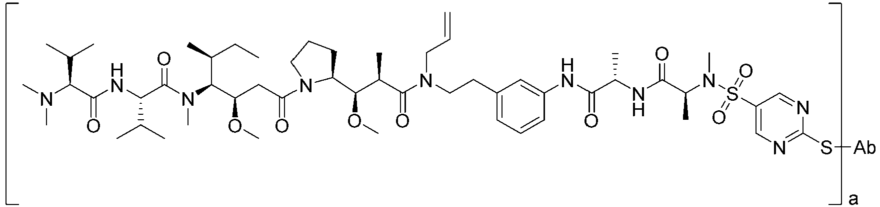 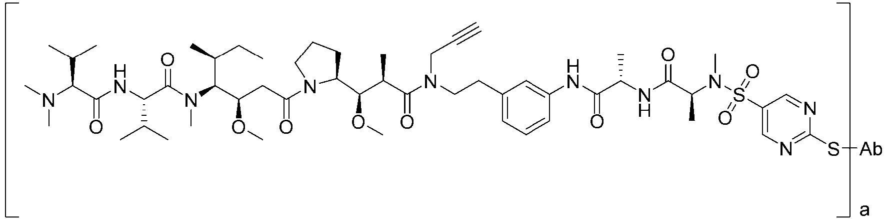 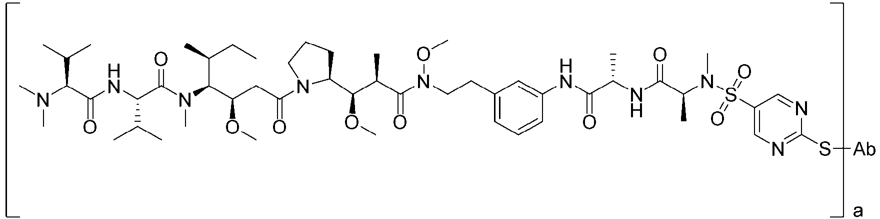 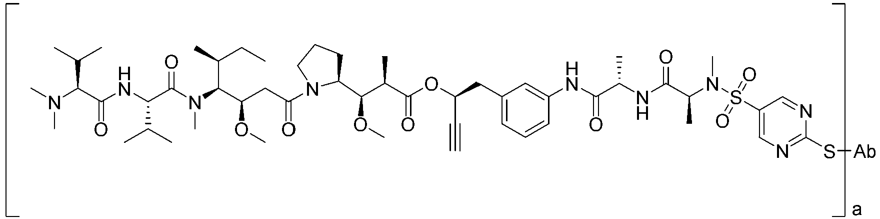 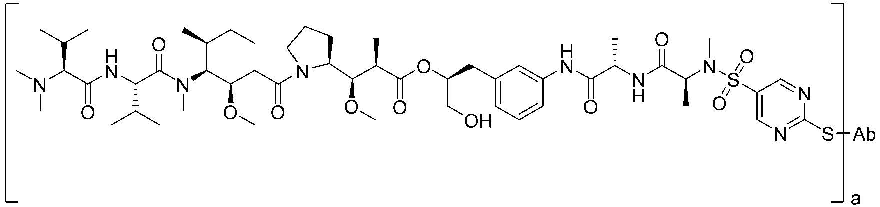 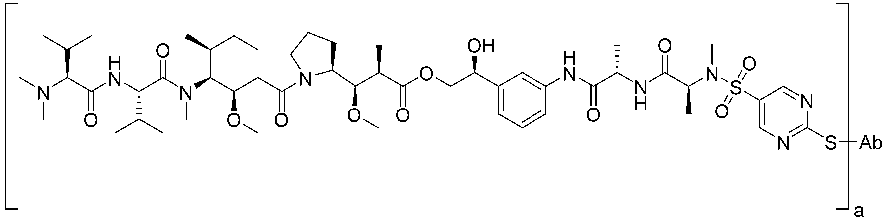 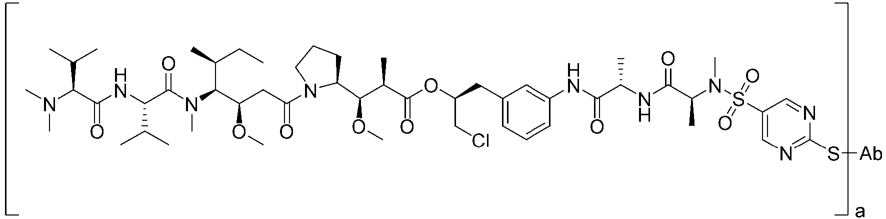 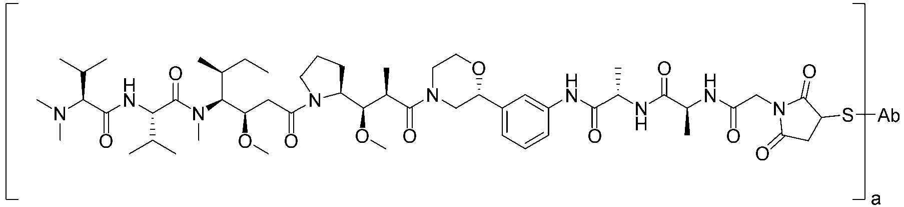 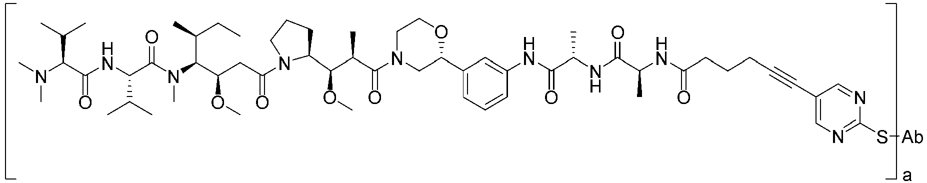 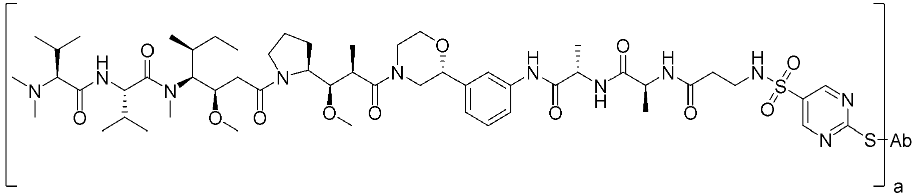 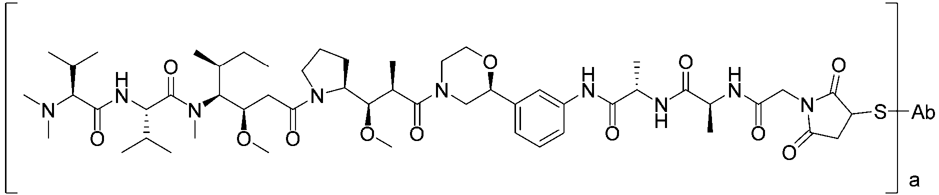 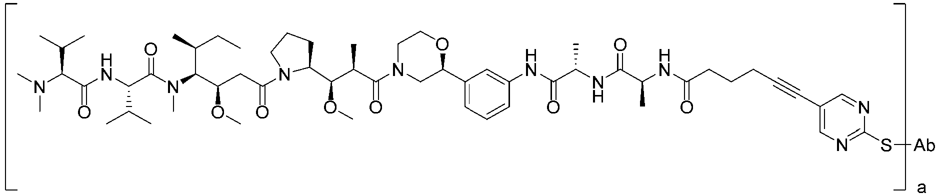 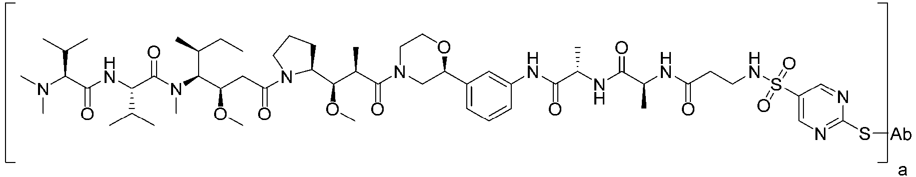 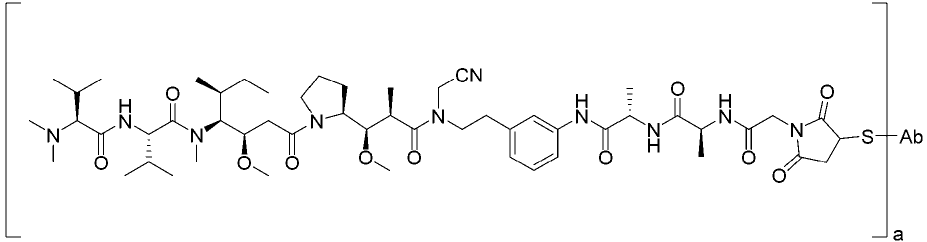 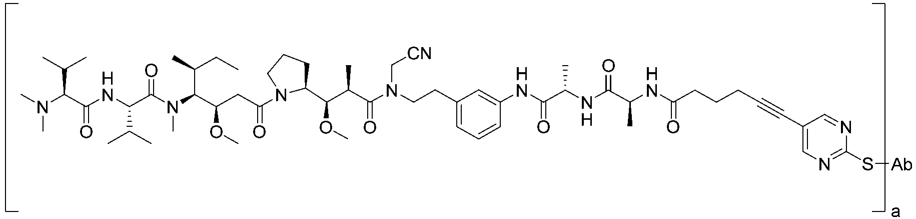 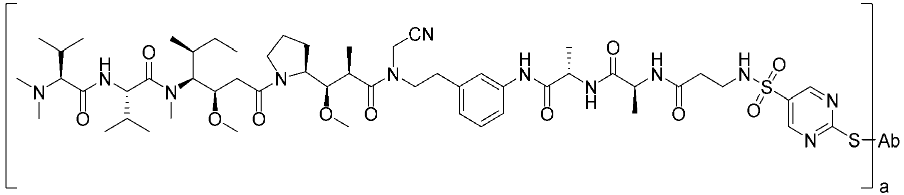 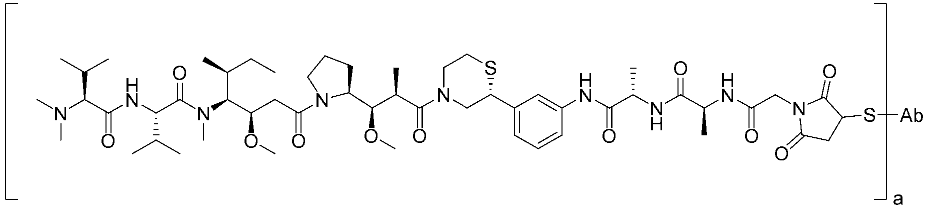 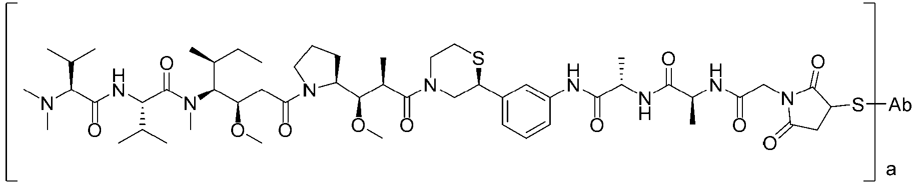 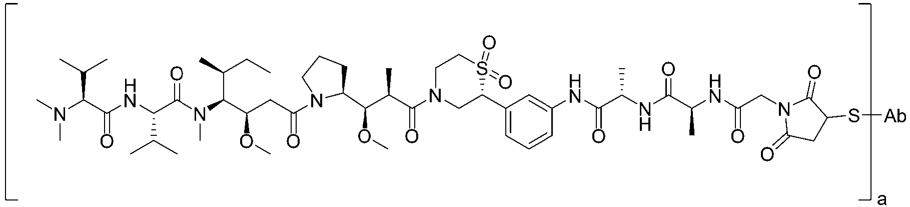 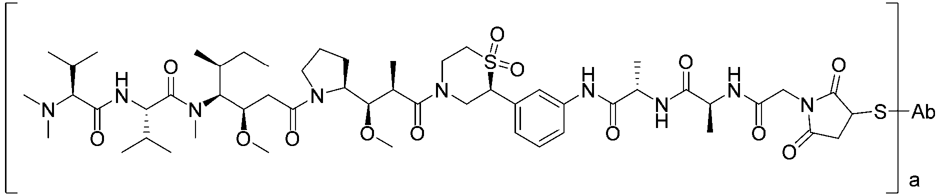 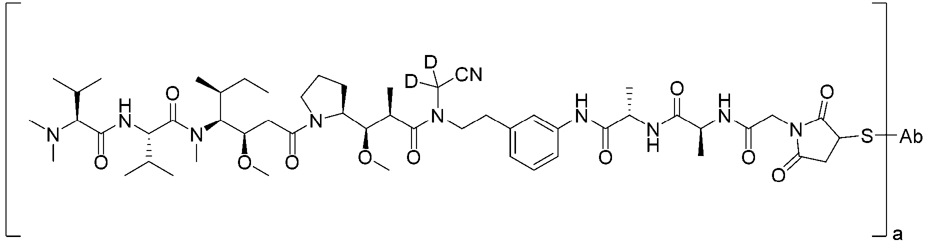 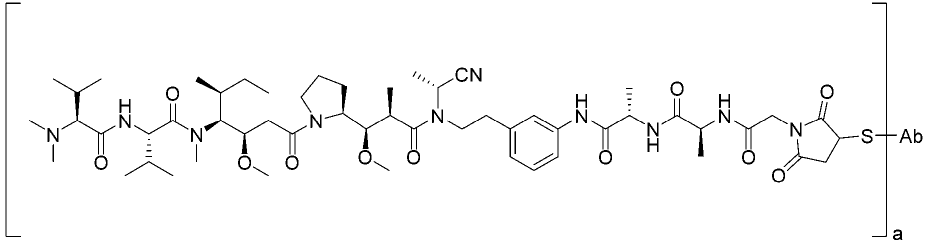 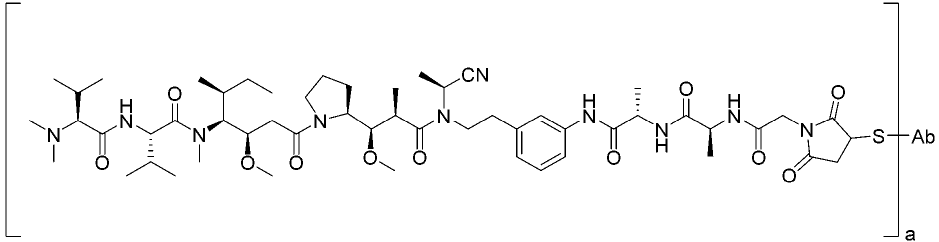 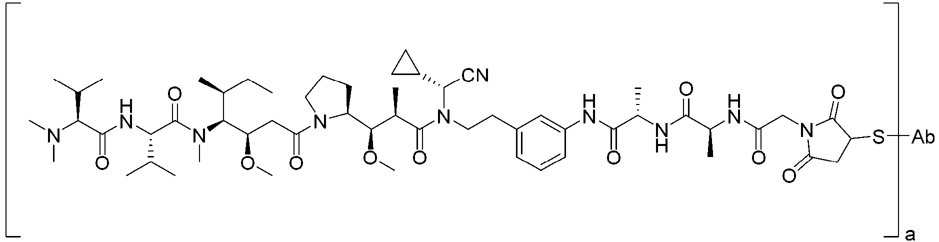 and 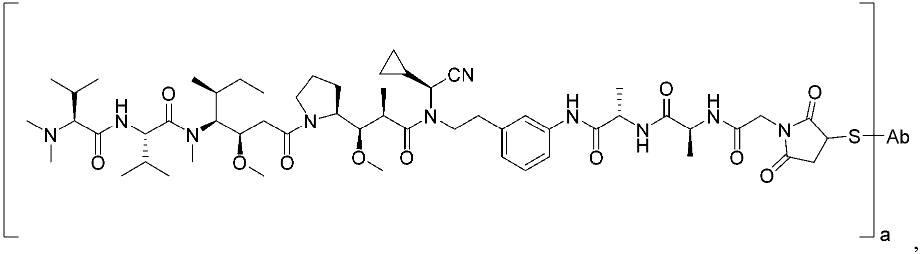
wherein Ab is an antibody, preferably a Her2 antibody, and more preferably trastuzumab;
a is selected from a numerical value of 2-8, preferably a numerical value of 4-8.

25. A pharmaceutical composition, comprising the cytotoxic drug or the stereoisomer, isotopic derivative, solvate, nitrogen oxide, prodrug, or pharmaceutically acceptable salt thereof according to any one of claims 1-16, or the antibody-drug conjugate or the stereoisomer, isotopic derivative, solvate, nitrogen oxide, prodrug, or pharmaceutically acceptable salt thereof according to any one of claims 17-20 or claim 24, and a pharmaceutically acceptable carrier.

26. Use of the cytotoxic drug or the stereoisomer, isotopic derivative, solvate, nitrogen oxide, prodrug, or pharmaceutically acceptable salt thereof according to any one of claims 1-16, the antibody-drug conjugate or the stereoisomer, isotopic derivative, solvate, nitrogen oxide, prodrug, or pharmaceutically acceptable salt thereof according to any one of claims 17-20 or claim 24, the cytotoxic drug-linker or the stereoisomer, isotopic derivative, solvate, nitrogen oxide, prodrug, or pharmaceutically acceptable salt thereof according to any one of claims 21-23, or the pharmaceutical composition according to claim 25 in preparing a medicament for treating a tumor.

27. The use according to claim 26, wherein the tumor is selected from breast cancer, ovarian cancer, cervical cancer, uterine cancer, prostate cancer, kidney cancer, urethral cancer, bladder cancer, liver cancer, gastric cancer, endometrial cancer, salivary gland cancer, esophageal cancer, melanoma, neuroglioma, neuroblastoma, sarcoma, lung cancer, head and neck cancer, colon cancer, rectal cancer, colorectal cancer, leukemia, skin cancer, thyroid cancer, pancreatic cancer, and lymphoma.
